# EUROPEAN PATENT APPLICATION

(11) **EP 2 172 552 A2**
(43) Date of publication of application: **07.04.2010**
(21) Application number: 10150509.7
(22) Date of filing: 10.10.2002
(51) Int. Cl.: C12N 15/40, C12N 15/51, C12N 15/85, C12N 15/86, C12N 15/861, A61K 48/00, C12N 15/34

(54) **Recombinant nucleic acids comprising regions of AD6**

(30) Priority: 11.10.2001 US 328655 P; 13.03.2002 US 363774 P
(62) Divisional of application: 02773740.2
(71) Applicant: Merck Sharp & Dohme Corp., Rahway, NJ 07065-0907 (US); Istituto di Richerche di Biologia Molecolare P. Angeletti S.p.A., 00040 Pomezia (Rome) (IT)
(72) Inventor: Emini, Emilio, A., Rahway, NJ 07065-0907 (US); Kaslow, David, C., Rahway, NJ 07065-0907 (US); Bett, Andrew, J., Rahway, NJ 07065-0907 (US); Shiver, John, W., Rahway, NJ 07065-0907 (US); Nicosia, Alfredo, 00040 Pomezia (IT); Lahm, Armin, 00040 Pomezia (IT); Luzzago, Alessandra, 00040 Pomezia (IT); Cortese, Riccardo, 00040 Pomezia (IT); Colloca, Stefano, 00040 Pomezia (IT)
(74) Representative: Horgan, James Michael Frederic

(57) **Abstract**

The present invention provides recombinant nucleic acids comprising one or more Ad6 regions and a region not present in Ad6, where the Ad6 region is selected from E1A, E1B, E2B, E2A, E4, L1, L2, L4 and L5, preferably where the adenovirus is defective in E1 but is able to replicate when E1 is supplied in *trans.*

## Description

### RELATED APPLICATIONS

The present application claims priority to provisional applications U.S. Serial No. 60/363,774, filed March 13, 2002, and U.S. Serial No. 60/328,655, filed October 11, 2001, each of which are hereby incorporated by reference herein.

### BACKGROUND OF THE INVENTION

The references cited in the present application are not admitted to be prior art to the claimed invention.

About 3% of the world's population are infected with the Hepatitis C virus (HCV). (Wasley et al., Semin. Liver Dis. 20, 1-16, 2000.) Exposure to HCV results in an overt acute disease in a small percentage of cases, while in most instances the virus establishes a chronic infection causing liver inflammation and slowly progresses into liver failure and cirrhosis. (Iwarson, FEMS Microbiol. Rev. 14, 201-204, 1994.) In addition, epidemiological surveys indicate an important role of HCV in the pathogenesis of hepatocellular carcinoma. (Kew, FEMS Microbiol. Rev. 14, 211-220, 1994, Alter, Blood 85, 1681-1695, 1995.)

Prior to the implementation of routine blood screening for HCV in 1992, most infections were contracted by inadvertent exposure to contaminated blood, blood products or transplanted organs. In those areas where blood screening of HCV is carried out, HCV is primarily contracted through direct percutaneous exposure to infected blood, *i*.*e*., intravenous drug use. Less frequent methods of transmission include perinatal exposure, hemodialysis, and sexual contact with an HCV infected person. (Alter et al., N. Engl. J. Med. 341(8), 556-562, 1999, Alter, J. Hepatol. 31 Suppl. 88-91, 1999. Semin. Liver. Dis. 201, 1-16, 2000.)

The HCV genome consists of a single strand RNA about 9.5 kb encoding a precursor polyprotein of about 3000 amino acids. (Choo et al., Science 244, 362-364, 1989, Choo et al., Science 244, 359-362, 1989, Takamizawa et al., J. Virol. 65, 1105-1113, 1991.) The HCV polyprotein contains the viral proteins in the order: C-E1-E2-p7-NS2-NS3-NS4A-NS4B-NS5A-NS5B.

Individual viral proteins are produced by proteolysis of the HCV polyprotein. Host cell proteases release the putative structural proteins C, E1, E2, and p7, and create the N-terminus of NS2 at amino acid 810. (Mizushima et al., J. Virol. 68, 2731-2734, 1994, Hijikata et al., P.N.A.S. USA 90, 10773-10777, 1993.)

The non-structural proteins NS3, NS4A, NS4B, NS5A and NS5B presumably form the virus replication machinery and are released from the polyprotein. A zinc-dependent protease associated with NS2 and the N-terminus of NS3 is responsible for cleavage between NS2 and NS3. (Grakoui et al., J. Virol. 67, 1385-1395, 1993, Hijikata et al., P.N.A.S. USA 90, 10773-10777, 1993.) A distinct serine protease located in the N-terminal domain of NS3 is responsible for proteolytic cleavages at the NS3/NS4A, NS4A/NS4B, NS4B/NS5A and NS5A/NS5B junctions. (Bartenschlager et al., J. Virol. 67, 3835-3844, 1993, Grakoui et al., Proc. Natl. Acad. Sci. USA 90, 10583-10587, 1993, Tomei et al., J. Virol. 67, 4017-4026, 1993.) NS4A provides a cofactor for NS3 activity. (Failla et al., J. Virol. 68, 3753-3760, 1994, De Francesco et al., U.S. Patent No. 5,739,002.)

NS5A is a highly phosphorylated protein conferring interferon resistance. (De Francesco et al., Semin. Liver Dis., 20(1), 69-83, 2000, Pawlotsky, Viral Hepat. Suppl. 1, 47-48, 1999.)

NS5B provides an RNA-dependent RNA polymerase. (De Francesco *et al.,* International Publication Number WO 96/37619, Behrens et al., EMBO 15, 12-22, 1996, Lohmann et al., Virology 249, 108-118, 1998.)

### SUMMARY OF THE INVENTION

The present invention features Ad6 vectors and a nucleic acid encoding a Met-NS3-NS4A-NS4B-NS5A-NS5B polypeptide containing an inactive NS5B RNA-dependent RNA polymerase region. The nucleic acid is particularly useful as a component of an adenovector or DNA plasmid vaccine providing a broad range of antigens for generating an HCV specific cell mediated immune (CMI) response against HCV.

A HCV specific CMI response refers to the production of cytotoxic T lymphocytes and T helper cells that recognize an HCV antigen. The CMI response may also include non-HCV specific immune effects.

Preferred nucleic acids encode a Met-NS3-NS4A-NS4B-NS5A-NS5B polypeptide that is substantially similar to SEQ. ID. NO. 1 and has sufficient protease activity to process itself to produce at least a polypeptide substantially similar to the NS5B region present in SEQ. ID. NO. 1. The produced polypeptide corresponding to NS5B is enzymatically inactive. More preferably, the HCV polypeptide has sufficient protease activity to produce polypeptides substantially similar to the NS3, NS4A, NS4B, NS5A, and NS5B regions present in SEQ. ID. NO. 1.

Reference to a "substantially similar sequence" indicates an identity of at least about 65% to a reference sequence. Thus, for example, polypeptides having an amino acid sequence substantially similar to SEQ. ID. NO. 1 have an overall amino acid identity of at least about 65% to SEQ. ID. NO. 1.

Polypeptides corresponding to NS3, NS4A, NS4B, NS5A, and NS5B have an amino acid sequence identity of at least about 65% to the corresponding region in SEQ. ID. NO. 1. Such corresponding polypeptides are also referred to herein as NS3, NS4A, NS4B, NS5A, and NS5B polypeptides.

Thus, a first aspect of the present invention describes a nucleic acid comprising a nucleotide sequence encoding a Met-NS3-NS4A-NS4B-NS5A-NS5B polypeptide substantially similar to SEQ. ID. NO. 1. The encoded polypeptide has sufficient protease activity to process itself to produce an NS5B polypeptide that is enzymatically inactive.

In a preferred embodiment, the nucleic acid is an expression vector capable of expressing the Met-NS3-NS4A-NS4B-NS5A-NS5B polypeptide in a desired human cell. Expression inside a human cell has therapeutic applications for actively treating an HCV infection and for prophylactically treating against an HCV infection.

An expression vector contains a nucleotide sequence encoding a polypeptide along with regulatory elements for proper transcription and processing. The regulatory elements that may be present include those naturally associated with the nucleotide sequence encoding the polypeptide and exogenous regulatory elements not naturally associated with the nucleotide sequence. Exogenous regulatory elements such as an exogenous promoter can be useful for expression in a particular host, such as in a human cell. Examples of regulatory elements useful for functional expression include a promoter, a terminator, a ribosome binding site, and a polyadenylation signal.

Another aspect of the present invention describes a nucleic acid comprising a gene expression cassette able to express in a human cell a Met-NS3-NS4A-NS4B-NS5A-NS5B polypeptide substantially similar to SEQ. ID. NO. 1. The polypeptide can process itself to produce an enzymatically inactive NS5B protein. The gene expression cassette contains at least the following:
a) a promoter transcriptionally coupled to a nucleotide sequence encoding a polypeptide;
b) a 5' ribosome binding site functionally coupled to the nucleotide sequence,
c) a terminator joined to the 3' end of the nucleotide sequence, and
d) a 3' polyadenylation signal functionally coupled to the nucleotide sequence.

Reference to "transcriptionally coupled" indicates that the promoter is positioned such that transcription of the nucleotide sequence can be brought about by RNA polymerase binding at the promoter. Transcriptionally coupled does not require that the sequence being transcribed is adjacent to the promoter.

Reference to "functionally coupled" indicates the ability to mediate an effect on the nucleotide sequence. Functionally coupled does not require that the coupled sequences be adjacent to each other. A 3' polyadenylation signal functionally coupled to the nucleotide sequence facilitates cleavage and polyadenylation of the transcribed RNA. A 5' ribosome binding site functionally coupled to the nucleotide sequence facilitates ribosome binding.

In preferred embodiments the nucleic acid is a DNA plasmid vector or an adenovector suitable for either therapeutic application in treating HCV or as an intermediate in the production of a therapeutic vector. Treating HCV includes actively treating an HCV infection and prophylactically treating against an HCV infection.

Another aspect of the present invention describes an adenovector comprising a Met-NS3-NS4A-NS4B-NS5A-NS5B expression cassette able to express a polypeptide substantially similar to SEQ. ID. NO. 1 that is produced by a process involving (a) homologous recombination and (b) adenovector rescue. The homologous recombinant step produces an adenovirus genome plasmid. The adenovector rescue step produces the adenovector from the adenogenome plasmid.

Adenovirus genome plasmids described herein contain a recombinant adenovirus genome having a deletion in the E1 region and optionally in the E3 region and a gene expression cassette inserted into one of the deleted regions. The recombinant adenovirus genome is made of regions substantially similar to one or more adenovirus serotypes.

Another aspect of the present invention describes an adenovector consisting of the nucleic acid sequence of SEQ. ID. NO. 4 or a derivative thereof, wherein said derivative thereof has the HCV polyprotein encoding sequence present in SEQ. ID. NO. 4 replaced with the HCV polyprotein encoding sequence of either SEQ. ID. NO. 3, SEQ. ID. NO. 10 or SEQ. ID. NO. 11.

Another aspect of the present invention describes a cultured recombinant cell comprising a nucleic acid containing a sequence encoding a Met-NS3-NS4A-NS4B-NS5A-NS5B polypeptide substantially similar to SEQ. ID. NO. 1. The recombinant cell has a variety of uses such as being used to replicate nucleic acid encoding the polypeptide in vector construction methods.

Another aspect of the present invention describes a method of making an adenovector comprising a Met-NS3-NS4A-NS4B-NS5A-NS5B expression cassette able to express a polypeptide substantially similar to SEQ. ID. NO. 1. The method involves the steps of (a) producing an adenovirus genome plasmid containing a recombinant adenovirus genome with deletions in the E1 and E3 regions and a gene expression cassette inserted into one of the deleted regions and (b) rescuing the adenovector from the adenovirus genome plasmid.

Another aspect of the present invention describes a pharmaceutical composition comprising a vector for expressing a Met-NS3-NS4A-NS4B-NS5A-NS5B polypeptide substantially similar to SEQ. ID. NO. 1 and a pharmaceutically acceptable carrier. The vector is suitable for administration and polypeptide expression in a patient.

A "patient" refers to a mammal capable of being infected with HCV. A patient may or may not be infected with HCV. Examples of patients are humans and chimpanzees.

Another aspect of the present invention describes a method of treating a patient comprising the step of administering to the patient an effective amount of a vector expressing a Met-NS3-NS4A-NS4B-NS5A-NS5B polypeptide substantially similar to SEQ. ID. NO. 1. The vector is suitable for administration and polypeptide expression in the patient.

The patient undergoing treatment may or may not be infected with HCV. For a patient infected with HCV, an effective amount is sufficient to achieve one or more of the following effects: reduce the ability of HCV to replicate, reduce HCV load, increase viral clearance, and increase one or more HCV specific CMI responses. For a patient not infected with HCV, an effective amount is sufficient to achieve one or more of the following: an increased ability to produce one or more components of a HCV specific CMI response to a HCV infection, a reduced susceptibility to HCV infection, and a reduced ability of the infecting virus to establish persistent infection for chronic disease.

Another aspect of the present invention features a recombinant nucleic acid comprising an Ad6 region and a region not present in Ad6. Reference to "recombinant" nucleic acid indicates the presence of two or more nucleic acid regions not naturally associated with each other. Preferably, the Ad6 recombinant nucleic acid contains Ad6 regions and a gene expression cassette coding for a polypeptide heterologous to Ad6.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the present invention.

### BRIEF DESCRIPTION OF THE DRAWINGS

Figures 1A and 1B illustrate SEQ. ID. NO. 1.
Figures 2A, 2B, 2C, and 2D illustrate SEQ. ID. NO. 2. SEQ. ID. NO. 2 provides a nucleotide sequence coding for SEQ. ID. NO. 1 along with an optimized internal ribosome entry site and TAAA termination. Nucleotides 1-6 provides an optimized internal ribosome entry site. Nucleotides 7-5961 code for a HCV Met-NS3-NS4A-NS4B-NS5A-NS5B polypeptide with nucleotides in positions 5137 to 5145 providing a AlaAlaGly sequence in amino acid positions 1711 to 1713 that renders NS5B inactive. Nucleotides 5962-5965 provide a TAAA termination.
Figures 3A, 3B, 3C, and 3D illustrate SEQ. ID. NO. 3. SEQ. ID. NO. 3 is a codon optimized version of SEQ. ID. NO. 2. Nucleotides 7-5961 encode a HCV Met-NS3-NS4A-NS4B-NS5A-NS5B polypeptide.
Figures 4A-4M illustrate MRKAd6-NSmut (SEQ. ID. NO. 4). SEQ. ID. NO. 4 is an adenovector containing an expression cassette where the polypeptide of SEQ. ID. NO. 1 is encoded by SEQ. ID. NO. 2. Base pairs 1-450 correspond to the Ad5 bp 1 to 450; base pairs 462 to 1252 correspond to the human CMV promoter; base pairs 1258 to 1267 correspond to the Kozak sequence; base pairs 1264 to 7222 correspond to the NS genes; base pairs 7231 to 7451 correspond to the BGH polyadenylation signal; base pairs 7469 to 9506 correspond to Ad5 base pairs 3511 to 5548; base pairs 9507 to 32121 correspond to Ad6 base pairs 5542 to 28156; base pairs 32122 to 35117 correspond to Ad6 base pairs 30789 to 33784; and base pairs 35118 to 37089 correspond to Ad5 base pairs 33967 to 35935.
Figures 5A-5O illustrate SEQ. ID. NOs. 5 and 6. SEQ. ID. NO. 5 encodes a HCV Met-NS3-NS4A-NS4B-NS5A-NS5B polypeptide with an active RNA dependent RNA polymerase. SEQ. ID. NO. 6 provides the amino acid sequence for the polypeptide.
Figures 6A-6C provide the nucleic acid sequence for pV1JnsA (SEQ. ID. NO. 7).
Figures 7A-7N provide the nucleic acid sequence for the Ad6 genome (SEQ. ID. NO. 8).
Figures 8A-8K provide the nucleic acid sequence for the Ad5 genome (SEQ. ID. NO. 9).

Figure 9 illustrates different regions of the Ad6 genome. The linear (35759 bp) ds DNA genome is indicated by two parallel lines and is divided into 100 map units. Transcription units are shown relative to their position and orientation in the genome. Early genes (E1A, E1B, E2A/B, E3 and E4 are indicated by gray arrows. Late genes (L1 to L5), indicated by black arrows, are produced by alternative splicing of a transcript produced from the major late promoter (MLP) and all contain the tripartite leader (1, 2, 3) at their 5' ends. The E1 region is located from approximately 1.0 to 11.5 map units, the E2 region from 75.0 to 11.5 map units, E3 from 76.1 to 86.7 map units, and E4 from 99.5 to 91.2 map units. The major late transcription unit is located between 16.0 and 91.2 map units.

Figure 10 illustrates homologous recombination to recover pAdE1-E3+ containing Ad6 and Ad5 regions.

Figure 11 illustrates homologous recombinant to recover a pAdE1-E3+ containing Ad6 regions.

Figure 12 illustrates a western blot on whole-cell extracts from 293 cells transfected with plasmid DNA expressing different HCV NS cassettes. Mature NS3 and NS5A products were detected with specific antibodies. "pV1Jns-NS" refers to a pV1JnsA plasmid where a Met-NS3-NS4A-NS4B-NS5A-NS5B polypeptide is encoded by SEQ. ID. NO. 5, and SEQ. ID. NO. 5 is inserted between bases 1881 and 1912 of SEQ. ID. NO. 7. "pV1Jns-NSmut" refers to a pV1JnsA plasmid where SEQ. ID. NO. 2 is inserted between bases 1882 and 1925 of SEQ. ID. NO. 7. "pV1Jns-NSOPTmut" refers to a pV1JnsA plasmid where SEQ. ID. NO. 3 is inserted between bases 1881 and 1905 of SEQ. ID. NO. 7.

Figures 13A and 13B illustrate T cell responses by IFNγ ELIspot induced in C57black6 mice (A) and BalbC mice (B) by two injections of 25µg and 50µg, respectively, of plasmid DNA encoding the different HCV NS cassettes with Gene Electro-Transfer (GET).

Figure 14 illustrates protein expression from different adenovectors upon infection of HeLa cells. MRKAd5-NSmut is an adenovector based on an Ad5 sequence (SEQ. ID. NO. 9), where the Ad5 genome has an E1 deletion of base pairs 451 to 3510, an E3 deletion of base pairs 28134 to 30817, and has the NS3-NS4A-NS4B-NS5A-NS5B expression cassette as provided in base pairs 451 to 7468 of SEQ. ID. NO. 4 inserted between positions 450 and 3511. Ad5-NS is an adenovector based on an Ad5 backbone with an E1 deletion of base pairs 342 to 3523, and E3 deletion of base pairs 28134 to 30817 and containing an expression cassette encoding a NS3-NS4A-NS4B-NS5A-NS5B from SEQ. ID. NO. 5. "MRKAd6-NSOPTmut" refers to an adenovector having a modified SEQ. ID. NO. 4 sequence, wherein base pairs 1258 to 7222 of SEQ. ID. NO. 4 is replaced with SEQ. ID. NO. 3.

Figure 15 illustrates T cell responses by IFNγ ELIspot induced in C57black6 mice by two injections of 10⁹ vp of adenovectors containing different HCV non-structural gene cassettes.

Figures 16A-16D illustrate T cell responses by IFNγ ELIspot induced in Rhesus monkeys by one or two injections of 10¹⁰ vp (A) or 10¹¹ vp (B) of adenovectors containing different HCV non-structural gene cassettes.

Figures 17A and 17B illustrates CD8+ T cell responses by IFNγ ICS induced in Rhesus monkeys by two injections of 10¹⁰ vp (A) or 10¹¹ vp (B) of adenovectors encoding the different HCV non-structural gene cassettes.

Figures 18A-18F illustrate T cell responses by bulk CTL assay induced in Rhesus monkeys by two injections of 10¹¹ vp of Ad5-NS (A), MRKAd5-NSmut (B), or MRKAd6-NSmut (C).

Figure 19 illustrates the plasmid pE2.

Figures 20A-D illustrates the partial codon optimized sequence NSsuboptmut (SEQ. ID. NO. 10). Coding sequence for the Met-NS3-NS4A-NS4B-NS5A-NS5B polypeptide is from base 7 to 5961.

### DETAILED DESCRIPTION OF THE INVENTION

The present invention features Ad6 vectors and nucleic acid encoding a Met-NS3-NS4A-NS4B-NS5A-NS5B polypeptide that contains an inactive NS5B region. Providing an inactive NS5B region supplies NS5B antigens while reducing the possibility of adverse side effects due to an active viral RNA polymerase. Uses of the featured nucleic acid include use as a vaccine component to introduce into a cell an HCV polypeptide that provides a broad range of antigens for generating a CMI response against HCV, and as an intermediate for producing such a vaccine component.

The adaptive cellular immune response can function to recognize viral antigens in HCV infected cells throughout the body due to the ubiquitous distribution of major histocompatibility complex (MHC) class I and II expression, to induce immunological memory, and to maintain immunological memory. These functions are attributed to antigen-specific CD4+ T helper (Th) and CD8+ cytotoxic T cells (CTL).

Upon activation via their specific T cell receptors, HCV specific Th cells fulfill a variety of immunoregulatory functions, most of them mediated by Th1 and Th2 cytokines. HCV specific Th cells assist in the activation and differentiation of B cells and induction and stimulation of virus-specific cytotoxic T cells. Together with CTL, Th cells may also secrete IFN-γ and TNF-α that inhibit replication and gene expression of several viruses. Additionally, Th cells and CTL, the main effector cells, can induce apoptosis and lysis of virus infected cells.

HCV specific CTL are generated from antigens processed by professional antigen presenting cells (pAPCs). Antigens can be either synthesized within or introduced into pAPCs. Antigen synthesis in a pAPC can be brought about by introducing into the cell an expression cassette encoding the antigen.

A preferred route of nucleic acid vaccine administration is an intramuscular route. Intramuscular administration appears to result in the introduction and expression of nucleic acid into somatic cells and pAPCs. HCV antigens produced in the somatic cells can be transferred to pAPCs for presentation in the context of MHC class I molecules. (Donnelly et al., Annu. Rev. Immunol. 15:617-648, 1997.)

pAPCs process longer length antigens into smaller peptide antigens in the proteasome complex. The antigen is translocated into the endoplasmic reticulum/Golgi complex secretory pathway for association with MHC class I proteins. CD8+ T lymphocytes recognize antigen associated with class I MHC via the T cell receptor (TCR) and the CD8 cell surface protein.

Using a nucleic acid encoding a Met-NS3-NS4A-NS4B-NS5A-NS5B polypeptide as a vaccine component allows for production of a broad range of antigens capable of generating CMI responses from a single vector. The polypeptide should be able to process itself sufficiently to produce at least a region corresponding to NS5B. Preferred nucleic acids encode an amino acid sequence substantially similar to SEQ. ID. NO. 1 that has sufficient protease activity to process itself to produce individual HCV polypeptides substantially similar to the NS3, NS4A, NS4B, NS5A, and NS5B regions present in SEQ. ID. NO. 1.

A polypeptide substantially similar to SEQ. ID. NO. 1 with sufficient protease activity to process itself in a cell provides the cell with T cell epitopes that are present in several different HCV strains. Protease activity is provided by NS3 and NS3/NS4A proteins digesting the Met-NS3-NS4A-NS4B-NS5A-NS5B polypeptide at the appropriate cleavage sites to release polypeptides corresponding to NS3, NS4A, NS4B, NS5A, and NS5B. Self- processing of the Met-NS3-NS4A-NS4B-NS5A-NS5B generates polypeptides that approximate naturally occurring HCV polypeptides.

Based on the guidance provided herein a sufficiently strong immune response can be generated to achieve beneficial effects in a patient. The provided guidance includes information concerning HCV sequence selection, vector selection, vector production, combination treatment, and administration.

### I. HCV SEQUENCES

A variety of different nucleic acid sequences can be used as a vaccine component to supply a HCV Met-NS3-NS4A-NS4B-NS5A-NS5B polypeptide to a cell or as an intermediate to produce vaccine components. The starting point for obtaining suitable nucleic acid sequences are preferably naturally occurring NS3-NS4A-NS4B-NS5A-NS5B polypeptide sequences modified to produce an inactive NS5B.

The use of a HCV nucleic acid sequence providing HCV non-structural antigens to generate a CMI response is mentioned by Cho et al., Vaccine 17:1136-1144, 1999, Paliard *et al*., International Publication Number WO 01/30812 (not admitted to be prior art to the claimed invention), and Coit *et al*., International Publication Number WO 01/38360 (not admitted to be prior art to the claimed invention). Such references fail to describe, for example, a polypeptide that processes itself to produce an inactive NS5B, and the particular combinations of HCV sequences and delivery vehicles employed herein.

Modifications to a HCV Met-NS3-NS4A-NS4B-NS5A-NS5B polypeptide sequence can be produced by altering the encoding nucleic acid. Alterations can be performed to create deletions, insertions and substitutions.

Small modifications can be made in NS5B to produce an inactive polymerase by targeting motifs essentially for replication. Examples of motifs critical for NS5B activity and modifications that can be made to produce an inactive NS5B are described by Lohmann et al., Journal of Virology 71:8416-8426, 1997, and Kolykhalov et al., Journal of Virology 74:2046-2051, 2000.

Additional factors to take into account when producing modifications to a HCV Met-NS3-NS4A-NS4B-NS5A-NS5B polypeptide include maintaining the ability to self-process and maintaining T cell antigens. The ability of the HCV polypeptide to process itself is determined to a large extent by a functional NS3 protease. Modifications that maintain NS3 activity protease activity can be obtained by taking into account the NS3 protein, NS4A which serves as a cofactor for NS3, and NS3 protease recognition sites present within the NS3-NS4A-NS4B-NS5A-NS5B polypeptide.

Different modifications can be made to naturally occurring NS3-NS4A-NS4B-NS5A-NS5B polypeptide sequences to produce polypeptides able to elicit a broad range of T cell responses. Factors influencing the ability of a polypeptide to elicit a broad T cell response include the preservation or introduction of HCV specific T cell antigen regions and prevalence of different T cell antigen regions in different HCV isolates.

Numerous examples of naturally occurring HCV isolates are well known in the art. HCV isolates can be classified into the following six major genotypes comprising one or more subtypes: HCV-1/(1a,1b,1c), HCV-2/(2a,2b,2c), HCV-3/(3a,3b,10a), HCV-4/(4a), HCV-5/(5a) and HCV-6/(6a,6b,7b,8b,9a,11a). (Simmonds, J. Gen. Virol., 693-712, 2001.) Examples of particular HCV sequences such as HCV-BK, HCV-J, HCV-N, HCV-H, have been deposited in GenBank and described in various publications. (See, for example, Chamberlain et al., J. Gen. Virol., 1341-1347, 1997.)

HCV T cell antigens can be identified by, for example, empirical experimentation. One way of identifying T cell antigens involves generating a series of overlapping short peptides from a longer length polypeptide and then screening the T-cell populations from infected patients for positive clones. Positive clones are activated/primed by a particular peptide. Techniques such as IFNγ-ELISPOT, IFNγ-Intracellular staining and bulk CTL assays can be used to measure peptide activity. Peptides thus identified can be considered to represent T-cell epitopes of the respective pathogen.

HCV T cell antigen regions from different HCV isolates can be introduced into a single sequence by, for example, producing a hybrid NS3-NS4A-NS4B-NS5A-NS5B polypeptide containing regions from two or more naturally occurring sequences. Such a hybrid can contain additional modifications, which preferably do not reduce the ability of the polypeptide to produce an HCV CMI response.

The ability of a modified Met-NS3-NS4A-NS4B-NS5A-NS5B polypeptide to process itself and produce a CMI response can be determined using techniques described herein or well known in the art. Such techniques include the use of IFNγ-ELISPOT, IFNγ-Intracellular staining and bulk CTL assays to measure a HCV specific CMI response.

### A. Met-NS3-NS4A-NS4B-NS5A-NS5B Sequences

SEQ. ID. NO. 1 provides a preferred Met-NS3-NS4A-NS4B-NS5A-NS5B sequence. SEQ. ID. NO. 1 contains a large number of HCV specific T cell antigens that are present in several different HCV isolates. SEQ. ID. NO. 1 is similar to the NS3-NS4A-NS4B-NS5A-NS5B portion of the HCV BK strain nucleotide sequence (GenBank accession number M58335).

In SEQ. ID. NO. 1 anchor positions important for recognition by MHC class I molecules are conserved or represent conservative substitutions for 18 out of 20 known T-cell epitopes in the NS3-NS4A-NS4B-NS5A-NS5B portion of HCV polyproteins. With respect to the remaining two known T-cell epitopes, one has a non-conservative anchor substitution in SEQ. ID. NO. 1 that may still be recognized by a different HLA supertype and one epitope has one anchor residue not conserved. HCV T-cell epitopes are described in Chisari et al., Curr. Top. Microbiol Immunol., 242:299-325, 2000, and Lechner et al. J. Exp. Med. 9:1499-1512, 2000.

Differences between the HCV-BK NS3-NS4A-NS4B-NS5A-NS5B nucleotide sequence and SEQ. ID. NO. 1 include the introduction of a methionine at the 5' end and the presence of modified NS5B active site residues in SEQ. ID. NO. 1. The modification replaces GlyAspAsp with AlaAlaGly (residues 1711-1713) to inactivate NS5B.

The encoded HCV Met-NS3-NS4A-NS4B-NS5A-NS5B polypeptide preferably has an amino acid sequence substantially similar to SEQ. ID. NO. 1. In different embodiments, the encoded HCV Met-NS3-NS4A-NS4B-NS5A-NS5B polypeptide has an amino acid identify to SEQ. ID. NO. 1 of at least 65%, at least 75%, at least 85%, at least 95%, at least 99% or 100%; or differs from SEQ. ID. NO. 1 by 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, or 1-20 amino acids.

Amino acid differences between a Met-NS3-NS4A-NS4B-NS5A-NS5B polypeptide and SEQ. ID. NO. 1 are calculated by determining the minimum number of amino acid modifications in which the two sequences differ. Amino acid modifications can be deletions, additions, substitutions or any combination thereof.

Amino acid sequence identity is determined by methods well known in the art that compare the amino acid sequence of one polypeptide to the amino acid sequence of a second polypeptide and generate a sequence alignment. Amino acid identity is calculated from the alignment by counting the number of aligned residue pairs that have identical amino acids.

Methods for determining sequence identity include those described by Schuler, G.D. in Bioinformatics: A Practical Guide to the Analysis of Genes and Proteins, Baxevanis, A.D. and Ouelette, B.F.F., eds., John Wiley & Sons, Inc, 2001; Yona, et al., in Bioinformatics: Sequence, structure and databanks, Higgins, D. and Taylor, W. eds, Oxford University Press, 2000; and Bioinformatics: Sequence and Genome Analysis, Mount, D.W., ed., Cold Spring Harbor Laboratory Press, 2001). Methods to determine amino acid sequence identity are codified in publicly available computer programs such as GAP (Wisconsin Package Version 10.2, Genetics Computer Group (GCG), Madison, Wisc.), BLAST (Altschul et al., J. Mol. Biol. 215(3):403-10, 1990), and FASTA (Pearson, Methods in Enzymology 183:63-98, 1990, R.F. Doolittle, ed.).

In an embodiment of the present invention sequence identity between two polypeptides is determined using the GAP program (Wisconsin Package Version 10.2, Genetics Computer Group (GCG), Madison, Wisc.). GAP uses the alignment method of Needleman and Wunsch. (Needleman, et al., J. Mol. Biol. 48:443-453, 1970.) GAP considers all possible alignments and gap positions between two sequences and creates a global alignment that maximizes the number of matched residues and minimizes the number and size of gaps. A scoring matrix is used to assign values for symbol matches. In addition, a gap creation penalty and a gap extension penalty are required to limit the insertion of gaps into the alignment. Default program parameters for polypeptide comparisons using GAP are the BLOSUM62 (Henikoff et al., Proc. Natl. Acad. Sci. USA, 89:10915-10919, 1992) amino acid scoring matrix

(MATrix=blosum62.cmp), a gap creation parameter (GAPweight=8) and a gap extension pararameter (LENgthweight=2).

More preferred HCV Met-NS3-NS4A-NS4B-NS5A-NS5B polypeptides in addition to being substantially similar to SEQ. ID. NO. 1 across their entire length produce individual NS3, NS4A, NS4B, NS5A and NS5B regions that are substantially similar to the corresponding regions present in SEQ. ID. NO. 1. The corresponding regions in SEQ. ID. NO. 1 are provided as follows: Met-NS3 amino acids 1-632; NS4A amino acids 633-686; NS4B amino acids 687-947; NS5A amino acids 948-1394; and NS5B amino acids 1395-1985.

In different embodiments a NS3, NS4A, NS4B, NS5A and/or NS5B region has an amino acid identity to the corresponding region in SEQ. ID. NO. 1 of at least 65%, at least 75%, at least 85%, at least 95%, at least 99%, or 100%; or an amino acid difference of 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, or 1-20 amino acids.

Amino acid modifications to SEQ. ID. NO. 1 preferably maintain all or most of the T-cell antigen regions. Differences in naturally occurring amino acids are due to different amino acid side chains (R groups). An R group affects different properties of the amino acid such as physical size, charge, and hydrophobicity. Amino acids can be divided into different groups as follows: neutral and hydrophobic (alanine, valine, leucine, isoleucine, proline, tyrptophan, phenylalanine, and methionine); neutral and polar (glycine, serine, threonine, tryosine, cysteine, asparagine, and glutamine); basic (lysine, arginine, and histidine); and acidic (aspartic acid and glutamic acid).

Generally, in substituting different amino acids it is preferable to exchange amino acids having similar properties. Substituting different amino acids within a particular group, such as substituting valine for leucine, arginine for lysine, and asparagine for glutamine are good candidates for not causing a change in polypeptide tertiary structure.

Starting with a particular amino acid sequence and the known degeneracy of the genetic code, a large number of different encoding nucleic acid sequences can be obtained. The degeneracy of the genetic code arises because almost all amino acids are encoded by different combinations of nucleotide triplets or "codons". The translation of a particular codon into a particular amino acid is well known in the art (*see*, *e*.*g*., Lewin GENES IV, p. 119, Oxford University Press, 1990). Amino acids are encoded by codons as follows:
A=Ala=Alanine: codons GCA, GCC, GCG, GCU
C=Cys=Cysteine: codons UGC, UGU
D=Asp=Aspartic acid: codons GAC, GAU
E=Glu=Glutamic acid: codons GAA, GAG
F=Phe=Phenylalanine: codons UUC, UUU
G=Gly=Glycine: codons GGA, GGC, GGG, GGU
H=His=Histidine: codons CAC, CAU
I=Ile=Isoleucine: codons AUA, AUC, AUU
K=Lys=Lysine: codons AAA, AAG
L=Leu=Leucine: codons UUA, UUG, CUA, CUC, CUG, CUU
M=Met=Methionine: codon AUG
N=Asn=Asparagine: codons AAC, AAU
P=Pro=Proline: codons CCA, CCC, CCG, CCU
Q=Gln=Glutamine: codons CAA, CAG
R=Arg=Arginine: codons AGA, AGG, CGA, CGC, CGG, CGU
S=Ser=Serine: codons AGC, AGU, UCA, UCC, UCG, UCU
T=Thr=Threonine: codons ACA, ACC, ACG, ACU
V=Val=Valine: codons GUA, GUC, GUG, GUU
W=Trp=Tryptophan: codon UGG
Y=Tyr=Tyrosine: codons UAC, UAU.

Nucleic acid sequences can be optimized in an effort to enhance expression in a host. Factors to be considered include C:G content, preferred codons, and the avoidance of inhibitory secondary structure. These factors can be combined in different ways in an attempt to obtain nucleic acid sequences having enhanced expression in a particular host. (See, for example, Donnelly *et al*., International Publication Number WO 97/47358.)

The ability of a particular sequence to have enhanced expression in a particular host involves some empirical experimentation. Such experimentation involves measuring expression of a prospective nucleic acid sequence and, if needed, altering the sequence.

### B. Encoding Nucleotide Sequences

SEQ. ID. NOs. 2 and 3 provide two examples of nucleotide sequences encoding a Met-NS3-NS4A-NS4B-NS5A-NS5B sequence. The coding sequence of SEQ. ID. NO. 2 is similar (99.4% nucleotide sequence identity) to the NS3-NS4A-NS4B-NS5A-NS5B region of the naturally occurring HCV-BK sequence (GenBank accession number M58335). SEQ. ID. NO. 3 is a codon-optimized version of SEQ. ID. NO. 2. SEQ. ID. NOs. 2 and 3 have a nucleotide sequence identity of 78.3%.

Differences between the HCV-BK NS3-NS4A-NS4B-NS5A-NS5B nucleotide (GenBank accession number M58335) and SEQ. ID. NO. 2, include SEQ. ID. NO. 2 having a ribosome binding site, an ATG methionine codon, a region coding for a modified NS5B catalytic domain, a TAAA stop signal and an additional 30 nucleotide differences. The modified catalytic domain codes for a AlaAlaGly (residues 1711-1713) instead of GlyAspAsp to inactivate NS5B.

A nucleotide sequence encoding a HCV Met-NS3-NS4A-NS4B-NS5A-NS5B polypeptide is preferably substantially similar to the SEQ. ID. NO. 2 coding region. In different embodiments, the nucleotide sequence encoding a HCV Met-NS3-NS4A-NS4B-NS5A-NS5B polypeptide has a nucleotide sequence identify to the SEQ. ID. NO. 2 coding region of at least 65%, at least 75%, at least 85%, at least 95%, at least 99%, or 100%; or differs from SEQ. ID. NO. 2 by 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-25, 1-30, 1-35, 1-40, 1-45, or 1-50 nucleotides.

Nucleotide differences between a sequence coding Met-NS3-NS4A-NS4B-NS5A-NS5B and the SEQ. ID. NO. 2 coding region are calculated by determining the minimum number of nucleotide modifications in which the two sequences differ. Nucleotide modifications can be deletions, additions, substitutions or any combination thereof.

Nucleotide sequence identity is determined by methods well known in the art that compare the nucleotide sequence of one sequence to the nucleotide sequence of a second sequence and generate a sequence alignment. Sequence identity is determined from the alignment by counting the number of aligned positions having identical nucleotides.

Methods for determining nucleotide sequence identity between two polynucleotides include those described by Schuler, in Bioinformatics: A Practical Guide to the Analysis of Genes and Proteins, Baxevanis, A.D. and Ouelette, B.F.F., eds., John Wiley & Sons, Inc, 2001; Yona et al.,. in Bioinformatics: Sequence, structure and databanks, Higgins, D. and Taylor, W. eds, Oxford University Press, 2000; and Bioinformatics: Sequence and Genome Analysis, Mount, D.W., ed., Cold Spring Harbor Laboratory Press, 2001). Methods to determine nucleotide sequence identity are codified in publicly available computer programs such as GAP (Wisconsin Package Version 10.2, Genetics Computer Group (GCG), Madison, Wisc.), BLAST (Altschul et al., J. Mol. Biol. 215(3):403-10, 1990), and FASTA (Pearson, W.R., Methods in Enzymology 183:63-98, 1990, R.F. Doolittle, ed.).

In an embodiment of the present ivnention, sequence identity between two polynucleotides is determined by application of GAP (Wisconsin Package Version 10.2, Genetics Computer Group (GCG), Madison, Wisc.). GAP uses the alignment method of Needleman and Wunsch. (Needleman et al., J. Mol. Biol. 48:443-453, 1970.) GAP considers all possible alignments and gap positions between two sequences and creates a global alignment that maximizes the number of matched residues and minimizes the number and size of gaps. A scoring matrix is used to assign values for symbol matches. In addition, a gap creation penalty and a gap extension penalty are required to limit the insertion of gaps into the alignment. Default program parameters for polynucleotide comparisons using GAP are the nwsgapdna.cmp scoring matrix (MATrix=nwsgapdna.cmp), a gap creation parameter (GAPweight=50) and a gap extension pararameter (LENgthweight=3).

More preferred HCV Met-NS3-NS4A-NS4B-NS5A-NS5B nucleotide sequences in addition to being substantially similar across its entire length, produce individual NS3, NS4A, NS4B, NS5A and NS5B regions that are substantially similar to the corresponding regions present in SEQ. ID. NO. 2. The corresponding coding regions in SEQ. ID. NO. 2 are provided as follows: Met-NS3, nucleotides 7-1902; NS4A nucleotides 1903-2064; NS4B nucleotides 2065-2847; NS5A nucleotides 2848-4188: NS5B nucleotides 4189-5661.

In different embodiments a NS3, NS4A, NS4B, NS5A and/or NS5B encoding region has a nucleotide sequence identity to the corresponding region in

SEQ. ID. NO. 2 of at least 65%, at least 75%, at least 85%, at least 95%, at least 99% or 100%; or a nucleotide difference to SEQ. ID. NO. 2 of 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-25, 1-30, 1-35, 1-40, 1-45, or 1-50 nucleotides.

### C. Gene Expression Cassettes

A gene expression cassette contains elements needed for polypeptide expression. Reference to "polypeptide" does not provide a size limitation and includes protein. Regulatory elements present in a gene expression cassette generally include: (a) a promoter transcriptionally coupled to a nucleotide sequence encoding the polypeptide, (b) a 5' ribosome binding site functionally coupled to the nucleotide sequence, (c) a terminator joined to the 3' end of the nucleotide sequence, and (d) a 3' polyadenylation signal functionally coupled to the nucleotide sequence. Additional regulatory elements useful for enhancing or regulating gene expression or polypeptide processing may also be present.

Promoters are genetic elements that are recognized by an RNA polymerase and mediate transcription of downstream regions. Preferred promoters are strong promoters that provide for increased levels of transcription. Examples of strong promoters are the immediate early human cytomegalovirus promoter (CMV), and CMV with intron A. (Chapman et al, Nucl. Acids Res. 19:3979-3986, 1991.) Additional examples of promoters include naturally occurring promoters such as the EF1 alpha promoter, the murine CMV promoter, Rous sarcoma virus promoter, and SV40 early/late promoters and the β-actin promoter; and artificial promoters such as a synthetic muscle specific promoter and a chimeric muscle-specific/CMV promoter (Li et al., Nat. Biotechnol. 17:241-245, 1999, Hagstrom et al., Blood 95:2536-2542, 2000).

The ribosome binding site is located at or near the initiation codon. Examples of preferred ribosome binding sites include CCACCAUGG, CCGCCAUGG, and ACCAUGG, where AUG is the initiation codon. (Kozak, Cell 44:283-292, 1986). Another example of a ribosome binding site is GCCACCAUGG (SEQ. ID. NO.12).

The polyadenylation signal is responsible for cleaving the transcribed RNA and the addition of a poly (A) tail to the RNA. The polyadenylation signal in higher eukaryotes contains an AAUAAA sequence about 11-30 nucleotides from the polyadenylation addition site. The AAUAAA sequence is involved in signaling RNA cleavage. (Lewin, Genes IV, Oxford University Press, NY, 1990.) The poly (A) tail is important for the mRNA processing.

Polyadenylation signals that can be used as part of a gene expression cassette include the minimal rabbit β -globin polyadenylation signal and the bovine growth hormone polyadenylation (BGH). (Xu et al., Gene 272:149-156, 2001, Post *et al.,* U.S. Patent U. S. 5,122,458.) Additional examples include the Synthetic Polyadenylation Signal (SPA) and SV40 polyadenylation signal. The SPA sequence is as follows: AAUAAAAGAUCUUUAUUUUCAUUAGAUCUGUGUG UUGGUUUUUUGUGUG (SEQ. ID. NO. 13).

Examples of additional regulatory elements useful for enhancing or regulating gene expression or polypeptide processing that may be present include an enhancer, a leader sequence and an operator. An enhancer region increases transcription. Examples of enhancer regions include the CMV enhancer and the SV40 enhancer. (Hitt et al., Methods in Molecular Genetics 7:13-30, 1995, Xu, et al., Gene 272:149-156, 2001.) An enhancer region can be associated with a promoter.

A leader sequence is an amino acid region on a polypeptide that directs the polypeptide into the proteasome. Nucleic acid encoding the leader sequence is 5' of a structural gene and is transcribed along the structural gene. An example of a leader sequences is tPA.

An operator sequence can be used to regulate gene expression. For example, the Tet operator sequence can be used to repress gene expression.

### II. THERAPEUTIC VECTORS

Nucleic acid encoding a Met-NS3-NS4A-NS4B-NS5A-NS5B polypeptide can be introduced into a patient using vectors suitable for therapeutic administration. Suitable vectors can deliver nucleic acid into a target cell without causing an unacceptable side effect.

Cellular expression is achieved using a gene expression cassette encoding a Met-NS3-NS4A-NS4B-NS5A-NS5B polypeptide. The gene expression cassette contains regulatory elements for producing and processing a sufficient amount of nucleic acid inside a target cell to achieve a beneficial effect.

Examples of vectors that can be used for therapeutic applications include first and second generation adenovectors, helper dependent adenovectors, adeno-associated viral vectors, retroviral vectors, alpha virus vectors, Venezuelan Equine Encephalitis virus vector, and plasmid vectors. (Hitt, et al., Advances in Pharmacology 40:137-206, 1997, Johnston et al., U.S. Patent No. 6,156,588, and Johnston *et al*., International Publication Number WO 95/32733.) Preferred vectors for introducing a Met-NS3-NS4A-NS4B-NS5A-NS5B polypeptide into a subject are first generation adenoviral vectors and plasmid DNA vectors.

### A. First Generation Adenovectors

First generation adenovector for expressing a gene expression cassette contain the expression cassette in an E1 and optionally E3 deleted recombinant adenovirus genome. The deletion in the E1 region is sufficiently large to remove elements needed for adenoviral replication.

First generation adenovectors for expressing a Met-NS3-NS4A-NS4B-NS5A-NS5B polypeptide contain a E1 and E3 deleted recombinant adenovirus genome. The deletion in the E 1 region is sufficiently large to remove elements needed for adenoviral replication. The combinations of deletions of the E1 and E3 regions are sufficiently large to accommodate a gene expression cassette encoding a Met-NS3-NS4A-NS4B-NS5A-NS5B polypeptide.

The adenovirus has a double-stranded linear genome with inverted terminal repeats at both ends. During viral replication, the genome is packaged inside a viral capsid to form a virion. The virus enters its target cell through viral attachment followed by internalization. (Hitt et al., Advances in Pharmacology 40:137-206, 1997.)

Adenovectors can be based on different adenovirus serotypes such as those found in humans or animals. Examples of animal adenoviruses include bovine, porcine, chimp, murine, canine, and avian (CELO). Preferred adenovectors are based on human serotypes, more preferably Group B, C, or D serotypes. Examples of human adenovirus Group B, C, D, or E serotypes include types 2 ("Ad2"), 4 ("Ad4"), 5 ("Ad5"), 6 ("Ad6"), 24 ("Ad24"), 26 ("Ad26"), 34 ("Ad34") and 35 ("Ad35"). Adenovectors can contain regions from a single adenovirus or from two or more adenovirus.

In different embodiments adenovectors are based on Ad5, Ad6, or a combination thereof. Ad5 is described by Chroboczek, et al., J. Virology 186:280-285, 1992. Ad6 is described in Figures 7A-7N. An Ad6 based vector containing Ad5 regions is described in the Example section provided below.

Adenovectors do not need to have their E1 and E3 regions completely removed. Rather, a sufficient amount the E1 region is removed to render the vector replication incompetent in the absence of the E1 proteins being supplied in *trans*; and the E1 deletion or the combination of the E1 and E3 deletions are sufficiently large enough to accommodate a gene expression cassette.

E1 deletions can be obtained starting at about base pair 342 going up to about base pair 3523 of Ad5, or a corresponding region from other adenoviruses. Preferably, the deleted region involves removing a region from about base pair 450 to about base pair 3511 of Ad5, or a corresponding region from other adenoviruses. Larger E1 region deletions starting at about base pair 341 removes elements that facilitate virus packaging.

E3 deletions can be obtained starting at about base pair 27865 to about base pair 30995 of Ad5, or the corresponding region of other adenovectors. Preferably the deletion region involves removing a region from about base pair 28134 up to about base pair 30817 of Ad5, or the corresponding region of other adenovectors.

The combination of deletions to the E1 region and optionally the E3 region should be sufficiently large so that the overall size of the recombinant genome containing the gene expression cassette does not exceed about 105% of the wild type adenovirus genome. For example, as recombinant adenovirus Ad5 genomes increase size above about 105% the genome becomes unstable. (Bett et al., Journal of Virology 67:5911-5921, 1993.)

Preferably, the size of the recombinant adenovirus genome containing the gene expression cassette is about 85% to about 105% the size of the wild type adenovirus genome. In different embodiments, the size of the recombinant adenovirus genome containing the expression cassette is about 100% to about 105.2%, or about 100%, the size of the wild type genome.

Approximately 7,500 kb can be inserted into an adenovirus genome with a E1 and E3 deletion. Without any deletion, the Ad5 genome is 35,935 base pairs and the Ad6 genome is 35,759 base pairs.

Replication of first generation adenovectors can be performed by supplying the E1 gene products in *trans.* The E1 gene product can be supplied in *trans*, for example, by using cell lines that have been transformed with the adenovirus E1 region. Examples of cells and cells lines transformed with the adenovirus E1 region are HEK 293 cells, 911 cells, PERC.6™ cells, and transfected primary human aminocytes cells. (Graham et al., Journal of Virology 36:59-72, 1977, Schiedner et al., Human Gene Therapy 11:2105-2116, 2000, Fallaux et al., Human Gene Therapy 9:1909-1917, 1998, Bout et al., U.S. Patent No. 6,033,908.)

A Met-NS3-NS4A-NS4B-NS5A-NS5B expression cassette should be inserted into a recombinant adenovirus genome in the region corresponding to the deleted E1 region or the deleted E3 region. The expression cassette can have a parallel or anti-parallel orientation. In a parallel orientation the transcription direction of the inserted gene is the same direction as the deleted E1 or E3 gene. In an anti-parallel orientation transcription the opposite strand serves as a template and the transcription direction is in the opposite direction.

In an embodiment of the present invention the adenovector has a gene expression cassette inserted in the E1 deleted region. The vector contains:
a) a first adenovirus region from about base pair 1 to about base pair 450 corresponding to either Ad5 or Ad6;
b) a gene expression cassette in a E1 parallel or E1 anti-parallel orientation joined to the first region;
c) a second adenovirus region from about base pair 3511 to about base pair 5548 corresponding to Ad5 or from about base pair 3508 to about base pair 5541 corresponding to Ad6, joined to the expression cassette;
d) a third adenovirus region from about base pair 5549 to about base pair 28133 corresponding to Ad5 or from about base pair 5542 to about base pair 28156 corresponding to Ad6, joined to the second region;
e) a fourth adenovirus region from about base pair 30818 to about base pair 33966 corresponding to Ad5 or from about base pair 30789 to about base pair 33784 corresponding to Ad6, joined to the third region; and
f) a fifth adenovirus region from about base pair 33967 to about base pair 35935 corresponding to Ad5 or from about base pair 33785 to about base pair 35759 corresponding to Ad6 joined to the fourth region.

In another embodiment of the present invention the adenovector has an expression cassette inserted in the E3 deleted region. The vector contains:
a) a first adenovirus region from about base pair 1 to about base pair 450 corresponding to either Ad5 or Ad6;
b) a second adenovirus region from about base pair 3511 to about base pair 5548 corresponding to Ad5 or from about base pair 3508 to about base pair 5541 corresponding to Ad6, joined to the first region;
c) a third adenovirus region from about base pair 5549 to about base pair 28133 corresponding to Ad5 or from about base pair 5542 to about base pair 28156 corresponding to Ad6, joined to the second region;
d) a gene expression cassette in a E3 parallel or E3 anti-parallel orientation joined to the third region;
e) a fourth adenovirus region from about base pair 30818 to about base pair 33966 corresponding to Ad5 or from about base pair 30789 to about base pair 33784 corresponding to Ad6, joined to the gene expression cassette; and
f) a fifth adenovirus region from about base pair 33967 to about base pair 35935 corresponding to Ad5 or from about base pair 33785 to about base pair 35759 corresponding to Ad6, joined to the fourth region.

In preferred different embodiments concerning adenovirus regions that are present: (1) the first, second, third, fourth, and fifth region corresponds to Ad5; (2) the first, second, third, fourth, and fifth region corresponds to Ad6; and (3) the first region corresponds to Ad5, the second region corresponds to Ad5, the third region corresponds to Ad6, the fourth region corresponds to Ad6, and the fifth region corresponds to Ad5.

### B. DNA Plasmid Vectors

DNA vaccine plasmid vectors contain a gene expression cassette along with elements facilitating replication and preferably vector selection. Preferred elements provide for replication in non-mammalian cells and a selectable marker. The vectors should not contain elements providing for replication in human cells or for integration into human nucleic acid.

The selectable marker facilitates selection of nucleic acids containing the marker. Preferred selectable markers are those that confer antibiotic resistance. Examples of antibiotic selection genes include nucleic acid encoding resistance to ampicillin, neomycin, and kanamycin.

Suitable DNA vaccine vectors can be produced starting with a plasmid containing a bacterial origin of replication and a selectable marker. Examples of bacterial origins of replication providing for higher yields include the ColE1 plasmid-derived bacterial origin of replication. (Donnelly et al., Annu. Rev. Immunol. 15:617-648, 1997.)

The presence of the bacterial origin of replication and selectable marker allows for the production of the DNA vector in a bacterial strain such as *E. coli.* The selectable marker is used to eliminate bacteria not containing the DNA vector.

### III. AD6 RECOMBINANT NUCLEIC ACID

Ad6 recombinant nucleic acid comprises an Ad6 region substantially similar to an Ad6 region found in SEQ. ID. NO. 8, and a region not present in Ad6 nucleic acid. Recombinant nucleic acid comprising Ad6 regions have different uses such as in producing different Ad6 regions, as intermediates in the production of Ad6 based vectors, and as a vector for delivering a recombinant gene.

As depicted in Figure 9, the genomic organization of Ad6 is very similar to the genomic organization of Ad5. The homology between Ad5 and Ad6 is approximately 98%.

In different embodiments, the Ad6 recombinant nucleic acid comprises a nucleotide region substantially similar to E1A, E1B, E2B, E2A, E3, E4, L1, L2, L3, or L4, or any combination thereof. A substantially similar nucleic acid region to an Ad6 region has a nucleotide sequence identity of at least 65%, at least 75%, at least 85%, at least 95%, at least 99% or 100%; or a nucleotide difference of 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-25, 1-30, 1-35, 1-40, 1-45, or 1-50 nucleotides. Techniques and embodiments for determining substantially similar nucleic acid sequences are described in Section I.B. *supra.*

Preferably, the recombinant Ad6 nucleic acid contains an expression cassette coding for a polypeptide not found in Ad6. Examples of expression cassettes include those coding for HCV regions and those coding for other types of polypeptides.

Different types of adenoviral vectors can be produced incorporating different amounts of Ad6, such as first and second generation adenovectors. As noted in Section II.A. *supra.* first generation adenovectors are defective in E1 and can replicate when E1 is supplied *in trans.*

Second generation adenovectors contain less adenoviral genome than first generation vectors and can be used in conjugation with complementing cell lines and/or helper vectors supplying adenoviral proteins. Second generation adenovectors are described in different references such as Russell, Journal of General Virology 81:2573-2604, 2000; Hitt et al., 1997, Human Ad vectors for Gene Transfer, Advances in Pharmacology, Vol 40 Academic Press.

In an embodiment of the present invention, the Ad6 recombinant nucleic acid is an adenovirus vector defective in E1 that is able to replicate when E1 is supplied *in trans.* Expression cassettes can be inserted into a deleted E1 region and/or a deleted E3 region.

An example of an Ad6 based adenoviral vector with an expression cassette provided in a deleted E1 region comprises or consists of:
a) a first adenovirus region from about base pair 1 to about base pair 450 corresponding to either Ad5 or Ad6;
b) a gene expression cassette in a E1 parallel or E1 anti-parallel orientation joined to the first region;
c) a second adenovirus region from about base pair 3511 to about base pair 5548 corresponding to Ad5 or from about base pair 3508 to about base pair 5541 corresponding to Ad6, joined to the expression cassette;
d) a third adenovirus region from about base pair 5549 to about base pair 28133 corresponding to Ad5 or from about base pair 5542 to about base pair 28156 corresponding to Ad6, joined to the second region;
e) an optionally present fourth region from about base pair 28134 to about base pair 30817 corresponding to Ad5, or from about base pair 28157 to about base pair 30788 corresponding to Ad6, joined to the third region;
f) a fifth adenovirus region from about base pair 30818 to about base pair 33966 corresponding to Ad5 or from about base pair 30789 to about base pair 33784 corresponding to Ad6, wherein the fifth region is joined to the fourth region if the fourth region is present, or the fifth is joined to the third region if the fourth region is not present; and
g) a sixth adenovirus region from about base pair 33967 to about base pair 35935 corresponding to Ad5 or from about base pair 33785 to about base pair 35759 corresponding to Ad6, joined to the fifth region;
wherein at least one Ad6 region is present.

In different embodiments of the invention, all of the regions are from Ad6; all of the regions expect for the first and second are from Ad6; and 1, 2, 3, or 4 regions selected from the second, third, fourth, and fifth regions are from Ad6.

An example of an Ad6 based adenoviral vector with an expression cassette provided in a deleted E3 region comprises or consists of:
a) a first adenovirus region from about base pair 1 to about base pair 450 corresponding to either Ad5 or Ad6;
b) a second adenovirus region from about base pair 3511 to about base pair 5548 corresponding to Ad5 or from about base pair 3508 to about base pair 5541 corresponding to Ad6, joined to the first region;
c) a third adenovirus region from about base pair 5549 to about base pair 28133 corresponding to Ad5 or from about base pair 5542 to about base pair 28156 corresponding to Ad6, joined to the second region;
d) a gene expression cassette in a E3 parallel or E3 anti-parallel orientation joined to the third region;
e) a fourth adenovirus region from about base pair 30818 to about base pair 33966 corresponding to Ad5 or from about base pair 30789 to about base pair 33784 corresponding to Ad6, joined to the gene expression cassette; and
f) a fifth adenovirus region from about base pair 33967 to about base pair 35935 corresponding to Ad5 or from about base pair 33785 to about base pair 35759 corresponding to Ad6, joined to the fourth region;
wherein at least one Ad6 region is present.

In different embodiment of the invention, all of the regions are from Ad6; all of the regions expect for the first and second are from Ad6; and 1, 2, 3, or 4 regions selected from the second, third, fourth and fifth regions are from Ad6.

### IV. VECTOR PRODUCTION

Vectors can be produced using recombinant nucleic acid techniques such as those involving the use of restriction enzymes, nucleic acid ligation, and homologous recombination. Recombinant nucleic acid techniques are well known in the art. (Ausubel, Current Protocols in Molecular Biology, John Wiley, 1987-1998, and Sambrook et al., Molecular Cloning, A Laboratory Manual, 2nd Edition, Cold Spring Harbor Laboratory Press, 1989.)

Intermediate vectors are used to derive a therapeutic vector or to transfer an expression cassette or portion thereof from one vector to another vector. Examples of intermediate vectors include adenovirus genome plasmids and shuttle vectors.

Useful elements in an intermediate vector include an origin of replication, a selectable marker, homologous recombination regions, and convenient restriction sites. Convenient restriction sites can be used to facilitate cloning or release of a nucleic acid sequence.

Homologous recombination regions provide nucleic acid sequence regions that are homologous to a target region in another nucleic acid molecule. The homologous regions flank the nucleic acid sequence that is being inserted into the target region. In different embodiments homologous regions are preferably about 150 to 600 nucleotides in length, or about 100 to 500 nucleotides in length.

An embodiment of the present invention describes a shuttle vector containing a Met-NS3-NS4A-NS4B-NS5A-NS5B expression cassette, a selectable marker, a bacterial origin of replication, a first adenovirus homology region and a second adenovirus homologous region that target the expression cassette to insert in or replace an E1 region. The first and second homology regions flank the expression cassette. The first homology region contains at least about 100 base pairs substantially homologous to at least the right end (3' end) of a wild-type adenovirus region from about base pairs 4-450. The second homology contains at least about 100 base pairs substantially homologous to at least the left end (5' end) of Ad5 from about base pairs 3511-5792, or the corresponding region from another adenovirus.

Reference to "substantially homologous" indicates a sufficient degree of homology to specifically recombine with a target region. In different embodiments substantially homologous refers to at least 85%, at least 95%, or 100% sequence identity. Sequence identity can be calculated as described in Section I.B. *supra.*

One method of producing adenovectors is through the creation of an adenovirus genome plasmid containing an expression cassette. The pre-Adenovirus plasmid contains all the adenovirus sequences needed for replication in the desired complimenting cell line. The pre-Adenovirus plasmid is then digested with a restriction enzyme to release the viral ITR's and transfected into the complementing cell line for virus rescue. The ITR's must be released from plasmid sequences to allow replication to occur. Adenovector rescue results in the production on an adenovector containing the expression cassette.

### A. Adenovirus Genome Plasmids

Adenovirus genome plasmids contain an adenovector sequence inside a longer-length plasmid (which may be a cosmid). The longer-length plasmid may contain additional elements such as those facilitating growth and selection in eukaryotic or bacterial cells depending upon the procedures employed to produce and maintain the plasmid. Techniques for producing adenovirus genome plasmids include those involving the use of shuttle vectors and homologous recombination, and those involving the insertion of a gene expression cassette into an adenovirus cosmid. (Hitt et al., Methods in Molecular Genetics 7:13-30, 1995, Danthinne et al., Gene Therapy 7:1707-1714, 2000.)

Adenovirus genome plasmids preferably have a gene expression cassette inserted into a E1 or E3 deleted region. In an embodiment of the present invention, the adenovirus genome plasmid contains a gene expression cassette inserted in the E1 deleted region, an origin of replication, a selectable marker, and the recombinant adenovirus region is made up of:
a) a first adenovirus region from about base pair 1 to about base 450 corresponding to either Ad5 or Ad6;
b) a gene expression cassette in a E1 parallel or E1 anti-parallel orientation joined to the first region;
c) a second adenovirus region from about base pair 3511 to about base pair 5548 corresponding to Ad5 or from about base pair 3508 to about base pair 5541 corresponding to Ad6, joined to the expression cassette;
d) a third adenovirus region from about base pair 5549 to about base pair 28133 corresponding to Ad5 or from about base pair 5542 to about base pair 28156 corresponding to Ad6, joined to the second region;
e) a fourth adenovirus region from about base pair 30818 to about base pair 33966 corresponding to Ad5 or from about base pair 30789 to about base pair 33784 corresponding to Ad6, joined to the third region;
f) a fifth adenovirus region from about base pair 33967 to about base pair 35935 corresponding to Ad5 or from about base pair 33785 to about base pair 35759 corresponding to Ad6, joined to the fourth region, and
g) an optionally present E3 region corresponding to all or part of the E3 region present in Ad5 or Ad6, which may be present for smaller inserts taking into account the overall size of the desired adenovector.

In another embodiment of the present invention the recombinant adenovirus genome plasmid has the gene expression cassette inserted in the E3 deleted region. The vector contains an origin of replication, a selectable marker, and the following:
a) a first adenovirus region from about base pair 1 to about base pair 450 corresponding to either Ad5 or Ad6;
b) a second adenovirus region from about base pair 3511 to about base pair 5548 corresponding to Ad5 or from about base pair 3508 to about base pair 5541 corresponding to Ad6, joined to the expression cassette;
c) a third adenovirus region from about base pair 5549 to about base pair 28133 corresponding to Ad5 or from about base pair 5542 to about base pair 28156 corresponding to Ad6, joined to the second region;
d) the gene expression cassette in a E3 parallel or E3 anti-parallel orientation joined to the third region;
e) a fourth adenovirus region from about base pair 30818 to about base pair 33966 corresponding to Ad5 or from about base pair 30789 to about base pair 33784 corresponding to Ad6, joined to the gene expression cassette; and
f) a fifth adenovirus region from about base pair 33967 to about base pair 35935 corresponding to Ad5 or from about base pair 33785 to about base pair 35759 corresponding to Ad6, joined to the fourth region.

In different embodiments concerning adenovirus regions that are present: (1) the first, second, third, fourth, and fifth region corresponds to Ad5; (2) the first, second, third, fourth, and fifth region corresponds to Ad6; and (3) the first region corresponds to Ad5, the second region corresponds to Ad5, the third region corresponds to Ad6, the fourth region corresponds to Ad6, and the fifth region corresponds to Ad5.

An embodiment of the present invention describes a method of making an adenovector involving a homologous recombination step to produce a adenovirus genome plasmid and an adenovirus rescue step. The homologous recombination step involves the use of a shuttle vector containing a Met-NS3-NS4A-NS4B-NS5A-NS5B expression cassette flanked by adenovirus homology regions. The adenovirus homology regions target the expression cassette into either the E1 or E3 deleted region.

In an embodiment of the present invention concerning the production of an adenovirus genome plasmid, the gene expression cassette is inserted into a vector comprising: a first adenovirus region from about base pair 1 to about base pair 450 corresponding to either Ad5 or Ad6; a second adenovirus region from about base pair 3511 to about base pair 5548 corresponding to Ad5 or from about base pair 3508 to about base pair 5541 corresponding to Ad6, joined to the second region; a third adenovirus region from about base pair 5549 to about base pair 28133 corresponding to Ad5 or from about base pair 5542 to about base pair 28156 corresponding to Ad6, joined to the second region; a fourth adenovirus region from about base pair 30818 to about base pair 33966 corresponding to Ad5 or from about base pair 30789 to about base pair 33784 corresponding to Ad6, joined to the third region; and a fifth adenovirus region from about 33967 to about 35935 corresponding to Ad5 or from about base pair 33785 to about base pair 35759 corresponding to Ad6, joined to the fourth region. The adenovirus genome plasmid should contain an origin of replication and a selectable marker, and may contain all or part of the Ad5 or Ad6 E3 region.

In different embodiments concerning adenovirus regions that are present: (1) the first, second, third, fourth, and fifth region corresponds to Ad5; (2) the first, second, third, fourth, and fifth region corresponds to Ad6; and (3) the first region corresponds to Ad5, the second region corresponds to Ad5, the third region corresponds to Ad6, the fourth region corresponds to Ad6, and the fifth region corresponds to Ad5.

### B. Adenovector Rescue

An adenovector can be rescued from a recombinant adenovirus genome plasmid using techniques known in the art or described herein. Examples of techniques for adenovirus rescue well known in the art are provided by Hitt et al., Methods in Molecular Genetics 7:13-30, 1995, and Danthinne et al., Gene Therapy 7:1707-1714, 2000.

A preferred method of rescuing an adenovector described herein involves boosting adenoviral replication. Boosting adenoviral replication can be performed, for example, by supplying adenoviral functions such as E2 proteins (polymerase, pre-terminal protein and DNA binding protein) as well as E4 orf6 on a separate plasmid. Example 10 *infra.* illustrates the boosting of adenoviral replication to rescue an adenovector containing a codon optimized Met-NS3-NS4A-NS4B-NS5A-NS5B expression cassette.

### V. PARTIAL-OPITIMIZED HCV ENCODING SEQUENCES

Partial optimization of HCV polyprotein encoding nucleic acid provides for a lesser amount of codons optimized for expression in a human than complete optimization. The overall objective is to provide the benefits of increased expression due to codon optimization, while facilitating the production of an adenovector containing HCV polyprotein encoding nucleic acid having optimized codons.

Complete optimization of an HCV polyprotein encoding sequence provides the most frequently observed human codon for each amino acid. Complete optimization can be performed using codon frequency tables well known in the art and using programs such as the BACKTRANSLATE program (Wisconsin Package version 10, Genetics Computer Group, GCG, Madison, Wisc.).

Partial optimization can be preformed on an entire HCV polyprotein encoding sequence that is present (*e*.*g*., NS3-NS5B), or one or more local regions that are present. In different embodiments the GC content for the entire HCV encoded polyprotein that is present is no greater than at least about 65%; and the GC content for one or more local regions is no greater than about 70%.

Local regions are regions present in HCV encoding nucleic acid, and can vary in size. For example, local regions can be about 60, about 70, about 80, about 90 or about 100 nucleotides in length.

Partial optimization can be achieved by initially constructing an HCV encoding polyprotein sequence to be partially optimized based on a naturally ocurring sequence. Alternatively, an optimized HCV encoding sequence can be used as basis of comparison to produce a partial optimized sequence.

### VI. HCV COMBINATION TREATMENT

The HCV Met-NS3-NS4A-NS4B-NS5A-NS5B vaccine can be used by itself to treat a patient, can be used in conjunction with other HCV therapeutics, and can be used with agents targeting other types of diseases. Additional therapeutics include additional therapeutic agents to treat HCV and diseases having a high prevalence in HCV infected persons. Agents targeting other types of disease include vaccines directed against HIV and HBV.

Additional therapeutics for treating HCV include vaccines and non-vaccine agents. (Zein, Expert Opin. Investig. Drugs 10:1457-1469, 2001.) Examples of additional HCV vaccines include vaccines designed to elicit an immune response against an HCV core antigen and the HCV E1, E2 or p7 region. Vaccine components can be naturally occurring HCV polypeptides, HCV mimotope polypeptides or nucleic acid encoding such polypeptides.

HCV mimotope polypeptides contain HCV epitopes, but have a different sequence than a naturally occurring HCV antigen. A HCV mimotope can be fused to a naturally occurring HCV antigen. References describing techniques for producing mimotopes in general and describing different HCV mimotopes are provided in Felici et al. U.S. Patent No. 5,994,083 and Nicosia *et al*., International Application Number WO 99/60132.

### VII. PHARMACEUTICAL ADMINISTRATION

HCV vaccines can be formulated and administered to a patient using the guidance provided herein along with techniques well known in the art. Guidelines for pharmaceutical administration in general are provided in, for example, *Modern Vaccinology*, Ed. Kurstak, Plenum Med. Co. 1994; *Remington's Pharmaceutical Sciences 18^{th} Edition*, Ed. Gennaro, Mack Publishing, 1990; and *Modern Pharmaceutics 2^{nd} Edition*, Eds. Banker and Rhodes, Marcel Dekker, Inc., 1990, each of which are hereby incorporated by reference herein.

HCV vaccines can be administered by different routes such intravenous, intraperitoneal, subcutaneous, intramuscular, intradermal, impression through the skin, or nasal. A preferred route is intramuscular.

Intramuscular administration can be preformed using different techniques such as by injection with or without one or more electric pulses. Electric mediated transfer can assist genetic immunization by stimulating both humoral and cellular immune responses.

Vaccine injection can be performed using different techniques, such as by employing a needle or a needless injection system. An example of a needless injection system is a jet injection device. (Donnelly *et al.,* International Publication Number WO 99/52463.)

### A. Electrically Mediated Transfer

Electrically mediated transfer or Gene Electro-Transfer (GET) can be performed by delivering suitable electric pulses after nucleic acid injection. (See Mathiesen, International Publication Number WO 98/43702). Plasmid injection and electroporation can be performed using stainless needles. Needles can be used in couples, triplets or more complex patterns. In one configuration the needles are soldered on a printed circuit board that is a mechanical support and connects the needles to the electrical field generator by means of suitable cables.

The electrical stimulus is given in the form of electrical pulses. Pulses can be of different forms (square, sinusoidal, triangular, exponential decay) and different polarity (monopolar of positive or negative polarity, bipolar). Pulses can be delivered either at constant voltage or constant current modality.

Different patterns of electric treatment can be used to introduce nucleic acid vaccines including HCV and other nucleic acid vaccines into a patient. Possible patterns of electric treatment include the following:
Treatment 1: 10 trains of 1000 square bipolar pulses delivered every other second, pulse length 0.2 msec/phase, frequency 1000 Hz, constant voltage mode, 45 Volts/phase, floating current.
Treatment 2: 2 trains of 100 square bipolar pulses delivered every other second, pulse length 2 msec/phase, frequency 100 Hz, constant current mode, 100 mA/phase, floating voltage.
Treatment 3: 2 trains of bipolar pulses at a pulse length of about 2 msec/phase, for a total length of about 3 seconds, where the actual current going through the tissue is fixed at about 50 mA.

Electric pulses are delivered through an electric field generator. A suitable generator can be composed of three independent hardware elements assembled in a common chassis and driven by a portable PC which runs the driving program. The software manages both basic and accessory functions. The elements of the device are: (1) signal generator driven by a microprocessor, (2) power amplifier and (3) digital oscilloscope.

The signal generator delivers signals having arbitrary frequency and shape in a given range under software control. The same software has an interactive editor for the waveform to be delivered. The generator features a digitally controlled current limiting device (a safety feature to control the maximal current output). The power amplifier can amplify the signal generated up to +/- 150 V. The oscilloscope is digital and is able to sample both the voltage and the current being delivered by the amplifier.

### B. Pharmaceutical Carriers

Pharmaceutically acceptable carriers facilitate storage and administration of a vaccine to a subject. Examples of pharmaceutically acceptable carriers are described herein. Additional pharmaceutical acceptable carriers are well known in the art.

Pharmaceutically acceptable carriers may contain different components such a buffer, normal saline or phosphate buffered saline, sucrose, salts and polysorbate. An example of a pharmaceutically acceptable carrier is follows: 2.5-10 mM TRIS buffer, preferably about 5 mM TRIS buffer; 25-100 mM NaCl, preferably about 75 mM NaCl; 2.5-10% sucrose, preferably about 5% sucrose; 0.01 -2 mM MgCl₂; and 0.001%-0.01% polysorbate 80 (plant derived). The pH is preferably from about 7.0-9.0, more preferably about 8.0. A specific example of a carrier contains 5 mM TRIS, 75 mM NaCl, 5% sucrose, 1 mM MgCl₂, 0.005% polysorbate 80 at pH 8.0.

### C. Dosing Regimes

Suitable dosing regimens can be determined taking into account the efficacy of a particular vaccine and factors such as age, weight, sex and medical condition of a patient; the route of administration; the desired effect; and the number of doses. The efficacy of a particular vaccine depends on different factors such as the ability of a particular vaccine to produce polypeptide that is expressed and processed in a cell and presented in the context of MHC class I and II complexes.

HCV encoding nucleic acid administered to a patient can be part of different types of vectors including viral vectors such as adenovector, and DNA plasmid vaccines. In different embodiments concerning administration of a DNA plasmid, about 0.1 to 10 mg of plasmid is administered to a patient, and about 1 to 5 mg of plasmid is administered to a patient. In different embodiments concerning administration of a viral vector, preferably an adenoviral vector, about 105 to 10¹¹ viral particles are administered to a patient, and about 10⁷ to 10¹⁰ viral particles are administered to a patient.

Viral vector vaccines and DNA plasmid vaccines may be administered alone, or may be part of a prime and boost administration regimen. A mixed modality priming and booster inoculation involves either priming with a DNA vaccine and boosting with viral vector vaccine, or priming with a viral vector vaccine and boosting with a DNA vaccine.

Multiple priming, for example, about to 2-4 or more may be used. The length of time between priming and boost may typically vary from about four months to a year, but other time frames may be used. The use of a priming regimen with a DNA vaccine may be preferred in situations where a person has a pre-existing anti-adenovirus immune response.

In an embodiment of the present invention, 1x10⁷ to 1x10¹² particles and preferably about 1x10¹⁰ to 1x10¹¹ particles of adenovector is administered directly into muscle tissue. Following initial vaccination a boost is performed with an adenovector or DNA vaccine.

In another embodiment of the present invention initial vaccination is performed with a DNA vaccine directly into muscle tissue. Following initial vaccination a boost is performed with an adenovector or DNA vaccine.

Agents such as interleukin-12, GM-CSF, B7-1, B7-2, IP10, Mig-1 can be coadministered to boost the immune response. The agents can be coadministered as proteins or through use of nucleic acid vectors.

### D. Heterologous Prime-Boost

Heterologous prime-boost is a mixed modality involving the use of one type of viral vector for priming and another type of viral vector for boosting. The heterologous prime-boost can involve related vectors such as vectors based on different adenovirus serotypes and more distantly related viruses such adenovirus and poxvirus. The use of poxvirus and adenovirus vectors to protect mice against malaria is illustrated by Gilbert et al., Vaccine 20:1039-1045, 2002.

Different embodiments concerning priming and boosting involve the following types of vectors expressing desired antigens such as Met-NS3-NS4A-NS4B-NS5A-NS5B: Ad5 vector followed by Ad6 vector; Ad6 vector followed by Ad5 vector; Ad5 vector followed by poxvirus vector; poxvirus vector followed by Ad5 vector; Ad6 vector followed by poxvirus vector; and poxvirus vector followed by Ad6 vector.

The length of time between priming and boosting typically varies from about four months to a year, but other time frames may be used. The minimum time frame should be sufficient to allow for an immunological rest. In an embodiment, this rest is for a period of at least 6 months. Priming may involve multiple priming with one type of vector, such as 2-4 primings.

Expression cassettes present in a poxvirus vector should contain a promoter either native to, or derived from, the poxvirus of interest or another poxvirus member. Different strategies for constructing and employing different types of poxvirus based vectors including those based on vaccinia virus, modified vaccinia virus, avipoxvirus, raccoon poxvirus, modified vaccinia virus Ankara, canarypoxviruses (such as ALVAC), fowlpoxviruses, cowpoxviruses, and NYVAC are well known in the art. (Moss, Current Topics in Microbiology and Immunology 158:25-38, 1982; Earl et al., In Current Protocols in Molecular Biology, Ausubel et al. eds., New York: Greene Publishing Associates & Wiley Interscience; 1991:16.16.1-16.16.7, Child et al., Virology 174(2):625-9, 1990; Tartaglia et al., Virology 188:217-232, 1992; U.S. Patent Nos., 4,603,112, 4,722,848, 4,769,330, 5,110,587, 5,174,993, 5,185,146, 5,266,313, 5,505,941, 5,863,542, and 5,942,235.

### E. Adjuvants

HCV vaccines can be formulated with an adjuvant. Adjuvants are particularly useful for DNA plasmid vaccines. Examples of adjuvants are alum, AlPO₄, alhydrogel, Lipid-A and derivatives or variants thereof, Freund's incomplete adjuvant, neutral liposomes, liposomes containing the vaccine and cytokines, non-ionic block copolymers, and chemokines.

Non-ionic block polymers containing polyoxyethylene (POE) and polyxylpropylene (POP), such as POE-POP-POE block copolymers may be used as an adjuvant. (Newman et al., Critical Reviews in Therapeutic Drug Carrier Systems 15:89-142, 1998.) The immune response of a nucleic acid can be enhanced using a non-ionic block copolymer combined with an anionic surfactant.

A specific example of an adjuvant formulation is one containing CRL-1005 (CytRx Research Laboratories), DNA, and benzylalkonium chloride (BAK). The formulation can be prepared by adding pure polymer to a cold (< 5°C) solution of plasmid DNA in PBS using a positive displacement pipette. The solution is then vortexed to solubilize the polymer. After complete solubilization of the polymer a clear solution is obtained at temperatures below the cloud point of the polymer (-6-7°C). Approximately 4 mM BAK is then added to the DNA/CRL-1005 solution in PBS, by slow addition of a dilute solution of BAK dissolved in PBS. The initial DNA concentration is approximately 6 mg/mL before the addition of polymer and BAK, and the final DNA concentration is about 5 mg/mL. After BAK addition the formulation is vortexed extensively, while the temperature is allowed to increase from ~ 2°C to above the cloud point. The formulation is then placed on ice to decrease the temperature below the cloud point. Then, the formulation is vortexed while the temperature is allowed to increase from ~2°C to above the cloud point. Cooling and mixing while the temperature is allowed to increase from ~2°C to above the cloud point is repeated several times, until the particle size of the formulation is about 200-500 nm, as measured by dynamic light scattering. The formulation is then stored on ice until the solution is clear, then placed in storage at -70°C. Before use, the formulation is allowed to thaw at room temperature.

### F. Vaccine Storage

Adenovector and DNA vaccines can be stored using different types of buffers. For example, buffer A105 described in Example 9 *infra.* can be used to for vector storage.

Storage of DNA can be enhanced by removal or chelation of trace metal ions. Reagents such as succinic or malic acid, and chelators can be used to enhance DNA vaccine stability. Examples of chelators include multiple phosphate ligands and EDTA. The inclusion of non-reducing free radical scavengers, such as ethanol or glycerol, can also be useful to prevent damage of DNA plasmid from free radical production. Furthermore, the buffer type, pH, salt concentration, light exposure, as well as the type of sterilization process used to prepare the vials, may be controlled in the formulation to optimize the stability of the DNA vaccine.

### VII. EXAMPLES

Examples are provided below to further illustrate different features of the present invention. The examples also illustrate useful methodology for practicing the invention. These examples do not limit the claimed invention.

### Example 1: Met-NS3-NS4A-NS4B-NS5A-NS5B Expression Cassettes

Different gene expression cassettes encoding HCV NS3-NS4A-NS4B-NS5A-NS5B were constructed based on a 1b subtype HCV BK strain. The encoded sequences had either (1) an active NS5B sequence ("NS"), (2) an inactive NS5B sequence ("NSmut"), (3) a codon optimized sequence with an inactive NS5B sequence ("NSOPTmut"). The expression cassettes also contained a CMV promoter/enhancer and the BGH polyadenylation signal.

The NS nucleotide sequence (SEQ. ID. NO. 5) differs from HCV BK strain GenBank accession number M58335 by 30 out of 5952 nucleotides. The NS amino acid sequence (SEQ. ID. NO. 6) differs from the corresponding 1b genotype HCV BK strain by 7 out of 1984 amino acids. To allow for initiation of translation an ATG codon is present at the 5' end of the NS sequence. A TGA termination sequence is present at the 3' end of the NS sequence.

The NSmut nucleotide sequence (SEQ. ID. NO. 2, Figure 2), is similar to the NS sequence. The differences between NSmut and NS include NSmut having an altered NS5B catalytic site; an optimal ribosome binding site at the 5' end; and a TAAA termination sequence at the 3' end. The alterations in NS5B comprise bases 5138 to 5146, which encode amino acids 1711 to 1713. The alterations result in a change of amino acids GlyAspAsp into AlaAlaGly and creates an inactive form of the NS5B RNA-dependent RNA-polymerase NS5B.

The NSOPTmut sequence (SEQ. ID. NO. 3, Figure 3) was designed based on the amino acid sequence encoded by NSmut. The NSmut amino acid sequence was back translated into a nucleotide sequence with the GCG (Wisconsin Package version 10, Genetics Computer Group, GCG, Madison, Wisc.) BACKTRANSLATE program. To generate a NSOPTmut nucleotide sequence where each amino acid is coded for by the corresponding most frequently observed human codon, the program was run choosing as parameter the generation of the most probable nucleotide sequence and specifying the codon frequency table of highly expressed human genes (human_high.cod) available within the GCG Package as translation scheme.

### Example 2: Generation pV1Jns plasmid with NS, NSmut or NSOPTmut Sequences

pV1Jns plasmids containing either the NS sequence, NSmut sequence or NSOPTmut sequences were generated and characterised as follows:

### pV1Jns Plasmid with the NS Sequence

The coding region Met-NS3-NS4A-NS4B-NS5A and the coding region Met-NS3-NS4A-NS4B-NS5A-NS5B from a HCV BK type strain (Tomei et al., J. Virol. 67:4017-4026, 1993) were cloned into pcDNA3 plasmid (Invitrogen), generating pcD3-5a and pcD3-5b vectors, respectively. PcD3-5A was digested with Hind III, blunt-ended with Klenow fill-in and subsequently digested with Xba I, to generate a fragment corresponding to the coding region of Met-NS3-NS4A-NS4B-NS5A. The fragment was cloned into pV1Jns-poly, digested with Bgl II blunt-ended with Klenow fill-in and subsequently digested with Xba I, generating pV1JnsNS3-5A.

pV1Jns-poly is a derivative of pV1JnsA plasmid (Montgomery et al., DNA and Cell Biol. 12:777-783, 1993), modified by insertion of a polylinker containing recognition sites for XbaI, PmeI, PacI into the unique BglII and NotI restriction sites. The pV1Jns plasmid with the NS sequence (pV1JnsNS3-5B) was obtained by homologous recombination into the bacterial strain BJ5183, co-transforming pV1JNS3-5A linearized with XbaI and NotI digestion and a PCR fragment containing approximately 200 bp of NS5A, NS5B coding sequence and approximately 60 bp of the BGH polyadenylation signal. The resulting plasmid represents pV1Jns-NS.

pV1Jns-NS can be summarized as follows:

| | |
|---|---|
| Bases an additional then the then the an additional NO. 14) | 1 to 1881 of pV1JnsA |
| | AGCTT |
| | Met-NS3-NS5B sequence (SEQ. ID. NO. 5) |
| | wt TGA stop |
| | TCTAGAGCGTTTAAACCCTTAATTAAGG (SEQ. ID. |
| Bases | 1912 to 4909 of pV1JnsA |

### pV1Jns Plasmid with the NSmut Sequence

The V1JnsNS3-5A plasmid was modified at the 5' of the NS3 coding sequence by addition of a full Kozak sequence. The plasmid (V1JNS3-5Akozak) was obtained by homologous recombination into the bacterial strain BJ5183, co-transforming V1JNS3-5A linearized by *Afl*II digestion and a PCR fragment containing the proximal part of Intron A, the restriction site BglII, a full Kozak translation initiation sequence and part of the NS3 coding sequence.

The resulting plasmid (V1JNS3-5Akozak) was linearized with Xba I digestion and co-transformed into the bacterial strain BJ5183 with a PCR fragment, containing approximately 200 bp of NS5A, the NS5B mutated sequence, the strong translation termination TAAA and approximately 60 bp of the BGH polyadenylation signal. The PCR fragment was obtained by assembling two 22bp-overlapping fragments where mutations were introduced by the oligonucleotides used for their amplification. The resulting plasmid represents pV1Jns-NSmut.

pV1Jns-NSmut can be summarized as follows:

| | |
|---|---|
| Bases then the an additional | 1 to 1882 of pV1JnsA |
| | kozak Met-NS3-NS5B(mut) TAAA sequence (SEQ. ID. NO. 2) |
| | TCTAGA |
| Bases | 1925 to 4909 of pV1JnsA |

### pV1Jns Plasmid with the NSOPTmut Sequence

The human codon-optimized synthetic gene (NSOPTmut) with mutated NS5B to abrogate enzymatic activity, full Kozak translation initiation sequence and a strong translation termination was digested with BamHI and SalI restriction sites present at the 5' and 3' end of the gene. The gene was then cloned into the BglII and SalI restriction sites present in the polylinker of pV1JnsA plasmid, generating pV1Jns-NSOPTmut.

pV1Jns-NSOPTmut can be summarized as follows:

| | |
|---|---|
| Bases an additional then an additional | 1 to 1881 of pV1JnsA |
| | C |
| | kozak Met-NS3-NS5B(optmut) TAAA sequence (SEQ. ID. NO. 3) |
| | TTTAAATGTTTAAAC (SEQ. ID. NO. 15) |
| Bases | 1905 to 4909 of pV1JnsA |

### Plasmids Characterization

Expression of HCV NS proteins was tested by transfection of HEK 293 cells, grown in 10% FCS/DMEM supplemented by L-glutamine (final 4 mM). Twenty-four hours before transfection, cells were plated in 6-well 35 mm diameter, to reach 90-95% confluence on the day of transfection. Forty nanograms of plasmid DNA (previously assessed as a non-saturating DNA amount) were co-transfected with 100 ng of pRSV-Luc plasmid containing the luciferase reporter gene under the control of Rous sarcoma virus promoter, using the LIPOFECTAMINE 2000 reagent. Cells were kept in a CO₂ incubator for 48 hours at 37 °C.

Cell extracts were prepared in 1% Triton/TEN buffer. The extracts were normalized for Luciferase activity, and run in serial dilution on 10% SDS-acrylamide gel. Proteins were transferred on nitrocellulose and assayed with antibodies directed against NS3, NS5A and NS5B to assess strength of expression and correct proteolytic cleavage. Mock-transfected cells were used as a negative control. Results from representative experiments testing pV1JnsNS, pV1JnsNSmut and pV1JnsNSOPTmut are shown in Figure 12.

### Example 3: Mice Immunization with Plasmid DNA Vectors

The DNA plasmids pV1Jns-NS, pV1Jns-NSmut and pV1Jns-NSOPTmut were injected in different mice strains to evaluate their potential to elicit anti-HCV immune responses. Two different strains (Balb/C and C57Black6, N=9-10) were injected intramuscularly with 25 or 50 µg of DNA followed by electrical pluses. Each animal received two doses at three weeks interval.

Humoral immune response elicited in C57Black6 mice against the NS3 protein was measured in post dose two sera by ELISA on bacterially expressed NS3 protease domain. Antibodies specific for the tested antigen were detected in animals immunized with all three vectors with geometric mean titers (GMT) ranging from 94000 to 133000 (Tables 1-3).

**Table 1: pV1jns-NS**

| | | | | | | | | | | GMT |
|---|---|---|---|---|---|---|---|---|---|---|
| Mice n. | 1 | 2 | 3 | 4 | 5 | 6 | 7 | 8 | 9 | |
| Titer | 105466 | 891980 | 78799 | 39496 | 543542 | 182139 | 32351 | 95028 | 67800 | 94553 |

**Table 2: pV1jns-NSmut**

| | | | | | | | | | | | GMT |
|---|---|---|---|---|---|---|---|---|---|---|---|
| Mice n. | 11 | 12 | 13 | 14 | 15 | 16 | 17 | 18 | 19 | 20 | |
| Titer | 202981 | 55670 | 130786 | 49748 | 17672 | 174958 | 44304 | 37337 | 78182 | 193695 | 75083 |

**Table 3: pV1jns-NSOPTmut**

| | | | | | | | | | | | GMT |
|---|---|---|---|---|---|---|---|---|---|---|---|
| Mice n. | 21 | 22 | 23 | 24 | 25 | 26 | 27 | 28 | 29 | 30 | |
| Titer | 310349 | 43645 | 63496 | 82174 | 630778 | 297259 | 66861 | 146735 | 173506 | 77732 | 133165 |

A T cell response was measured in C57Black6 mice immunized with two intramuscular injections at three weeks interval with 25 µg of plasmid DNA. Quantitative ELIspot assay was performed to determine the number of IFNγ secreting T cells in response to five pools of 20mer peptides overlapping by ten residues encompassing the NS3-NS5B sequence. Specific CD8+ response was analyzed by the same assay using a 20mer peptide encompassing a CD8+ epitope for C57Black6 mice (pep1480).

Cells secreting IFNγ in an antigen specific-manner were detected using a standard ELIspot assay. T cell response in C57Black6 mice immunized with two intramuscular injections at three weeks interval with 50 µg of plasmid DNA, was analyzed by the same ELIspot assay measuring the number of IFNγ secreting T cells in response to five pools of 20mer peptides overlapping by ten residues encompassing the NS3-NS5B sequence.

Spleen cells were prepared from immunized mice and re-suspended in R10 medium (RPMI 1640 supplemented with 10% FCS, 2 mM L-Glutamine, 50 U/ml-50µg/ml Penicillin/Streptomycin, 10 mM Hepes, 50 µM 2-mercapto-ethanol). Multiscreen 96-well Filtration Plates (Millipore, Cat. No. MAIPS4510, Millipore Corporation, 80 Ashby Road Bedford, MA) were coated with purified rat anti-mouse INFγ antibody (PharMingen, Cat. No. 18181D, PharmiMingen, 10975 Torreyana Road, San Diego, California 92121-1111 USA). After overnight incubation, plates were washed with PBS 1X/0.005% Tween and blocked with 250 µl/well of R10 medium.

Splenocytes from immunized mice were prepared and incubated for twenty-four hours in the presence or absence of 10 µM peptide at a density of 2.5 X 10⁵/well or 5 X 10⁵/well. After extensive washing (PBS 1X/0.005% Tween), biotinylated rat anti-mouse IFNγ antibody (PharMingen, Cat. No. 18112D, PharMingen, 10975 Torreyana Road, San Diego, California 92121-1111 USA) was added and incubated overnight at 4° C. For development, streptavidin-AKP (PharMingen, Cat. No. 13043E, PharMingen, 10975 Torreyana Road, San Diego, California 92121-1111 USA) and 1-Step^{™} NBT-BCIP development solution (Pierce, Cat. No. 34042, Pierce, P.O. Box 117, Rockford, IL 61105 USA) were added.

Pools of 20mer overlapping peptides encompassing the entire sequence of the HCV BK strain NS3 to NS5B were used to reveal HCV-specific IFNγ-secreting T cells. Similarly a single 20mer peptide encompassing a CD8+ epitope for C57Black6 mice was used to detect CD8 response. Representative data from groups of C57Black6 and Balb/C mice (N=9-10) immunized with two injections of 25 or 50 µg of plasmid vectors pV1Jns-NS, pV1Jns-NSmut and pV1Jns-NSOPTmut are shown in Figures 13A and 13B.

### Example 4: Immunization of Rhesus Macaques

Rhesus macaques (N=3) were immunized by intramuscular injection with 5mg of plasmid pV1Jns-NSOPTmut in 7.5mg/ml CRL1005, Benzalkonium chloride 0.6 mM. Each animal received two doses in the deltoid muscle at 0, and 4 weeks.

CMI was measured at different time points by IFN-γ ELISPOT. This assay measures HCV antigen-specific CD8+ and CD4+ T lymphocyte responses, and can be used for a variety of mammals, such as humans, rhesus monkeys, mice, and rats.

The use of a specific peptide or a pool of peptides can simplify antigen presentation in CTL cytotoxicity assays, interferon-gamma ELISPOT assays and interferon-gamma intracellular staining assays. Peptides based on the amino acid sequence of various HCV proteins (core, E2, NS3, NS4A, NS4B, NS5A, NS5B) were prepared for use in these assays to measure immune responses in HCV DNA and adenovirus vector vaccinated rhesus monkeys, as well as in HCV-infected humans. The individual peptides are overlapping 20-mers, offset by 10 amino acids. Large pools of peptides can be used to detect an overall response to HCV proteins while smaller pools and individual peptides may be used to define the epitope specificity of a response.

### IFNγ ELISPOT

The IFNγ-ELISPOT assay provides a quantitative determination of HCV-specific T lymphocyte responses. PBMC are serially diluted and placed in microplate wells coated with anti-rhesus IFN-γ antibody (MD-1 U-Cytech). They are cultured with a HCV peptide pool for 20 hours, resulting in the restimulation of the precursor cells and secretion of IFN-γ. The cells are washed away, leaving the secreted IFN bound to the antibody-coated wells in concentrated areas where the cells were sitting. The captured IFN is detected with biotinylated anti-rhesus IFN antibody (detector Ab U-Cytech) followed by alkaline phosphatase-conjugated streptavidin (Pharmingen 13043E). The addition of insoluble alkaline phosphatase substrate results in dark spots in the wells at the sites where the cells were located, leaving one spot for each T cell that secreted IFN-γ.

The number of spots per well is directly related to the precursor frequency of antigen-specific T cells. Gamma interferon was selected as the cytokine visualized in this assay (using species specific anti-gamma interferon monoclonal antibodies) because it is the most common, and one of the most abundant cytokines synthesized and secreted by activated T lymphocytes. For this assay, the number of spot forming cells (SFC) per million PBMCs is determined for samples in the presence and absence (media control) of peptide antigens. Data from Rhesus macaques on PBMC from post dose two material are shown in Table 4.

**Table 4**

| | PV1J-NSOPTmut | | |
|---|---|---|---|
| Pep pools | 21G | 99C161 | 99C166 |
| F (NS3p) | 8 | 10 | 170 |
| G (NS3h) | 7 | 592 | 229 |
| H (NS4) | 3 | 14 | 16 |
| I(NS5a) | 5 | 71 | 36 |
| L (NS5b) | 14 | 23 | 11 |
| M (NS5b) | 3 | 35 | 8 |
| DMSO | 2 | 4 | 5 |

| | | | |
|---|---|---|---|
| INFγELISPOT on PBMC from Rhesus monkeys immunized with two injections of 5 mg DNA/dose in OPTIVAX/BAK of plasmid pV1Jns-NSOPTmut. Data are expressed as SFC7 106 PBMC. | | | |

### Example 5: Construction of Ad6 Pre-Adenovirus Plasmids

Ad6 pre-adenovirus plasmids were obtained as follows:

### Construction of pAd6 E1-E3+ Pre-adenovirus Plasmid

An Ad6 based pre-adenovirus plasmid which can be used to generate first generation Ad6 vectors was constructed either taking advantage of the extensive sequence identity (approx. 98%) between Ad5 and Ad6 or containing only Ad6 regions. Homologous recombination was used to clone wtAd6 sequences into a bacterial plasmid.

A general strategy used to recover pAd6E1-E3+ as a bacterial plasmid containing Ad5 and Ad6 regions is illustrated in Figure 10. Cotransformation of BJ 5183 bacteria with purified wt Ad6 viral DNA and a second DNA fragment termed the Ad5 ITR cassette resulted in the circularization of the viral genome by homologous recombination. The ITR cassette contains sequences from the right (bp 33798 to 35935) and left (bp 1 to 341 and bp 3525 to 5767) end of the Ad5 genome separated by plasmid sequences containing a bacterial origin of replication and an ampicillin resistance gene. The ITR cassette contains a deletion of E1 sequences from Ad5 342 to 3524. The Ad5 sequences in the ITR cassette provide regions of homology with the purified Ad6 viral DNA in which recombination can occur.

Potential clones were screened by restriction analysis and one clone was selected as pAd6E1-E3+. This clone was then sequenced in it entirety. pAd6E1-E3+ contains Ad5 sequences from bp 1 to 341 and from bp 3525 to 5548, Ad6 bp 5542 to 33784, and Ad5 bp 33967 to 35935 (bp numbers refer to the wt sequence for both Ad5 and Ad6). pAd6E1-E3+ contains the coding sequences for all Ad6 virion structural proteins which constitute its serotype specificity.

A general strategy used to recover pAd6E1-E3+ as a bacterial plasmid containing Ad6 regions is illustrated in Figure 11. Cotransformation of BJ 5183 bacteria with purified wt Ad6 viral DNA and a second DNA fragment termed the Ad6 ITR cassette resulted in the circularization of the viral genome by homologous recombination. The ITR cassette contains sequences from the right (bp 35460 to 35759) and left (bp 1 to 450 and bp 3508 to 3807) end of the Ad6 genome separated by plasmid sequences containing a bacterial origin of replication and an ampicillin resistance gene. These three segments were generated by PCR and cloned sequentially into pNEB193, generating pNEBAd6-3 (the ITR cassette). The ITR cassette contains a deletion of E1 sequences from Ad5 451 to 3507. The Ad6 sequences in the ITR cassette provide regions of homology with the purified Ad6 viral DNA in which recombination can occur.

### Construction of pAd6 E1-E3- pre-adenovirus plasmids

Ad6 based vectors containing A5 regions and deleted in the E3 region were constructed starting with pAd6E1-E3+ containing Ad5 regions. A 5322 bp subfragment of pAd6E1-E3+ containing the E3 region (Ad6 bp 25871 to 31192) was subcloned into pABS.3 generating pABSAd6E3. Three E3 deletions were then made in this plasmid generating three new plasmids pABSAd6E3(1.8Kb) (deleted for Ad6 bp 28602 to 30440), pABSAd6E3(2.3Kb) (deleted for Ad6 bp 28157 to 30437) and pABSAd6E3(2.6Kb) (deleted for Ad6 bp 28157 to 30788). Bacterial recombination was then used to substitute the three E3 deletions back into pAd6E1-E3+ generating the Ad6 genome plasmids pAd6E1-E3-1.8Kb, pAd6E1-E3-2.3Kb and pAd6E1-E3-2.6Kb.

### Example 6: Generation of Ad5 Genome Plasmid with the NS Sequence

A pcDNA3 plasmid (Invitrogen) containing the coding region NS3-NS4A-NS4B-NS5A was digested with *Xmn*I and *Nru*I restriction sites and the DNA fragment containing the CMV promoter, the NS3-NS4A-NS4B-NS5A coding sequence and the Bovine Growth Hormone (BGH) polyadenylation signal was cloned into the unique *Ecor*V restriction site of the shuttle vector pDelE1Spa, generating the Sva3-5A vector.

A pcDNA3 plasmid containing the coding region NS3-NS4A-NS4B-NS5A-NS5B was digested with *Xmn*I and *Ecor*I (partial digestion), and the DNA fragment containing part of NS5A, NS5B gene and the BGH polyadenylation signal was cloned into the Sva3-5A vector, digested *Ecor*I and *Bgl*II blunted with Klenow, generating the Sva3-5B vector.

The Sva3-5B vector was finally digested *Ssp*I and *Bst*1107I restriction sites and the DNA fragment containing the expression cassette (CMV promoter, NS3-NS4A-NS4B-NS5A-NS5B coding sequence and the BGH polyadenylation signal) flanked by adenovirus sequences was co-transformed with pAd5HVO (E1-,E3-) ClaI linearized genome plasmid into the bacterial strain BJ5183, to generate pAd5HVONS. pAd5HVO contains Ad5 bp 1 to 341, bp 3525 to 28133 and bp 30818 to 35935.

### Example 7: Generation of Adenovirus Genome Plasmids with the NSmut Sequence

Adenovirus genome plasmids containing an NS-mut sequence were generated in an Ad5 or Ad6 background. The Ad6 background contained Ad5 regions at bases 1 to 450, 3511 to 5548 and 33967 to 35935.

pV1JNS3-5Akozak was digested with *Bgl*II and *Xba*I restriction enzymes and the DNA fragment containing the Kozak sequence and the sequence coding NS3-NS4A-NS4B-NS5A was cloned into a *Bgl*II and XbaI digested polypMRKpdelE1 shuttle vector. The resulting vector was designated shNS3-5Akozak.

PolypMRKpdelE1 is a derivative of RKpdelE1(Pac/pIX/pack450) + CMVmin+BGHpA(str.) modified by the insertion of a polylinker containing recognition sites for BglII, PmeI, SwaI, XbaI, SalI, into the unique BglII restriction site present downstream the CMV promoter. MRKpdelE1(Pac/pIX/pack450) + CMVmin + BGHpA(str.) contains Ad5 sequences from bp 1 to 5792 with a deletion of E1 sequences from bp 451 to 3510. The human CMV promoter and BGH polyadenylation signal were inserted into the E1 deletion in an E1 parallel orientation with a unique BglII site separating them.

The NS5B fragment, mutated to abrogate enzymatic activity and with a strong translation termination at the 3' end, was obtained by assembly PCR and inserted into the shNS3-5Akozak vector via homologous recombination, generating polypMRKpdelE1NSmut. In polypMRKpdelE1NSmut the NS-mut coding sequence is under the control of CMV promoter and the BGH polyadenylation signal is present downstream.

The gene expression cassette and the flanking regions which contain adenovirus sequences allowing homologous recombination were excised by digestion with *Pac*I and *Bst*1107I restriction enzymes and co-transformed with either pAd5HVO (E1-,E3-) or pAd6E1-E3-2.6Kb *Cla*I linearized genome plasmids into the bacterial strain BJ5183, to generate pAd5HVONSmut and pAd6E1-,E3-NSmut, respectively.

pAd6E1-E3-2.6Kb contains Ad5 bp 1 to 341 and from bp 3525 to 5548, Ad6 bp 5542 to 28157 and from bp 30788 to 33784, and Ad5 bp 33967 to 35935 (bp numbers refer to the wt sequence for both Ad5 and Ad6). In both plasmids the viral ITR's are joined by plasmid sequences that contain the bacterial origin of replication and an ampicillin resistance gene.

### Example 8: Generation of Adenovirus Genome Plasmids with the NSOPTmut

The human codon-optimized synthetic gene (NSOPTmut) provided by SEQ. ID. NO. 3 cloned into a pCRBlunt vector (Invitrogen) was digested with *BamH*1 and *Sal*I restriction enzymes and cloned into *Bgl*I*I* and *Sal*I restriction sites present in the shuttle vector polypMRKpdelE1. The resulting clone (polypMRKpdelE1NSOPTmut) was digested with *Pac*I and *Bst*1107I restriction enzymes and co-transformed with either pAd5HVO (E1-,E3-) or pAd6E1-E3-2.6Kb *Cla*I linearized genome plasmids, into the bacterial strain BJ5183, to generate pAd5HVONSOPTmut and pAd6E1-,E3-NSOPTmut, respectively.

### Example 9: Rescue and Amplification of Adenovirus Vectors

Adenovectors were rescued in Per.6 cells. Per.C6 were grown in 10% FCS / DMEM supplemented by L-glutamine (final 4mM), penicillin/streptomycin (final 100 IU/ml) and 10 mM MgCl₂. After infection, cells were kept in the same medium supplemented by 5% horse serum (HS). For viral rescue, 2.5 X 10⁶ Per.C6 were plated in 6 cm ø Petri dishes.

Twenty-four hours after plating, cells were transfected by calcium phosphate method with 10 µg of the *Pac I* linearized adenoviral DNA. The DNA precipitate was left on the cells for 4 hours. The medium was removed and 5% HS/DMEM was added.

Cells were kept in a CO₂ incubator until a cytopathic effect was visible (1 week). Cells and supernatant were recovered and subjected to 3X freeze/thawing cycles (liquid nitrogen / water bath at 37°C). The lysate was centrifuged at 3000 rpm at - 4°C for 20 minutes and the recovered supernatant (corresponding to a cell lysate containing virus passed on cells only once; P1) was used, in the amount of 1 ml/ dish, to infect 80-90% confluent Per.C6 in 10 cm ø Petri dishes. The infected cells were incubated until a cytopathic effect was visible, cells and supernatant recovered and the lysate prepared as described above (P2).

P2 lysate (4 ml) were used to infect 2 X 15 cm ø Petri dishes. The lysate recovered from this infection (P3) was kept in aliquots at -80°C as a stock of virus to be used as starting point for big viral preparations. In this case, 1 ml of the stock was enough to infect 2 X 15 cm ø Petri dishes and resulting lysate (P4) was used for the infection of the Petri dishes devoted to the large scale infection.

Further amplification was obtained from the P4 lysate which was diluted in medium without FCS and used to infect 30 X 15 cm ø Petri dishes (with Per.C6 80%-90% confluent) in the amount of 10 ml/dish. Cells were incubated 1 hour in the CO₂ incubator, mixing gently every 20 minutes. 12 ml / dish of 5% HS / DMEM was added and cells were incubated until a cytopathic effect was visible (about 48 hours).

Cells and supernatant were collected and centrifuged at 2K rpm for 20 minutes at 4°C. The pellet was resuspended in 15 ml of 0.1 M Tris pH=8.0. Cells were lysed by 3X freeze/thawing cycles (liquid nitrogen / water bath at 37°C). 150 µl of 2 M MgCl₂ and 75 µl of DNAse (10 mg of bovine pancreatic deoxyribonuclease I in 10 ml of 20 mM Tris-HCl pH= 7.4, 50 mM NaCl, 1 mM dithiothreitol, 0.1 mg/ml bovine serum albumin, 50% glycerol) were added. After a 1 hour incubation at 37°C in a water bath (vortex every 15 minutes) the lysate was centrifuged at 4K rpm for 15 minutes at 4°C. The recovered supernatant was ready to be applied on CsCl gradient.

The CsCl gradients were prepared in SW40 ultra-clear tubes as follows:
0.5 ml of 1.5d CsCl
3 ml of 1.35d CsCl
3 ml of 1.25d CsCl
5-ml/ tube of viral supernatant was applied.

If necessary, the tubes were topped up with 0.1 M tris-Cl pH=8.0. Tubes were centrifuged at 35K rpm for 1 hour at -10°C with rotor SW40. The viral bands (located at the 1.25/1.35 interface) were collected using a syringe.

The virus was transferred into a new SW40 ultraclear tube and 1.35d CsCl was added to top the tube up. After centrifugation at 35K rpm for 24 hours at 10°C in the rotor SW40, the virus was collected in the smallest possible volume and dialyzed extensively against buffer A105 (5 mM Tris, 5% sucrose, 75 mM NaCl, 1 mM MgCl₂, 0.005% polysorbate 80 pH=8.0). After dialysis, glycerol was added to final 10% and the virus was stored in aliquots at - 80°C.

### Example 10: Enhanced Adenovector Rescue

First generation Ad5 and Ad6 vectors carrying HCV NSOPTmut transgene were found to be difficult to rescue. A possible block in the rescue process might be attributed to an inefficient replication of plasmid DNA that is a sub-optimal template for the replication machinery of adenovirus. The absence of the terminal protein linked to the 5'ends of the DNA (normally present in the viral DNA), associated with the very high G-C content of the transgene inserted in the E1 region of the vector, may be causing a substantial reduction in replication rate of the plasmid-derived adenovirus.

To set up a more efficient and reproducible procedure for rescuing Ad vectors, an expression vector (pE2; Figure 19) containing all E2 proteins (polymerase, pre-terminal protein and DNA binding protein) as well as E4 orf6 under the control of tet-inducible promoter was employed. The transfection of pE2 in combination with a normal preadeno plasmid in PerC6 and in 293 leads to a strong increase of Ad DNA replication and to a more efficient production of complete infectious adenovirus particles.

### Plasmid Constriction

pE2 is based on the cloning vector pBI (CLONTECH) with the addition of two elements to allow episomal replication and selection in cell culture: (1) the EBV-OriP (EBV [nt] 7421-8042) region permitting plasmid replication in synchrony with the cell cycle when EBNA-1 is expressed and (2) the hygromycin-B phosphotransferase (HPH)-resistance gene allowing a positive selection of transformed cells. The two transcriptional units for the adenoviral genes E2 a and b and E4-Orf6 were constructed and assembled in pE2 as described below.

The Ad5-Polymerase *ClaI*/*SphI* fragment and the Ad5-pTP *Acc65*/*EcoRV* fragment were obtained from pVac-Pol and pVac-pTP (Stunnemberg et al. NAR 16:2431-2444, 1988). Both fragments were filled with Klenow and cloned into the *SalI* (filled) and *EcoRV* sites of pBI, respectively obtaining pBI-Pol/pTP.

EBV-OriP element from pCEP4 (Invitrogen) was first inserted within two chicken β-globin insulator dimers by cloning it into *BamHI* site of pJC13-1 (Chung et al., Cell 74(3):505-14, 1993). HS4-OriP fragment from pJC13-OriP was then cloned inside pSA1mv (a plasmid containing tk-Hygro-B resistance gene expression cassette as well as Ad5 replication origin), the ITR's arranged as head-to-tail junction, obtained by PCR from pFG140 (Graham, EMBO J. 3:2917-2922, 1984) using the following primers: 5'-TCGAATCGATACGCGAACCTACGC-3' (SEQ. ID. NO. 16) and 5'-TCGACGTGTCGACTTCGAAGCGCACACCAAAAACGTC-3' (SEQ. ID. NO. 17), thus generating pMVHS4Orip. A DNA fragment from pMVHS4Orip, containing the insulated OriP, Ad5 ITR junction and tk-HygroB cassette, was then inserted into pBI-Pol/pTP vector restricted *AseI*/*AatII* generating pBI-Pol/pTPHS4.

To construct the second transcriptional unit expressing Ad5-Orf6 as well as Ad5-DBP, E4orf6 (Ad 5 [nt] 33193-34077) obtained by PCR was first inserted into pBI vector, generating pBI-Orf6. Subsequently, DBP coding DNA sequence (Ad 5 [nt] 22443-24032) was inserted into pBI-Orf6 obtaining the second bi-directional Tet-regulated expression vector (pBI-DBP/E4orf6). The original polyA signals present in pBI were substituted with BGH and SV40 polyA.

pBI-DBP/E4orf6 was then modified by inserting a DNA fragment containing the Adeno5-ITRs arranged in head-to-tail junction plus the hygromicin B resistance gene obtained from plasmid pSA-lmv. The new plasmid pBI-DBP/E4orf6shuttle was then used as donor plasmid to insert the second tet-regulated transcriptional unit into pBI-Pol/pTPHS4 by homologous recombination using *E. coli* strain BJ5183 obtaining pE2.

### Cell lines, Transfections and Virus Amplification

PerC6 cells were cultured in Dulbecco's modified Eagle's Medium (DMEM) plus 10% fetal bovine serum (FBS), 10 mM MgCl₂, penicillin (100 U/ml), streptomycin (100 µg/ml) and 2 mM glutamine.

All transient transfections were performed using Lipofectamine2000 (Invitrogen) as described by the manufacturer. 90% confluent PERC.6™ planted in 6-cm plates were transfected with 3.5 µg of Ad5/6NSOPTmut pre-adeno plasmids, digested with PacI, alone or in combination with 5 µg pE2 plus 1 µg pUHD52.1. pUHD52.1 is the expression vector for the reverse tet transactivator 2 (rtTA2) (Urlinger et al., Proc. Natl. Acad. Sci. U.S.A. 97(14):7963-7968, 2000). Upon transfection, cells were cultivated in the presence of 1 µg/ml of doxycycline to activate pE2 expression. 7 days post-transfection cells were harvested and cell lysate was obtained by three cycles of freeze-thaw. Two ml of cell lysate were used to infect a second 6-cm dish of PerC6. Infected cells were cultivated until a full CPE was observed then harvested. The virus was serially passaged five times as described above, then purified on CsCl gradient. The DNA structure of the purified virus was controlled by endonuclease digestion and agarose gel electrophoresis analysis and compared to the original pre-adeno plasmid restriction pattern.

### Example 11: Partial Optimizeation of HCV Polyprotein Encoding Nucleic acid

Partial optimization of HCV polyprotein encoding nucleic acid was performed to facilitate the production of adenovectors containing codons optimized for expression in a human host. The overall objective was to provide for increased expression due to codon optimization, while facilitating the production of an adenovector encoding HCV polyprotein.

Several difficulties were encountered in producing an adenovector encoding HCV polyprotein with codons optimized for expression in a human host. An adenovector containing an optimized sequence (SEQ. ID. NO. 3) was found to be more difficult to synthesize and rescue than an adenovector containing a non-optimized sequence (SEQ. ID. NO. 2).

The difficulties in producing an adenovector containing SEQ. ID. NO. 3 were attributed to a high GC content. A particularly problemetic region was the region at about position 3900 ofNSOPTmut (SEQ. ID. NO. 3).

Alternative versions of optimized HCV encoding nucleic acid sequence were designed to facilitate its use in an adenovector. The alternative versions, compared to NSOPTmut, were designed to have a lower overall GC content, to reduce/avoid the presence of potentially problematic motifis of consecutive G's or C's, while maintaining a high level of codon optimization to allow improved expression of the encoded polyprotein and the individual cleavage products.

A starting point for the generation of a suboptimally codon-optimized sequence is the coding region of the NSOPTmut nucleotide sequence (bases 7 to 5961 of SEQ. ID. NO. 3). Values for codon usage frequencies (normalized to a total of 1.0 for each amino acid) were taken from the file human_high.cod available in the Wisconsin Package Version 10.3 (Accelrys Inc., a wholly owned subsidiary of Pharmacopeia, Inc). To reduce the local and overall GC content a table defining preferred codon substitutions for each amino acid was manually generated. For each amino acid the codon having 1) a lower GC content as compared to the most frequent codon and 2) a relativly high observed codon usage frequency (as defined in human_high.cod) was choosen as the replacement codon. For example for Arg the codon with the highest frequency is CGC. Out of the other five alternative codons encoding Arg (CGG, AGG, AGA, CGT, CGA) three (AGG, CGT, CGA) reduce the GC content by 1 base, one (AGA) by two bases and one (CGG) by 0 bases. Since the AGA codon is listed in human_high.cod as having a relatively low usage frequency (0.1), the codon substituting CGC was therefore choosen to be AGG with a relative frequency of 0.18. Similar criteria were applied in order to establish codon replacements for the other amino acids resulting in the list shown in Table 5. Parameters applied in the following optimization procedure were determined empirically such that the resulting sequence maintained a considerably improved codon usage (for each amino acid) and the GC content (overall and in form of local stretches of consecutive G's and/or C's) was decreased. Two examples of partial optimized HCV encoding sequences are provided by SEQ. ID. NO. 10 and SEQ. ID. NO. 11. SEQ. ID. NO. 10 provides a HCV encoding sequence that is partially optimized throughout. SEQ. ID. NO. 11 provides an HCV encoding sequence fully optimized for codon usage with the exception of a region that was partially optimized.

Codon optimization was performed using the following procedure:
Step 1) The coding region of the input fully optimized NSOPTmut sequence was analyzed using a sliding window of 3 codons (9 bases) shifting the window by one codon after each cycle. Whenever a stretch containing 5 or more consecutive C's and/or G's was detected in the window the following replacement rule was applied: Let N indicate the number of codon replacements previously performed. If N is odd replace the middle codon in the window with the codon specified in Table 5, if N is even replace the third terminal codon in the window with the codon specified in a codon optimization table such as human_high.cod. If Leu or Val is present at the second or third codon do not apply any replacement in order not to introduce Leu or Val codons with very low relative codon usage frequency (see, for example, human_high.cod). In the following cycle analysis of the shifted window was then applied to a sequence containing the replacements of the previous cycle.
   The alternating replacement of the middle and terminal codon in the 3 codon window was found empirically to give a more satisfying overall maintenance of optimized codon usage while also reducing GC content (as judged from the final sequence after the procedure). In general, however, the precise replacement strategy depends on the amino acid sequence encoded by the nucelotide sequence under analysis and will have to be determined empirically.
Step 2) The sequence containing all the codon replacements performed during step 1) was then subjected to an additional analysis using a sliding window of 21 codons (63 bases) in length: according to an adjustable parameter the overall GC content in the window was determined. If the GC content in the window was higher than 70% the following codon replacement strategy was applied: In the window replace the codons for the amino acids Asn, Asp, Cys, Glu, His, Ile, Lys, Phe, Tyr by the codons given in Table 5. Restriction of the replacement to this set of amino acids was motivated by the fact that a) the replacement codon still has an accetably high frequency of usage in human_high.cod and b) the average overall human codon usage in CUTG for the replacement codon is nearly as high as the most frequent codon. In the following cycle analysis of the shifted window is then applied to a sequence containing the replacements of the previous cycle.
   The threshold 70% was determined empirically by compromising between an overall reduction in GC content and maintenance of a high codon optimization for the individual amino acids. As in step 1) the precise replacement strategy (choice of amino acids and GC content threshold value) will again depend on the amino acid sequence encoded by the nucleotide sequence under analysis and will have to be determined empirically.
Step 3) The sequence generated by steps 1) and 2) was then manually edited and additional codons were changed according to the following criteria: Regions still having a GC content higher than 70% over a window of 21 codons were examined manually and a few codons were replaced again following the scheme given in Table 5.
   Subsequent steps were performed to provide for useful restriction sites, remove possible open reading frames on the complementary strand, to add homologous recombinant regions, to add a Kozac signal, and to add a terminator. These steps are numbered 4-7
Step 4) The sequence generated in step 3 was examined for the absence of certain restriction sites (BglII, PmeI and XbaI) and presence of only 1 StuI site to allow a subsequent cloning strategy using a subset of restriction enzymes. Two sites (one for BglII and one for StuI) were removed from the sequence by replacing codons that were part of the respective recognition sites.
Step 5) The sequence generated by steps 1) through 4) was then modified according to allow subsequent generation of a modified NSOPTmut sequence (by homologous recombination). In the sequence obtained from steps 1) through 4) the segment comprising base 3556 to 3755 and the segment comprising base 4456 to 4656 were replaced by the corresponding segments from NSOPTmut. The segment comprising bases 3556 to 4656 of SEQ. ID. NO. 10 can be used to replace the problematic region in NSOPTmut (around position 3900) by homologous recombination thus creating the variant of NSOPTmut having the sequence of SEQ. ID. NO. 11.
Step 6) Analysis of the sequence generated through steps 1) to 5) revealed a potential open reading frame spanning nearly the complete fragment on the complementary strand. Removal of all codons CTA and TTA (Leu) and TCA (Ser) from the sense strand effectively removed all stop codons in one of the reading frames on the complementary strand. Although the likelyhood for transcription of this complementary strand open reading frame and subsequent translation into protein is very small, in order to exclude a potential interference with the transcription and subsequent translation of the sequence encoded on the sense strand, TCA codons for Ser were introduced on the sense approximately every 500 bases. No changes were introduced in the segments introduced during step 5) to allow homologous recombination. The TCA codon for Ser was preferred over the CTA and TTA codons for Leu because of the higher relative frequency for TCA (0.05) as compared to CTA (0.02) and TTA (0.03) in human_high.cod. In addition, the average human codon usage from CUTG favored TCA (0.14 against 0.07 for CTA and TTA).
Step 7) In a final step GCCACC was added at the 5' end of the sequence to generate an optimized internal ribosome entry site (Kozak signal) and a TAAA stop sgnal was added at the 3'. To maintain the initiation of translation properties of NSsuboptmut the first 8 codons of the coding region were kept identical to the NSOPTmut sequence. The resulting sequence was again checked for the absence of BgIII, PmeI and XbaI recognition sites and the presence of only 1 StuI site.

The NSsuboptmut sequence (SEQ. ID. NO. 10) has an overall reduced GC content (63.5%) as compared to NSOPTmut (70.3%) and maintains a well optimized level of codon usage optimization. Nucleotide sequence identity of NSsuboptmut is 77.2% with respect to NSmut.

**Table 5: Definition of codon replacements performed during steps 1) and 2).**

| Amino Acid | Most frequent codon | Relative frequency | Reduction in GC content (bases) | Replacement codon | Relative frequency |
|---|---|---|---|---|---|
| Amino Acids where the replacement codon reduces the codon GC-content by 1 base | | | | | |
| Ala | GCC | 0.51 | 1 | GCT | 0.17 |
| Arg | CGC | 0.37 | 1 | AGG | 0.18 |
| Asn | AAC | 0.78 | 1 | AAT | 0.22 |
| Asp | GAC | 0.75 | 1 | GAT | 0.25 |
| Cys | TGC | 0.68 | 1 | TGT | 0.32 |
| Glu | GAG | 0.75 | 1 | GAA | 0.25 |
| Gln | CAG | 0.88 | 1 | CAA | 0.12 |
| Gly | GGC | 0.50 | 1 | GGA | 0.14 |
| His | CAC | 0.79 | 1 | CAT | 0.21 |
| Ile | ATC | 0.77 | 1 | ATT | 0.18 |
| Lys | AAG | 0.82 | 1 | AAA | 0.18 |
| Phe | TTC | 0.80 | 1 | TTT | 0.20 |
| Pro | CCC | 0.48 | 1 | CCT | 0.19 |
| Ser | AGC | 0.34 | 1 | TCT | 0.13 |
| Thr | ACC | 0.51 | 1 | ACA | 0.14 |
| Tyr | TAC | 0.74 | 1 | TAT | 0.26 |

| Amino Acids with no alternative codon | | | | | |
|---|---|---|---|---|---|
| Met | ATG | 1.00 | 0 | ATG | 1.00 |
| Trp | TGG | 1.00 | 0 | TGG | 1.00 |

| Amino Acids where the replacement codon has a very low relative frequency. These amino acids were excluded from the replacement procedure | | | | | |
|---|---|---|---|---|---|
| Leu | CTG | 0.58 | 1 | TTG | 0.06 |
| Val | GTG | 0.64 | 1 | GTT | 0.07 |

### Example 12: Virus Characterization

Adenovectors were characterized by: (a) measuring the physical particles/ml; (b) running a TaqMan PCR assay; and (c) checking protein expression after infection of HeLa cells.

### a) Physical Particles Determination

CsCl purified virus was diluted 1/10 and 1/100 in 0.1% SDS PBS. As a control, buffer A105 was used. These dilutions were incubated 10 minutes at 55°C. After spinning the tubes briefly, O.D. at 260 nm was measured. The amount of viral particles was calculated as follows: 1 OD 260 nm = 1.1 X 10¹² physical particles/ml. The results were typically between 5 X 10¹¹ and 1 X 10¹² physical particles /ml.

### b) TaqMan PCR Assay

TaqMan PCR assay was used for adenovectors genome quantification (Q-PCR particles/ml). TaqMan PCR assay was performed using the ABI Prism 7700-sequence detector. The reaction was performed in a final 50 µl volume in the presence of oligonucleotides (at final 200 nM) and probe (at final 200 µM) specific for the adenoviral backbone. The virus was diluted 1/10 in 0.1% SDS PBS and incubated 10 minutes at 55°C. After spinning the tube briefly, serial 1/10 dilutions (in water) were prepared. 10 µl the 10⁻³, 10⁻⁵ and 10⁻⁷ dilutions were used as templates in the PCR assay.

The amount of particles present in each sample was calculated on the basis of a standard curve run in the same experiment. Typically results were between 1 X 10¹² and 3 X 10¹² Q-PCR particles /ml.

### c) Expression of HCV Non-Structural Proteins

Expression of HCV NS proteins was tested by infection of HeLa cells. Cells were plated the day before the infection at 1.5 X 10⁶ cells/dish (10 cm ø Petri dishes). Different amounts of CsCl purified virus corresponding to m.o.i. of 50, 250 and 1250 pp/cell were diluted in medium (FCS free) up to a final volume of 5 ml. The diluted virus was added on the cells and incubated for 1 hour at 37°C in a CO₂ incubator (gently mixing every 20 minutes). 5 ml of 5% HS-DMEM was added and the cells were incubated at 37°C for 48 hours.

Cell extracts were prepared in 1% Triton/TEN buffer. The extracts were run on 10% SDS-acrylamide gel, blotted on nitrocellulose and assayed with antibodies directed against NS3, NS5a and NS5b in order to check the correct polyprotein cleavage. Mock-infected cells were used as a negative control. Results from representative experiments testing the Ad5-NS, MRKAd5-NSmut, MRKAd6-NSmut and MRKAd6-NSOPTmut are shown in Figure 14.

### Example 13: Mice Immunization with Adenovectors Encoding Different NS Cassettes

The adenovectors Ad5-NS, MRKAd5-NSmut, MRKAd6-NSmut and MRKAd6-NSOPTmut were injected in C57Black6 mice strains to evaluate their potential to elicit anti-HCV immune responses. Groups of animals (N=9-10) were injected intramuscularly with 109 pp of CsCl purified virus. Each animal received two doses at three weeks interval.

Humoral immune response against the NS3 protein was measured in post dose two sera from C57Black6 immunized mice by ELISA on bacterially expressed NS3 protease domain. Antibodies specific for the tested antigen were detected with geometric mean titers (GMT) ranging from 100 to 46000 (Tables 6, 7, 8 and 9).

**Table 6: Ad5-NS**

| | | | | | | | | | | | GMT |
|---|---|---|---|---|---|---|---|---|---|---|---|
| Mice n. | 1 | 2 | 3 | 4 | 5 | 6 | 7 | 8 | 9 | 10 | |
| Titer | 50 | 253 | 50 | 50 | 50 | 2257 | 504 | 50 | 50 | 50 | 108 |

**Table 7: Ad5-NSmut**

| | | | | | | | | | | | GMT |
|---|---|---|---|---|---|---|---|---|---|---|---|
| Mice n. | 11 | 12 | 13 | 14 | 15 | 16 | 17 | 18 | 19 | 20 | |
| Titer | 3162 | 78850 | 87241 | 6796 | 12134 | 3340 | 18473 | 13093 | 76167 | 49593 | 23645 |

**Table 8: MRKAd6-NSmut**

| | | | | | | | | | | | GMT |
|---|---|---|---|---|---|---|---|---|---|---|---|
| Mice n. | 21 | 22 | 23 | 24 | 25 | 26 | 27 | 28 | 29 | 30 | |
| Titer | 125626 | 39751 | 40187 | 65834 | 60619 | 69933 | 21555 | 49348 | 29290 | 26859 | 46461 |

**Table 9: MRKAd6-NSOPTmut**

| | | | | | | | | GMT |
|---|---|---|---|---|---|---|---|---|
| Mice n. | 31 | 32 | 33 | 34 | 35 | 36 | 37 | |
| Titer | 25430 | 3657 | 893 | 175 | 10442 | 49540 | 173 | 2785 |

T cell response in C57Black6 mice was analyzed by the quantitative ELISPOT assay measuring the number of IFNγ secreting T cells in response to five pools (named from F to L+M) of 20mer peptides overlapping by ten residues encompassing the NS3-NS5B sequence. Specific CD8+ response induced in C57Black6 mice was analyzed by the same assay using a 20mer peptide encompassing a CD8+ epitope for C57Black6 mice (pep1480). Cells secreting IFNγ in an antigen specific-manner were detected using a standard ELIspot assay.

Spleen cells, splenocytes and peptides were produced and treated as described in Example 3, *supra.* Representative data from groups of C57Black6 mice (N=9-10) immunized with two injections of 10⁹ viral particles of vectors Ad5-NS, MRKAd5-NSmut and MRKAd6-NSmut are shown in Figure 15.

### Example 14: Immunization of Rhesus macaques with Adenovectors

Rhesus macaques (N=3-4) were immunized by intramuscular injection of CsCl purified Ad5-NS, MRKAd5-NSmut, MRKAd6-NSmut or MRKAd6-NSOPTmut virus. Each animal received two doses of 10¹¹ or 10¹⁰ vp in the deltoid muscle at 0, and 4 weeks.

CMI was measured at different time points by a) IFN-γ ELISPOT (see Example 3, *supra*), b) IFN-γ ICS and c) bulk CTL assays. These assays measure HCV antigen-specific CD8+ and CD4+ T lymphocyte responses, and can be used for a variety of mammals, such as humans, rhesus monkeys, mice, and rats.

The use of a specific peptide or a pool of peptides can simplify antigen presentation in CTL cytotoxicity assays, interferon-gamma ELISPOT assays and interferon-gamma intracellular staining assays. Peptides based on the amino acid sequence of various HCV proteins (core, E2, NS3, NS4A, NS4B, NS5a, NS5b) were prepared for use in these assays to measure immune responses in HCV DNA and adenovirus vector vaccinated rhesus monkeys, as well as in HCV-infected humans. The individual peptides are overlapping 20-mers, offset by 10 amino acids. Large pools of peptides can be used to detect an overall response to HCV proteins while smaller pools and individual peptides may be used to define the epitope specificity of a response.

### IFN-γ ICS

For IFN-γ ICS, 2 x 106 PBMC in 1 ml R10 (RPMI medium, supplemented with 10% FCS) were stimulated with peptide pool antigens. Final concentration of each peptide was 2 µg/ml. Cells were incubated for 1 hour in a CO₂ incubator at 37°C and then Brefeldin A was added to a final concentration of 10 µg /ml to inhibit the secretion of soluble cytokines. Cells were incubated for additional 14-16 hours at 37°C.

Stimulation was done in the presence of co-stimulatory antibodies: CD28 and CD49d (anti-humanCD28 BD340975 and anti-humanCD49d BD340976). After incubation, cells were stained with fluorochrome-conjugated antibodies for surface antigens: anti-CD3, anti-CD4, anti-CD8 (CD3-APC Biosource APS0301, CD4-PE BD345769, CD8-PerCP BD345774).

To detect intracellular cytokines, cells were treated with FACS permeabilization buffer 2 (BD340973), 2x final concentration. Once fixed and permeabilized, cells were incubated with an antibody against human IFN-γ, IFN-γFITC (Biosource AHC4338).

Cells were resuspended in 1% formaldehyde in PBS and analyzed at FACS within 24 hours. Four color FACS analysis was performed on a FACSCalibur instrument (Becton Dickinson) equipped with two lasers. Acquisition was done gating on the lymphocyte population in the Forward versus Side Scatter plot coupled with the CD3, CD8 positive populations. At least 30,000 events of the gate were taken. The positive cells are expressed as number of IFN-γ expressing cells over 10⁶ lymphocytes.

IFN-γ ELISPOT and IFN-γ ICS data from immunized monkeys after one or two injections of 10¹⁰ or 10¹¹ vp of the different adenovectors are reported in Figures 16A-16D, 17A, and 17B.

### Bulk CTL Assays

A distinguishing effector function of T lymphocytes is the ability of subsets of this cell population to directly lyse cells exhibiting appropriate MHC-associated antigenic peptides. This cytotoxic activity is most often associated with CD8+ T lymphocytes.

PBMC samples were infected with recombinant vaccine viruses expressing HCV antigens *in vitro* for approximately 14 days to provide antigen restimulation and expansion of memory T cells. Cytotoxicity against autologous B cell lines treated with peptide antigen pools was tested.

The lytic function of the culture is measured as a percentage of specific lysis resulted from chromium released from target cells during 4 hours incubation with CTL effector cells. Specific cytotoxicity is measured and compared to irrelevant antigen or excipient-treated B cell lines. This assay is semi-quantitative and is the preferred means for determining whether CTL responses were elicited by the vaccine. Data after two injections from monkeys immunized with 10¹¹ vp/dose with adenovectors Ad5-NS, MRKAd5-NSmut and MRKAd6-NSmut are reported in Figures 18A-18F.

Other embodiments are within the following claims. While several embodiments have been shown and described, various modifications may be made without departing from the spirit and scope of the present invention.

## Claims

1. A recombinant nucleic acid comprising one or more Ad6 regions and a region not present in Ad6, wherein at least one Ad6 region is selected from the group consisting of: E1A, E1B, E2B, E2A, E4, L1, L2, L4, and L5.

2. The recombinant nucleic acid of claim 1, wherein said region not present in Ad6, is an expression cassette coding for a polypeptide not found in Ad6.

3. The recombinant nucleic acid of claim 2, wherein said recombinant nucleic acid is an adenovirus vector defective in at least E1 that is able to replicate when E1 is supplied *in trans.*

4. The recombinant nucleic acid of claim 3, wherein said vector consists of:
a) a first adenovirus region from about base pair 1 to about base pair 450 corresponding to either Ad5 or Ad6;
b) said gene expression cassette in an E1 parallel or E1 anti-parallel orientation joined to said first region;
c) a second adenovirus region from about base pair 3511 to about base pair 5548 corresponding to Ad5 or from about base pair 3508 to about base pair 5541 corresponding to Ad6, joined to said gene expression cassette;
d) a third adenovirus region from about base pair 5549 to about base pair 28133 corresponding to Ad5 or from about base pair 5542 to about base pair 28156 corresponding to Ad6, joined to said second region;
e) an optionally present fourth region from about base pair 28134 to about base pair 30817 corresponding to Ad5, or from about base pair 28157 to about 30789 corresponding to Ad6, joined to said third region;
f) a fifth adenovirus region from about base pair 30818 to about base pair 33966 corresponding to Ad5 or from about base pair 30789 to about base pair 33784 corresponding to Ad6, wherein said fifth region is joined to said fourth region if said fourth region is present, or said fifth is joined to said third region if said fourth region is not present; and
g) a sixth adenovirus region from about base pair 33967 to about base pair 35935 corresponding to Ad5 or from about base pair 33785 to about base pair 35759 corresponding to Ad6, joined to said fourth region;
provided that at least one of said second, third, and fifth regions is from Ad6.

5. The recombinant nucleic acid of claim 3, wherein said vector consists of:
a) a first adenovirus region from about base pair 1 to about base pair 450 corresponding to either Ad5 or Ad6;
b) a second adenovirus region from about base pair 3511 to about base pair 5548 corresponding to Ad5 or from about base pair 3508 to about base pair 5541 corresponding to Ad6, joined to said first region;
c) a third adenovirus region from about base pair 5549 to about base pair 28133 corresponding to Ad5 or from about base pair 5542 to about base pair 28156 corresponding to Ad6, joined to said second region;
d) said gene expression cassette in a E3 parallel or E3 anti-parallel orientation joined to said third region;
e) a fourth adenovirus region from about base pair 30818 to about base pair 33966 corresponding to Ad5 or from about base pair 30789 to about base pair 33784 corresponding to Ad6, joined to said gene expression cassette; and
f) a fifth adenovirus region from about base pair 33967 to about base pair 35935 corresponding to Ad5 or from about base pair 33785 to about base pair 35759 corresponding to Ad6, joined to said fourth region;
provided that at least one of said second, third, and fourth regions is from Ad6.


SEQUENCE LISTING

<110> Merck & Co. Inc.,
      Istituto di Ricerche di Biologia Molecolare P. Angeletti S.p.A.

<120> HEPATITIS C VIRUS VACCINE

<130> ITR0015Y PCT

<140> PCT/US02/32512
<141> 2002-10-10

<150> 60/363,774
<151> 2002-03-13

<150> 60/328,655
<151> 2001-10-11

<160> 17

<170> FastSEQ for Windows Version 4.0

<210> 1
<211> 1985
<212> PRT
<213> Artificial Sequence

<220>
<223> Met-NS3-NS4A-NS4B-NS5A-NS5B polypeptide

<400> 1
Met Ala Pro Ile Thr Ala Tyr Ser Gln Gln Thr Arg Gly Leu Leu Gly
 1               5                  10                  15
Cys Ile Ile Thr Ser Leu Thr Gly Arg Asp Lys Asn Gln Val Glu Gly
            20                  25                  30
Glu Val Gln Val Val Ser Thr Ala Thr Gln Ser Phe Leu Ala Thr Cys
        35                  40                  45
Val Asn Gly Val Cys Trp Thr Val Tyr His Gly Ala Gly Ser Lys Thr
    50                  55                  60
Leu Ala Gly Pro Lys Gly Pro Ile Thr Gln Met Tyr Thr Asn Val Asp
65                  70                  75                  80
Gln Asp Leu Val Gly Trp Gln Ala Pro Pro Gly Ala Arg Ser Leu Thr
                85                  90                  95
Pro Cys Thr Cys Gly Ser Ser Asp Leu Tyr Leu Val Thr Arg His Ala
            100                 105                 110
Asp Val Ile Pro Val Arg Arg Arg Gly Asp Ser Arg Gly Ser Leu Leu
        115                 120                 125
Ser Pro Arg Pro Val Ser Tyr Leu Lys Gly Ser Ser Gly Gly Pro Leu
    130                 135                 140
Leu Cys Pro Ser Gly His Ala Val Gly Ile Phe Arg Ala Ala Val Cys
145                 150                 155                 160
Thr Arg Gly Val Ala Lys Ala Val Asp Phe Val Pro Val Glu Ser Met
                165                 170                 175
Glu Thr Thr Met Arg Ser Pro Val Phe Thr Asp Asn Ser Ser Pro Pro
            180                 185                 190
Ala Val Pro Gln Ser Phe Gln Val Ala His Leu His Ala Pro Thr Gly
        195                 200                 205
Ser Gly Lys Ser Thr Lys Val Pro Ala Ala Tyr Ala Ala Gln Gly Tyr
    210                 215                 220
Lys Val Leu Val Leu Asn Pro Ser Val Ala Ala Thr Leu Gly Phe Gly
225                 230                 235                 240
Ala Tyr Met Ser Lys Ala His Gly Ile Asp Pro Asn Ile Arg Thr Gly
                245                 250                 255
Val Arg Thr Ile Thr Thr Gly Ala Pro Val Thr Tyr Ser Thr Tyr Gly
            260                 265                 270
Lys Phe Leu Ala Asp Gly Gly Cys Ser Gly Gly Ala Tyr Asp Ile Ile

        275                 280                 285
Ile Cys Asp Glu Cys His Ser Thr Asp Ser Thr Thr Ile Leu Gly Ile
    290                 295                 300
Gly Thr Val Leu Asp Gln Ala Glu Thr Ala Gly Ala Arg Leu Val Val
305                 310                 315                 320
Leu Ala Thr Ala Thr Pro Pro Gly Ser Val Thr Val Pro His Pro Asn
                325                 330                 335
Ile Glu Glu Val Ala Leu Ser Asn Thr Gly Glu Ile Pro Phe Tyr Gly
            340                 345                 350
Lys Ala Ile Pro Ile Glu Ala Ile Arg Gly Gly Arg His Leu Ile Phe
        355                 360                 365
Cys His Ser Lys Lys Lys Cys Asp Glu Leu Ala Ala Lys Leu Ser Gly
    370                 375                 380
Leu Gly Ile Asn Ala Val Ala Tyr Tyr Arg Gly Leu Asp Val Ser Val
385                 390                 395                 400
Ile Pro Thr Ile Gly Asp Val Val Val Val Ala Thr Asp Ala Leu Met
                405                 410                 415
Thr Gly Tyr Thr Gly Asp Phe Asp Ser Val Ile Asp Cys Asn Thr Cys
            420                 425                 430
Val Thr Gln Thr Val Asp Phe Ser Leu Asp Pro Thr Phe Thr Ile Glu
        435                 440                 445
Thr Thr Thr Val Pro Gln Asp Ala Val Ser Arg Ser Gln Arg Arg Gly
    450                 455                 460
Arg Thr Gly Arg Gly Arg Arg Gly Ile Tyr Arg Phe Val Thr Pro Gly
465                 470                 475                 480
Glu Arg Pro Ser Gly Met Phe Asp Ser Ser Val Leu Cys Glu Cys Tyr
                485                 490                 495
Asp Ala Gly Cys Ala Trp Tyr Glu Leu Thr Pro Ala Glu Thr Ser Val
            500                 505                 510
Arg Leu Arg Ala Tyr Leu Asn Thr Pro Gly Leu Pro Val Cys Gln Asp
        515                 520                 525
His Leu Glu Phe Trp Glu Ser Val Phe Thr Gly Leu Thr His Ile Asp
    530                 535                 540
Ala His Phe Leu Ser Gln Thr Lys Gln Ala Gly Asp Asn Phe Pro Tyr
545                 550                 555                 560
Leu Val Ala Tyr Gln Ala Thr Val Cys Ala Arg Ala Gln Ala Pro Pro
                565                 570                 575
Pro Ser Trp Asp Gln Met Trp Lys Cys Leu Ile Arg Leu Lys Pro Thr
            580                 585                 590
Leu His Gly Pro Thr Pro Leu Leu Tyr Arg Leu Gly Ala Val Gln Asn
        595                 600                 605
Glu Val Thr Leu Thr His Pro Ile Thr Lys Tyr Ile Met Ala Cys Met
    610                 615                 620
Ser Ala Asp Leu Glu Val Val Thr Ser Thr Trp Val Leu Val Gly Gly
625                 630                 635                 640
Val Leu Ala Ala Leu Ala Ala Tyr Cys Leu Thr Thr Gly Ser Val Val
                645                 650                 655
Ile Val Gly Arg Ile Ile Leu Ser Gly Arg Pro Ala Ile Val Pro Asp
            660                 665                 670
Arg Glu Phe Leu Tyr Gln Glu Phe Asp Glu Met Glu Glu Cys Ala Ser
        675                 680                 685
His Leu Pro Tyr Ile Glu Gln Gly Met Gln Leu Ala Glu Gln Phe Lys
    690                 695                 700
Gln Lys Ala Leu Gly Leu Leu Gln Thr Ala Thr Lys Gln Ala Glu Ala
705                 710                 715                 720
Ala Ala Pro Val Val Glu Ser Lys Trp Arg Ala Leu Glu Thr Phe Trp
                725                 730                 735
Ala Lys His Met Trp Asn Phe Ile Ser Gly Ile Gln Tyr Leu Ala Gly
            740                 745                 750
Leu Ser Thr Leu Pro Gly Asn Pro Ala Ile Ala Ser Leu Met Ala Phe
        755                 760                 765
Thr Ala Ser Ile Thr Ser Pro Leu Thr Thr Gln Ser Thr Leu Leu Phe
    770                 775                 780
Asn Ile Leu Gly Gly Trp Val Ala Ala Gln Leu Ala Pro Pro Ser Ala
785                 790                 795                 800
Ala Ser Ala Phe Val Gly Ala Gly Ile Ala Gly Ala Ala Val Gly Ser
                805                 810                 815
Ile Gly Leu Gly Lys Val Leu Val Asp Ile Leu Ala Gly Tyr Gly Ala

            820                 825                 830
Gly Val Ala Gly Ala Leu Val Ala Phe Lys Val Met Ser Gly Glu Met
        835                 840                 845
Pro Ser Thr Glu Asp Leu Val Asn Leu Leu Pro Ala Ile Leu Ser Pro
    850                 855                 860
Gly Ala Leu Val Val Gly Val Val Cys Ala Ala Ile Leu Arg Arg His
865                 870                 875                 880
Val Gly Pro Gly Glu Gly Ala Val Gln Trp Met Asn Arg Leu Ile Ala
                885                 890                 895
Phe Ala Ser Arg Gly Asn His Val Ser Pro Thr His Tyr Val Pro Glu
            900                 905                 910
Ser Asp Ala Ala Ala Arg Val Thr Gln Ile Leu Ser Ser Leu Thr Ile
        915                 920                 925
Thr Gln Leu Leu Lys Arg Leu His Gln Trp Ile Asn Glu Asp Cys Ser
    930                 935                 940
Thr Pro Cys Ser Gly Ser Trp Leu Arg Asp Val Trp Asp Trp Ile Cys
945                 950                 955                 960
Thr Val Leu Thr Asp Phe Lys Thr Trp Leu Gln Ser Lys Leu Leu Pro
                965                 970                 975
Gln Leu Pro Gly Val Pro Phe Phe Ser Cys Gln Arg Gly Tyr Lys Gly
            980                 985                 990
Val Trp Arg Gly Asp Gly Ile Met Gln Thr Thr Cys Pro Cys Gly Ala
        995                 1000                1005
Gln Ile Thr Gly His Val Lys Asn Gly Ser Met Arg Ile Val Gly Pro
    1010                1015                1020
Lys Thr Cys Ser Asn Thr Trp His Gly Thr Phe Pro Ile Asn Ala Tyr
1025                1030                1035                1040
Thr Thr Gly Pro Cys Thr Pro Ser Pro Ala Pro Asn Tyr Ser Arg Ala
                1045                1050                1055
Leu Trp Arg Val Ala Ala Glu Glu Tyr Val Glu Val Thr Arg Val Gly
            1060                1065                1070
Asp Phe His Tyr Val Thr Gly Met Thr Thr Asp Asn Val Lys Cys Pro
        1075                1080                1085
Cys Gln Val Pro Ala Pro Glu Phe Phe Thr Glu Val Asp Gly Val Arg
    1090                1095                1100
Leu His Arg Tyr Ala Pro Ala Cys Arg Pro Leu Leu Arg Glu Glu Val
1105                1110                1115                1120
Thr Phe Gln Val Gly Leu Asn Gln Tyr Leu Val Gly Ser Gln Leu Pro
                1125                1130                1135
Cys Glu Pro Glu Pro Asp Val Ala Val Leu Thr Ser Met Leu Thr Asp
            1140                1145                1150
Pro Ser His Ile Thr Ala Glu Thr Ala Lys Arg Arg Leu Ala Arg Gly
        1155                1160                1165
Ser Pro Pro Ser Leu Ala Ser Ser Ser Ala Ser Gln Leu Ser Ala Pro
    1170                1175                1180
Ser Leu Lys Ala Thr Cys Thr Thr His His Val Ser Pro Asp Ala Asp
1185                1190                1195                1200
Leu Ile Glu Ala Asn Leu Leu Trp Arg Gln Glu Met Gly Gly Asn Ile
                1205                1210                1215
Thr Arg Val Glu Ser Glu Asn Lys Val Val Val Leu Asp Ser Phe Asp
            1220                1225                1230
Pro Leu Arg Ala Glu Glu Asp Glu Arg Glu Val Ser Val Pro Ala Glu
        1235                1240                1245
Ile Leu Arg Lys Ser Lys Lys Phe Pro Ala Ala Met Pro Ile Trp Ala
    1250                1255                1260
Arg Pro Asp Tyr Asn Pro Pro Leu Leu Glu Ser Trp Lys Asp Pro Asp
1265                1270                1275                1280
Tyr Val Pro Pro Val Val His Gly Cys Pro Leu Pro Pro Ile Lys Ala
                1285                1290                1295
Pro Pro Ile Pro Pro Pro Arg Arg Lys Arg Thr Val Val Leu Thr Glu
            1300                1305                1310
Ser Ser Val Ser Ser Ala Leu Ala Glu Leu Ala Thr Lys Thr Phe Gly
        1315                1320                1325
Ser Ser Glu Ser Ser Ala Val Asp Ser Gly Thr Ala Thr Ala Leu Pro
    1330                1335                1340
Asp Gln Ala Ser Asp Asp Gly Asp Lys Gly Ser Asp Val Glu Ser Tyr
1345                1350                1355                1360
Ser Ser Met Pro Pro Leu Glu Gly Glu Pro Gly Asp Pro Asp Leu Ser

                    1365                1370                1375
Asp Gly Ser Trp Ser Thr Val Ser Glu Glu Ala Ser Glu Asp Val Val
            1380                1385                1390
Cys Cys Ser Met Ser Tyr Thr Trp Thr Gly Ala Leu Ile Thr Pro Cys
        1395                1400                1405
Ala Ala Glu Glu Ser Lys Leu Pro Ile Asn Ala Leu Ser Asn Ser Leu
    1410                1415                1420
Leu Arg His His Asn Met Val Tyr Ala Thr Thr Ser Arg Ser Ala Gly
1425                1430                1435                1440
Leu Arg Gln Lys Lys Val Thr Phe Asp Arg Leu Gln Val Leu Asp Asp
                1445                1450                1455
His Tyr Arg Asp Val Leu Lys Glu Met Lys Ala Lys Ala Ser Thr Val
            1460                1465                1470
Lys Ala Lys Leu Leu Ser Val Glu Glu Ala Cys Lys Leu Thr Pro Pro
        1475                1480                1485
His Ser Ala Lys Ser Lys Phe Gly Tyr Gly Ala Lys Asp Val Arg Asn
    1490                1495                1500
Leu Ser Ser Lys Ala Val Asn His Ile His Ser Val Trp Lys Asp Leu
1505                1510                1515                1520
Leu Glu Asp Thr Val Thr Pro Ile Asp Thr Thr Ile Met Ala Lys Asn
                1525                1530                1535
Glu Val Phe Cys Val Gln Pro Glu Lys Gly Gly Arg Lys Pro Ala Arg
            1540                1545                1550
Leu Ile Val Phe Pro Asp Leu Gly Val Arg Val Cys Glu Lys Met Ala
        1555                1560                1565
Leu Tyr Asp Val Val Ser Thr Leu Pro Gln Val Val Met Gly Ser Ser
    1570                1575                1580
Tyr Gly Phe Gln Tyr Ser Pro Gly Gln Arg Val Glu Phe Leu Val Asn
1585                1590                1595                1600
Thr Trp Lys Ser Lys Lys Asn Pro Met Gly Phe Ser Tyr Asp Thr Arg
                1605                1610                1615
Cys Phe Asp Ser Thr Val Thr Glu Asn Asp Ile Arg Val Glu Glu Ser
            1620                1625                1630
Ile Tyr Gln Cys Cys Asp Leu Ala Pro Glu Ala Arg Gln Ala Ile Lys
        1635                1640                1645
Ser Leu Thr Glu Arg Leu Tyr Ile Gly Gly Pro Leu Thr Asn Ser Lys
    1650                1655                1660
Gly Gln Asn Cys Gly Tyr Arg Arg Cys Arg Ala Ser Gly Val Leu Thr
1665                1670                1675                1680
Thr Ser Cys Gly Asn Thr Leu Thr Cys Tyr Leu Lys Ala Ser Ala Ala
                1685                1690                1695
Cys Arg Ala Ala Lys Leu Gln Asp Cys Thr Met Leu Val Asn Ala Ala
            1700                1705                1710
Gly Leu Val Val Ile Cys Glu Ser Ala Gly Thr Gln Glu Asp Ala Ala
        1715                1720                1725
Ser Leu Arg Val Phe Thr Glu Ala Met Thr Arg Tyr Ser Ala Pro Pro
    1730                1735                1740
Gly Asp Pro Pro Gln Pro Glu Tyr Asp Leu Glu Leu Ile Thr Ser Cys
1745                1750                1755                1760
Ser Ser Asn Val Ser Val Ala His Asp Ala Ser Gly Lys Arg Val Tyr
                1765                1770                1775
Tyr Leu Thr Arg Asp Pro Thr Thr Pro Leu Ala Arg Ala Ala Trp Glu
            1780                1785                1790
Thr Ala Arg His Thr Pro Val Asn Ser Trp Leu Gly Asn Ile Ile Met
        1795                1800                1805
Tyr Ala Pro Thr Leu Trp Ala Arg Met Ile Leu Met Thr His Phe Phe
    1810                1815                1820
Ser Ile Leu Leu Ala Gln Glu Gln Leu Glu Lys Ala Leu Asp Cys Gln
1825                1830                1835                1840
Ile Tyr Gly Ala Cys Tyr Ser Ile Glu Pro Leu Asp Leu Pro Gln Ile
                1845                1850                1855
Ile Glu Arg Leu His Gly Leu Ser Ala Phe Ser Leu His Ser Tyr Ser
            1860                1865                1870
Pro Gly Glu Ile Asn Arg Val Ala Ser Cys Leu Arg Lys Leu Gly Val
        1875                1880                1885
Pro Pro Leu Arg Val Trp Arg His Arg Ala Arg Ser Val Arg Ala Arg
    1890                1895                1900
Leu Leu Ser Gln Gly Gly Arg Ala Ala Thr Cys Gly Lys Tyr Leu Phe

1905                1910                1915                1920
Asn Trp Ala Val Lys Thr Lys Leu Lys Leu Thr Pro Ile Pro Ala Ala
                1925                1930                1935
Ser Gln Leu Asp Leu Ser Gly Trp Phe Val Ala Gly Tyr Ser Gly Gly
            1940                1945                1950
Asp Ile Tyr His Ser Leu Ser Arg Ala Arg Pro Arg Trp Phe Met Leu
        1955                1960                1965
Cys Leu Leu Leu Leu Ser Val Gly Val Gly Ile Tyr Leu Leu Pro Asn
    1970                1975                1980
Arg
1985


<210> 2
<211> 5965
<212> DNA
<213> Artificial Sequence

<220>
<223> Non-optimized cDNA sequence encoding SEQ. ID. NO.
      1

<400> 2
gccaccatgg cgcccatcac ggcctactcc caacagacgc ggggcctact tggttgcatc 60
atcactagcc ttacaggccg ggacaagaac caggtcgagg gagaggttca ggtggtttcc 120
accgcaacac aatccttcct ggcgacctgc gtcaacggcg tgtgttggac cgtttaccat 180
ggtgctggct caaagacctt agccggccca aaggggccaa tcacccagat gtacactaat 240
gtggaccagg acctcgtcgg ctggcaggcg ccccccgggg cgcgttcctt gacaccatgc 300
acctgtggca gctcagacct ttacttggtc acgagacatg ctgacgtcat tccggtgcgc 360
cggcggggcg acagtagggg gagcctgctc tcccccaggc ctgtctccta cttgaagggc 420
tcttcgggtg gtccactgct ctgcccttcg gggcacgctg tgggcatctt ccgggctgcc 480
gtatgcaccc ggggggttgc gaaggcggtg gactttgtgc ccgtagagtc catggaaact 540
actatgcggt ctccggtctt cacggacaac tcatcccccc cggccgtacc gcagtcattt 600
caagtggccc acctacacgc tcccactggc agcggcaaga gtactaaagt gccggctgca 660
tatgcagccc aagggtacaa ggtgctcgtc ctcaatccgt ccgttgccgc taccttaggg 720
tttggggcgt atatgtctaa ggcacacggt attgacccca acatcagaac tggggtaagg 780
accattacca caggcgcccc cgtcacatac tctacctatg gcaagtttct tgccgatggt 840
ggttgctctg ggggcgctta tgacatcata atatgtgatg agtgccattc aactgactcg 900
actacaatct tgggcatcgg cacagtcctg gaccaagcgg agacggctgg agcgcggctt 960
gtcgtgctcg ccaccgctac gcctccggga tcggtcaccg tgccacaccc aaacatcgag 1020
gaggtggccc tgtctaatac tggagagatc cccttctatg gcaaagccat ccccattgaa 1080
gccatcaggg ggggaaggca tctcattttc tgtcattcca agaagaagtg cgacgagctc 1140
gccgcaaagc tgtcaggcct cggaatcaac gctgtggcgt attaccgggg gctcgatgtg 1200
tccgtcatac caactatcgg agacgtcgtt gtcgtggcaa cagacgctct gatgacgggc 1260
tatacgggcg actttgactc agtgatcgac tgtaacacat gtgtcaccca gacagtcgac 1320
ttcagcttgg atcccacctt caccattgag acgacgaccg tgcctcaaga cgcagtgtcg 1380
cgctcgcagc ggcggggtag gactggcagg ggtaggagag gcatctacag gtttgtgact 1440
ccgggagaac ggccctcggg catgttcgat tcctcggtcc tgtgtgagtg ctatgacgcg 1500
ggctgtgctt ggtacgagct cacccccgcc gagacctcgg ttaggttgcg ggcctacctg 1560
aacacaccag ggttgcccgt ttgccaggac cacctggagt tctgggagag tgtcttcaca 1620
ggcctcaccc acatagatgc acacttcttg tcccagacca agcaggcagg agacaacttc 1680
ccctacctgg tagcatacca agccacggtg tgcgccaggg ctcaggcccc acctccatca 1740
tgggatcaaa tgtggaagtg tctcatacgg ctgaaaccta cgctgcacgg gccaacaccc 1800
ttgctgtaca ggctgggagc cgtccaaaat gaggtcaccc tcacccaccc cataaccaaa 1860
tacatcatgg catgcatgtc ggctgacctg gaggtcgtca ctagcacctg ggtgctggtg 1920
ggcggagtcc ttgcagctct ggccgcgtat tgcctgacaa caggcagtgt ggtcattgtg 1980
ggtaggatta tcttgtccgg gaggccggct attgttcccg acagggagtt tctctaccag 2040
gagttcgatg aaatggaaga gtgcgcctcg cacctccctt acatcgagca gggaatgcag 2100
ctcgccgagc aattcaagca gaaagcgctc gggttactgc aaacagccac caaacaagcg 2160
gaggctgctg ctcccgtggt ggagtccaag tggcgagccc ttgagacatt ctgggcgaag 2220
cacatgtgga atttcatcag cgggatacag tacttagcag gcttatccac tctgcctggg 2280
aaccccgcaa tagcatcatt gatggcattc acagcctcta tcaccagccc gctcaccacc 2340
caaagtaccc tcctgtttaa catcttgggg gggtgggtgg ctgcccaact cgcccccccc 2400
agcgccgctt cggctttcgt gggcgccggc atcgccggtg cggctgttgg cagcataggc 2460
cttgggaagg tgcttgtgga cattctggcg ggttatggag caggagtggc cggcgcgctc 2520
gtggccttca aggtcatgag cggcgagatg ccctccaccg aggacctggt caatctactt 2580
cctgccatcc tctctcctgg cgccctggtc gtcggggtcg tgtgtgcagc aatactgcgt 2640
cgacacgtgg gtccgggaga gggggctgtg cagtggatga accggctgat agcgttcgcc 2700

tcgcggggta atcatgtttc ccccacgcac tatgtgcctg agagcgacgc cgcagcgcgt 2760
gttactcaga tcctctccag ccttaccatc actcagctgc tgaaaaggct ccaccagtgg 2820
attaatgaag actgctccac accgtgttcc ggctcgtggc taagggatgt ttgggactgg 2880
atatgcacgg tgttgactga cttcaagacc tggctccagt ccaagctcct gccgcagcta 2940
ccgggagtcc cttttttctc gtgccaacgc gggtacaagg gagtctggcg gggagacggc 3000
atcatgcaaa ccacctgccc atgtggagca cagatcaccg gacatgtcaa aaacggttcc 3060
atgaggatcg tcggccctaa gacctgcagc aacacgtggc atggaacatt ccccatcaac 3120
gcatacacca cgggcccctg cacaccctct ccagcgccaa actattctag ggcgctgtgg 3180
cgggtggccg ctgaggagta cgtggaggtc acgcgggtgg gggatttcca ctacgtgacg 3240
ggcatgacca ctgacaacgt aaagtgccca tgccaggttc cggctcctga attcttcacg 3300
gaggtggacg gagtgcggtt gcacaggtac gctccggcgt gcaggcctct cctacgggag 3360
gaggttacat tccaggtcgg gctcaaccaa tacctggttg ggtcacagct accatgcgag 3420
cccgaaccgg atgtagcagt gctcacttcc atgctcaccg acccctccca catcacagca 3480
gaaacggcta agcgtaggtt ggccaggggg tctcccccct ccttggccag ctcttcagct 3540
agccagttgt ctgcgccttc cttgaaggcg acatgcacta cccaccatgt ctctccggac 3600
gctgacctca tcgaggccaa cctcctgtgg cggcaggaga tgggcgggaa catcacccgc 3660
gtggagtcgg agaacaaggt ggtagtcctg gactctttcg acccgcttcg agcggaggag 3720
gatgagaggg aagtatccgt tccggcggag atcctgcgga aatccaagaa gttccccgca 3780
gcgatgccca tctgggcgcg cccggattac aaccctccac tgttagagtc ctggaaggac 3840
ccggactacg tccctccggt ggtgcacggg tgcccgttgc cacctatcaa ggcccctcca 3900
ataccacctc cacggagaaa gaggacggtt gtcctaacag agtcctccgt gtcttctgcc 3960
ttagcggagc tcgctactaa gaccttcggc agctccgaat catcggccgt cgacagcggc 4020
acggcgaccg cccttcctga ccaggcctcc gacgacggtg acaaaggatc cgacgttgag 4080
tcgtactcct ccatgccccc ccttgagggg gaaccggggg accccgatct cagtgacggg 4140
tcttggtcta ccgtgagcga ggaagctagt gaggatgtcg tctgctgctc aatgtcctac 4200
acatggacag gcgccttgat cacgccatgc gctgcggagg aaagcaagct gcccatcaac 4260
gcgttgagca actctttgct gcgccaccat aacatggttt atgccacaac atctcgcagc 4320
gcaggcctgc ggcagaagaa ggtcaccttt gacagactgc aagtcctgga cgaccactac 4380
cgggacgtgc tcaaggagat gaaggcgaag gcgtccacag ttaaggctaa actcctatcc 4440
gtagaggaag cctgcaagct gacgccccca cattcggcca aatccaagtt tggctatggg 4500
gcaaaggacg tccggaacct atccagcaag gccgttaacc acatccactc cgtgtggaag 4560
gacttgctgg aagacactgt gacaccaatt gacaccacca tcatggcaaa aaatgaggtt 4620
ttctgtgtcc aaccagagaa aggaggccgt aagccagccc gccttatcgt attcccagat 4680
ctgggagtcc gtgtatgcga gaagatggcc ctctatgatg tggtctccac ccttcctcag 4740
gtcgtgatgg gctcctcata cggattccag tactctcctg ggcagcgagt cgagttcctg 4800
gtgaatacct ggaaatcaaa gaaaaacccc atgggctttt catatgacac tcgctgtttc 4860
gactcaacgg tcaccgagaa cgacatccgt gttgaggagt caatttacca atgttgtgac 4920
ttggcccccg aagccagaca ggccataaaa tcgctcacag agcggcttta tatcgggggt 4980
cctctgacta attcaaaagg gcagaactgc ggttatcgcc ggtgccgcgc gagcggcgtg 5040
ctgacgacta gctgcggtaa caccctcaca tgttacttga aggcctctgc agcctgtcga 5100
gctgcgaagc tccaggactg cacgatgctc gtgaacgccg ccggccttgt cgttatctgt 5160
gaaagcgcgg gaacccaaga ggacgcggcg agcctacgag tcttcacgga ggctatgact 5220
aggtactctg ccccccccgg ggacccgccc caaccagaat acgacttgga gctgataaca 5280
tcatgttcct ccaatgtgtc ggtcgcccac gatgcatcag gcaaaagggt gtactacctc 5340
acccgtgatc ccaccacccc cctcgcacgg gctgcgtggg aaacagctag acacactcca 5400
gttaactcct ggctaggcaa cattatcatg tatgcgccca ctttgtgggc aaggatgatt 5460
ctgatgactc acttcttctc catccttcta gcacaggagc aacttgaaaa agccctggac 5520
tgccagatct acggggcctg ttactccatt gagccacttg acctacctca gatcattgaa 5580
cgactccatg gccttagcgc attttcactc catagttact ctccaggtga gatcaatagg 5640
gtggcttcat gcctcaggaa acttggggta ccacccttgc gagtctggag acatcgggcc 5700
aggagcgtcc gcgctaggct actgtcccag ggggggaggg ccgccacttg tggcaagtac 5760
ctcttcaact gggcagtgaa gaccaaactc aaactcactc caatcccggc tgcgtcccag 5820
ctggacttgt ccggctggtt cgttgctggt tacagcgggg gagacatata tcacagcctg 5880
tctcgtgccc gaccccgctg gttcatgctg tgcctactcc tactttctgt aggggtaggc 5940
atctacctgc tccccaaccg ataaa 5965

<210> 3
<211> 5965
<212> DNA
<213> Artificial Sequence

<220>
<223> Optimized cDNA encoding SEQ ID NO: 1

<400> 3
gccaccatgg cccccatcac cgcctacagc cagcagaccc gcggcctgct gggctgcatc 60

atcaccagcc tgaccggccg cgacaagaac caggtggagg gcgaggtgca ggtggtgagc 120
accgccaccc agagcttcct ggccacctgc gtgaacggcg tgtgctggac cgtgtaccac 180
ggcgccggca gcaagaccct ggccggcccc aagggcccca tcacccagat gtacaccaac 240
gtggaccagg acctggtggg ctggcaggcc ccccccggcg cccgcagcct gaccccctgc 300
acctgcggca gcagcgacct gtacctggtg acccgccacg ccgacgtgat ccccgtgcgc 360
cgccgcggcg acagccgcgg cagcctgctg agcccccgcc ccgtgagcta cctgaagggc 420
agcagcggcg gccccctgct gtgccccagc ggccacgccg tgggcatctt ccgcgccgcc 480
gtgtgcaccc gcggcgtggc caaggccgtg gacttcgtgc ccgtggagag catggagacc 540
accatgcgca gccccgtgtt caccgacaac agcagccccc ccgccgtgcc ccagagcttc 600
caggtggccc acctgcacgc ccccaccggc agcggcaaga gcaccaaggt gcccgccgcc 660
tacgccgccc agggctacaa ggtgctggtg ctgaacccca gcgtggccgc caccctgggc 720
ttcggcgcct acatgagcaa ggcccacggc atcgacccca acatccgcac cggcgtgcgc 780
accatcacca ccggcgcccc cgtgacctac agcacctacg gcaagttcct ggccgacggc 840
ggctgcagcg gcggcgccta cgacatcatc atctgcgacg agtgccacag caccgacagc 900
accaccatcc tgggcatcgg caccgtgctg gaccaggccg agaccgccgg cgcccgcctg 960
gtggtgctgg ccaccgccac cccccccggc agcgtgaccg tgccccaccc caacatcgag 1020
gaggtggccc tgagcaacac cggcgagatc cccttctacg gcaaggccat ccccatcgag 1080
gccatccgcg gcggccgcca cctgatcttc tgccacagca agaagaagtg cgacgagctg 1140
gccgccaagc tgagcggcct gggcatcaac gccgtggcct actaccgcgg cctggacgtg 1200
agcgtgatcc ccaccatcgg cgacgtggtg gtggtggcca ccgacgccct gatgaccggc 1260
tacaccggcg acttcgacag cgtgatcgac tgcaacacct gcgtgaccca gaccgtggac 1320
ttcagcctgg accccacctt caccatcgag accaccaccg tgccccagga cgccgtgagc 1380
cgcagccagc gccgcggccg caccggccgc ggccgccgcg gcatctaccg cttcgtgacc 1440
cccggcgagc gccccagcgg catgttcgac agcagcgtgc tgtgcgagtg ctacgacgcc 1500
ggctgcgcct ggtacgagct gacccccgcc gagaccagcg tgcgcctgcg cgcctacctg 1560
aacacccccg gcctgcccgt gtgccaggac cacctggagt tctgggagag cgtgttcacc 1620
ggcctgaccc acatcgacgc ccacttcctg agccagacca agcaggccgg cgacaacttc 1680
ccctacctgg tggcctacca ggccaccgtg tgcgcccgcg cccaggcccc cccccccagc 1740
tgggaccaga tgtggaagtg cctgatccgc ctgaagccca ccctgcacgg ccccaccccc 1800
ctgctgtacc gcctgggcgc cgtgcagaac gaggtgaccc tgacccaccc catcaccaag 1860
tacatcatgg cctgcatgag cgccgacctg gaggtggtga ccagcacctg ggtgctggtg 1920
ggcggcgtgc tggccgccct ggccgcctac tgcctgacca ccggcagcgt ggtgatcgtg 1980
ggccgcatca tcctgagcgg ccgccccgcc atcgtgcccg accgcgagtt cctgtaccag 2040
gagttcgacg agatggagga gtgcgccagc cacctgccct acatcgagca gggcatgcag 2100
ctggccgagc agttcaagca gaaggccctg ggcctgctgc agaccgccac caagcaggcc 2160
gaggccgccg cccccgtggt ggagagcaag tggcgcgccc tggagacctt ctgggccaag 2220
cacatgtgga acttcatcag cggcatccag tacctggccg gcctgagcac cctgcccggc 2280
aaccccgcca tcgccagcct gatggccttc accgccagca tcaccagccc cctgaccacc 2340
cagagcaccc tgctgttcaa catcctgggc ggctgggtgg ccgcccagct ggcccccccc 2400
agcgccgcca gcgccttcgt gggcgccggc atcgccggcg ccgccgtggg cagcatcggc 2460
ctgggcaagg tgctggtgga catcctggcc ggctacggcg ccggcgtggc cggcgccctg 2520
gtggccttca aggtgatgag cggcgagatg cccagcaccg aggacctggt gaacctgctg 2580
cccgccatcc tgagccccgg cgccctggtg gtgggcgtgg tgtgcgccgc catcctgcgc 2640
cgccacgtgg gccccggcga gggcgccgtg cagtggatga accgcctgat cgccttcgcc 2700
agccgcggca accacgtgag ccccacccac tacgtgcccg agagcgacgc cgccgcccgc 2760
gtgacccaga tcctgagcag cctgaccatc acccagctgc tgaagcgcct gcaccagtgg 2820
atcaacgagg actgcagcac cccctgcagc ggcagctggc tgcgcgacgt gtgggactgg 2880
atctgcaccg tgctgaccga cttcaagacc tggctgcaga gcaagctgct gccccagctg 2940
cccggcgtgc ccttcttcag ctgccagcgc ggctacaagg gcgtgtggcg cggcgacggc 3000
atcatgcaga ccacctgccc ctgcggcgcc cagatcaccg gccacgtgaa gaacggcagc 3060
atgcgcatcg tgggccccaa gacctgcagc aacacctggc acggcacctt ccccatcaac 3120
gcctacacca ccggcccctg cacccccagc cccgccccca actacagccg cgccctgtgg 3180
cgcgtggccg ccgaggagta cgtggaggtg acccgcgtgg gcgacttcca ctacgtgacc 3240
ggcatgacca ccgacaacgt gaagtgcccc tgccaggtgc ccgcccccga gttcttcacc 3300
gaggtggacg gcgtgcgcct gcaccgctac gcccccgcct gccgccccct gctgcgcgag 3360
gaggtgacct tccaggtggg cctgaaccag tacctggtgg gcagccagct gccctgcgag 3420
cccgagcccg acgtggccgt gctgaccagc atgctgaccg accccagcca catcaccgcc 3480
gagaccgcca agcgccgcct ggcccgcggc agccccccca gcctggccag cagcagcgcc 3540
agccagctga gcgcccccag cctgaaggcc acctgcacca cccaccacgt gagccccgac 3600
gccgacctga tcgaggccaa cctgctgtgg cgccaggaga tgggcggcaa catcacccgc 3660
gtggagagcg agaacaaggt ggtggtgctg gacagcttcg accccctgcg cgccgaggag 3720
gacgagcgcg aggtgagcgt gcccgccgag atcctgcgca agagcaagaa gttccccgcc 3780
gccatgccca tctgggcccg ccccgactac aacccccccc tgctggagag ctggaaggac 3840
cccgactacg tgccccccgt ggtgcacggc tgccccctgc cccccatcaa ggcccccccc 3900
atcccccccc cccgccgcaa gcgcaccgtg gtgctgaccg agagcagcgt gagcagcgcc 3960
ctggccgagc tggccaccaa gaccttcggc agcagcgaga gcagcgccgt ggacagcggc 4020
accgccaccg ccctgcccga ccaggccagc gacgacggcg acaagggcag cgacgtggag 4080
agctacagca gcatgccccc cctggagggc gagcccggcg accccgacct gagcgacggc 4140

agctggagca ccgtgagcga ggaggccagc gaggacgtgg tgtgctgcag catgagctac 4200
acctggaccg gcgccctgat caccccctgc gccgccgagg agagcaagct gcccatcaac 4260
gccctgagca acagcctgct gcgccaccac aacatggtgt acgccaccac cagccgcagc 4320
gccggcctgc gccagaagaa ggtgaccttc gaccgcctgc aggtgctgga cgaccactac 4380
cgcgacgtgc tgaaggagat gaaggccaag gccagcaccg tgaaggccaa gctgctgagc 4440
gtggaggagg cctgcaagct gacccccccc cacagcgcca agagcaagtt cggctacggc 4500
gccaaggacg tgcgcaacct gagcagcaag gccgtgaacc acatccacag cgtgtggaag 4560
gacctgctgg aggacaccgt gacccccatc gacaccacca tcatggccaa gaacgaggtg 4620
ttctgcgtgc agcccgagaa gggcggccgc aagcccgccc gcctgatcgt gttccccgac 4680
ctgggcgtgc gcgtgtgcga gaagatggcc ctgtacgacg tggtgagcac cctgccccag 4740
gtggtgatgg gcagcagcta cggcttccag tacagccccg gccagcgcgt ggagttcctg 4800
gtgaacacct ggaagagcaa gaagaacccc atgggcttca gctacgacac ccgctgcttc 4860
gacagcaccg tgaccgagaa cgacatccgc gtggaggaga gcatctacca gtgctgcgac 4920
ctggcccccg aggcccgcca ggccatcaag agcctgaccg agcgcctgta catcggcggc 4980
cccctgacca acagcaaggg ccagaactgc ggctaccgcc gctgccgcgc cagcggcgtg 5040
ctgaccacca gctgcggcaa caccctgacc tgctacctga aggccagcgc cgcctgccgc 5100
gccgccaagc tgcaggactg caccatgctg gtgaacgccg ccggcctggt ggtgatctgc 5160
gagagcgccg gcacccagga ggacgccgcc agcctgcgcg tgttcaccga ggccatgacc 5220
cgctacagcg ccccccccgg cgaccccccc cagcccgagt acgacctgga gctgatcacc 5280
agctgcagca gcaacgtgag cgtggcccac gacgccagcg gcaagcgcgt gtactacctg 5340
acccgcgacc ccaccacccc cctggcccgc gccgcctggg agaccgcccg ccacaccccc 5400
gtgaacagct ggctgggcaa catcatcatg tacgccccca ccctgtgggc ccgcatgatc 5460
ctgatgaccc acttcttcag catcctgctg gcccaggagc agctggagaa ggccctggac 5520
tgccagatct acggcgcctg ctacagcatc gagcccctgg acctgcccca gatcatcgag 5580
cgcctgcacg gcctgagcgc cttcagcctg cacagctaca gccccggcga gatcaaccgc 5640
gtggccagct gcctgcgcaa gctgggcgtg ccccccctgc gcgtgtggcg ccaccgcgcc 5700
cgcagcgtgc gcgcccgcct gctgagccag ggcggccgcg ccgccacctg cggcaagtac 5760
ctgttcaact gggccgtgaa gaccaagctg aagctgaccc ccatccccgc cgccagccag 5820
ctggacctga gcggctggtt cgtggccggc tacagcggcg gcgacatcta ccacagcctg 5880
agccgcgccc gcccccgctg gttcatgctg tgcctgctgc tgctgagcgt gggcgtgggc 5940
atctacctgc tgcccaaccg ctaaa 5965

<210> 4
<211> 37090
<212> DNA
<213> Artificial Sequence

<220>
<223> MRKAd6-NSmut nucleic acid

<400> 4
catcatcaat aatatacctt attttggatt gaagccaata tgataatgag ggggtggagt 60
ttgtgacgtg gcgcggggcg tgggaacggg gcgggtgacg tagtagtgtg gcggaagtgt 120
gatgttgcaa gtgtggcgga acacatgtaa gcgacggatg tggcaaaagt gacgtttttg 180
gtgtgcgccg gtgtacacag gaagtgacaa ttttcgcgcg gttttaggcg gatgttgtag 240
taaatttggg cgtaaccgag taagatttgg ccattttcgc gggaaaactg aataagagga 300
agtgaaatct gaataatttt gtgttactca tagcgcgtaa tatttgtcta gggccgcggg 360
gactttgacc gtttacgtgg agactcgccc aggtgttttt ctcaggtgtt ttccgcgttc 420
cgggtcaaag ttggcgtttt attattatag gcggccgcga tccattgcat acgttgtatc 480
catatcataa tatgtacatt tatattggct catgtccaac attaccgcca tgttgacatt 540
gattattgac tagttattaa tagtaatcaa ttacggggtc attagttcat agcccatata 600
tggagttccg cgttacataa cttacggtaa atggcccgcc tggctgaccg cccaacgacc 660
cccgcccatt gacgtcaata atgacgtatg ttcccatagt aacgccaata gggactttcc 720
attgacgtca atgggtggag tatttacggt aaactgccca cttggcagta catcaagtgt 780
atcatatgcc aagtacgccc cctattgacg tcaatgacgg taaatggccc gcctggcatt 840
atgcccagta catgacctta tgggactttc ctacttggca gtacatctac gtattagtca 900
tcgctattac catggtgatg cggttttggc agtacatcaa tgggcgtgga tagcggtttg 960
actcacgggg atttccaagt ctccacccca ttgacgtcaa tgggagtttg ttttggcacc 1020
aaaatcaacg ggactttcca aaatgtcgta acaactccgc cccattgacg caaatgggcg 1080
gtaggcgtgt acggtgggag gtctatataa gcagagctcg tttagtgaac cgtcagatcg 1140
cctggagacg ccatccacgc tgttttgacc tccatagaag acaccgggac cgatccagcc 1200
tccgcggccg ggaacggtgc attggaacgc ggattccccg tgccaagagt gagatctgcc 1260
accatggcgc ccatcacggc ctactcccaa cagacgcggg gcctacttgg ttgcatcatc 1320
actagcctta caggccggga caagaaccag gtcgagggag aggttcaggt ggtttccacc 1380
gcaacacaat ccttcctggc gacctgcgtc aacggcgtgt gttggaccgt ttaccatggt 1440
gctggctcaa agaccttagc cggcccaaag gggccaatca cccagatgta cactaatgtg 1500

gaccaggacc tcgtcggctg gcaggcgccc cccggggcgc gttccttgac accatgcacc 1560
tgtggcagct cagaccttta cttggtcacg agacatgctg acgtcattcc ggtgcgccgg 1620
cggggcgaca gtagggggag cctgctctcc cccaggcctg tctcctactt gaagggctct 1680
tcgggtggtc cactgctctg cccttcgggg cacgctgtgg gcatcttccg ggctgccgta 1740
tgcacccggg gggttgcgaa ggcggtggac tttgtgcccg tagagtccat ggaaactact 1800
atgcggtctc cggtcttcac ggacaactca tcccccccgg ccgtaccgca gtcatttcaa 1860
gtggcccacc tacacgctcc cactggcagc ggcaagagta ctaaagtgcc ggctgcatat 1920
gcagcccaag ggtacaaggt gctcgtcctc aatccgtccg ttgccgctac cttagggttt 1980
ggggcgtata tgtctaaggc acacggtatt gaccccaaca tcagaactgg ggtaaggacc 2040
attaccacag gcgcccccgt cacatactct acctatggca agtttcttgc cgatggtggt 2100
tgctctgggg gcgcttatga catcataata tgtgatgagt gccattcaac tgactcgact 2160
acaatcttgg gcatcggcac agtcctggac caagcggaga cggctggagc gcggcttgtc 2220
gtgctcgcca ccgctacgcc tccgggatcg gtcaccgtgc cacacccaaa catcgaggag 2280
gtggccctgt ctaatactgg agagatcccc ttctatggca aagccatccc cattgaagcc 2340
atcagggggg gaaggcatct cattttctgt cattccaaga agaagtgcga cgagctcgcc 2400
gcaaagctgt caggcctcgg aatcaacgct gtggcgtatt accgggggct cgatgtgtcc 2460
gtcataccaa ctatcggaga cgtcgttgtc gtggcaacag acgctctgat gacgggctat 2520
acgggcgact ttgactcagt gatcgactgt aacacatgtg tcacccagac agtcgacttc 2580
agcttggatc ccaccttcac cattgagacg acgaccgtgc ctcaagacgc agtgtcgcgc 2640
tcgcagcggc ggggtaggac tggcaggggt aggagaggca tctacaggtt tgtgactccg 2700
ggagaacggc cctcgggcat gttcgattcc tcggtcctgt gtgagtgcta tgacgcgggc 2760
tgtgcttggt acgagctcac ccccgccgag acctcggtta ggttgcgggc ctacctgaac 2820
acaccagggt tgcccgtttg ccaggaccac ctggagttct gggagagtgt cttcacaggc 2880
ctcacccaca tagatgcaca cttcttgtcc cagaccaagc aggcaggaga caacttcccc 2940
tacctggtag cataccaagc cacggtgtgc gccagggctc aggccccacc tccatcatgg 3000
gatcaaatgt ggaagtgtct catacggctg aaacctacgc tgcacgggcc aacacccttg 3060
ctgtacaggc tgggagccgt ccaaaatgag gtcaccctca cccaccccat aaccaaatac 3120
atcatggcat gcatgtcggc tgacctggag gtcgtcacta gcacctgggt gctggtgggc 3180
ggagtccttg cagctctggc cgcgtattgc ctgacaacag gcagtgtggt cattgtgggt 3240
aggattatct tgtccgggag gccggctatt gttcccgaca gggagtttct ctaccaggag 3300
ttcgatgaaa tggaagagtg cgcctcgcac ctcccttaca tcgagcaggg aatgcagctc 3360
gccgagcaat tcaagcagaa agcgctcggg ttactgcaaa cagccaccaa acaagcggag 3420
gctgctgctc ccgtggtgga gtccaagtgg cgagcccttg agacattctg ggcgaagcac 3480
atgtggaatt tcatcagcgg gatacagtac ttagcaggct tatccactct gcctgggaac 3540
cccgcaatag catcattgat ggcattcaca gcctctatca ccagcccgct caccacccaa 3600
agtaccctcc tgtttaacat cttggggggg tgggtggctg cccaactcgc cccccccagc 3660
gccgcttcgg ctttcgtggg cgccggcatc gccggtgcgg ctgttggcag cataggcctt 3720
gggaaggtgc ttgtggacat tctggcgggt tatggagcag gagtggccgg cgcgctcgtg 3780
gccttcaagg tcatgagcgg cgagatgccc tccaccgagg acctggtcaa tctacttcct 3840
gccatcctct ctcctggcgc cctggtcgtc ggggtcgtgt gtgcagcaat actgcgtcga 3900
cacgtgggtc cgggagaggg ggctgtgcag tggatgaacc ggctgatagc gttcgcctcg 3960
cggggtaatc atgtttcccc cacgcactat gtgcctgaga gcgacgccgc agcgcgtgtt 4020
actcagatcc tctccagcct taccatcact cagctgctga aaaggctcca ccagtggatt 4080
aatgaagact gctccacacc gtgttccggc tcgtggctaa gggatgtttg ggactggata 4140
tgcacggtgt tgactgactt caagacctgg ctccagtcca agctcctgcc gcagctaccg 4200
ggagtccctt ttttctcgtg ccaacgcggg tacaagggag tctggcgggg agacggcatc 4260
atgcaaacca cctgcccatg tggagcacag atcaccggac atgtcaaaaa cggttccatg 4320
aggatcgtcg ggcctaagac ctgcagcaac acgtggcatg gaacattccc catcaacgca 4380
tacaccacgg gcccctgcac accctctcca gcgccaaact attctagggc gctgtggcgg 4440
gtggccgctg aggagtacgt ggaggtcacg cgggtggggg atttccacta cgtgacgggc 4500
atgaccactg acaacgtaaa gtgcccatgc caggttccgg ctcctgaatt cttcacggag 4560
gtggacggag tgcggttgca caggtacgct ccggcgtgca ggcctctcct acgggaggag 4620
gttacattcc aggtcggcct caaccaatac ctggttgggt cacagctacc atgcgagccc 4680
gaaccggatg tagcagtgct cacttccatg ctcaccgacc cctcccacat cacagcagaa 4740
acggctaagc gtaggttggc cagggggtct cccccctcct tggccagctc ttcagctagc 4800
cagttgtctg cgccttcctt gaaggcgaca tgcactaccc accatgtctc tccggacgct 4860
gacctcatcg aggccaacct cctgtggcgg caggagatgg gcgggaacat cacccgcgtg 4920
gagtcggaga acaaggtggt agtcctggac tctttcgacc cgcttcgagc ggaggaggat 4980
gagagggaag tatccgttcc ggcggagatc ctgcggaaat ccaagaagtt ccccgcagcg 5040
atgcccatct gggcgcgccc ggattacaac cctccactgt tagagtcctg gaaggacccg 5100
gactacgtcc ctccggtggt gcacgggtgc ccgttgccac ctatcaaggc ccctccaata 5160
ccacctccac ggagaaagag gacggttgtc ctaacagagt cctccgtgtc ttctgcctta 5220
gcggagctcg ctactaagac cttcggcagc tccgaatcat cggccgtcga cagcggcacg 5280
gcgaccgccc ttcctgacca ggcctccgac gacggtgaca aaggatccga cgttgagtcg 5340
tactcctcca tgccccccct tgagggggaa ccgggggacc ccgatctcag tgacgggtct 5400
tggtctaccg tgagcgagga agctagtgag gatgtcgtct gctgctcaat gtcctacaca 5460
tggacaggcg ccttgatcac gccatgcgct gcggaggaaa gcaagctgcc catcaacgcg 5520
ttgagcaact ctttgctgcg ccaccataac atggtttatg ccacaacatc tcgcagcgca 5580

ggcctgcggc agaagaaggt cacctttgac agactgcaag tcctggacga ccactaccgg 5640
gacgtgctca aggagatgaa ggcgaaggcg tccacagtta aggctaaact cctatccgta 5700
gaggaagcct gcaagctgac gcccccacat tcggccaaat ccaagtttgg ctatggggca 5760
aaggacgtcc ggaacctatc cagcaaggcc gttaaccaca tccactccgt gtggaaggac 5820
ttgctggaag acactgtgac accaattgac accaccatca tggcaaaaaa tgaggttttc 5880
tgtgtccaac cagagaaagg aggccgtaag ccagcccgcc ttatcgtatt cccagatctg 5940
ggagtccgtg tatgcgagaa gatggccctc tatgatgtgg tctccaccct tcctcaggtc 6000
gtgatgggct cctcatacgg attccagtac tctcctgggc agcgagtcga gttcctggtg 6060
aatacctgga aatcaaagaa aaaccccatg ggcttttcat atgacactcg ctgtttcgac 6120
tcaacggtca ccgagaacga catccgtgtt gaggagtcaa tttaccaatg ttgtgacttg 6180
gcccccgaag ccagacaggc cataaaatcg ctcacagagc ggctttatat cgggggtcct 6240
ctgactaatt caaaagggca gaactgcggt tatcgccggt gccgcgcgag cggcgtgctg 6300
acgactagct gcggtaacac cctcacatgt tacttgaagg cctctgcagc ctgtcgagct 6360
gcgaagctcc aggactgcac gatgctcgtg aacgccgccg gccttgtcgt tatctgtgaa 6420
agcgcgggaa cccaagagga cgcggcgagc ctacgagtct tcacggaggc tatgactagg 6480
tactctgccc cccccgggga cccgccccaa ccagaatacg acttggagct gataacatca 6540
tgttcctcca atgtgtcggt cgcccacgat gcatcaggca aaagggtgta ctacctcacc 6600
cgtgatccca ccacccccct cgcacgggct gcgtgggaaa cagctagaca cactccagtt 6660
aactcctggc taggcaacat tatcatgtat gcgcccactt tgtgggcaag gatgattctg 6720
atgactcact tcttctccat ccttctagca caggagcaac ttgaaaaagc cctggactgc 6780
cagatctacg gggcctgtta ctccattgag ccacttgacc tacctcagat cattgaacga 6840
ctccatggcc ttagcgcatt ttcactccat agttactctc caggtgagat caatagggtg 6900
gcttcatgcc tcaggaaact tggggtacca cccttgcgag tctggagaca tcggccaggg 6960
agcgtccgcg ctaggctact gtcccagggg gggagggccg ccacttgtgg caagtacctc 7020
ttcaactggg cagtgaagac caaactcaaa ctcactccaa tcccggctgc gtcccagctg 7080
gacttgtccg gctggttcgt tgctggttac agcgggggag acatatatca cagcctgtct 7140
cgtgcccgac cccgctggtt catgctgtgc ctactcctac tttctgtagg ggtaggcatc 7200
tacctgctcc ccaaccggta aatctagagc tgtgccttct agttgccagc catctgttgt 7260
ttgcccctcc cccgtgcctt ccttgaccct ggaaggtgcc actcccactg tcctttccta 7320
ataaaatgag gaaattgcat cgcattgtct gagtaggtgt cattctattc tggggggtgg 7380
ggtggggcag gacagcaagg gggaggattg ggaagacaat agcaggcatg ctggggatgc 7440
ggtgggctct atggccgatc ggcgcgccgt actgaaatgt gtgggcgtgg cttaagggtg 7500
ggaaagaata tataaggtgg gggtcttatg tagttttgta tctgttttgc agcagccgcc 7560
gccgccatga gcaccaactc gtttgatgga agcattgtga gctcatattt gacaacgcgc 7620
atgcccccat gggccggggt gcgtcagaat gtgatgggct ccagcattga tggtcgcccc 7680
gtcctgcccg caaactctac taccttgacc tacgagaccg tgtctggaac gccgttggag 7740
actgcagcct ccgccgccgc ttcagccgct gcagccaccg cccgcgggat tgtgactgac 7800
tttgctttcc tgagcccgct tgcaagcagt gcagcttccc gttcatccgc ccgcgatgac 7860
aagttgacgg ctcttttggc acaattggat tctttgaccc gggaacttaa tgtcgtttct 7920
cagcagctgt tggatctgcg ccagcaggtt tctgccctga aggcttcctc ccctcccaat 7980
gcggtttaaa acataaataa aaaaccagac tctgtttgga tttggatcaa gcaagtgtct 8040
tgctgtcttt atttaggggt tttgcgcgcg cggtaggccc gggaccagcg gtctcggtcg 8100
ttgagggtcc tgtgtatttt ttccaggacg tggtaaaggt gactctggat gttcagatac 8160
atgggcataa gcccgtctct ggggtggagg tagcaccact gcagagcttc atgctgcggg 8220
gtggtgttgt agatgatcca gtcgtagcag gagcgctggg cgtggtgcct aaaaatgtct 8280
ttcagtagca agctgattgc caggggcagg cccttggtgt aagtgtttac aaagcggtta 8340
agctgggatg ggtgcatacg tggggatatg agatgcatct tggactgtat ttttaggttg 8400
gctatgttcc cagccatatc cctccgggga ttcatgttgt gcagaaccac cagcacagtg 8460
tatccggtgc acttgggaaa tttgtcatgt agcttagaag gaaatgcgtg gaagaacttg 8520
gagacgccct tgtgacctcc aagattttcc atgcattcgt ccataatgat ggcaatgggc 8580
ccacgggcgg cggcctgggc gaagatattt ctgggatcac taacgtcata gttgtgttcc 8640
aggatgagat cgtcataggc catttttaca aagcgcgggc ggagggtgcc agactgcggt 8700
ataatggttc catccggccc aggggcgtag ttaccctcac agatttgcat ttcccacgct 8760
ttgagttcag atggggggat catgtctacc tgcggggcga tgaagaaaac ggtttccggg 8820
gtaggggaga tcagctggga agaaagcagg ttcctgagca gctgcgactt accgcagccg 8880
gtgggcccgt aaatcacacc tattaccggc tgcaactggt agttaagaga gctgcagctg 8940
ccgtcatccc tgagcagggg ggccacttcg ttaagcatgt ccctgactcg catgttttcc 9000
ctgaccaaat ccgccagaag gcgctcgccg cccagcgata gcagttcttg caaggaagca 9060
aagtttttca acggtttgag accgtccgcc gtaggcatgc ttttgagcgt ttgaccaagc 9120
agttccaggc ggtcccacag ctcggtcacc tgctctacgg catctcgatc cagcatatct 9180
cctcgtttcg cgggttgggg cggctttcgc tgtacggcag tagtcggtgc tcgtccagac 9240
gggccagggt catgtctttc cacgggcgca gggtcctcgt cagcgtagtc tgggtcacgg 9300
tgaaggggtg cgctccgggc tgcgcgctgg ccagggtgcg cttgaggctg gtcctgctgg 9360
tgctgaagcg ctgccggtct tcgccctgcg cgtcggccag gtagcatttg accatggtgt 9420
catagtccag cccctccgcg gcgtggccct tggcgcgcag cttgcccttg gaggaggcgc 9480
cgcacgaggg gcagtgcaga cttttgaggg cgtagagctt gggcgcgaga aataccgatt 9540
ccggggagta ggcatccgcg ccgcaggccc cgcagacggt ctcgcattcc acgagccagg 9600
tgagctctgg ccgttcgggg tcaaaaacca ggtttccccc atgctttttg atgcgtttct 9660

tacctctggt ttccatgagc cggtgtccac gctcggtgac gaaaaggctg tccgtgtccc 9720
cgtatacaga cttgagaggc ctgtcctcga gcggtgttcc gcggtcctcc tcgtatagaa 9780
actcggacca ctctgagacg aaggctcgcg tccaggccag cacgaaggag gctaagtggg 9840
aggggtagcg gtcgttgtcc actagggggt ccactcgctc cagggtgtga agacacatgt 9900
cgccctcttc ggcatcaagg aaggtgattg gtttataggt gtaggccacg tgaccgggtg 9960
ttcctgaagg ggggctataa aagggggtgg gggcgcgttc gtcctcactc tcttccgcat 10020
cgctgtctgc gagggccagc tgttggggtg agtactccct ctcaaaagcg ggcatgactt 10080
ctgcgctaag attgtcagtt tccaaaaacg aggaggattt gatattcacc tggcccgcgg 10140
tgatgccttt gagggtggcc gcgtccatct ggtcagaaaa gacaatcttt ttgttgtcaa 10200
gcttggtggc aaacgacccg tagagggcgt tggacagcaa cttggcgatg gagcgcaggg 10260
tttggttttt gtcgcgatcg gcgcgctcct tggccgcgat gtttagctgc acgtattcgc 10320
gcgcaacgca ccgccattcg ggaaagacgg tggtgcgctc gtcgggcact aggtgcacgc 10380
gccaaccgcg gttgtgcagg gtgacaaggt caacgctggt ggctacctct ccgcgtaggc 10440
gctcgttggt ccagcagagg cggccgccct tgcgcgagca gaatggcggt agtgggtcta 10500
gctgcgtctc gtccgggggg tctgcgtcca cggtaaagac cccgggcagc aggcgcgcgt 10560
cgaagtagtc tatcttgcat ccttgcaagt ctagcgcctg ctgccatgcg cgggcggcaa 10620
gcgcgcgctc gtatgggttg agtgggggac cccatggcat ggggtgggtg agcgcggagg 10680
cgtacatgcc gcaaatgtcg taaacgtaga ggggctctct gagtattcca agatatgtag 10740
ggtagcatct tccaccgcgg atgctggcgc gcacgtaatc gtatagttcg tgcgagggag 10800
cgaggaggtc gggaccgagg ttgctacggg cgggctgctc tgctcggaag actatctgcc 10860
tgaagatggc atgtgagttg gatgatatgg ttggacgctg gaagacgttg aagctggcgt 10920
ctgtgagacc taccgcgtca cgcacgaagg aggcgtagga gtcgcgcagc ttgttgacca 10980
gctcggcggt gacctgcacg tctagggcgc agtagtccag ggtttccttg atgatgtcat 11040
acttatcctg tccctttttt ttccacagct cgcggttgag gacaaactct tcgcggtctt 11100
tccagtactc ttggatcgga aacccgtcgg cctccgaacg gtaagagcct agcatgtaga 11160
actggttgac ggcctggtag gcgcagcatc ccttttctac gggtagcgcg tatgcctgcg 11220
cggccttccg gagcgaggtg tgggtgagcg caaaggtgtc cctaaccatg actttgaggt 11280
actggtattt gaagtcagtg tcgtcgcatc cgccctgctc ccagagcaaa aagtccgtgc 11340
gctttttgga acgcgggttt ggcagggcga aggtgacatc gttgaagagt atctttcccg 11400
cgcgaggcat aaagttgcgt gtgatgcgga agggtcccgg cacctcggaa cggttgttaa 11460
ttacctgggc ggcgagcacg atctcgtcaa agccgttgat gttgtggccc acaatgtaaa 11520
gttccaagaa gcgcgggatg cccttgatgg aaggcaattt tttaagttcc tcgtaggtga 11580
gctcttcagg ggagctgagc ccgtgctctg aaagggccca gtctgcaaga tgagggttgg 11640
aagcgacgaa tgagctccac aggtcacggg ccattagcat ttgcaggtgg tcgcgaaagg 11700
tcctaaactg gcgacctatg gccatttttt ctggggtgat gcagtagaag gtaagcgggt 11760
cttgttccca gcggtcccat ccaaggtccg cggctaggtc tcgcgcggcg gtcactagag 11820
gctcatctcc gccgaacttc atgaccagca tgaagggcac gagctgcttc ccaaaggccc 11880
ccatccaagt ataggtctct acatcgtagg tgacaaagag acgctcggtg cgaggatgcg 11940
agccgatcgg gaagaactgg atctcccgcc accagttgga ggagtggctg ttgatgtggt 12000
gaaagtagaa gtccctgcga cgggccgaac actcgtgctg gcttttgtaa aaacgtgcgc 12060
agtactggca gcggtgcacg ggctgtacat cctgcacgag gttgacctga cgaccgcgca 12120
caaggaagca gagtgggaat ttgagcccct cgcctggcgg gtttggctgg tggtcttcta 12180
cttcggctgc ttgtccttga ccgtctggct gctcgagggg agttacggtg gatcggacca 12240
ccacgccgcg cgagcccaaa gtccagatgt ccgcgcgcgg cggtcggagc ttgatgacaa 12300
catcgcgcag atgggagctg tccatggtct ggagctcccg cggcgtcagg tcaggcggga 12360
gctcctgcag gtttacctcg catagccggg tcagggcgcg ggctaggtcc aggtgatacc 12420
tgatttccag gggctggttg gtggcggcgt cgatggcttg caagaggccg catccccgcg 12480
gcgcgactac ggtaccgcgc ggcgggcggt gggccgcggg ggtgtccttg gatgatgcat 12540
ctaaaagcgg tgacgcgggc gggcccccgg aggtaggggg ggctcgggac ccgccgggag 12600
agggggcagg ggcacgtcgg cgccgcgcgc gggcaggagc tggtgctgcg cgcggaggtt 12660
gctggcgaac gcgacgacgc ggcggttgat ctcctgaatc tggcgcctct gcgtgaagac 12720
gacgggcccg gtgagcttga acctgaaaga gagttcgaca gaatcaattt cggtgtcgtt 12780
gacggcggcc tggcgcaaaa tctcctgcac gtctcctgag ttgtcttgat aggcgatctc 12840
ggccatgaac tgctcgatct cttcctcctg gagatctccg cgtccggctc gctccacggt 12900
ggcggcgagg tcgttggaga tgcgggccat gagctgcgag aaggcgttga ggcctccctc 12960
gttccagacg cggctgtaga ccacgccccc ttcggcatcg cgggcgcgca tgaccacctg 13020
cgcgagattg agctccacgt gccgggcgaa gacggcgtag tttcgcaggc gctgaaagag 13080
gtagttgagg gtggtggcgg tgtgttctgc cacgaagaag tacataaccc agcgccgcaa 13140
cgtggattcg ttgatatccc ccaaggcctc aaggcgctcc atggcctcgt agaagtccac 13200
ggcgaagttg aaaaactggg agttgcgcgc cgacacggtt aactcctcct ccagaagacg 13260
gatgagctcg gcgacagtgt cgcgcacctc gcgctcaaag gctacagggg cctcttcttc 13320
ttcttcaatc tcctcttcca taagggcctc cccttcttct tcttctggcg gcggtggggg 13380
aggggggaca cggcggcgac gacggcgcac cgggaggcgg tcgacaaagc gctcgatcat 13440
ctccccgcgg cgacggcgca tggtctcggt gacggcgcgg ccgttctcgc gggggcgcag 13500
ttggaagacg ccgcccgtca tgtcccggtt atgggttggc ggggggctgc cgtgcggcag 13560
ggatacggcg ctaacgatgc atctcaacaa ttgttgtgta ggtactccgc caccgaggga 13620
cctgagcgag tccgcatcga ccggatcgga aaacctctcg agaaaggcgt ctaaccagtc 13680
acagtcgcaa ggtaggctga gcaccgtggc gggcggcagc gggcggcggt cggggttgtt 13740

tctggcggag gtgctgctga tgatgtaatt aaagtaggcg gtcttgagac ggcggatggt 13800
cgacagaagc accatgtcct tgggtccggc ctgctgaatg cgcaggcggt cggccatgcc 13860
ccaggcttcg ttttgacatc ggcgcaggtc tttgtagtag tcttgcatga gcctttctac 13920
cggcacttct tcttctcctt cctcttgtcc tgcatctctt gcatctatcg ctgcggcggc 13980
ggcggagttt ggccgtaggt ggcgccctct tcctcccatg cgtgtgaccc cgaagcccct 14040
catcggctga agcagggcca ggtcggcgac aacgcgctcg gctaatatgg cctgctgcac 14100
ctgcgtgagg gtagactgga agtcgtccat gtccacaaag cggtggtatg cgcccgtgtt 14160
gatggtgtaa gtgcagttgg ccataacgga ccagttaacg gtctggtgac ccggctgcga 14220
gagctcggtg tacctgagac gcgagtaagc ccttgagtca aagacgtagt cgttgcaagt 14280
ccgcaccagg tactggtatc ccaccaaaaa gtgcggcggc ggctggcggt agaggggcca 14340
gcgtagggtg gccggggctc cgggggcgag gtcttccaac ataaggcgat gatatccgta 14400
gatgtacctg gacatccagg tgatgccggc ggcggtggtg gaggcgcgcg gaaagtcacg 14460
gacgcggttc cagatgttgc gcagcggcaa aaagtgctcc atggtcggga cgctctggcc 14520
ggtcaggcgc gcgcagtcgt tgacgctcta gaccgtgcaa aaggagagcc tgtaagcggg 14580
cactcttccg tggtctggtg gataaattcg caagggtatc atggcggacg accggggttc 14640
gaaccccgga tccggccgtc cgccgtgatc catgcggtta ccgcccgcgt gtcgaaccca 14700
ggtgtgcgac gtcagacaac gggggagcgc tccttttggc ttccttccag gcgcggcgga 14760
tgctgcgcta gcttttttgg ccactggccg cgcgcggcgt aagcggttag gctggaaagc 14820
gaaagcatta agtggctcgc tccctgtagc cggagggtta ttttccaagg gttgagtcgc 14880
gggacccccg gttcgagtct cgggccggcc ggactgcggc gaacgggggt ttgcctcccc 14940
gtcatgcaag accccgcttg caaattcctc cggaaacagg gacgagcccc ttttttgctt 15000
ttcccagatg catccggtgc tgcggcagat gcgcccccct cctcagcagc ggcaagagca 15060
agagcagcgg cagacatgca gggcaccctc cccttctcct accgcgtcag gaggggcaac 15120
atccgcggct gacgcggcgg cagatggtga ttacgaaccc ccgcggcgcc ggacccggca 15180
ctacttggac ttggaggagg gcgagggcct ggcgcggcta ggagcgccct ctcctgagcg 15240
acacccaagg gtgcagctga agcgtgacac gcgcgaggcg tacgtgccgc ggcagaacct 15300
gtttcgcgac cgcgagggag aggagcccga ggagatgcgg gatcgaaagt tccatgcagg 15360
gcgcgagttg cggcatggcc tgaaccgcga gcggttgctg cgcgaggagg actttgagcc 15420
cgacgcgcgg accgggatta gtcccgcgcg cgcacacgtg gcggccgccg acctggtaac 15480
cgcgtacgag cagacggtga accaggagat taactttcaa aaaagcttta acaaccacgt 15540
gcgcacgctt gtggcgcgcg aggaggtggc tataggactg atgcatctgt gggactttgt 15600
aagcgcgctg gagcaaaacc caaatagcaa gccgctcatg gcgcagctgt tccttatagt 15660
gcagcacagc agggacaacg aggcattcag ggatgcgctg ctaaacatag tagagcccga 15720
gggccgctgg ctgctcgatt tgataaacat tctgcagagc atagtggtgc aggagcgcag 15780
cttgagcctg gctgacaagg tggccgccat taactattcc atgctcagtc tgggcaagtt 15840
ttacgcccgc aagatatacc atacccctta cgttcccata gacaaggagg taaagatcga 15900
ggggttctac atgcgcatgg cgctgaaggt gcttaccttg agcgacgacc tgggcgttta 15960
tcgcaacgag cgcatccaca aggccgtgag cgtgagccgg cggcgcgagc tcagcgaccg 16020
cgagctgatg cacagcctgc aaagggccct ggctggcacg ggcagcggcg atagagaggc 16080
cgagtcctac tttgacgcgg gcgctgacct gcgctgggcc ccaagccgac gcgccctgga 16140
ggcagctggg gccggacctg ggctggcggt ggcacccgcg cgcgctggca acgtcggcgg 16200
cgtggaggaa tatgacgagg acgatgagta cgagccagag gacggcgagt actaagcggt 16260
gatgtttctg atcagatgat gcaagacgca acggacccgg cggtgcgggc ggcgctgcag 16320
agccagccgt ccggccttaa ctccacggac gactggcgcc aggtcatgga ccgcatcatg 16380
tcgctgactg cgcgcaaccc tgacgcgttc cggcagcagc cgcaggccaa ccggctctcc 16440
gcaattctgg aagcggtggt cccggcgcgc gcaaacccca cgcacgagaa ggtgctggcg 16500
atcgtaaacg cgctggccga aaacaggccc atccggcccg atgaggccgg cctggtctac 16560
gacgcgctgc ttcagcgcgt ggctcgttac aacagcagca acgtgcagac caacctggac 16620
cggctggtgg gggatgtgcg cgaggccgtg gcgcagcgtg agcgcgcgca gcagcagggc 16680
aacctgggct ccatggttgc actaaacgcc ttcctgagta cacagcccgc caacgtgccg 16740
cggggacagg aggactacac caactttgtg agcgcactgc ggctaatggt gactgagaca 16800
ccgcaaagtg aggtgtatca gtccgggcca gactattttt tccagaccag tagacaaggc 16860
ctgcagaccg taaacctgag ccaggctttc aagaacttgc aggggctgtg gggggtgcgg 16920
gctcccacag gcgaccgcgc gaccgtgtct agcttgctga cgcccaactc gcgcctgttg 16980
ctgctgctaa tagcgccctt cacggacagt ggcagcgtgt cccgggacac atacctaggt 17040
cacttgctga cactgtaccg cgaggccata ggtcaggcgc atgtggacga gcatactttc 17100
caggagatta caagtgttag ccgcgcgctg gggcaggagg acacgggcag cctggaggca 17160
accctgaact acctgctgac caaccggcgg caaaaaatcc cctcgttgca cagtttaaac 17220
agcgaggagg agcgcatttt gcgctatgtg cagcagagcg tgagccttaa cctgatgcgc 17280
gacggggtaa cgcccagcgt ggcgctggac atgaccgcgc gcaacatgga accgggcatg 17340
tatgcctcaa accggccgtt tatcaatcgc ctaatggact acttgcatcg cgcggccgcc 17400
gtgaaccccg agtatttcac caatgccatc ttgaacccgc actggctacc gccccctggt 17460
ttctacaccg ggggattcga ggtgcccgag ggtaacgatg gattcctctg ggacgacata 17520
gacgacagcg tgttttcccc gcaaccgcag accctgctag agttgcaaca acgcgagcag 17580
gcagaggcgg cgctgcgaaa ggaaagcttc cgcaggccaa gcagcttgtc cgatctaggc 17640
gctgcggccc cgcggtcaga tgctagtagc ccatttccaa gcttgatagg gtctcttacc 17700
agcactcgca ccacccgccc gcgcctgctg ggcgaggagg agtacctaaa caactcgctg 17760
ctgcagccgc agcgcgaaaa gaacctgcct ccggcgtttc ccaacaacgg gatagagagc 17820

ctagtggaca agatgagtag atggaagacg tatgcgcagg agcacaggga tgtgcccggc 17880
ccgcgcccgc ccacccgtcg tcaaaggcac gaccgtcagc ggggtctggt gtgggaggac 17940
gatgactcgg cagacgacag cagcgtcttg gatttgggag ggagtggcaa cccgtttgca 18000
caccttcgcc ccaggctggg gagaatgttt taaaaaaaag catgatgcaa aataaaaaac 18060
tcaccaaggc catggcaccg agcgttggtt ttcttgtatt ccccttagta tgcggcgcgc 18120
ggcgatgtat gaggaaggtc ctcctccctc ctacgagagc gtggtgagcg cggcgccagt 18180
ggcggcggcg ctgggttcac ccttcgatgc tcccctggac ccgccgttcg tgcctccgcg 18240
gtacctgcgg cctaccgggg ggagaaacag catccgttac tctgagttgg cacccctatt 18300
cgacaccacc cgtgtgtacc ttgtggacaa caagtcaacg gatgtggcat ccctgaacta 18360
ccagaacgac cacagcaact ttctaaccac ggtcattcaa aacaatgact acagcccggg 18420
ggaggcaagc acacagacca tcaatcttga cgaccggtcg cactggggcg gcgacctgaa 18480
aaccatcctg cataccaaca tgccaaatgt gaacgagttc atgtttacca ataagtttaa 18540
ggcgcgggtg atggtgtcgc gctcgcttac taaggacaaa caggtggagc tgaaatacga 18600
gtgggtggag ttcacgctgc ccgagggcaa ctactccgag accatgacca tagaccttat 18660
gaacaacgcg atcgtggagc actacttgaa agtgggcagg cagaacgggg ttctggaaag 18720
cgacatcggg gtaaagtttg acacccgcaa cttcagactg gggtttgacc cagtcactgg 18780
tcttgtcatg cctggggtat atacaaacga agccttccat ccagacatca ttttgctgcc 18840
aggatgcggg gtggacttca cccacagccg cctgagcaac ttgttgggca tccgcaagcg 18900
gcaacccttc caggagggct ttaggatcac ctacgatgac ctggagggtg gtaacattcc 18960
cgcactgttg gatgtggacg cctaccaggc aagcttgaaa gatgacaccg aacagggcgg 19020
gggtggcgca ggcggcggca acaacagtgg cagcggcgcg gaagagaact ccaacgcggc 19080
agctgcggca atgcagccgg tggaggacat gaacgatcat gccattcgcg gcgacacctt 19140
tgccacacgg gcggaggaga agcgcgctga ggccgaggca gcggccgaag ctgccgcccc 19200
cgctgcggag gctgcacaac ccgaggtcga gaagcctcag aagaaaccgg tgattaaacc 19260
cctgacagag gacagcaaga aacgcagtta caacctaata agcaatgaca gcaccttcac 19320
ccagtaccgc agctggtacc ttgcatacaa ctacggcgac cctcaggccg ggatccgctc 19380
atggaccctg ctttgcactc ctgacgtaac ctgcggctcg gagcaggtat actggtcgtt 19440
gcccgacatg atgcaagacc ccgtgacctt ccgctccacg cgccagatca gcaactttcc 19500
ggtggtgggc gccgagctgt tgcccgtgca ctccaagagc ttctacaacg accaggccgt 19560
ctactcccag ctcatccgcc agtttacctc tctgacccac gtgttcaatc gctttcccga 19620
gaaccagatt ttggcgcgcc cgccagcccc caccatcacc accgtcagtg aaaacgttcc 19680
tgctctcaca gatcacggga cgctaccgct gcgcaacagc atcggaggag tccagcgagt 19740
gaccattact gacgccagac gccgcacctg cccctacgtt tacaaggccc tgggcatagt 19800
ctcgccgcgc gtcctatcga gccgcacttt ttgagcaagc atgtccatcc ttatatcgcc 19860
cagcaataac acaggctggg gcctgcgctt cccaagcaag atgtttggcg gggccaagaa 19920
gcgctccgac caacacccag tgcgcgtgcg cgggcactac cgcgcgccct ggggcgcgca 19980
caaacgcggc cgcactgggc gcaccaccgt cgatgacgcc atcgacgcgg tggtggagga 20040
ggcgcgcaac tacacgccca cgccgccgcc agtgtccacc gtggacgcgg ccattcagac 20100
cgtggtgcgc ggagcccggc gctacgctaa aatgaagaga cggcggaggc gcgtagcacg 20160
tcgccaccgc cgccgacccg gcactgccgc ccaacgcgcg gcggcggccc tgcttaaccg 20220
cgcacgtcgc accggccgac gggcggccat gcgagccgct cgaaggctgg ccgcgggtat 20280
tgtcactgtg ccccccaggt ccaggcgacg agcggccgcc gcagcagccg cggccattag 20340
tgctatgact cagggtcgca ggggcaacgt gtactgggtg cgcgactcgg ttagcggcct 20400
gcgcgtgccc gtgcgcaccc gccccccgcg caactagatt gcaataaaaa actacttaga 20460
ctcgtactgt tgtatgtatc cagcggcggc ggcgcgcatc gaagctatgt ccaagcgcaa 20520
aatcaaagaa gagatgctcc aggtcatcgc gccggagatc tatggccccc cgaagaagga 20580
agagcaggat tacaagcccc gaaagctaaa gcgggtcaaa aagaaaaaga aagatgatga 20640
tgatgatgaa cttgacgacg aggtggaact gttgcacgcg accgcgccca ggcgacgggt 20700
acagtggaaa ggtcgacgcg taagacgtgt tttgcgaccc ggcaccaccg tagtctttac 20760
gcccggtgag cgctccaccc gcacctacaa gcgcgtgtat gatgaggtgt acggcgacga 20820
ggacctgctt gagcaggcca acgagcgcct cggggagttt gcctacggaa agcggcataa 20880
ggacatgctg gcgttgccgc tggacgaggg caacccaaca cctagcctaa agcccgtgac 20940
actgcagcag gtgctgcccg cgcttgcacc gtccgaagaa aagcgcggcc taaagcgcga 21000
gtctggtgac ttggcaccca ccgtgcagct gatggtaccc aagcgtcagc gactggaaga 21060
tgtcttggaa aaaatgaccg tggagcctgg gctggagccc gaggtccgcg tgcggccaat 21120
caagcaggtg gcaccgggac tgggcgtgca gaccgtggac gttcagatac ccaccaccag 21180
tagcactagt attgccactg ccacagaggg catggagaca caaacgtccc cggttgcctc 21240
ggcggtggca gatgccgcgg tgcaggcggc cgctgcggcc gcgtccaaga cctctacgga 21300
ggtgcaaacg gacccgtgga tgtttcgtgt ttcagccccc cggcgtccgc gccgttcaag 21360
gaagtacggc gccgccagcg cgctactgcc cgaatatgcc ctacatcctt ccatcgcgcc 21420
tacccccggc tatcgtggct acacctaccg ccccagaaga cgagcaacta cccgacgccg 21480
aaccaccact ggaacccgcc gccgccgtcg ccgtcgccag cccgtgctgg ccccgatttc 21540
cgtgcgcagg gtggctcgcg aaggaggcag gaccctggtg ctgccaacag cgcgctacca 21600
ccccagcatc gtttaaaagc cggtctttgt ggttcttgca gatatggccc tcacctgccg 21660
cctccgtttc ccggtgccgg gattccgagg aagaatgcac cgtaggaggg gcatggccgg 21720
ccacggcctg acgggcggca tgcgtcgtgc gcaccaccgg cggcggcgcg cgtcgcaccg 21780
tcgcatgcgc ggcggtatcc tgcccctcct tattccactg atcgccgcgg cgattggcgc 21840
cgtgcccgga attgcatccg tggccttgca ggcgcagaga cactgattaa aaacaagtta 21900

catgtggaaa aatcaaaata aaagtctgga ctctcacgct cgcttggtcc tgtaactatt 21960
ttgtagaatg gaagacatca actttgcgtc actggccccg cgacacggct cgcgcccgtt 22020
catgggaaac tggcaagata tcggcaccag caatatgagc ggtggcgcct tcagctgggg 22080
ctcgctgtgg agcggcatta aaaatttcgg ttccgccgtt aagaactatg gcagcaaagc 22140
ctggaacagc agcacaggcc agatgctgag ggacaagttg aaagagcaaa atttccaaca 22200
aaaggtggta gatggcctgg cctctggcat tagcggggtg gtggacctgg ccaaccaggc 22260
agtgcaaaat aagattaaca gtaagcttga tccccgccct cccgtagagg agcctccacc 22320
ggccgtggag acagtgtctc cagaggggcg tggcgaaaag cgtccgcgac ccgacaggga 22380
agaaactctg gtgacgcaaa tagacgagcc tccctcgtac gaggaggcac taaagcaagg 22440
cctgcccacc acccgtccca tcgcgcccat ggctaccgga gtgctgggcc agcacacacc 22500
cgtaacgctg gacctgcctc cccccgccga cacccagcag aaacctgtgc tgccaggccc 22560
gtccgccgtt gttgtaaccc gtcctagccg cgcgtccctg cgccgcgccg ccagcggtcc 22620
gcgatcgttg cggcccgtag ccagtggcaa ctggcaaagc acactgaaca gcatcgtggg 22680
tttgggggtg caatccctga agcgccgacg atgcttctga tagctaacgt gtcgtatgtg 22740
tgtcatgtat gcgtccatgt cgccgccaga ggagctgctg agccgccgcg cgcccgcttt 22800
ccaagatggc taccccttcg atgatgccgc agtggtctta catgcacatc tcgggccagg 22860
acgcctcgga gtacctgagc cccgggctgg tgcagttcgc ccgcgccacc gagacgtact 22920
tcagcctgaa taacaagttt agaaacccca cggtggcgcc tacgcacgac gtgaccacag 22980
accggtctca gcgtttgacg ctgcggttca tccccgtgga ccgcgaggat actgcgtact 23040
cgtacaaggc gcggttcacc ctagctgtgg gtgataaccg tgtgctagac atggcttcca 23100
cgtactttga catccgcggc gtgctggaca ggggccctac ttttaagccc tactctggca 23160
ctgcctacaa cgcactggcc cccaagggtg cccccaactc gtgcgagtgg gaacaaaatg 23220
aaactgcaca agtggatgct caagaacttg acgaagagga gaatgaagcc aatgaagctc 23280
aggcgcgaga acaggaacaa gctaagaaaa cccatgtata tgcccaggct ccactgtccg 23340
gaataaaaat aactaaagaa ggtctacaaa taggaactgc cgacgccaca gtagcaggtg 23400
ccggcaaaga aattttcgca gacaaaactt ttcaacctga accacaagta ggagaatctc 23460
aatggaacga agcggatgcc acagcagctg gtggaagggt tcttaaaaag acaactccca 23520
tgaaaccctg ctatggctca tacgctagac ccaccaattc caacggcgga cagggcgtta 23580
tggttgaaca aaatggtaaa ttggaaagtc aagtcgaaat gcaatttttt tccacatcca 23640
caaatgccac aaatgaagtt aacaatatac aaccaacagt tgtattgtac agcgaagatg 23700
taaacatgga aactccagat actcatcttt cttataaacc taaaatgggg gataaaaatg 23760
ccaaagtcat gcttggacaa caagcaatgc caaacagacc aaattacatt gcttttagag 23820
acaattttat tggtctcatg tattacaaca gcacaggtaa catgggtgtc cttgctggtc 23880
aggcatcgca gttgaacgct gttgtagatt tgcaagacag aaacacagag ctgtcctacc 23940
agcttttgct tgattcaatt ggcgacagaa caagatactt ttcaatgtgg aatcaagctg 24000
ttgacagcta tgatccagat gtcagaatta ttgagaacca tggaactgag gatgagttgc 24060
caaattattg ctttcctctt ggtggaattg ggattactga cacttttcaa gctgttaaaa 24120
caactgctgc taacggggac caaggcaata ctacctggca aaaagattca acatttgcag 24180
aacgcaatga aataggggtg ggaaataact ttgccatgga aattaacctg aatgccaacc 24240
tatggagaaa tttcctttac tccaatattg cgctgtacct gccagacaag ctaaaataca 24300
accccaccaa tgtggaaata tctgacaacc ccaacaccta cgactacatg aacaagcgag 24360
tggtggctcc tgggcttgta gactgctaca ttaaccttgg ggcgcgctgg tctctggact 24420
acatggacaa cgttaatccc tttaaccacc accgcaatgc gggcctgcgt taccgctcca 24480
tgttgttggg aaacggccgc tacgtgccct ttcacattca ggtgccccaa aagttttttg 24540
ccattaaaaa cctcctcctc ctgccaggct catacacata tgaatggaac ttcaggaagg 24600
atgttaacat ggttctgcag agctctctgg gaaacgacct tagagttgac ggggctagca 24660
ttaagtttga cagcatttgt ctttacgcca ccttcttccc catggcccac aacacggcct 24720
ccacgctgga agccatgctc agaaatgaca ccaacgacca gtcctttaat gactaccttt 24780
ccgccgccaa catgctatat cccatacccg ccaacgccac caacgtgccc atctccatcc 24840
catcgcgcaa ctgggcagca tttcgcggtt gggccttcac acgcttgaag acaaaggaaa 24900
ccccttccct gggatcaggc tacgaccctt actacaccta ctctggctcc ataccatacc 24960
ttgacggaac cttctatctt aatcacacct ttaagaaggt ggccattact tttgactctt 25020
ctgttagctg gccgggcaac gaccgcctgc ttactcccaa tgagtttgag attaagcgct 25080
cagttgacgg ggagggctat aacgtagctc agtgcaacat gacaaaggac tggttcctag 25140
tgcagatgtt ggccaactac aatattggct accagggctt ctacattcca gaaagctaca 25200
aagaccgcat gtactcgttc ttcagaaact tccagcccat gagccggcaa gtggtggacg 25260
atactaaata caaagattat cagcaggttg gaattatcca ccagcataac aactcaggct 25320
tcgtaggcta cctcgctccc accatgcgcg agggacaagc ttaccccgct aatgttccct 25380
acccactaat aggcaaaacc gcggttgata gtattaccca gaaaaagttt ctttgcgacc 25440
gcaccctgtg gcgcatcccc ttctccagta actttatgtc catgggtgcg ctcacagacc 25500
tgggccaaaa ccttctctac gcaaactccg cccacgcgct agacatgacc tttgaggtgg 25560
atcccatgga cgagcccacc cttctttatg ttttgtttga agtctttgac gtggtccgtg 25620
tgcaccagcc gcaccgcggc gtcatcgaga ccgtgtacct gcgcacgccc ttctcggccg 25680
gcaacgccac aacataaaga agcaagcaac atcaacaaca gctgccgcca tgggctccag 25740
tgagcaggaa ctgaaagcca ttgtcaaaga tcttggttgt gggccatatt ttttgggcac 25800
ctatgacaag cgcttcccag gctttgtttc cccacacaag ctcgcctgcg ccatagttaa 25860
cacggccggt cgcgagactg ggggcgtaca ctggatggcc tttgcctgga acccgcgctc 25920
aaaaacatgc tacctctttg agccctttgg cttttctgac caacgtctca agcaggttta 25980

ccagtttgag tacgagtcac tcctgcgccg tagcgccatt gcctcttccc ccgaccgctg 26040
tataacgctg gaaaagtcca cccaaagcgt gcaggggccc aactcggccg cctgtggcct 26100
attctgctgc atgtttctcc acgcctttgc caactggccc caaactccca tggatcacaa 26160
ccccaccatg aaccttatta ccggggtacc caactccatg cttaacagtc cccaggtaca 26220
gcccaccctg cgccgcaacc aggaacagct ctacagcttc ctggagcgcc actcgcccta 26280
cttccgcagc cacagtgcgc aaattaggag cgccacttct ttttgtcact tgaaaaacat 26340
gtaaaaataa tgtactagga gacactttca ataaaggcaa atgtttttat ttgtacactc 26400
tcgggtgatt atttaccccc acccttgccg tctgcgccgt ttaaaaatca aaggggttct 26460
gccgcgcatc gctatgcgcc actggcaggg acacgttgcg atactggtgt ttagtgctcc 26520
acttaaactc aggcacaacc atccgcggca gctcggtgaa gttttcactc cacaggctgc 26580
gcaccatcac caacgcgttt agcaggtcgg gcgccgatat cttgaagtcg cagttggggc 26640
ctccgccctg cgcgcgcgag ttgcgataca cagggttaca gcactggaac actatcagcg 26700
ccgggtggtg cacgctggcc agcacgctct tgtcggagat cagatccgcg tccaggtcct 26760
ccgcgttgct cagggcgaac ggagtcaact ttggtagctg ccttcccaaa aagggtgcat 26820
gcccaggctt tgagttgcac tcgcaccgta gtggcatcag aaggtgaccg tgcccagtct 26880
gggcgttagg atacagcgcc tgcatgaaag ccttgatctg cttaaaagcc acctgagcct 26940
ttgcgccttc agagaagaac atgccgcaag acttgccgga aaactgattg gccggacagg 27000
ccgcgtcatg cacgcagcac cttgcgtcgg tgttggagat ctgcaccaca tttcggcccc 27060
accggttctt cacgatcttg gccttgctag actgctcctt cagcgcgcgc tgcccgtttt 27120
cgctcgtcac atccatttca atcacgtgct ccttatttat cataatgctc ccgtgtagac 27180
acttaagctc gccttcgatc tcagcgcagc ggtgcagcca caacgcgcag cccgtgggct 27240
cgtggtgctt gtaggttacc tctgcaaacg actgcaggta cgcctgcagg aatcgcccca 27300
tcatcgtcac aaaggtcttg ttgctggtga aggtcagctg caacccgcgg tgctcctcgt 27360
ttagccaggt cttgcatacg gccgccagag cttccacttg gtcaggcagt agcttgaagt 27420
ttgcctttag atcgttatcc acgtggtact tgtccatcaa cgcgcgcgca gcctccatgc 27480
ccttctccca cgcagacacg atcggcaggc tcagcgggtt tatcaccgtg ctttcacttt 27540
ccgcttcact ggactcttcc ttttcctctt gcatccgcat accccgcgcc actgggtcgt 27600
cttcattcag ccgccgcacc gtgcgcttac ctcccttgcc gtgcttgatt agcaccggtg 27660
ggttgctgaa acccaccatt tgtagcgcca catcttctct ttcttcctcg ctgtccacga 27720
tcacctctgg ggatggcggg cgctcgggct tgggagaggg gcgcttcttt ttctttttgg 27780
acgcaatggc caaatccgcc gtcgaggtcg atggccgcgg gctgggtgtg cgcggcacca 27840
gcgcatcttg tgacgagtct tcttcgtcct cggactcgag acgccgcctc agccgctttt 27900
ttgggggcgc gcggggaggc ggcggcgacg gcgacgggga cgagacgtcc tccatggttg 27960
gtggacgtcg cgccgcaccg cgtccgcgct cgggggtggt ttcgcgctgc tcctcttccc 28020
gactggccat ttccttctcc tataggcaga aaaagatcat ggagtcagtc gagaaggagg 28080
acagcctaac cgcccccttt gagttcgcca ccaccgcctc caccgatgcc gccaacgcgc 28140
ctaccacctt ccccgtcgag gcacccccgc ttgaggagga ggaagtgatt atcgagcagg 28200
acccaggttt tgtaagcgaa gacgacgaag atcgctcagt accaacagag gataaaaagc 28260
aagaccagga cgacgcagag gcaaacgagg aacaagtcgg gcggggggac caaaggcatg 28320
gcgactacct agatgtggga gacgacgtgc tgttgaagca tctgcagcgc cagtgcgcca 28380
ttatctgcga cgcgttgcaa gagcgcagcg atgtgcccct cgccatagcg gatgtcagcc 28440
ttgcctacga acgccacctg ttctcaccgc gcgtaccccc caaacgccaa gaaaacggca 28500
catgcgagcc caacccgcgc ctcaacttct accccgtatt tgccgtgcca gaggtgcttg 28560
ccacctatca catctttttc caaaactgca agatacccct atcctgccgt gccaaccgca 28620
gccgagcgga caagcagctg gccttgcggc agggcgctgt catacctgat atcgcctcgc 28680
tcgacgaagt gccaaaaatc tttgagggtc ttggacgcga cgagaagcgc gcggcaaacg 28740
ctctgcaaca agaaaacagc gaaaatgaaa gtcactgtgg agtgctggtg gaacttgagg 28800
gtgacaacgc gcgcctagcc gtgctgaaac gcagcatcga ggtcacccac tttgcctacc 28860
cggcacttaa cctacccccc aaggttatga gcacagtcat gagcgagctg atcgtgcgcc 28920
gtgcacgacc cctggagagg gatgcaaact tgcaagaaca aaccgaggag ggcctacccg 28980
cagttggcga tgagcagctg gcgcgctggc ttgagacgcg cgagcctgcc gacttggagg 29040
agcgacgcaa gctaatgatg gccgcagtgc ttgttaccgt ggagcttgag tgcatgcagc 29100
ggttctttgc tgacccggag atgcagcgca agctagagga aacgttgcac tacacctttc 29160
gccagggcta cgtgcgccag gcctgcaaaa tttccaacgt ggagctctgc aacctggtct 29220
cctaccttgg aattttgcac gaaaaccgcc ttgggcaaaa cgtgcttcat tccacgctca 29280
agggcgaggc gcgccgcgac tacgtccgcg actgcgttta cttatttctg tgctacacct 29340
ggcaaacggc catgggcgtg tggcagcagt gcctggagga gcgcaacctg aaggagctgc 29400
agaagctgct aaagcaaaac ttgaaggacc tatggacggc cttcaacgag cgctccgtgg 29460
ccgcgcacct ggcggacatt atcttccccg aacgcctgct taaaaccctg caacagggtc 29520
tgccagactt caccagtcaa agcatgttgc aaaactttag gaactttatc ctagagcgtt 29580
caggaattct gcccgccacc tgctgtgcgc ttcctagcga ctttgtgccc attaagtacc 29640
gtgaatgccc tccgccgctt tggggtcact gctaccttct gcagctagcc aactaccttg 29700
cctaccactc cgacatcatg gaagacgtga gcggtgacgg cctactggag tgtcactgtc 29760
gctgcaacct atgcaccccg caccgctccc tggtctgcaa ttcacaactg cttagcgaaa 29820
gtcaaattat cggtaccttt gagctgcagg gtccctcgcc tgacgaaaag tccgcggctc 29880
cggggttgaa actcactccg gggctgtgga cgtcggctta ccttcgcaaa tttgtacctg 29940
aggactacca cgcccacgag attaggttct acgaagacca atcccgcccg ccaaatgcgg 30000
agcttaccgc ctgcgtcatt acccagggcc acatccttgg ccaattgcaa gccattaaca 30060

aagcccgcca agagtttctg ctacgaaagg gacggggggt ttacttggac ccccagtccg 30120
gcgaggagct caacccaatc cccccgccgc cgcagcccta tcagcagccg cgggcccttg 30180
cttcccagga tggcacccaa aaagaagctg cagctgccgc cgccgccacc cacggacgag 30240
gaggaatact gggacagtca ggcagaggag gttttggacg aggaggagga gatgatggaa 30300
gactgggaca gcctagacga ggaagcttcc gaggccgaag aggtgtcaga cgaaacaccg 30360
tcaccctcgg tcgcattccc ctcgccggcg ccccagaaat cggcaaccgt tcccagcatt 30420
gctacaacct ccgctcctca ggcgccgccg gcactgcccg ttcgccgacc caaccgtaga 30480
tgggacacca ctggaaccag ggccggtaag tctaagcagc cgccgccgtt agcccaagag 30540
caacaacagc gccaaggcta ccgctcgtgg cgcgtgcaca agaacgccat agttgcttgc 30600
ttgcaagact gtgggggcaa catctccttc gcccgccgct ttcttctcta ccatcacggc 30660
gtggccttcc cccgtaacat cctgcattac taccgtcatc tctacagccc ctactgcacc 30720
ggcggcagcg gcagcaacag cagcggccac gcagaagcaa aggcgaccgg atagcaagac 30780
tctgacaaag cccaagaaat ccacagcggc ggcagcagca ggaggaggag cactgcgtct 30840
ggcgcccaac gaacccgtat cgacccgcga gcttagaaac aggatttttc ccactctgta 30900
tgctatattt caacagagca ggggccaaga acaagagctg aaaataaaaa acaggtctct 30960
gcgctccctc acccgcagct gcctgtatca caaaagcgaa gatcagcttc ggcgcacgct 31020
ggaagacgcg gaggctctct tcagcaaata ctgcgcgctg actcttaagg actagtttcg 31080
cgccctttct caaatttaag cgcgaaaact acgtcatctc cagcggccac acccggcgcc 31140
agcacctgtc gtcagcgcca ttatgagcaa ggaaattccc acgccctaca tgtggagtta 31200
ccagccacaa atgggacttg cggctggagc tgcccaagac tactcaaccc gaataaacta 31260
catgagcgcg ggaccccaca tgatatcccg ggtcaacgga atccgcgccc accgaaaccg 31320
aattctcctc gaacaggcgg ctattaccac cacacctcgt aataacctta atccccgtag 31380
ttggcccgct gccctggtgt accaggaaag tcccgctccc accactgtgg tacttcccag 31440
agacgcccag gccgaagttc agatgactaa ctcaggggcg cagcttgcgg gcggctttcg 31500
tcacagggtg cggtcgcccg ggcagggtat aactcacctg aaaatcagag ggcgaggtat 31560
tcagctcaac gacgagtcgg tgagctcctc tcttggtctc cgtccggacg ggacatttca 31620
gatcggcggc gctggccgct cttcatttac gccccgtcag gcgatcctaa ctctgcagac 31680
ctcgtcctcg gagccgcgct ccggaggcat tggaactcta caatttattg aggagttcgt 31740
gccttcggtt tacttcaacc ccttttctgg acctcccggc cactacccgg accagtttat 31800
tcccaacttt gacgcggtaa aagactcggc ggacggctac gactgaatga ccagtggaga 31860
ggcagagcaa ctgcgcctga cacacctcga ccactgccgc cgccacaagt gctttgcccg 31920
cggctccggt gagttttgtt actttgaatt gcccgaagag catatcgagg gcccggcgca 31980
cggcgtccgg ctcaccaccc aggtagagct tacacgtagc ctgattcggg agtttaccaa 32040
gcgccccctg ctagtggagc gggagcgggg tccctgtgtt ctgaccgtgg tttgcaactg 32100
tcctaaccct ggattacatc aagatcttat tccattcaac taacaataaa cacacaataa 32160
attacttact taaaatcagt cagcaaatct ttgtccagct tattcagcat cacctccttt 32220
ccctcctccc aactctggta tttcagcagc cttttagctg cgaactttct ccaaagtcta 32280
aatgggatgt caaattcctc atgttcttgt ccctccgcac ccactatctt catattgttg 32340
cagatgaaac gcgccagacc gtctgaagac accttcaacc ctgtgtaccc atatgacacg 32400
gaaaccggcc ctccaactgt gcctttcctt acccctccct ttgtgtcgcc aaatgggttc 32460
caagaaagtc cccccggagt gctttctttg cgtctttcag aacctttggt tacctcacac 32520
ggcatgcttg cgctaaaaat gggcagcggc ctgtccctgg atcaggcagg caaccttaca 32580
tcaaatacaa tcactgtttc tcaaccgcta aaaaaaacaa agtccaatat aactttggaa 32640
acatccgcgc cccttacagt cagctcaggc gccctaacca tggccacaac ttcgcctttg 32700
gtggtctctg acaacactct taccatgcaa tcacaagcac cgctaaccgt gcaagactca 32760
aaacttagca ttgctaccaa agagccactt acagtgttag atggaaaact ggccctgcag 32820
acatcagccc ccctctctgc cactgataac aacgccctca ctatcactgc ctcacctcct 32880
cttactactg caaatggtag tctggctgtt accatggaaa acccacttta caacaacaat 32940
ggaaaacttg ggctcaaaat tggcggtcct ttgcaagtgg ccaccgactc acatgcacta 33000
acactaggta ctggtcaggg ggttgcagtt cataacaatt tgctacatac aaaagttaca 33060
ggcgcaatag ggtttgatac atctggcaac atggaactta aaactggaga tggcctctat 33120
gtggatagcg ccggtcctaa ccaaaaacta catattaatc taaataccac aaaaggcctt 33180
gcttttgaca acaccgcaat aacaattaac gctggaaaag ggttggaatt tgaaacagac 33240
tcctcaaacg gaaatcccat aaaaacaaaa attggatcag gcatacaata taataccaat 33300
ggagctatgg ttgcaaaact tggaacaggc ctcagttttg acagctccgg agccataaca 33360
atgggcagca taaacaatga cagacttact ctttggacaa caccagaccc atccccaaat 33420
tgcagaattg cttcagataa agactgcaag ctaactctgg cgctaacaaa atgtggcagt 33480
caaattttgg gcactgtttc agctttggca gtatcaggta atatggcctc catcaatgga 33540
actctaagca gtgtaaactt ggttcttaga tttgatgaca acggagtgct tatgtcaaat 33600
tcatcactgg acaaacagta ttggaacttt agaaacgggg actccactaa cggtcaacca 33660
tacacttatg ctgttgggtt tatgccaaac ctaaaagctt acccaaaaac tcaaagtaaa 33720
actgcaaaaa gtaatattgt tagccaggtg tatcttaatg gtgacaagtc taaaccattg 33780
cattttacta ttacgctaaa tggaacagat gaaaccaacc aagtaagcaa atactcaata 33840
tcattcagtt ggtcctggaa cagtggacaa tacactaatg acaaatttgc caccaattcc 33900
tataccttct cctacattgc ccaggaataa agaatcgtga acctgttgca tgttatgttt 33960
caacgtgttt atttttcaat tgcagaaaat ttcaagtcat ttttcattca gtagtatagc 34020
cccaccacca catagcttat actaatcacc gtaccttaat caaactcaca gaaccctagt 34080
attcaacctg ccacctccct cccaacacac agagtacaca gtcctttctc cccggctggc 34140

cttaaacagc atcatatcat gggtaacaga catattctta ggtgttatat tccacacggt 34200
ctcctgtcga gccaaacgct catcagtgat gttaataaac tccccgggca gctcgcttaa 34260
gttcatgtcg ctgtccagct gctgagccac aggctgctgt ccaacttgcg gttgctcaac 34320
gggcggcgaa ggagaagtcc acgcctacat gggggtagag tcataatcgt gcatcaggat 34380
agggcggtgg tgctgcagca gcgcgcgaat aaactgctgc cgccgccgct ccgtcctgca 34440
ggaatacaac atggcagtgg tctcctcagc gatgattcgc accgcccgca gcataaggcg 34500
ccttgtcctc cgggcacagc agcgcaccct gatctcactt aagtcagcac agtaactgca 34560
gcacagtacc acaatattgt ttaaaatccc acagtgcaag gcgctgtatc caaagctcat 34620
ggcggggacc acagaaccca cgtggccatc ataccacaag cgcaggtaga ttaagtggcg 34680
acccctcata aacacgctgg acataaacat tacctctttt ggcatgttgt aattcaccac 34740
ctcccggtac catataaacc tctgattaaa catggcgcca tccaccacca tcctaaacca 34800
gctggccaaa acctgcccgc cggctatgca ctgcagggaa ccgggactgg aacaatgaca 34860
gtggagagcc caggactcgt aaccatggat catcatgctc gtcatgatat caatgttggc 34920
acaacacagg cacacgtgca tacacttcct caggattaca agctcctccc gcgtcagaac 34980
catatcccag ggaacaaccc attcctgaat cagcgtaaat cccacactgc agggaagacc 35040
tcgcacgtaa ctcacgttgt gcattgtcaa agtgttacat tcgggcagca gcggatgatc 35100
ctccagtatg gtagcgcggg tttctgtctc aaaaggaggt agacgatccc tactgtacgg 35160
agtgcgccga gacaaccgag atcgtgttgg tcgtagtgtc atgccaaatg gaacgccgga 35220
cgtagtcata tttcctgaag caaaaccagg tgcgggcgtg acaaacagat ctgcgtctcc 35280
ggtctcgccg cttagatcgc tctgtgtagt agttgtagta tatccactct ctcaaagcat 35340
ccaggcgccc cctggcttcg ggttctatgt aaactccttc atgcgccgct gccctgataa 35400
catccaccac cgcagaataa gccacaccca gccaacctac acattcgttc tgcgagtcac 35460
acacgggagg agcgggaaga gctggaagaa ccatgttttt tttttattc caaaagatta 35520
tccaaaacct caaaatgaag atctattaag tgaacgcgct cccctccggt ggcgtggtca 35580
aactctacag ccaaagaaca gataatggca tttgtaagat gttgcacaat ggcttccaaa 35640
aggcaaacgg ccctcacgtc caagtggacg taaaggctaa acccttcagg gtgaatctcc 35700
tctataaaca ttccagcacc ttcaaccatg cccaaataat tctcatctcg ccaccttctc 35760
aatatatctc taagcaaatc ccgaatatta agtccggcca ttgtaaaaat ctgctccaga 35820
gcgccctcca ccttcagcct caagcagcga atcatgattg caaaaattca ggttcctcac 35880
agacctgtat aagattcaaa agcggaacat taacaaaaat accgcgatcc cgtaggtccc 35940
ttcgcagggc cagctgaaca taatcgtgca ggtctgcacg gaccagcgcg gccacttccc 36000
cgccaggaac catgacaaaa gaacccacac tgattatgac acgcatactc ggagctatgc 36060
taaccagcgt agccccgatg taagcttgtt gcatgggcgg cgatataaaa tgcaaggtgc 36120
tgctcaaaaa atcaggcaaa gcctcgcgca aaaaagaaag cacatcgtag tcatgctcat 36180
gcagataaag gcaggtaagc tccggaacca ccacagaaaa agacaccatt tttctctcaa 36240
acatgtctgc gggtttctgc ataaacacaa aataaaataa caaaaaaaca tttaaacatt 36300
agaagcctgt cttacaacag gaaaaacaac ccttataagc ataagacgga ctacggccat 36360
gccggcgtga ccgtaaaaaa actggtcacc gtgattaaaa agcaccaccg acagctcctc 36420
ggtcatgtcc ggagtcataa tgtaagactc ggtaaacaca tcaggttgat tcacatcggt 36480
cagtgctaaa aagcgaccga aatagcccgg gggaatacat acccgcaggc gtagagacaa 36540
cattacagcc cccataggag gtataacaaa attaatagga gagaaaaaca cataaacacc 36600
tgaaaaaccc tcctgcctag gcaaaatagc accctcccgc tccagaacaa catacagcgc 36660
ttccacagcg gcagccataa cagtcagcct taccagtaaa aaagaaaacc tattaaaaaa 36720
acaccactcg acacggcacc agctcaatca gtcacagtgt aaaaaagggc caagtgcaga 36780
gcgagtatat ataggactaa aaaatgacgt aacggttaaa gtccacaaaa aacacccaga 36840
aaaccgcacg cgaacctacg cccagaaacg aaagccaaaa aacccacaac ttcctcaaat 36900
cgtcacttcc gttttcccac gttacgtcac ttcccatttt aagaaaacta caattcccaa 36960
cacatacaag ttactccgcc ctaaaaccta cgtcacccgc cccgttccca cgccccgcgc 37020
cacgtcacaa actccacccc ctcattatca tattggcttc aatccaaaat aaggtatatt 37080
attgatgatg 37090

<210> 5
<211> 5955
<212> DNA
<213> Artificial Sequence

<220>
<223> NS cDNA sequence

<220>
<221> CDS
<222> (1)...(5955)

<400> 5
atg gcg ccc atc acg gcc tac tcc caa cag acg cgg ggc cta ctt ggt 48
Met Ala Pro Ile Thr Ala Tyr Ser Gln Gln Thr Arg Gly Leu Leu Gly

1                 5                   10                  15

tgc atc atc act agc ctt aca ggc cgg gac aag aac cag gtc gag gga    96
Cys Ile Ile Thr Ser Leu Thr Gly Arg Asp Lys Asn Gln Val Glu Gly
                20                  25                  30

gag gtt cag gtg gtt tcc acc gca aca caa tcc ttc ctg gcg acc tgc    144
Glu Val Gln Val Val Ser Thr Ala Thr Gln Ser Phe Leu Ala Thr Cys
            35                  40                  45

gtc aac ggc gtg tgt tgg acc gtt tac cat ggt gct ggc tca aag acc    192
Val Asn Gly Val Cys Trp Thr Val Tyr His Gly Ala Gly Ser Lys Thr
    50                  55                  60

tta gcc ggc cca aag ggg cca atc acc cag atg tac act aat gtg gac    240
Leu Ala Gly Pro Lys Gly Pro Ile Thr Gln Met Tyr Thr Asn Val Asp
65                  70                  75                  80

cag gac ctc gtc ggc tgg cag gcg ccc ccc ggg gcg cgt tcc ttg aca    288
Gln Asp Leu Val Gly Trp Gln Ala Pro Pro Gly Ala Arg Ser Leu Thr
                85                  90                  95

cca tgc acc tgt ggc agc tca gac ctt tac ttg gtc acg aga cat gct    336
Pro Cys Thr Cys Gly Ser Ser Asp Leu Tyr Leu Val Thr Arg His Ala
            100                 105                 110

gac gtc att ccg gtg cgc cgg cgg ggc gac agt agg ggg agc ctg ctc    384
Asp Val Ile Pro Val Arg Arg Arg Gly Asp Ser Arg Gly Ser Leu Leu
        115                 120                 125

tcc ccc agg cct gtc tcc tac ttg aag ggc tct tcg ggt ggt cca ctg    432
Ser Pro Arg Pro Val Ser Tyr Leu Lys Gly Ser Ser Gly Gly Pro Leu
    130                 135                 140

ctc tgc cct tcg ggg cac gct gtg ggc atc ttc cgg gct gcc gta tgc    480
Leu Cys Pro Ser Gly His Ala Val Gly Ile Phe Arg Ala Ala Val Cys
145                 150                 155                 160

acc cgg ggg gtt gcg aag gcg gtg gac ttt gtg ccc gta gag tcc atg    528
Thr Arg Gly Val Ala Lys Ala Val Asp Phe Val Pro Val Glu Ser Met
                165                 170                 175

gaa act act atg cgg tct ccg gtc ttc acg gac aac tca tcc ccc ccg    576
Glu Thr Thr Met Arg Ser Pro Val Phe Thr Asp Asn Ser Ser Pro Pro
            180                 185                 190

gcc gta ccg cag tca ttt caa gtg gcc cac cta cac gct ccc act ggc    624
Ala Val Pro Gln Ser Phe Gln Val Ala His Leu His Ala Pro Thr Gly
        195                 200                 205

agc ggc aag agt act aaa gtg ccg gct gca tat gca gcc caa ggg tac    672
Ser Gly Lys Ser Thr Lys Val Pro Ala Ala Tyr Ala Ala Gln Gly Tyr
    210                 215                 220

aag gtg ctc gtc ctc aat ccg tcc gtt gcc gct acc tta ggg ttt ggg    720
Lys Val Leu Val Leu Asn Pro Ser Val Ala Ala Thr Leu Gly Phe Gly
225                 230                 235                 240

gcg tat atg tct aag gca cac ggt att gac ccc aac atc aga act ggg    768
Ala Tyr Met Ser Lys Ala His Gly Ile Asp Pro Asn Ile Arg Thr Gly
                245                 250                 255

gta agg acc att acc aca ggc gcc ccc gtc aca tac tct acc tat ggc    816
Val Arg Thr Ile Thr Thr Gly Ala Pro Val Thr Tyr Ser Thr Tyr Gly
            260                 265                 270

aag ttt ctt gcc gat ggt ggt tgc tct ggg ggc gct tat gac atc ata    864
Lys Phe Leu Ala Asp Gly Gly Cys Ser Gly Gly Ala Tyr Asp Ile Ile

        275                 280                 285

ata tgt gat gag tgc cat tca act gac tcg act aca atc ttg ggc atc 912
Ile Cys Asp Glu Cys His Ser Thr Asp Ser Thr Thr Ile Leu Gly Ile
    290                 295                 300

ggc aca gtc ctg gac caa gcg gag acg gct gga gcg cgg ctt gtc gtg 960
Gly Thr Val Leu Asp Gln Ala Glu Thr Ala Gly Ala Arg Leu Val Val
305                 310                 315                 320

ctc gcc acc gct acg cct ccg gga tcg gtc acc gtg cca cac cca aac 1008
Leu Ala Thr Ala Thr Pro Pro Gly Ser Val Thr Val Pro His Pro Asn
                325                 330                 335

atc gag gag gtg gcc ctg tct aat act gga gag atc ccc ttc tat ggc 1056
Ile Glu Glu Val Ala Leu Ser Asn Thr Gly Glu Ile Pro Phe Tyr Gly
            340                 345                 350

aaa gcc atc ccc att gaa gcc atc agg ggg gga agg cat ctc att ttc 1104
Lys Ala Ile Pro Ile Glu Ala Ile Arg Gly Gly Arg His Leu Ile Phe
        355                 360                 365

tgt cat tcc aag aag aag tgc gac gag ctc gcc gca aag ctg tca ggc 1152
Cys His Ser Lys Lys Lys Cys Asp Glu Leu Ala Ala Lys Leu Ser Gly
    370                 375                 380

ctc gga atc aac gct gtg gcg tat tac cgg ggg ctc gat gtg tcc gtc 1200
Leu Gly Ile Asn Ala Val Ala Tyr Tyr Arg Gly Leu Asp Val Ser Val
385                 390                 395                 400

ata cca act atc gga gac gtc gtt gtc gtg gca aca gac gct ctg atg 1248
Ile Pro Thr Ile Gly Asp Val Val Val Val Ala Thr Asp Ala Leu Met
                405                 410                 415

acg ggc tat acg ggc gac ttt gac tca gtg atc gac tgt aac aca tgt 1296
Thr Gly Tyr Thr Gly Asp Phe Asp Ser Val Ile Asp Cys Asn Thr Cys
            420                 425                 430

gtc acc cag aca gtc gac ttc agc ttg gat ccc acc ttc acc att gag 1344
Val Thr Gln Thr Val Asp Phe Ser Leu Asp Pro Thr Phe Thr Ile Glu
        435                 440                 445

acg acg acc gtg cct caa gac gca gtg tcg cgc tcg cag cgg cgg ggt 1392
Thr Thr Thr Val Pro Gln Asp Ala Val Ser Arg Ser Gln Arg Arg Gly
    450                 455                 460

agg act ggc agg ggt agg aga ggc atc tac agg ttt gtg act ccg gga 1440
Arg Thr Gly Arg Gly Arg Arg Gly Ile Tyr Arg Phe Val Thr Pro Gly
465                 470                 475                 480

gaa cgg ccc tcg ggc atg ttc gat tcc tcg gtc ctg tgt gag tgc tat 1488
Glu Arg Pro Ser Gly Met Phe Asp Ser Ser Val Leu Cys Glu Cys Tyr
                485                 490                 495

gac gcg ggc tgt gct tgg tac gag ctc acc ccc gcc gag acc tcg gtt 1536
Asp Ala Gly Cys Ala Trp Tyr Glu Leu Thr Pro Ala Glu Thr Ser Val
            500                 505                 510

agg ttg cgg gcc tac ctg aac aca cca ggg ttg ccc gtt tgc cag gac 1584
Arg Leu Arg Ala Tyr Leu Asn Thr Pro Gly Leu Pro Val Cys Gln Asp
        515                 520                 525

cac ctg gag ttc tgg gag agt gtc ttc aca ggc ctc acc cac ata gat 1632
His Leu Glu Phe Trp Glu Ser Val Phe Thr Gly Leu Thr His Ile Asp
    530                 535                 540

gca cac ttc ttg tcc cag acc aag cag gca gga gac aac ttc ccc tac 1680
Ala His Phe Leu Ser Gln Thr Lys Gln Ala Gly Asp Asn Phe Pro Tyr

545                 550                 555                 560

ctg gta gca tac caa gcc acg gtg tgc gcc agg gct cag gcc cca cct     1728
Leu Val Ala Tyr Gln Ala Thr Val Cys Ala Arg Ala Gln Ala Pro Pro
                565                 570                 575

cca tca tgg gat caa atg tgg aag tgt ctc ata cgg ctg aaa cct acg     1776
Pro Ser Trp Asp Gln Met Trp Lys Cys Leu Ile Arg Leu Lys Pro Thr
            580                 585                 590

ctg cac ggg cca aca ccc ttg ctg tac agg ctg gga gcc gtc caa aat     1824
Leu His Gly Pro Thr Pro Leu Leu Tyr Arg Leu Gly Ala Val Gln Asn
        595                 600                 605

gag gtc acc ctc acc cac ccc ata acc aaa tac atc atg gca tgc atg     1872
Glu Val Thr Leu Thr His Pro Ile Thr Lys Tyr Ile Met Ala Cys Met
    610                 615                 620

tcg gct gac ctg gag gtc gtc act agc acc tgg gtg ctg gtg ggc gga     1920
Ser Ala Asp Leu Glu Val Val Thr Ser Thr Trp Val Leu Val Gly Gly
625                 630                 635                 640

gtc ctt gca gct ctg gcc gcg tat tgc ctg aca aca ggc agt gtg gtc     1968
Val Leu Ala Ala Leu Ala Ala Tyr Cys Leu Thr Thr Gly Ser Val Val
                645                 650                 655

att gtg ggt agg att atc ttg tcc ggg agg ccg gct att gtt ccc gac     2016
Ile Val Gly Arg Ile Ile Leu Ser Gly Arg Pro Ala Ile Val Pro Asp
            660                 665                 670

agg gag ttt ctc tac cag gag ttc gat gaa atg gaa gag tgc gcc tcg     2064
Arg Glu Phe Leu Tyr Gln Glu Phe Asp Glu Met Glu Glu Cys Ala Ser
        675                 680                 685

cac ctc cct tac atc gag cag gga atg cag ctc gcc gag caa ttc aag     2112
His Leu Pro Tyr Ile Glu Gln Gly Met Gln Leu Ala Glu Gln Phe Lys
    690                 695                 700

cag aaa gcg ctc ggg tta ctg caa aca gcc acc aaa caa gcg gag gct     2160
Gln Lys Ala Leu Gly Leu Leu Gln Thr Ala Thr Lys Gln Ala Glu Ala
705                 710                 715                 720

gct gct ccc gtg gtg gag tcc aag tgg cga gcc ctt gag aca ttc tgg     2208
Ala Ala Pro Val Val Glu Ser Lys Trp Arg Ala Leu Glu Thr Phe Trp
                725                 730                 735

gcg aag cac atg tgg aat ttc atc agc ggg ata cag tac tta gca ggc     2256
Ala Lys His Met Trp Asn Phe Ile Ser Gly Ile Gln Tyr Leu Ala Gly
            740                 745                 750

tta tcc act ctg cct ggg aac ccc gca ata gca tca ttg atg gca ttc     2304
Leu Ser Thr Leu Pro Gly Asn Pro Ala Ile Ala Ser Leu Met Ala Phe
        755                 760                 765

aca gcc tct atc acc agc ccg ctc acc acc caa agt acc ctc ctg ttt     2352
Thr Ala Ser Ile Thr Ser Pro Leu Thr Thr Gln Ser Thr Leu Leu Phe
    770                 775                 780

aac atc ttg ggg ggg tgg gtg gct gcc caa ctc gcc ccc ccc agc gcc     2400
Asn Ile Leu Gly Gly Trp Val Ala Ala Gln Leu Ala Pro Pro Ser Ala
785                 790                 795                 800

gct tcg gct ttc gtg ggc gcc ggc atc gcc ggt gcg gct gtt ggc agc     2448
Ala Ser Ala Phe Val Gly Ala Gly Ile Ala Gly Ala Ala Val Gly Ser
                805                 810                 815

ata ggc ctt ggg aag gtg ctt gtg gac att ctg gcg ggt tat gga gca     2496
Ile Gly Leu Gly Lys Val Leu Val Asp Ile Leu Ala Gly Tyr Gly Ala

            820                 825                 830

gga gtg gcc ggc gcg ctc gtg gcc ttc aag gtc atg agc ggc gag atg 2544
Gly Val Ala Gly Ala Leu Val Ala Phe Lys Val Met Ser Gly Glu Met
        835                 840                 845

ccc tcc acc gag gac ctg gtc aat cta ctt cct gcc atc ctc tct cct 2592
Pro Ser Thr Glu Asp Leu Val Asn Leu Leu Pro Ala Ile Leu Ser Pro
    850                 855                 860

ggc gcc ctg gtc gtc ggg gtc gtg tgt gca gca ata ctg cgt cga cac 2640
Gly Ala Leu Val Val Gly Val Val Cys Ala Ala Ile Leu Arg Arg His
865                 870                 875                 880

gtg ggt ccg gga gag ggg gct gtg cag tgg atg aac cgg ctg ata gcg 2688
Val Gly Pro Gly Glu Gly Ala Val Gln Trp Met Asn Arg Leu Ile Ala
                885                 890                 895

ttc gcc tcg cgg ggt aat cat gtt tcc ccc acg cac tat gtg cct gag 2736
Phe Ala Ser Arg Gly Asn His Val Ser Pro Thr His Tyr Val Pro Glu
            900                 905                 910

agc gac gcc gca gcg cgt gtt act cag atc ctc tcc agc ctt acc atc 2784
Ser Asp Ala Ala Ala Arg Val Thr Gln Ile Leu Ser Ser Leu Thr Ile
        915                 920                 925

act cag ctg ctg aaa agg ctc cac cag tgg att aat gaa gac tgc tcc 2832
Thr Gln Leu Leu Lys Arg Leu His Gln Trp Ile Asn Glu Asp Cys Ser
    930                 935                 940

aca ccg tgt tcc ggc tcg tgg cta agg gat gtt tgg gac tgg ata tgc 2880
Thr Pro Cys Ser Gly Ser Trp Leu Arg Asp Val Trp Asp Trp Ile Cys
945                 950                 955                 960

acg gtg ttg act gac ttc aag acc tgg ctc cag tcc aag ctc ctg ccg 2928
Thr Val Leu Thr Asp Phe Lys Thr Trp Leu Gln Ser Lys Leu Leu Pro
                965                 970                 975

cag cta ccg gga gtc cct ttt ttc tcg tgc caa cgc ggg tac aag gga 2976
Gln Leu Pro Gly Val Pro Phe Phe Ser Cys Gln Arg Gly Tyr Lys Gly
            980                 985                 990

gtc tgg cgg gga gac ggc atc atg caa acc acc tgc cca tgt gga gca 3024
Val Trp Arg Gly Asp Gly Ile Met Gln Thr Thr Cys Pro Cys Gly Ala
        995                 1000                1005

cag atc acc gga cat gtc aaa aac ggt tcc atg agg atc gtc ggg cct 3072
Gln Ile Thr Gly His Val Lys Asn Gly Ser Met Arg Ile Val Gly Pro
    1010                1015                1020

aag acc tgc agc aac acg tgg cat gga aca ttc ccc atc aac gca tac 3120
Lys Thr Cys Ser Asn Thr Trp His Gly Thr Phe Pro Ile Asn Ala Tyr
1025                1030                1035                1040

acc acg ggc ccc tgc aca ccc tct cca gcg cca aac tat tct agg gcg 3168
Thr Thr Gly Pro Cys Thr Pro Ser Pro Ala Pro Asn Tyr Ser Arg Ala
                1045                1050                1055

ctg tgg cgg gtg gcc gct gag gag tac gtg gag gtc acg cgg gtg ggg 3216
Leu Trp Arg Val Ala Ala Glu Glu Tyr Val Glu Val Thr Arg Val Gly
            1060                1065                1070

gat ttc cac tac gtg acg ggc atg acc act gac aac gta aag tgc cca 3264
Asp Phe His Tyr Val Thr Gly Met Thr Thr Asp Asn Val Lys Cys Pro
        1075                1080                1085

tgc cag gtt ccg gct cct gaa ttc ttc acg gag gtg gac gga gtg cgg 3312
Cys Gln Val Pro Ala Pro Glu Phe Phe Thr Glu Val Asp Gly Val Arg

1090                1095                1100

ttg cac agg tac gct ccg gcg tgc agg cct ctc cta cgg gag gag gtt     3360
Leu His Arg Tyr Ala Pro Ala Cys Arg Pro Leu Leu Arg Glu Glu Val
1105                1110                1115                1120

aca ttc cag gtc ggg ctc aac caa tac ctg gtt ggg tca cag cta cca     3408
Thr Phe Gln Val Gly Leu Asn Gln Tyr Leu Val Gly Ser Gln Leu Pro
                1125                1130                1135

tgc gag ccc gaa ccg gat gta gca gtg ctc act tcc atg ctc acc gac     3456
Cys Glu Pro Glu Pro Asp Val Ala Val Leu Thr Ser Met Leu Thr Asp
            1140                1145                1150

ccc tcc cac atc aca gca gaa acg gct aag cgt agg ttg gcc agg ggg     3504
Pro Ser His Ile Thr Ala Glu Thr Ala Lys Arg Arg Leu Ala Arg Gly
        1155                1160                1165

tct ccc ccc tcc ttg gcc agc tct tca gct agc cag ttg tct gcg cct     3552
Ser Pro Pro Ser Leu Ala Ser Ser Ser Ala Ser Gln Leu Ser Ala Pro
    1170                1175                1180

tcc ttg aag gcg aca tgc act acc cac cat gtc tct ccg gac gct gac     3600
Ser Leu Lys Ala Thr Cys Thr Thr His His Val Ser Pro Asp Ala Asp
1185                1190                1195                1200

ctc atc gag gcc aac ctc ctg tgg cgg cag gag atg ggc ggg aac atc     3648
Leu Ile Glu Ala Asn Leu Leu Trp Arg Gln Glu Met Gly Gly Asn Ile
                1205                1210                1215

acc cgc gtg gag tcg gag aac aag gtg gta gtc ctg gac tct ttc gac     3696
Thr Arg Val Glu Ser Glu Asn Lys Val Val Val Leu Asp Ser Phe Asp
            1220                1225                1230

ccg ctt cga gcg gag gag gat gag agg gaa gta tcc gtt ccg gcg gag     3744
Pro Leu Arg Ala Glu Glu Asp Glu Arg Glu Val Ser Val Pro Ala Glu
        1235                1240                1245

atc ctg cgg aaa tcc aag aag ttc ccc gca gcg atg ccc atc tgg gcg     3792
Ile Leu Arg Lys Ser Lys Lys Phe Pro Ala Ala Met Pro Ile Trp Ala
    1250                1255                1260

cgc ccg gat tac aac cct cca ctg tta gag tcc tgg aag gac ccg gac     3840
Arg Pro Asp Tyr Asn Pro Pro Leu Leu Glu Ser Trp Lys Asp Pro Asp
1265                1270                1275                1280

tac gtc cct ccg gtg gtg cac ggg tgc ccg ttg cca cct atc aag gcc     3888
Tyr Val Pro Pro Val Val His Gly Cys Pro Leu Pro Pro Ile Lys Ala
                1285                1290                1295

cct cca ata cca cct cca cgg aga aag agg acg gtt gtc cta aca gag     3936
Pro Pro Ile Pro Pro Pro Arg Arg Lys Arg Thr Val Val Leu Thr Glu
            1300                1305                1310

tcc tcc gtg tct tct gcc tta gcg gag ctc gct act aag acc ttc ggc     3984
Ser Ser Val Ser Ser Ala Leu Ala Glu Leu Ala Thr Lys Thr Phe Gly
        1315                1320                1325

agc tcc gaa tca tcg gcc gtc gac agc ggc acg gcg acc gcc ctt cct     4032
Ser Ser Glu Ser Ser Ala Val Asp Ser Gly Thr Ala Thr Ala Leu Pro
    1330                1335                1340

gac cag gcc tcc gac gac ggt gac aaa gga tcc gac gtt gag tcg tac     4080
Asp Gln Ala Ser Asp Asp Gly Asp Lys Gly Ser Asp Val Glu Ser Tyr
1345                1350                1355                1360

tcc tcc atg ccc ccc ctt gag ggg gaa ccg ggg gac ccc gat ctc agt     4128
Ser Ser Met Pro Pro Leu Glu Gly Glu Pro Gly Asp Pro Asp Leu Ser

1365                1370                1375

gac ggg tct tgg tct acc gtg agc gag gaa gct agt gag gat gtc gtc    4176
Asp Gly Ser Trp Ser Thr Val Ser Glu Glu Ala Ser Glu Asp Val Val
            1380                1385                1390

tgc tgc tca atg tcc tac aca tgg aca ggc gcc ttg atc acg cca tgc    4224
Cys Cys Ser Met Ser Tyr Thr Trp Thr Gly Ala Leu Ile Thr Pro Cys
        1395                1400                1405

gct gcg gag gaa agc aag ctg ccc atc aac gcg ttg agc aac tct ttg    4272
Ala Ala Glu Glu Ser Lys Leu Pro Ile Asn Ala Leu Ser Asn Ser Leu
    1410                1415                1420

ctg cgc cac cat aac atg gtt tat gcc aca aca tct cgc agc gca ggc    4320
Leu Arg His His Asn Met Val Tyr Ala Thr Thr Ser Arg Ser Ala Gly
1425                1430                1435                1440

ctg cgg cag aag aag gtc acc ttt gac aga ctg caa gtc ctg gac gac    4368
Leu Arg Gln Lys Lys Val Thr Phe Asp Arg Leu Gln Val Leu Asp Asp
                1445                1450                1455

cac tac cgg gac gtg ctc aag gag atg aag gcg aag gcg tcc aca gtt    4416
His Tyr Arg Asp Val Leu Lys Glu Met Lys Ala Lys Ala Ser Thr Val
            1460                1465                1470

aag gct aaa ctc cta tcc gta gag gaa gcc tgc aag ctg acg ccc cca    4464
Lys Ala Lys Leu Leu Ser Val Glu Glu Ala Cys Lys Leu Thr Pro Pro
        1475                1480                1485

cat tcg gcc aaa tcc aag ttt ggc tat ggg gca aag gac gtc cgg aac    4512
His Ser Ala Lys Ser Lys Phe Gly Tyr Gly Ala Lys Asp Val Arg Asn
    1490                1495                1500

cta tcc agc aag gcc gtt aac cac atc cac tcc gtg tgg aag gac ttg    4560
Leu Ser Ser Lys Ala Val Asn His Ile His Ser Val Trp Lys Asp Leu
1505                1510                1515                1520

ctg gaa gac act gtg aca cca att gac acc acc atc atg gca aaa aat    4608
Leu Glu Asp Thr Val Thr Pro Ile Asp Thr Thr Ile Met Ala Lys Asn
                1525                1530                1535

gag gtt ttc tgt gtc caa cca gag aaa gga ggc cgt aag cca gcc cgc    4656
Glu Val Phe Cys Val Gln Pro Glu Lys Gly Gly Arg Lys Pro Ala Arg
            1540                1545                1550

ctt atc gta ttc cca gat ctg gga gtc cgt gta tgc gag aag atg gcc    4704
Leu Ile Val Phe Pro Asp Leu Gly Val Arg Val Cys Glu Lys Met Ala
        1555                1560                1565

ctc tat gat gtg gtc tcc acc ctt cct cag gtc gtg atg ggc tcc tca    4752
Leu Tyr Asp Val Val Ser Thr Leu Pro Gln Val Val Met Gly Ser Ser
    1570                1575                1580

tac gga ttc cag tac tct cct ggg cag cga gtc gag ttc ctg gtg aat    4800
Tyr Gly Phe Gln Tyr Ser Pro Gly Gln Arg Val Glu Phe Leu Val Asn
1585                1590                1595                1600

acc tgg aaa tca aag aaa aac ccc atg ggc ttt tca tat gac act cgc    4848
Thr Trp Lys Ser Lys Lys Asn Pro Met Gly Phe Ser Tyr Asp Thr Arg
                1605                1610                1615

tgt ttc gac tca acg gtc acc gag aac gac atc cgt gtt gag gag tca    4896
Cys Phe Asp Ser Thr Val Thr Glu Asn Asp Ile Arg Val Glu Glu Ser
            1620                1625                1630

att tac caa tgt tgt gac ttg gcc ccc gaa gcc aga cag gcc ata aaa    4944
Ile Tyr Gln Cys Cys Asp Leu Ala Pro Glu Ala Arg Gln Ala Ile Lys

1635                 1640                 1645

tcg ctc aca gag cgg ctt tat atc ggg ggt cct ctg act aat tca aaa     4992
Ser Leu Thr Glu Arg Leu Tyr Ile Gly Gly Pro Leu Thr Asn Ser Lys
    1650                 1655                 1660

ggg cag aac tgc ggt tat cgc cgg tgc cgc gcg agc ggc gtg ctg acg     5040
Gly Gln Asn Cys Gly Tyr Arg Arg Cys Arg Ala Ser Gly Val Leu Thr
1665                 1670                 1675                 1680

act agc tgc ggt aac acc ctc aca tgt tac ttg aag gcc tct gca gcc     5088
Thr Ser Cys Gly Asn Thr Leu Thr Cys Tyr Leu Lys Ala Ser Ala Ala
                1685                 1690                 1695

tgt cga gct gcg aag ctc cag gac tgc acg atg ctc gtg aac gga gac     5136
Cys Arg Ala Ala Lys Leu Gln Asp Cys Thr Met Leu Val Asn Gly Asp
            1700                 1705                 1710

gac ctt gtc gtt atc tgt gaa agc gcg gga acc caa gag gac gcg gcg     5184
Asp Leu Val Val Ile Cys Glu Ser Ala Gly Thr Gln Glu Asp Ala Ala
        1715                 1720                 1725

agc cta cga gtc ttc acg gag gct atg act agg tac tct gcc ccc ccc     5232
Ser Leu Arg Val Phe Thr Glu Ala Met Thr Arg Tyr Ser Ala Pro Pro
    1730                 1735                 1740

ggg gac ccg ccc caa cca gaa tac gac ttg gag ctg ata aca tca tgt     5280
Gly Asp Pro Pro Gln Pro Glu Tyr Asp Leu Glu Leu Ile Thr Ser Cys
1745                 1750                 1755                 1760

tcc tcc aat gtg tcg gtc gcc cac gat gca tca ggc aaa agg gtg tac     5328
Ser Ser Asn Val Ser Val Ala His Asp Ala Ser Gly Lys Arg Val Tyr
                1765                 1770                 1775

tac ctc acc cgt gat ccc acc acc ccc ctc gca cgg gct gcg tgg gaa     5376
Tyr Leu Thr Arg Asp Pro Thr Thr Pro Leu Ala Arg Ala Ala Trp Glu
            1780                 1785                 1790

aca gct aga cac act cca gtt aac tcc tgg cta ggc aac att atc atg     5424
Thr Ala Arg His Thr Pro Val Asn Ser Trp Leu Gly Asn Ile Ile Met
        1795                 1800                 1805

tat gcg ccc act ttg tgg gca agg atg att ctg atg act cac ttc ttc     5472
Tyr Ala Pro Thr Leu Trp Ala Arg Met Ile Leu Met Thr His Phe Phe
    1810                 1815                 1820

tcc atc ctt cta gca cag gag caa ctt gaa aaa gcc ctg gac tgc cag     5520
Ser Ile Leu Leu Ala Gln Glu Gln Leu Glu Lys Ala Leu Asp Cys Gln
1825                 1830                 1835                 1840

atc tac ggg gcc tgt tac tcc att gag cca ctt gac cta cct cag atc     5568
Ile Tyr Gly Ala Cys Tyr Ser Ile Glu Pro Leu Asp Leu Pro Gln Ile
                1845                 1850                 1855

att gaa cga ctc cat ggc ctt agc gca ttt tca ctc cat agt tac tct     5616
Ile Glu Arg Leu His Gly Leu Ser Ala Phe Ser Leu His Ser Tyr Ser
            1860                 1865                 1870

cca ggt gag atc aat agg gtg gct tca tgc ctc agg aaa ctt ggg gta     5664
Pro Gly Glu Ile Asn Arg Val Ala Ser Cys Leu Arg Lys Leu Gly Val
        1875                 1880                 1885

cca ccc ttg cga gtc tgg aga cat cgg gcc agg agc gtc cgc gct agg     5712
Pro Pro Leu Arg Val Trp Arg His Arg Ala Arg Ser Val Arg Ala Arg
    1890                 1895                 1900

cta ctg tcc cag ggg ggg agg gcc gcc act tgt ggc aag tac ctc ttc     5760
Leu Leu Ser Gln Gly Gly Arg Ala Ala Thr Cys Gly Lys Tyr Leu Phe

1905                1910                1915                1920

aac tgg gca gtg aag acc aaa ctc aaa ctc act cca atc ccg gct gcg     5808
Asn Trp Ala Val Lys Thr Lys Leu Lys Leu Thr Pro Ile Pro Ala Ala
                1925                1930                1935

tcc cag ctg gac ttg tcc ggc tgg ttc gtt gct ggt tac agc ggg gga     5856
Ser Gln Leu Asp Leu Ser Gly Trp Phe Val Ala Gly Tyr Ser Gly Gly
            1940                1945                1950

gac ata tat cac agc ctg tct cgt gcc cga ccc cgc tgg ttc atg ctg     5904
Asp Ile Tyr His Ser Leu Ser Arg Ala Arg Pro Arg Trp Phe Met Leu
        1955                1960                1965

tgc cta ctc cta ctt tct gta ggg gta ggc atc tac ctg ctc ccc aac     5952
Cys Leu Leu Leu Leu Ser Val Gly Val Gly Ile Tyr Leu Leu Pro Asn
    1970                1975                1980

cga                                                                 5955
Arg
1985


<210> 6
<211> 1985
<212> PRT
<213> Artificial Sequence

<220>
<223> NS sequence

<400> 6
Met Ala Pro Ile Thr Ala Tyr Ser Gln Gln Thr Arg Gly Leu Leu Gly
1               5                   10                  15
Cys Ile Ile Thr Ser Leu Thr Gly Arg Asp Lys Asn Gln Val Glu Gly
            20                  25                  30
Glu Val Gln Val Val Ser Thr Ala Thr Gln Ser Phe Leu Ala Thr Cys
        35                  40                  45
Val Asn Gly Val Cys Trp Thr Val Tyr His Gly Ala Gly Ser Lys Thr
    50                  55                  60
Leu Ala Gly Pro Lys Gly Pro Ile Thr Gln Met Tyr Thr Asn Val Asp
65                  70                  75                  80
Gln Asp Leu Val Gly Trp Gln Ala Pro Pro Gly Ala Arg Ser Leu Thr
                85                  90                  95
Pro Cys Thr Cys Gly Ser Ser Asp Leu Tyr Leu Val Thr Arg His Ala
            100                 105                 110
Asp Val Ile Pro Val Arg Arg Arg Gly Asp Ser Arg Gly Ser Leu Leu
        115                 120                 125
Ser Pro Arg Pro Val Ser Tyr Leu Lys Gly Ser Ser Gly Gly Pro Leu
    130                 135                 140
Leu Cys Pro Ser Gly His Ala Val Gly Ile Phe Arg Ala Ala Val Cys
145                 150                 155                 160
Thr Arg Gly Val Ala Lys Ala Val Asp Phe Val Pro Val Glu Ser Met
                165                 170                 175
Glu Thr Thr Met Arg Ser Pro Val Phe Thr Asp Asn Ser Ser Pro Pro
            180                 185                 190
Ala Val Pro Gln Ser Phe Gln Val Ala His Leu His Ala Pro Thr Gly
        195                 200                 205
Ser Gly Lys Ser Thr Lys Val Pro Ala Ala Tyr Ala Ala Gln Gly Tyr
    210                 215                 220
Lys Val Leu Val Leu Asn Pro Ser Val Ala Ala Thr Leu Gly Phe Gly
225                 230                 235                 240
Ala Tyr Met Ser Lys Ala His Gly Ile Asp Pro Asn Ile Arg Thr Gly
                245                 250                 255
Val Arg Thr Ile Thr Thr Gly Ala Pro Val Thr Tyr Ser Thr Tyr Gly
            260                 265                 270
Lys Phe Leu Ala Asp Gly Gly Cys Ser Gly Gly Ala Tyr Asp Ile Ile
        275                 280                 285

Ile Cys Asp Glu Cys His Ser Thr Asp Ser Thr Thr Ile Leu Gly Ile
        290                 295                 300
Gly Thr Val Leu Asp Gln Ala Glu Thr Ala Gly Ala Arg Leu Val Val
305                 310                 315                 320
Leu Ala Thr Ala Thr Pro Pro Gly Ser Val Thr Val Pro His Pro Asn
                325                 330                 335
Ile Glu Glu Val Ala Leu Ser Asn Thr Gly Glu Ile Pro Phe Tyr Gly
            340                 345                 350
Lys Ala Ile Pro Ile Glu Ala Ile Arg Gly Gly Arg His Leu Ile Phe
        355                 360                 365
Cys His Ser Lys Lys Lys Cys Asp Glu Leu Ala Ala Lys Leu Ser Gly
    370                 375                 380
Leu Gly Ile Asn Ala Val Ala Tyr Tyr Arg Gly Leu Asp Val Ser Val
385                 390                 395                 400
Ile Pro Thr Ile Gly Asp Val Val Val Val Ala Thr Asp Ala Leu Met
                405                 410                 415
Thr Gly Tyr Thr Gly Asp Phe Asp Ser Val Ile Asp Cys Asn Thr Cys
            420                 425                 430
Val Thr Gln Thr Val Asp Phe Ser Leu Asp Pro Thr Phe Thr Ile Glu
        435                 440                 445
Thr Thr Thr Val Pro Gln Asp Ala Val Ser Arg Ser Gln Arg Arg Gly
    450                 455                 460
Arg Thr Gly Arg Gly Arg Arg Gly Ile Tyr Arg Phe Val Thr Pro Gly
465                 470                 475                 480
Glu Arg Pro Ser Gly Met Phe Asp Ser Ser Val Leu Cys Glu Cys Tyr
                485                 490                 495
Asp Ala Gly Cys Ala Trp Tyr Glu Leu Thr Pro Ala Glu Thr Ser Val
            500                 505                 510
Arg Leu Arg Ala Tyr Leu Asn Thr Pro Gly Leu Pro Val Cys Gln Asp
        515                 520                 525
His Leu Glu Phe Trp Glu Ser Val Phe Thr Gly Leu Thr His Ile Asp
    530                 535                 540
Ala His Phe Leu Ser Gln Thr Lys Gln Ala Gly Asp Asn Phe Pro Tyr
545                 550                 555                 560
Leu Val Ala Tyr Gln Ala Thr Val Cys Ala Arg Ala Gln Ala Pro Pro
                565                 570                 575
Pro Ser Trp Asp Gln Met Trp Lys Cys Leu Ile Arg Leu Lys Pro Thr
            580                 585                 590
Leu His Gly Pro Thr Pro Leu Leu Tyr Arg Leu Gly Ala Val Gln Asn
        595                 600                 605
Glu Val Thr Leu Thr His Pro Ile Thr Lys Tyr Ile Met Ala Cys Met
    610                 615                 620
Ser Ala Asp Leu Glu Val Val Thr Ser Thr Trp Val Leu Val Gly Gly
625                 630                 635                 640
Val Leu Ala Ala Leu Ala Ala Tyr Cys Leu Thr Thr Gly Ser Val Val
                645                 650                 655
Ile Val Gly Arg Ile Ile Leu Ser Gly Arg Pro Ala Ile Val Pro Asp
            660                 665                 670
Arg Glu Phe Leu Tyr Gln Glu Phe Asp Glu Met Glu Glu Cys Ala Ser
        675                 680                 685
His Leu Pro Tyr Ile Glu Gln Gly Met Gln Leu Ala Glu Gln Phe Lys
    690                 695                 700
Gln Lys Ala Leu Gly Leu Leu Gln Thr Ala Thr Lys Gln Ala Glu Ala
705                 710                 715                 720
Ala Ala Pro Val Val Glu Ser Lys Trp Arg Ala Leu Glu Thr Phe Trp
                725                 730                 735
Ala Lys His Met Trp Asn Phe Ile Ser Gly Ile Gln Tyr Leu Ala Gly
            740                 745                 750
Leu Ser Thr Leu Pro Gly Asn Pro Ala Ile Ala Ser Leu Met Ala Phe
        755                 760                 765
Thr Ala Ser Ile Thr Ser Pro Leu Thr Thr Gln Ser Thr Leu Leu Phe
    770                 775                 780
Asn Ile Leu Gly Gly Trp Val Ala Ala Gln Leu Ala Pro Pro Ser Ala
785                 790                 795                 800
Ala Ser Ala Phe Val Gly Ala Gly Ile Ala Gly Ala Ala Val Gly Ser
                805                 810                 815
Ile Gly Leu Gly Lys Val Leu Val Asp Ile Leu Ala Gly Tyr Gly Ala
            820                 825                 830

Gly Val Ala Gly Ala Leu Val Ala Phe Lys Val Met Ser Gly Glu Met
        835                 840                 845
Pro Ser Thr Glu Asp Leu Val Asn Leu Leu Pro Ala Ile Leu Ser Pro
    850                 855                 860
Gly Ala Leu Val Val Gly Val Val Cys Ala Ala Ile Leu Arg Arg His
865                 870                 875                 880
Val Gly Pro Gly Glu Gly Ala Val Gln Trp Met Asn Arg Leu Ile Ala
                885                 890                 895
Phe Ala Ser Arg Gly Asn His Val Ser Pro Thr His Tyr Val Pro Glu
            900                 905                 910
Ser Asp Ala Ala Ala Arg Val Thr Gln Ile Leu Ser Ser Leu Thr Ile
        915                 920                 925
Thr Gln Leu Leu Lys Arg Leu His Gln Trp Ile Asn Glu Asp Cys Ser
    930                 935                 940
Thr Pro Cys Ser Gly Ser Trp Leu Arg Asp Val Trp Asp Trp Ile Cys
945                 950                 955                 960
Thr Val Leu Thr Asp Phe Lys Thr Trp Leu Gln Ser Lys Leu Leu Pro
                965                 970                 975
Gln Leu Pro Gly Val Pro Phe Phe Ser Cys Gln Arg Gly Tyr Lys Gly
            980                 985                 990
Val Trp Arg Gly Asp Gly Ile Met Gln Thr Thr Cys Pro Cys Gly Ala
        995                 1000                1005
Gln Ile Thr Gly His Val Lys Asn Gly Ser Met Arg Ile Val Gly Pro
    1010                1015                1020
Lys Thr Cys Ser Asn Thr Trp His Gly Thr Phe Pro Ile Asn Ala Tyr
1025                1030                1035                1040
Thr Thr Gly Pro Cys Thr Pro Ser Pro Ala Pro Asn Tyr Ser Arg Ala
                1045                1050                1055
Leu Trp Arg Val Ala Ala Glu Glu Tyr Val Glu Val Thr Arg Val Gly
            1060                1065                1070
Asp Phe His Tyr Val Thr Gly Met Thr Thr Asp Asn Val Lys Cys Pro
        1075                1080                1085
Cys Gln Val Pro Ala Pro Glu Phe Phe Thr Glu Val Asp Gly Val Arg
    1090                1095                1100
Leu His Arg Tyr Ala Pro Ala Cys Arg Pro Leu Leu Arg Glu Glu Val
1105                1110                1115                1120
Thr Phe Gln Val Gly Leu Asn Gln Tyr Leu Val Gly Ser Gln Leu Pro
                1125                1130                1135
Cys Glu Pro Glu Pro Asp Val Ala Val Leu Thr Ser Met Leu Thr Asp
            1140                1145                1150
Pro Ser His Ile Thr Ala Glu Thr Ala Lys Arg Arg Leu Ala Arg Gly
        1155                1160                1165
Ser Pro Pro Ser Leu Ala Ser Ser Ser Ala Ser Gln Leu Ser Ala Pro
    1170                1175                1180
Ser Leu Lys Ala Thr Cys Thr Thr His His Val Ser Pro Asp Ala Asp
1185                1190                1195                1200
Leu Ile Glu Ala Asn Leu Leu Trp Arg Gln Glu Met Gly Gly Asn Ile
                1205                1210                1215
Thr Arg Val Glu Ser Glu Asn Lys Val Val Val Leu Asp Ser Phe Asp
            1220                1225                1230
Pro Leu Arg Ala Glu Glu Asp Glu Arg Glu Val Ser Val Pro Ala Glu
        1235                1240                1245
Ile Leu Arg Lys Ser Lys Lys Phe Pro Ala Ala Met Pro Ile Trp Ala
    1250                1255                1260
Arg Pro Asp Tyr Asn Pro Pro Leu Leu Glu Ser Trp Lys Asp Pro Asp
1265                1270                1275                1280
Tyr Val Pro Pro Val Val His Gly Cys Pro Leu Pro Pro Ile Lys Ala
                1285                1290                1295
Pro Pro Ile Pro Pro Pro Arg Arg Lys Arg Thr Val Val Leu Thr Glu
            1300                1305                1310
Ser Ser Val Ser Ser Ala Leu Ala Glu Leu Ala Thr Lys Thr Phe Gly
        1315                1320                1325
Ser Ser Glu Ser Ser Ala Val Asp Ser Gly Thr Ala Thr Ala Leu Pro
    1330                1335                1340
Asp Gln Ala Ser Asp Asp Gly Asp Lys Gly Ser Asp Val Glu Ser Tyr
1345                1350                1355                1360
Ser Ser Met Pro Pro Leu Glu Gly Glu Pro Gly Asp Pro Asp Leu Ser
                1365                1370                1375

Asp Gly Ser Trp Ser Thr Val Ser Glu Glu Ala Ser Glu Asp Val Val
            1380                1385                1390
Cys Cys Ser Met Ser Tyr Thr Trp Thr Gly Ala Leu Ile Thr Pro Cys
        1395                1400                1405
Ala Ala Glu Glu Ser Lys Leu Pro Ile Asn Ala Leu Ser Asn Ser Leu
    1410                1415                1420
Leu Arg His His Asn Met Val Tyr Ala Thr Thr Ser Arg Ser Ala Gly
1425                1430                1435                1440
Leu Arg Gln Lys Lys Val Thr Phe Asp Arg Leu Gln Val Leu Asp Asp
                1445                1450                1455
His Tyr Arg Asp Val Leu Lys Glu Met Lys Ala Lys Ala Ser Thr Val
            1460                1465                1470
Lys Ala Lys Leu Leu Ser Val Glu Glu Ala Cys Lys Leu Thr Pro Pro
        1475                1480                1485
His Ser Ala Lys Ser Lys Phe Gly Tyr Gly Ala Lys Asp Val Arg Asn
    1490                1495                1500
Leu Ser Ser Lys Ala Val Asn His Ile His Ser Val Trp Lys Asp Leu
1505                1510                1515                1520
Leu Glu Asp Thr Val Thr Pro Ile Asp Thr Thr Ile Met Ala Lys Asn
                1525                1530                1535
Glu Val Phe Cys Val Gln Pro Glu Lys Gly Gly Arg Lys Pro Ala Arg
            1540                1545                1550
Leu Ile Val Phe Pro Asp Leu Gly Val Arg Val Cys Glu Lys Met Ala
        1555                1560                1565
Leu Tyr Asp Val Val Ser Thr Leu Pro Gln Val Val Met Gly Ser Ser
    1570                1575                1580
Tyr Gly Phe Gln Tyr Ser Pro Gly Gln Arg Val Glu Phe Leu Val Asn
1585                1590                1595                1600
Thr Trp Lys Ser Lys Lys Asn Pro Met Gly Phe Ser Tyr Asp Thr Arg
                1605                1610                1615
Cys Phe Asp Ser Thr Val Thr Glu Asn Asp Ile Arg Val Glu Glu Ser
            1620                1625                1630
Ile Tyr Gln Cys Cys Asp Leu Ala Pro Glu Ala Arg Gln Ala Ile Lys
        1635                1640                1645
Ser Leu Thr Glu Arg Leu Tyr Ile Gly Gly Pro Leu Thr Asn Ser Lys
    1650                1655                1660
Gly Gln Asn Cys Gly Tyr Arg Arg Cys Arg Ala Ser Gly Val Leu Thr
1665                1670                1675                1680
Thr Ser Cys Gly Asn Thr Leu Thr Cys Tyr Leu Lys Ala Ser Ala Ala
                1685                1690                1695
Cys Arg Ala Ala Lys Leu Gln Asp Cys Thr Met Leu Val Asn Gly Asp
            1700                1705                1710
Asp Leu Val Val Ile Cys Glu Ser Ala Gly Thr Gln Glu Asp Ala Ala
        1715                1720                1725
Ser Leu Arg Val Phe Thr Glu Ala Met Thr Arg Tyr Ser Ala Pro Pro
    1730                1735                1740
Gly Asp Pro Pro Gln Pro Glu Tyr Asp Leu Glu Leu Ile Thr Ser Cys
1745                1750                1755                1760
Ser Ser Asn Val Ser Val Ala His Asp Ala Ser Gly Lys Arg Val Tyr
                1765                1770                1775
Tyr Leu Thr Arg Asp Pro Thr Thr Pro Leu Ala Arg Ala Ala Trp Glu
            1780                1785                1790
Thr Ala Arg His Thr Pro Val Asn Ser Trp Leu Gly Asn Ile Ile Met
        1795                1800                1805
Tyr Ala Pro Thr Leu Trp Ala Arg Met Ile Leu Met Thr His Phe Phe
    1810                1815                1820
Ser Ile Leu Leu Ala Gln Glu Gln Leu Glu Lys Ala Leu Asp Cys Gln
1825                1830                1835                1840
Ile Tyr Gly Ala Cys Tyr Ser Ile Glu Pro Leu Asp Leu Pro Gln Ile
                1845                1850                1855
Ile Glu Arg Leu His Gly Leu Ser Ala Phe Ser Leu His Ser Tyr Ser
            1860                1865                1870
Pro Gly Glu Ile Asn Arg Val Ala Ser Cys Leu Arg Lys Leu Gly Val
        1875                1880                1885
Pro Pro Leu Arg Val Trp Arg His Arg Ala Arg Ser Val Arg Ala Arg
    1890                1895                1900
Leu Leu Ser Gln Gly Gly Arg Ala Ala Thr Cys Gly Lys Tyr Leu Phe
1905                1910                1915                1920

Asn Trp Ala Val Lys Thr Lys Leu Lys Leu Thr Pro Ile Pro Ala Ala
                1925                1930                1935
Ser Gln Leu Asp Leu Ser Gly Trp Phe Val Ala Gly Tyr Ser Gly Gly
            1940                1945                1950
Asp Ile Tyr His Ser Leu Ser Arg Ala Arg Pro Arg Trp Phe Met Leu
        1955                1960                1965
Cys Leu Leu Leu Leu Ser Val Gly Val Gly Ile Tyr Leu Leu Pro Asn
    1970                1975                1980
Arg
1985

<210> 7
<211> 4909
<212> DNA
<213> Artificial Sequence

<220>
<223> pV1J nucleic acid

<400> 7
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320
ggatggggtc ccatttatta tttacaaatt cacatataca acaacgccgt cccccgtgcc 1380
cgcagttttt attaaacata gcgtgggatc tccacgcgaa tctcgggtac gtgttccgga 1440
catgggctct tctccggtag cggcggagct tccacatccg agccctggtc ccatgcctcc 1500
agcggctcat ggtcgctcgg cagctccttg ctcctaacag tggaggccag acttaggcac 1560
agcacaatgc ccaccaccac cagtgtgccg cacaaggccg tggcggtagg gtatgtgtct 1620
gaaaatgagc gtggagattg ggctcgcacg gctgacgcag atggaagact taaggcagcg 1680
gcagaagaag atgcaggcag ctgagttgtt gtattctgat aagagtcaga ggtaactccc 1740
gttgcggtgc tgttaacggt ggagggcagt gtagtctgag cagtactcgt tgctgccgcg 1800
cgcgccacca gacataatag ctgacagact aacagactgt tcctttccat gggtcttttc 1860
tgcagtcacc gtccttagat ctaggtacca gatatcagaa ttcagtcgac agcggccgcg 1920
atctgctgtg ccttctagtt gccagccatc tgttgtttgc ccctcccccg tgccttcctt 1980
gaccctggaa ggtgccactc ccactgtcct ttcctaataa aatgaggaaa ttgcatcgca 2040
ttgtctgagt aggtgtcatt ctattctggg gggtggggtg gggcaggaca gcaaggggga 2100
ggattgggaa gacaatagca ggcatgctgg ggatgcggtg ggctctatgg ccgctgcggc 2160
caggtgctga agaattgacc cggttcctcc tgggccagaa agaagcaggc acatcccctt 2220
ctctgtgaca caccctgtcc acgcccctgg ttcttagttc cagccccact cataggacac 2280
tcatagctca ggagggctcc gccttcaatc ccacccgcta aagtacttgg agcggtctct 2340
ccctccctca tcagcccacc aaaccaaacc tagcctccaa gagtgggaag aaattaaagc 2400
aagataggct attaagtgca gagggagaga aaatgcctcc aacatgtgag gaagtaatga 2460
gagaaatcat agaatttctt ccgcttcctc gctcactgac tcgctgcgct cggtcgttcg 2520
gctgcggcga gcggtatcag ctcactcaaa ggcggtaata cggttatcca cagaatcagg 2580
ggataacgca ggaaagaaca tgtgagcaaa aggccagcaa aaggccagga accgtaaaaa 2640
ggccgcgttg ctggcgtttt tccataggct ccgcccccct gacgagcatc acaaaaatcg 2700
acgctcaagt cagaggtggc gaaacccgac aggactataa agataccagg cgtttccccc 2760
tggaagctcc ctcgtgcgct ctcctgttcc gaccctgccg cttaccggat acctgtccgc 2820

ctttctccct tcgggaagcg tggcgctttc tcatagctca cgctgtaggt atctcagttc 2880
ggtgtaggtc gttcgctcca agctgggctg tgtgcacgaa ccccccgttc agcccgaccg 2940
ctgcgcctta tccggtaact atcgtcttga gtccaacccg gtaagacacg acttatcgcc 3000
actggcagca gccactggta acaggattag cagagcgagg tatgtaggcg gtgctacaga 3060
gttcttgaag tggtggccta actacggcta cactagaaga acagtatttg gtatctgcgc 3120
tctgctgaag ccagttacct tcggaaaaag agttggtagc tcttgatccg gcaaacaaac 3180
caccgctggt agcggtggtt tttttgtttg caagcagcag attacgcgca gaaaaaaagg 3240
atctcaagaa gatcctttga tcttttctac ggggtctgac gctcagtgga acgaaaactc 3300
acgttaaggg attttggtca tgagattatc aaaaaggatc ttcacctaga tccttttaaa 3360
ttaaaaatga agttttaaat caatctaaag tatatatgag taaacttggt ctgacagtta 3420
ccaatgctta atcagtgagg cacctatctc agcgatctgt ctatttcgtt catccatagt 3480
tgcctgactc gggggggggg ggcgctgagg tctgcctcgt gaagaaggtg ttgctgactc 3540
ataccaggcc tgaatcgccc catcatccag ccagaaagtg agggagccac ggttgatgag 3600
agctttgttg taggtggacc agttggtgat tttgaacttt tgctttgcca cggaacggtc 3660
tgcgttgtcg ggaagatgcg tgatctgatc cttcaactca gcaaaagttc gatttattca 3720
acaaagccgc cgtcccgtca agtcagcgta atgctctgcc agtgttacaa ccaattaacc 3780
aattctgatt agaaaaactc atcgagcatc aaatgaaact gcaatttatt catatcagga 3840
ttatcaatac catatttttg aaaaagccgt ttctgtaatg aaggagaaaa ctcaccgagg 3900
cagttccata ggatggcaag atcctggtat cggtctgcga ttccgactcg tccaacatca 3960
atacaaccta ttaatttccc ctcgtcaaaa ataaggttat caagtgagaa atcaccatga 4020
gtgacgactg aatccggtga gaatggcaaa agcttatgca tttctttcca gacttgttca 4080
acaggccagc cattacgctc gtcatcaaaa tcactcgcat caaccaaacc gttattcatt 4140
cgtgattgcg cctgagcgag acgaaatacg cgatcgctgt taaaaggaca attacaaaca 4200
ggaatcgaat gcaaccggcg caggaacact gccagcgcat caacaatatt ttcacctgaa 4260
tcaggatatt cttctaatac ctggaatgct gttttcccgg ggatcgcagt ggtgagtaac 4320
catgcatcat caggagtacg gataaaatgc ttgatggtcg gaagaggcat aaattccgtc 4380
agccagttta gtctgaccat ctcatctgta acatcattgg caacgctacc tttgccatgt 4440
ttcagaaaca actctggcgc atcgggcttc ccatacaatc gatagattgt cgcacctgat 4500
tgcccgacat tatcgcgagc ccatttatac ccatataaat cagcatccat gttggaattt 4560
aatcgcggcc tcgagcaaga cgtttcccgt tgaatatggc tcataacacc ccttgtatta 4620
ctgtttatgt aagcagacag ttttattgtt catgatgata tatttttatc ttgtgcaatg 4680
taacatcaga gattttgaga cacaacgtgg ctttcccccc ccccccatta ttgaagcatt 4740
tatcagggtt attgtctcat gagcggatac atatttgaat gtatttagaa aaataaacaa 4800
ataggggttc cgcgcacatt tccccgaaaa gtgccacctg acgtctaaga aaccattatt 4860
atcatgacat taacctataa aaataggcgt atcacgaggc cctttcgtc 4909

<210> 8
<211> 35759
<212> DNA
<213> Adenovirus serotype 6

<400> 8
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgtaa gtgtggcgga acacatgtaa gcgccggatg tggtaaaagt gacgtttttg 180
   gtgtgcgccg gtgtacacgg gaagtgacaa ttttcgcgcg gttttaggcg gatgttgtag 240
   taaatttggg cgtaaccaag taatatttgg ccattttcgc gggaaaactg aataagagga 300
   agtgaaatct gaataattct gtgttactca tagcgcgtaa tatttgtcta gggccgcggg 360
   gactttgacc gtttacgtgg agactcgccc aggtgttttt ctcaggtgtt ttccgcgttc 420
   cgggtcaaag ttggcgtttt attattatag tcagctgacg cgcagtgtat ttatacccgg 480
   tgagttcctc aagaggccac tcttgagtgc cagcgagtag agttttctcc tccgagccgc 540
   tccgacaccg ggactgaaaa tgagacatat tatctgccac ggaggtgtta ttaccgaaga 600
   aatggccgcc agtcttttgg accagctgat cgaagaggta ctggctgata atcttccacc 660
   tcctagccat tttgaaccac ctacccttca cgaactgtat gatttagacg tgacggcccc 720
   cgaagatccc aacgaggagg cggtttcgca gatttttccc gagtctgtaa tgttggcggt 780
   gcaggaaggg attgacttat tcacttttcc gccggcgccc ggttctccgg agccgcctca 840
   cctttcccgg cagcccgagc agccggagca gagagccttg ggtccggttt ctatgccaaa 900
   ccttgtgccg gaggtgatcg atcttacctg ccacgaggct ggctttccac ccagtgacga 960
   cgaggatgaa gagggtgagg agtttgtgtt agattatgtg gagcaccccg ggcacggttg 1020
   caggtcttgt cattatcacc ggaggaatac gggggaccca gatattatgt gttcgctttg 1080
   ctatatgagg acctgtggca tgtttgtcta cagtaagtga aaattatggg gcagtgggtg 1140
   atagagtggt gggtttggtg tggtaatttt ttttttaatt tttacagttt tgtggtttaa 1200
   agaattttgt attgtgattt tttaaaaggt cctgtgtctg aacctgagcc tgagcccgag 1260
   ccagaaccgg agcctgcaag acctacccgg cgtcctaaat tggtgcctgc tatcctgaga 1320
   cgcccgacat cacctgtgtc tagagaatgc aatagtagta cggatagctg tgactccggt 1380
   ccttctaaca cacctcctga gatacacccg gtggtcccgc tgtgccccat taaaccagtt 1440
   gccgtgagag ttggtgggcg tcgccaggct gtggaatgta tcgaggactt gcttaacgag 1500
   tctgggcaac ctttggactt gagctgtaaa cgccccaggc cataaggtgt aaacctgtga 1560
   ttgcgtgtgt ggttaacgcc tttgtttgct gaatgagttg atgtaagttt aataaagggt 1620
   gagataatgt ttaacttgca tggcgtgtta aatggggcgg ggcttaaagg gtatataatg 1680
   cgccgtgggc taatcttggt tacatctgac ctcatggagg cttgggagtg tttggaagat 1740
   ttttctgctg tgcgtaactt gctggaacag agctctaaca gtacctcttg gttttggagg 1800
   tttctgtggg gctcctccca ggcaaagtta gtctgcagaa ttaaggagga ttacaagtgg 1860
   gaatttgaag agcttttgaa atcctgtggt gagctgtttg attctttgaa tctgggtcac 1920
   caggcgcttt tccaagagaa ggtcatcaag actttggatt tttccacacc ggggcgcgct 1980
   gcggctgctg ttgctttttt gagttttata aaggataaat ggagcgaaga aacccatctg 2040
   agcggggggt acctgctgga ttttctggcc atgcatctgt ggagagcggt ggtgagacac 2100
   aagaatcgcc tgctactgtt gtcttccgtc cgcccggcaa taataccgac ggaggagcaa 2160
   cagcaggagg aagccaggcg gcggcggcgg caggagcaga gcccatggaa cccgagagcc 2220
   ggcctggacc ctcgggaatg aatgttgtac aggtggctga actgtttcca gaactgagac 2280
   gcattttaac cattaacgag gatgggcagg ggctaaaggg ggtaaagaag gagcgggggg 2340
   cttctgaggc tacagaggag gctaggaatc taacttttag cttaatgacc agacaccgtc 2400
   ctgagtgtgt tacttttcag cagattaagg ataattgcgc taatgagctt gatctgctgg 2460
   cgcagaagta ttccatagag cagctgacca cttactggct gcagccaggg gatgattttg 2520
   aggaggctat tagggtatat gcaaaggtgg cacttaggcc agattgcaag tacaagatta 2580
   gcaaacttgt aaatatcagg aattgttgct acatttctgg gaacggggcc gaggtggaga 2640
   tagatacgga ggatagggtg gcctttagat gtagcatgat aaatatgtgg ccgggggtgc 2700
   ttggcatgga cggggtggtt attatgaatg tgaggtttac tggtcccaat tttagcggta 2760
   cggttttcct ggccaatacc aatcttatcc tacacggtgt aagcttctat gggtttaaca 2820
   atacctgtgt ggaagcctgg accgatgtaa gggttcgggg ctgtgccttt tactgctgct 2880
   ggaagggggt ggtgtgtcgc cccaaaagca gggcttcaat taagaaatgc ctgtttgaaa 2940
   ggtgtacctt gggtatcctg tctgagggta actccagggt gcgccacaat gtggcctccg 3000
   actgtggttg ctttatgcta gtgaaaagcg tggctgtgat taagcataac atggtgtgtg 3060
   gcaactgcga ggacagggcc tctcagatgc tgacctgctc ggacggcaac tgtcacttgc 3120
   tgaagaccat tcacgtagcc agccactctc gcaaggcctg gccagtgttt gagcacaaca 3180
   tactgacccg ctgttccttg catttgggta acaggagggg ggtgttccta ccttaccaat 3240
   gcaatttgag tcacactaag atattgcttg agcccgagag catgtccaag gtgaacctga 3300
   acggggtgtt tgacatgacc atgaagatct ggaaggtgct gaggtacgat gagacccgca 3360
   ccaggtgcag accctgcgag tgtggcggta aacatattag gaaccagcct gtgatgctgg 3420
   atgtgaccga ggagctgagg cccgatcact tggtgctggc ctgcacccgc gctgagtttg 3480
   gctctagcga tgaagataca gattgaggta ctgaaatgtg tgggcgtggc ttaagggtgg 3540
   gaaagaatat ataaggtggg ggtctcatgt agttttgtat ctgttttgca gcagccgccg 3600
   ccatgagcgc caactcgttt gatggaagca ttgtgagctc atatttgaca acgcgcatgc 3660
   ccccatgggc cggggtgcgt cagaatgtga tgggctccag cattgatggt cgccccgtcc 3720
   tgcccgcaaa ctctactacc ttgacctacg agaccgtgtc tggaacgccg ttggagactg 3780
   cagcctccgc cgccgcttca gccgctgcag ccaccgcccg cgggattgtg actgactttg 3840
   ctttcctgag cccgcttgca agcagtgcag cttcccgttc atccgcccgc gatgacaagt 3900
   tgacggctct tttggcacaa ttggattctt tgacccggga acttaatgtc gtttctcagc 3960
   agctgttgga tctgcgccag caggtttctg ccctgaaggc ttcctcccct cccaatgcgg 4020
   tttaaaacat aaataaaaac cagactctgt ttggatttgg atcaagcaag tgtcttgctg 4080
   tctttattta ggggttttgc gcgcgcggta ggcccgggac cagcggtctc ggtcgttgag 4140
   ggtcctgtgt attttttcca ggacgtggta aaggtgactc tggatgttca gatacatggg 4200
   cataagcccg tctctggggt ggaggtagca ccactgcaga gcttcatgct gcggggtggt 4260
   gttgtagatg atccagtcgt agcaggagcg ctgggcgtgg tgcctaaaaa tgtctttcag 4320
   tagcaagctg attgccaggg gcaggccctt ggtgtaagtg tttacaaagc ggttaagctg 4380
   ggatgggtgc atacgtgggg atatgagatg catcttggac tgtattttta ggttggctat 4440
   gttcccagcc atatccctcc ggggattcat gttgtgcaga accaccagca cagtgtatcc 4500
   ggtgcacttg ggaaatttgt catgtagctt agaaggaaat gcgtggaaga acttggagac 4560
   gcccttgtga cctccaagat tttccatgca ttcgtccata atgatggcaa tgggcccacg 4620
   ggcggcggcc tgggcgaaga tatttctggg atcactaacg tcatagttgt gttccaggat 4680
   gagatcgtca taggccattt ttacaaagcg cgggcggagg gtgccagact gcggtataat 4740
   ggttccatcc ggcccagggg cgtagttacc ctcacagatt tgcatttccc acgctttgag 4800
   ttcagatggg gggatcatgt ctacctgcgg ggcgatgaag aaaaccgttt ccgggtaggg 4860
   ggagatcagc tgggaagaaa gcaggttcct aagcagctgc gacttaccgc agccggtggg 4920
   cccgtaaatc acacctatta ccggctgcaa ctggtagtta agagagctgc agctgccgtc 4980
   atccctgagc aggggggcca cttcgttaag catgtccctg acttgcatgt tttccctgac 5040
   caaatccgcc agaaggcgct cgccgcccag cgatagcagt tcttgcaagg aagcaaagtt 5100
   tttcaacggt ttgaggccgt ccgccgtagg catgcttttg agcgtttgac caagcagttc 5160
   caggcggtcc cacagctcgg tcacgtgctc tacggcatct cgatccagca tatctcctcg 5220
   tttcgcgggt tggggcggct ttcgctgtac ggcagtagtc ggtgctcgtc cagacgggcc 5280
   agggtcatgt ctttccacgg gcgcagggtc ctcgtcagcg tagtctgggt cacggtgaag 5340
   gggtgcgctc cgggttgcgc gctggccagg gtgcgcttga ggctggtcct gctggtgctg 5400
   aagcgctgcc ggtcttcgcc ctgcgcgtcg gccaggtagc atttgaccat ggtgtcatag 5460
   tccagcccct ccgcggcgtg gcccttggcg cgcagcttgc ccttggagga ggcgccgcac 5520
   gaggggcagt gcagactttt aagggcgtag agcttgggcg cgagaaatac cgattccggg 5580
   gagtaggcat ccgcgccgca ggccccgcag acggtctcgc attccacgag ccaggtgagc 5640
   tctggccgtt cggggtcaaa aaccaggttt cccccatgct ttttgatgcg tttcttacct 5700
   ctggtttcca tgagccggtg tccacgctcg gtgacgaaaa ggctgtccgt gtccccgtat 5760
   acagacttga gaggcctgtc ctcgagcggt gttccgcggt cctcctcgta tagaaactcg 5820
   gaccactctg agacgaaggc tcgcgtccag gccagcacga aggaggctaa gtgggagggg 5880
   tagcggtcgt tgtccactag ggggtccact cgctccaggg tgtgaagaca catgtcgccc 5940
   tcttcggcat caaggaaggt gattggttta taggtgtagg ccacgtgacc gggtgttcct 6000
   gaaggggggc tataaaaggg ggtgggggcg cgttcgtcct cactctcttc cgcatcgctg 6060
   tctgcgaggg ccagctgttg gggtgagtac tccctctcaa aagcgggcat gacttctgcg 6120
   ctaagattgt cagtttccaa aaacgaggag gatttgatat tcacctggcc cgcggtgatg 6180
   cctttgaggg tggccgcgtc catctggtca gaaaagacaa tctttttgtt gtcaagcttg 6240
   gtggcaaacg acccgtagag ggcgttggac agcaacttgg cgatggagcg cagggtttgg 6300
   tttttgtcgc gatcggcgcg ctccttggcc gcgatgttta gctgcacgta ttcgcgcgca 6360
   acgcaccgcc attcgggaaa gacggtggtg cgctcgtcgg gcactaggtg cacgcgccaa 6420
   ccgcggttgt gcagggtgac aaggtcaacg ctggtggcta cctctccgcg taggcgctcg 6480
   ttggtccagc agaggcggcc gcccttgcgc gagcagaatg gcggtagtgg gtctagctgc 6540
   gtctcgtccg gggggtctgc gtccacggta aagaccccgg gcagcaggcg cgcgtcgaag 6600
   tagtctatct tgcatccttg caagtctagc gcctgctgcc atgcgcgggc ggcaagcgcg 6660
   cgctcgtatg ggttgagtgg gggaccccat ggcatggggt gggtgagcgc ggaggcgtac 6720
   atgccgcaaa tgtcgtaaac gtagaggggc tctctgagta ttccaagata tgtagggtag 6780
   catcttccac cgcggatgct ggcgcgcacg taatcgtata gttcgtgcga gggagcgagg 6840
   aggtcgggac cgaggttgct acgggcgggc tgctctgctc ggaagactat ctgcctgaag 6900
   atggcatgtg agttggatga tatggttgga cgctggaaga cgttgaagct ggcgtctgtg 6960
   agacctaccg cgtcacgcac gaaggaggcg taggagtcgc gcagcttgtt gaccagctcg 7020
   gcggtgacct gcacgtctag ggcgcagtag tccagggttt ccttgatgat gtcatactta 7080
   tcctgtccct tttttttcca cagctcgcgg ttgaggacaa actcttcgcg gtctttccag 7140
   tactcttgga tcggaaaccc gtcggcctcc gaacggtaag agcctagcat gtagaactgg 7200
   ttgacggcct ggtaggcgca gcatcccttt tctacgggta gcgcgtatgc ctgcgcggcc 7260
   ttccggagcg aggtgtgggt gagcgcaaag gtgtccctaa ccatgacttt gaggtactgg 7320
   tatttgaagt cagtgtcgtc gcatccgccc tgctcccaga gcaaaaagtc cgtgcgcttt 7380
   ttggaacgcg ggtttggcag ggcgaaggtg acatcgttga agagtatctt tcccgcgcga 7440
   ggcataaagt tgcgtgtgat gcggaagggt cccggcacct cggaacggtt gttaattacc 7500
   tgggcggcga gcacgatctc gtcaaagccg ttgatgttgt ggcccacaat gtaaagttcc 7560
   aagaagcgcg ggatgccctt gatggaaggc aattttttaa gttcctcgta ggtgagctct 7620
   tcaggggagc tgagcccgtg ctctgaaagg gcccagtctg caagatgagg gttggaagcg 7680
   acgaatgagc tccacaggtc acgggccatt agcatttgca ggtggtcgcg aaaggtccta 7740
   aactggcgac ctatggccat tttttctggg gtgatgcagt agaaggtaag cgggtcttgt 7800
   tcccagcggt cccatccaag gtccgcggct aggtctcgcg cggcggtcac tagaggctca 7860
   tctccgccga acttcatgac cagcatgaag ggcacgagct gcttcccaaa ggcccccatc 7920
   caagtatagg tctctacatc gtaggtgaca aagagacgct cggtgcgagg atgcgagccg 7980
   atcgggaaga actggatctc ccgccaccag ttggaggagt ggctgttgat gtggtgaaag 8040
   tagaagtccc tgcgacgggc cgaacactcg tgctggcttt tgtaaaaacg tgcgcagtac 8100
   tggcagcggt gcacgggctg tacatcctgc acgaggttga cctgacgacc gcgcacaagg 8160
   aagcagagtg ggaatttgag cccctcgcct ggcgggtttg gctggtggtc ttctacttcg 8220
   gctgcttgtc cttgaccgtc tggctgctcg aggggagtta cggtggatcg gaccaccacg 8280
   ccgcgcgagc ccaaagtcca gatgtccgcg cgcggcggtc ggagcttgat gacaacatcg 8340
   cgcagatggg agctgtccat ggtctggagc tcccgcggcg tcaggtcagg cgggagctcc 8400
   tgcaggttta cctcgcatag ccgggtcagg gcgcgggcta ggtccaggtg atacctgatt 8460
   tccaggggct ggttggtggc ggcgtcgatg gcttgcaaga ggccgcatcc ccgcggcgcg 8520
   actacggtac cgcgcggcgg gcggtgggcc gcgggggtgt ccttggatga tgcatctaaa 8580
   agcggtgacg cgggcgggcc cccggaggta gggggggctc gggacccgcc gggagagggg 8640
   gcaggggcac gtcggcgccg cgcgcgggca ggagctggtg ctgcgcgcgg aggttgctgg 8700
   cgaacgcgac gacgcggcgg ttgatctcct gaatctggcg cctctgcgtg aagacgacgg 8760
   gcccggtgag cttgaacctg aaagagagtt cgacagaatc aatttcggtg tcgttgacgg 8820
   cggcctggcg caaaatctcc tgcacgtctc ctgagttgtc ttgataggcg atctcggcca 8880
   tgaactgctc gatctcttcc tcctggagat ctccgcgtcc ggctcgctcc acggtggcgg 8940
   cgaggtcgtt ggagatgcgg gccatgagct gcgagaaggc gttgaggcct ccctcgttcc 9000
   agacgcggct gtagaccacg cccccttcgg catcgcgggc gcgcatgacc acctgcgcga 9060
   gattgagctc cacgtgccgg gcgaagacgg cgtagtttcg caggcgctga aagaggtagt 9120
   tgagggtggt ggcggtgtgt tctgccacga agaagtacat aacccagcgc cgcaacgtgg 9180
   attcgttgat atcccccaag gcctcaaggc gctccatggc ctcgtagaag tccacggcga 9240
   agttgaaaaa ctgggagttg cgcgccgaca cggttaactc ctcctccaga agacggatga 9300
   gctcggcgac agtgtcgcgc acctcgcgct caaaggctac aggggcctct tcttcttctt 9360
   caatctcctc ttccataagg gcctcccctt cttcttcttc tggcggcggt gggggagggg 9420
   ggacacggcg gcgacgacgg cgcaccggga ggcggtcgac aaagcgctcg atcatctccc 9480
   cgcggcgacg gcgcatggtc tcggtgacgg cgcggccgtt ctcgcggggg cgcagttgga 9540
   agacgccgcc cgtcatgtcc cggttatggg ttggcggggg gctgccgtgc ggcagggata 9600
   cggcgctaac gatgcatctc aacaattgtt gtgtaggtac tccgccaccg agggacctga 9660
   gcgagtccgc atcgaccgga tcggaaaacc tctcgagaaa ggcgtctaac cagtcacagt 9720
   cgcaaggtag gctgagcacc gtggcgggcg gcagcgggcg gcggtcgggg ttgtttctgg 9780
   cggaggtgct gctgatgatg taattaaagt aggcggtctt gagacggcgg atggtcgaca 9840
   gaagcaccat gtccttgggt ccggcctgct gaatgcgcag gcggtcggcc atgccccagg 9900
   cttcgttttg acatcggcgc aggtctttgt agtagtcttg catgagcctt tctaccggca 9960
   cttcttcttc tccttcctct tgtcctgcat ctcttgcatc tatcgctgcg gcggcggcgg 10020
   agtttggccg taggtggcgc cctcttcctc ccatgcgtgt gaccccgaag cccctcatcg 10080
   gctgaagcag ggccaggtcg gcgacaacgc gctcggctaa tatggcctgc tgcacctgcg 10140
   tgagggtaga ctggaagtcg tccatgtcca caaagcggtg gtatgcgccc gtgttgatgg 10200
   tgtaagtgca gttggccata acggaccagt taacggtctg gtgacccggc tgcgagagct 10260
   cggtgtacct gagacgcgag taagcccttg agtcaaagac gtagtcgttg caagtccgca 10320
   ccaggtactg gtatcccacc aaaaagtgcg gcggcggctg gcggtagagg ggccagcgta 10380
   gggtggccgg ggctccgggg gcgaggtctt ccaacataag gcgatgatat ccgtagatgt 10440
   acctggacat ccaggtgatg ccggcggcgg tggtggaggc gcgcggaaag tcacggacgc 10500
   ggttccagat gttgcgcagc ggcaaaaagt gctccatggt cgggacgctc tggccggtca 10560
   ggcgcgcgca gtcgttgacg ctctagaccg tgcaaaagga gagcctgtaa gcgggcactc 10620
   ttccgtggtc tggtggataa attcgcaagg gtatcatggc ggacgaccgg ggttcgaacc 10680
   ccggatccgg ccgtccgccg tgatccatgc ggttaccgcc cgcgtgtcga acccaggtgt 10740
   gcgacgtcag acaacggggg agcgctcctt ttggcttcct tccaggcgcg gcggatgctg 10800
   cgctagcttt tttggccact ggccgcgcgc ggcgtaagcg gttaggctgg aaagcgaaag 10860
   cattaagtgg ctcgctccct gtagccggag ggttattttc caagggttga gtcgcgggac 10920
   ccccggttcg agtctcgggc cggccggact gcggcgaacg ggggtttgcc tccccgtcat 10980
   gcaagacccc gcttgcaaat tcctccggaa acagggacga gccccttttt tgcttttccc 11040
   agatgcatcc ggtgctgcgg cagatgcgcc cccctcctca gcagcggcaa gagcaagagc 11100
   agcggcagac atgcagggca ccctcccctt ctcctaccgc gtcaggaggg gcaacatccg 11160
   cggctgacgc ggcggcagat ggtgattacg aacccccgcg gcgccggacc cggcactact 11220
   tggacttgga ggagggcgag ggcctggcgc ggctaggagc gccctctcct gagcgacacc 11280
   caagggtgca gctgaagcgt gacacgcgcg aggcgtacgt gccgcggcag aacctgtttc 11340
   gcgaccgcga gggagaggag cccgaggaga tgcgggatcg aaagttccat gcagggcgcg 11400
   agttgcggca tggcctgaac cgcgagcggt tgctgcgcga ggaggacttt gagcccgacg 11460
   cgcggaccgg gattagtccc gcgcgcgcac acgtggcggc cgccgacctg gtaaccgcgt 11520
   acgagcagac ggtgaaccag gagattaact ttcaaaaaag ctttaacaac cacgtgcgca 11580
   cgcttgtggc gcgcgaggag gtggctatag gactgatgca tctgtgggac tttgtaagcg 11640
   cgctggagca aaacccaaat agcaagccgc tcatggcgca gctgttcctt atagtgcagc 11700
   acagcaggga caacgaggca ttcagggatg cgctgctaaa catagtagag cccgagggcc 11760
   gctggctgct cgatttgata aacattctgc agagcatagt ggtgcaggag cgcagcttga 11820
   gcctggctga caaggtggcc gccattaact attccatgct cagtctgggc aagttttacg 11880
   cccgcaagat ataccatacc ccttacgttc ccatagacaa ggaggtaaag atcgaggggt 11940
   tctacatgcg catggcgctg aaggtgctta ccttgagcga cgacctgggc gtttatcgca 12000
   acgagcgcat ccacaaggcc gtgagcgtga gccggcggcg cgagctcagc gaccgcgagc 12060
   tgatgcacag cctgcaaagg gccctggctg gcacgggcag cggcgataga gaggccgagt 12120
   cctactttga cgcgggcgct gacctgcgct gggccccaag ccgacgcgcc ctggaggcag 12180
   ctggggccgg acctgggctg gcggtggcac ccgcgcgcgc tggcaacgtc ggcggcgtgg 12240
   aggaatatga cgaggacgat gagtacgagc cagaggacgg cgagtactaa gcggtgatgt 12300
   ttctgatcag atgatgcaag acgcaacgga cccggcggtg cgggcggcgc tgcagagcca 12360
   gccgtccggc cttaactcca cggacgactg gcgccaggtc atggaccgca tcatgtcgct 12420
   gactgcgcgc aaccctgacg cgttccggca gcagccgcag gccaaccggc tctccgcaat 12480
   tctggaagcg gtggtcccgg cgcgcgcaaa ccccacgcac gagaaggtgc tggcgatcgt 12540
   aaacgcgctg gccgaaaaca gggccatccg gcccgatgag gccggcctgg tctacgacgc 12600
   gctgcttcag cgcgtggctc gttacaacag cagcaacgtg cagaccaacc tggaccggct 12660
   ggtgggggat gtgcgcgagg ccgtggcgca gcgtgagcgc gcgcagcagc agggcaacct 12720
   gggctccatg gttgcactaa acgccttcct gagtacacag cccgccaacg tgccgcgggg 12780
   acaggaggac tacaccaact ttgtgagcgc actgcggcta atggtgactg agacaccgca 12840
   aagtgaggtg tatcagtccg ggccagacta ttttttccag accagtagac aaggcctgca 12900
   gaccgtaaac ctgagccagg ctttcaagaa cttgcagggg ctgtgggggg tgcgggctcc 12960
   cacaggcgac cgcgcgaccg tgtctagctt gctgacgccc aactcgcgcc tgttgctgct 13020
   gctaatagcg cccttcacgg acagtggcag cgtgtcccgg gacacatacc taggtcactt 13080
   gctgacactg taccgcgagg ccataggtca ggcgcatgtg gacgagcata ctttccagga 13140
   gattacaagt gttagccgcg cgctggggca ggaggacacg ggcagcctgg aggcaaccct 13200
   gaactacctg ctgaccaacc ggcggcaaaa aatcccctcg ttgcacagtt taaacagcga 13260
   ggaggagcgc attttgcgct atgtgcagca gagcgtgagc cttaacctga tgcgcgacgg 13320
   ggtaacgccc agcgtggcgc tggacatgac cgcgcgcaac atggaaccgg gcatgtatgc 13380
   ctcaaaccgg ccgtttatca atcgcctaat ggactacttg catcgcgcgg ccgccgtgaa 13440
   ccccgagtat ttcaccaatg ccatcttgaa cccgcactgg ctaccgcccc ctggtttcta 13500
   caccggggga ttcgaggtgc ccgagggtaa cgatggattc ctctgggacg acatagacga 13560
   cagcgtgttt tccccgcaac cgcagaccct gctagagttg caacaacgcg agcaggcaga 13620
   ggcggcgctg cgaaaggaaa gcttccgcag gccaagcagc ttgtccgatc taggcgctgc 13680
   ggccccgcgg tcagatgcta gtagcccatt tccaagcttg atagggtctc ttaccagcac 13740
   tcgcaccacc cgcccgcgcc tgctgggcga ggaggagtac ctaaacaact cgctgctgca 13800
   gccgcagcgc gaaaagaacc tgcctccggc gtttcccaac aacgggatag agagcctagt 13860
   ggacaagatg agtagatgga agacgtatgc gcaggagcac agggatgtgc ccggcccgcg 13920
   cccgcccacc cgtcgtcaaa ggcacgaccg tcagcggggt ctggtgtggg aggacgatga 13980
   ctcggcagac gacagcagcg tcttggattt gggagggagt ggcaacccgt ttgcacacct 14040
   tcgccccagg ctggggagaa tgttttaaaa aaagcatgat gcaaaataaa aaactcacca 14100
   aggccatggc accgagcgtt ggttttcttg tattcccctt agtatgcggc gcgcggcgat 14160
   gtatgaggaa ggtcctcctc cctcctacga gagcgtggtg agcgcggcgc cagtggcggc 14220
   ggcgctgggt tcacccttcg atgctcccct ggacccgccg ttcgtgcctc cgcggtacct 14280
   gcggcctacc ggggggagaa acagcatccg ttactctgag ttggcacccc tattcgacac 14340
   cacccgtgtg taccttgtgg acaacaagtc aacggatgtg gcatccctga actaccagaa 14400
   cgaccacagc aactttctaa ccacggtcat tcaaaacaat gactacagcc cgggggaggc 14460
   aagcacacag accatcaatc ttgacgaccg gtcgcactgg ggcggcgacc tgaaaaccat 14520
   cctgcatacc aacatgccaa atgtgaacga gttcatgttt accaataagt ttaaggcgcg 14580
   ggtgatggtg tcgcgctcgc ttactaagga caaacaggtg gagctgaaat acgagtgggt 14640
   ggagttcacg ctgcccgagg gcaactactc cgagaccatg accatagacc ttatgaacaa 14700
   cgcgatcgtg gagcactact tgaaagtggg caggcagaac ggggttctgg aaagcgacat 14760
   cggggtaaag tttgacaccc gcaacttcag actggggttt gacccagtca ctggtcttgt 14820
   catgcctggg gtatatacaa acgaagcctt ccatccagac atcattttgc tgccaggatg 14880
   cggggtggac ttcacccaca gccgcctgag caacttgttg ggcatccgca agcggcaacc 14940
   cttccaggag ggctttagga tcacctacga tgacctggag ggtggtaaca ttcccgcact 15000
   gttggatgtg gacgcctacc aggcaagctt gaaagatgac accgaacagg gcgggggtgg 15060
   cgcaggcggc ggcaacaaca gtggcagcgg cgcggaagag aactccaacg cggcagctgc 15120
   ggcaatgcag ccggtggagg acatgaacga tcatgccatt cgcggcgaca cctttgccac 15180
   acgggcggag gagaagcgcg ctgaggccga ggcagcggcc gaagctgccg cccccgctgc 15240
   ggaggctgca caacccgagg tcgagaagcc tcagaagaaa ccggtgatta aacccctgac 15300
   agaggacagc aagaaacgca gttacaacct aataagcaat gacagcacct tcacccagta 15360
   ccgcagctgg taccttgcat acaactacgg cgaccctcag gccgggatcc gctcatggac 15420
   cctgctttgc actcctgacg taacctgcgg ctcggagcag gtatactggt cgttgcccga 15480
   catgatgcaa gaccccgtga ccttccgctc cacgcgccag atcagcaact ttccggtggt 15540
   gggcgccgag ctgttgcccg tgcactccaa gagcttctac aacgaccagg ccgtctactc 15600
   ccagctcatc cgccagttta cctctctgac ccacgtgttc aatcgctttc ccgagaacca 15660
   gattttggcg cgcccgccag cccccaccat caccaccgtc agtgaaaacg ttcctgctct 15720
   cacagatcac gggacgctac cgctgcgcaa cagcatcgga ggagtccagc gagtgaccat 15780
   tactgacgcc agacgccgca cctgccccta cgtttacaag gccctgggca tagtctcgcc 15840
   gcgcgtccta tcgagccgca ctttttgagc aagcatgtcc atccttatat cgcccagcaa 15900
   taacacaggc tggggcctgc gcttcccaag caagatgttt ggcggggcca agaagcgctc 15960
   cgaccaacac ccagtgcgcg tgcgcgggca ctaccgcgcg ccctggggcg cgcacaaacg 16020
   cggccgcact gggcgcacca ccgtcgatga cgccatcgac gcggtggtgg aggaggcgcg 16080
   caactacacg cccacgccgc cgccagtgtc caccgtggac gcggccattc agaccgtggt 16140
   gcgcggagcc cggcgctacg ctaaaatgaa gagacggcgg aggcgcgtag cacgtcgcca 16200
   ccgccgccga cccggcactg ccgcccaacg cgcggcggcg gccctgctta accgcgcacg 16260
   tcgcaccggc cgacgggcgg ccatgcgagc cgctcgaagg ctggccgcgg gtattgtcac 16320
   tgtgcccccc aggtccaggc gacgagcggc cgccgcagca gccgcggcca ttagtgctat 16380
   gactcagggt cgcaggggca acgtgtactg ggtgcgcgac tcggttagcg gcctgcgcgt 16440
   gcccgtgcgc acccgccccc cgcgcaacta gattgcaata aaaaactact tagactcgta 16500
   ctgttgtatg tatccagcgg cggcggcgcg catcgaagct atgtccaagc gcaaaatcaa 16560
   agaagagatg ctccaggtca tcgcgccgga gatctatggc cccccgaaga aggaagagca 16620
   ggattacaag ccccgaaagc taaagcgggt caaaaagaaa aagaaagatg atgatgatga 16680
   tgaacttgac gacgaggtgg aactgttgca cgcgaccgcg cccaggcgac gggtacagtg 16740
   gaaaggtcga cgcgtaagac gtgttttgcg acccggcacc accgtagtct ttacgcccgg 16800
   tgagcgctcc acccgcacct acaagcgcgt gtatgatgag gtgtacggcg acgaggacct 16860
   gcttgagcag gccaacgagc gcctcgggga gtttgcctac ggaaagcggc ataaggacat 16920
   gctggcgttg ccgctggacg agggcaaccc aacacctagc ctaaagcccg tgacactgca 16980
   gcaggtgctg cccgcgcttg caccgtccga agaaaagcgc ggcctaaagc gcgagtctgg 17040
   tgacttggca cccaccgtgc agctgatggt acccaagcgt cagcgactgg aagatgtctt 17100
   ggaaaaaatg accgtggagc ctgggctgga gcccgaggtc cgcgtgcggc caatcaagca 17160
   ggtggcaccg ggactgggcg tgcagaccgt ggacgttcag atacccacca ccagtagcac 17220
   tagtattgcc actgccacag agggcatgga gacacaaacg tccccggttg cctcggcggt 17280
   ggcagatgcc gcggtgcagg cggccgctgc ggccgcgtcc aagacctcta cggaggtgca 17340
   aacggacccg tggatgtttc gtgtttcagc cccccggcgt ccgcgccgtt caaggaagta 17400
   cggcgccgcc agcgcgctac tgcccgaata tgccctacat ccttccatcg cgcctacccc 17460
   cggctatcgt ggctacacct accgccccag aagacgagca actacccgac gccgaaccac 17520
   cactggaacc cgccgccgcc gtcgccgtcg ccagcccgtg ctggccccga tttccgtgcg 17580
   cagggtggct cgcgaaggag gcaggaccct ggtgctgcca acagcgcgct accaccccag 17640
   catcgtttaa aagccggtct ttgtggttct tgcagatatg gccctcacct gccgcctccg 17700
   tttcccggtg ccgggattcc gaggaagaat gcaccgtagg aggggcatgg ccggccacgg 17760
   cctgacgggc ggcatgcgtc gtgcgcacca ccggcggcgg cgcgcgtcgc accgtcgcat 17820
   gcgcggcggt atcctgcccc tccttattcc actgatcgcc gcggcgattg gcgccgtgcc 17880
   cggaattgca tccgtggcct tgcaggcgca gagacactga ttaaaaacaa gttacatgtg 17940
   gaaaaatcaa aataaaagtc tggactctca cgctcgcttg gtcctgtaac tattttgtag 18000
   aatggaagac atcaactttg cgtcactggc cccgcgacac ggctcgcgcc cgttcatggg 18060
   aaactggcaa gatatcggca ccagcaatat gagcggtggc gccttcagct ggggctcgct 18120
   gtggagcggc attaaaaatt tcggttccgc cgttaagaac tatggcagca aagcctggaa 18180
   cagcagcaca ggccagatgc tgagggacaa gttgaaagag caaaatttcc aacaaaaggt 18240
   ggtagatggc ctggcctctg gcattagcgg ggtggtggac ctggccaacc aggcagtgca 18300
   aaataagatt aacagtaagc ttgatccccg ccctcccgta gaggagcctc caccggccgt 18360
   ggagacagtg tctccagagg ggcgtggcga aaagcgtccg cgacccgaca gggaagaaac 18420
   tctggtgacg caaatagacg agcctccctc gtacgaggag gcactaaagc aaggcctgcc 18480
   caccacccgt cccatcgcgc ccatggctac cggagtgctg ggccagcaca cacccgtaac 18540
   gctggacctg cctccccccg ccgacaccca gcagaaacct gtgctgccag gcccgtccgc 18600
   cgttgttgta acccgtccta gccgcgcgtc cctgcgccgc gccgccagcg gtccgcgatc 18660
   gttgcggccc gtagccagtg gcaactggca aagcacactg aacagcatcg tgggtttggg 18720
   ggtgcaatcc ctgaagcgcc gacgatgctt ctgatagcta acgtgtcgta tgtgtgtcat 18780
   gtatgcgtcc atgtcgccgc cagaggagct gctgagccgc cgcgcgcccg ctttccaaga 18840
   tggctacccc ttcgatgatg ccgcagtggt cttacatgca catctcgggc caggacgcct 18900
   cggagtacct gagccccggg ctggtgcagt tcgcccgcgc caccgagacg tacttcagcc 18960
   tgaataacaa gtttagaaac cccacggtgg cgcctacgca cgacgtgacc acagaccggt 19020
   ctcagcgttt gacgctgcgg ttcatccccg tggaccgcga ggatactgcg tactcgtaca 19080
   aggcgcggtt caccctagct gtgggtgata accgtgtgct agacatggct tccacgtact 19140
   ttgacatccg cggcgtgctg gacaggggcc ctacttttaa gccctactct ggcactgcct 19200
   acaacgcact ggcccccaag ggtgccccca actcgtgcga gtgggaacaa aatgaaactg 19260
   cacaagtgga tgctcaagaa cttgacgaag aggagaatga agccaatgaa gctcaggcgc 19320
   gagaacagga acaagctaag aaaacccatg tatatgccca ggctccactg tccggaataa 19380
   aaataactaa agaaggtcta caaataggaa ctgccgacgc cacagtagca ggtgccggca 19440
   aagaaatttt cgcagacaaa acttttcaac ctgaaccaca agtaggagaa tctcaatgga 19500
   acgaagcgga tgccacagca gctggtggaa gggttcttaa aaagacaact cccatgaaac 19560
   cctgctatgg ctcatacgct agacccacca attccaacgg cggacagggc gttatggttg 19620
   aacaaaatgg taaattggaa agtcaagtcg aaatgcaatt tttttccaca tccacaaatg 19680
   ccacaaatga agttaacaat atacaaccaa cagttgtatt gtacagcgaa gatgtaaaca 19740
   tggaaactcc agatactcat ctttcttata aacctaaaat gggggataaa aatgccaaag 19800
   tcatgcttgg acaacaagca atgccaaaca gaccaaatta cattgctttt agagacaatt 19860
   ttattggtct catgtattac aacagcacag gtaacatggg tgtccttgct ggtcaggcat 19920
   cgcagttgaa cgctgttgta gatttgcaag acagaaacac agagctgtcc taccagcttt 19980
   tgcttgattc aattggcgac agaacaagat acttttcaat gtggaatcaa gctgttgaca 20040
   gctatgatcc agatgtcaga attattgaga accatggaac tgaggatgag ttgccaaatt 20100
   attgctttcc tcttggtgga attgggatta ctgacacttt tcaagctgtt aaaacaactg 20160
   ctgctaacgg ggaccaaggc aatactacct ggcaaaaaga ttcaacattt gcagaacgca 20220
   atgaaatagg ggtgggaaat aactttgcca tggaaattaa cctgaatgcc aacctatgga 20280
   gaaatttcct ttactccaat attgcgctgt acctgccaga caagctaaaa tacaacccca 20340
   ccaatgtgga aatatctgac aaccccaaca cctacgacta catgaacaag cgagtggtgg 20400
   ctcctggcct tgtagactgc tacattaacc ttgggcgcgc ctggtctctg gactacatgg 20460
   acaacgttaa tccctttaac caccaccgca atgcgggcct gcgttaccgc tccatgttgt 20520
   tgggaaacgg ccgctacgtg ccctttcaca ttcaggtgcc ccaaaagttt tttgccatta 20580
   aaaacctcct cctcctgcca ggctcataca catatgaatg gaacttcagg aaggatgtta 20640
   acatggttct gcagagctct ctgggaaacg accttagagt tgacggggct agcattaagt 20700
   ttgacagcat ttgtctttac gccaccttct tccccatggc ccacaacacg gcctccacgc 20760
   tggaagccat gctcagaaat gacaccaacg accagtcctt taatgactac ctttccgccg 20820
   ccaacatgct atatcccata cccgccaacg ccaccaacgt gcccatctcc atcccatcgc 20880
   gcaactgggc agcatttcgc ggttgggcct tcacacgctt gaagacaaag gaaacccctt 20940
   ccctgggatc aggctacgac ccttactaca cctactctgg ctccatacca taccttgacg 21000
   gaaccttcta tcttaatcac acctttaaga aggtggccat tacttttgac tcttctgtta 21060
   gctggccggg caacgaccgc ctgcttactc ccaatgagtt tgagattaag cgctcagttg 21120
   acggggaggg ctataacgta gctcagtgca acatgacaaa ggactggttc ctagtgcaga 21180
   tgttggccaa ctacaatatt ggctaccagg gcttctacat tccagaaagc tacaaagacc 21240
   gcatgtactc gttcttcaga aacttccagc ccatgagccg gcaagtggtg gacgatacta 21300
   aatacaaaga ttatcagcag gttggaatta tccaccagca taacaactca ggcttcgtag 21360
   gctacctcgc tcccaccatg cgcgagggac aagcttaccc cgctaatgtt ccctacccac 21420
   taataggcaa aaccgcggtt gatagtatta cccagaaaaa gtttctttgc gaccgcaccc 21480
   tgtggcgcat ccccttctcc agtaacttta tgtccatggg tgcgctcaca gacctgggcc 21540
   aaaaccttct ctacgcaaac tccgcccacg cgctagacat gacctttgag gtggatccca 21600
   tggacgagcc cacccttctt tatgttttgt ttgaagtctt tgacgtggtc cgtgtgcacc 21660
   agccgcaccg cggcgtcatc gagaccgtgt acctgcgcac gcccttctcg gccggcaacg 21720
   ccacaacata aagaagcaag caacatcaac aacagctgcc gccatgggct ccagtgagca 21780
   ggaactgaaa gccattgtca aagatcttgg ttgtgggcca tattttttgg gcacctatga 21840
   caagcgcttc ccaggctttg tttccccaca caagctcgcc tgcgccatag ttaacacggc 21900
   cggtcgcgag actgggggcg tacactggat ggcctttgcc tggaacccgc gctcaaaaac 21960
   atgctacctc tttgagccct ttggcttttc tgaccaacgt ctcaagcagg tttaccagtt 22020
   tgagtacgag tcactcctgc gccgtagcgc cattgcctct tcccccgacc gctgtataac 22080
   gctggaaaag tccacccaaa gcgtgcaggg gcccaactcg gccgcctgtg gcctattctg 22140
   ctgcatgttt ctccacgcct ttgccaactg gccccaaact cccatggatc acaaccccac 22200
   catgaacctt attaccgggg tacccaactc catgcttaac agtccccagg tacagcccac 22260
   cctgcgccgc aaccaggaac agctctacag cttcctggag cgccactcgc cctacttccg 22320
   cagccacagt gcgcaaatta ggagcgccac ttctttttgt cacttgaaaa acatgtaaaa 22380
   ataatgtact aggagacact ttcaataaag gcaaatgttt ttatttgtac actctcgggt 22440
   gattatttac ccccaccctt gccgtctgcg ccgtttaaaa atcaaagggg ttctgccgcg 22500
   catcgctatg cgccactggc agggacacgt tgcgatactg gtgtttagtg ctccacttaa 22560
   actcaggcac aaccatccgc ggcagctcgg tgaagttttc actccacagg ctgcgcacca 22620
   tcaccaacgc gtttagcagg tcgggcgccg atatcttgaa gtcgcagttg gggcctccgc 22680
   cctgcgcgcg cgagttgcga tacacagggt tacagcactg gaacactatc agcgccgggt 22740
   ggtgcacgct ggccagcacg ctcttgtcgg agatcagatc cgcgtccagg tcctccgcgt 22800
   tgctcagggc gaacggagtc aactttggta gctgccttcc caaaaagggt gcatgcccag 22860
   gctttgagtt gcactcgcac cgtagtggca tcagaaggtg accgtgccca gtctgggcgt 22920
   taggatacag cgcctgcatg aaagccttga tctgcttaaa agccacctga gcctttgcgc 22980
   cttcagagaa gaacatgccg caagacttgc cggaaaactg attggccgga caggccgcgt 23040
   catgcacgca gcaccttgcg tcggtgttgg agatctgcac cacatttcgg ccccaccggt 23100
   tcttcacgat cttggccttg ctagactgct ccttcagcgc gcgctgcccg ttttcgctcg 23160
   tcacatccat ttcaatcacg tgctccttat ttatcataat gctcccgtgt agacacttaa 23220
   gctcgccttc gatctcagcg cagcggtgca gccacaacgc gcagcccgtg ggctcgtggt 23280
   gcttgtaggt tacctctgca aacgactgca ggtacgcctg caggaatcgc cccatcatcg 23340
   tcacaaaggt cttgttgctg gtgaaggtca gctgcaaccc gcggtgctcc tcgtttagcc 23400
   aggtcttgca tacggccgcc agagcttcca cttggtcagg cagtagcttg aagtttgcct 23460
   ttagatcgtt atccacgtgg tacttgtcca tcaacgcgcg cgcagcctcc atgcccttct 23520
   cccacgcaga cacgatcggc aggctcagcg ggtttatcac cgtgctttca ctttccgctt 23580
   cactggactc ttccttttcc tcttgcatcc gcataccccg cgccactggg tcgtcttcat 23640
   tcagccgccg caccgtgcgc ttacctccct tgccgtgctt gattagcacc ggtgggttgc 23700
   tgaaacccac catttgtagc gccacatctt ctctttcttc ctcgctgtcc acgatcacct 23760
   ctggggatgg cgggcgctcg ggcttgggag aggggcgctt ctttttcttt ttggacgcaa 23820
   tggccaaatc cgccgtcgag gtcgatggcc gcgggctggg tgtgcgcggc accagcgcat 23880
   cttgtgacga gtcttcttcg tcctcggact cgagacgccg cctcagccgc ttttttgggg 23940
   gcgcgcgggg aggcggcggc gacggcgacg gggacgagac gtcctccatg gttggtggac 24000
   gtcgcgccgc accgcgtccg cgctcggggg tggtttcgcg ctgctcctct tcccgactgg 24060
   ccatttcctt ctcctatagg cagaaaaaga tcatggagtc agtcgagaag gaggacagcc 24120
   taaccgcccc ctttgagttc gccaccaccg cctccaccga tgccgccaac gcgcctacca 24180
   ccttccccgt cgaggcaccc ccgcttgagg aggaggaagt gattatcgag caggacccag 24240
   gttttgtaag cgaagacgac gaagatcgct cagtaccaac agaggataaa aagcaagacc 24300
   aggacgacgc agaggcaaac gaggaacaag tcgggcgggg ggaccaaagg catggcgact 24360
   acctagatgt gggagacgac gtgctgttga agcatctgca gcgccagtgc gccattatct 24420
   gcgacgcgtt gcaagagcgc agcgatgtgc ccctcgccat agcggatgtc agccttgcct 24480
   acgaacgcca cctgttctca ccgcgcgtac cccccaaacg ccaagaaaac ggcacatgcg 24540
   agcccaaccc gcgcctcaac ttctaccccg tatttgccgt gccagaggtg cttgccacct 24600
   atcacatctt tttccaaaac tgcaagatac ccctatcctg ccgtgccaac cgcagccgag 24660
   cggacaagca gctggccttg cggcagggcg ctgtcatacc tgatatcgcc tcgctcgacg 24720
   aagtgccaaa aatctttgag ggtcttggac gcgacgagaa gcgcgcggca aacgctctgc 24780
   aacaagaaaa cagcgaaaat gaaagtcact gtggagtgct ggtggaactt gagggtgaca 24840
   acgcgcgcct agccgtgctg aaacgcagca tcgaggtcac ccactttgcc tacccggcac 24900
   ttaacctacc ccccaaggtt atgagcacag tcatgagcga gctgatcgtg cgccgtgcac 24960
   gacccctgga gagggatgca aacttgcaag aacaaaccga ggagggccta cccgcagttg 25020
   gcgatgagca gctggcgcgc tggcttgaga cgcgcgagcc tgccgacttg gaggagcgac 25080
   gcaagctaat gatggccgca gtgcttgtta ccgtggagct tgagtgcatg cagcggttct 25140
   ttgctgaccc ggagatgcag cgcaagctag aggaaacgtt gcactacacc tttcgccagg 25200
   gctacgtgcg ccaggcctgc aaaatttcca acgtggagct ctgcaacctg gtctcctacc 25260
   ttggaatttt gcacgaaaac cgccttgggc aaaacgtgct tcattccacg ctcaagggcg 25320
   aggcgcgccg cgactacgtc cgcgactgcg tttacttatt tctgtgctac acctggcaaa 25380
   cggccatggg cgtgtggcag cagtgcctgg aggagcgcaa cctgaaggag ctgcagaagc 25440
   tgctaaagca aaacttgaag gacctatgga cggccttcaa cgagcgctcc gtggccgcgc 25500
   acctggcgga cattatcttc cccgaacgcc tgcttaaaac cctgcaacag ggtctgccag 25560
   acttcaccag tcaaagcatg ttgcaaaact ttaggaactt tatcctagag cgttcaggaa 25620
   ttctgcccgc cacctgctgt gcgcttccta gcgactttgt gcccattaag taccgtgaat 25680
   gccctccgcc gctttggggt cactgctacc ttctgcagct agccaactac cttgcctacc 25740
   actccgacat catggaagac gtgagcggtg acggcctact ggagtgtcac tgtcgctgca 25800
   acctatgcac cccgcaccgc tccctggtct gcaattcaca actgcttagc gaaagtcaaa 25860
   ttatcggtac ctttgagctg cagggtccct cgcctgacga aaagtccgcg gctccggggt 25920
   tgaaactcac tccggggctg tggacgtcgg cttaccttcg caaatttgta cctgaggact 25980
   accacgccca cgagattagg ttctacgaag accaatcccg cccgccaaat gcggagctta 26040
   ccgcctgcgt cattacccag ggccacatcc ttggccaatt gcaagccatt aacaaagccc 26100
   gccaagagtt tctgctacga aagggacggg gggtttactt ggacccccag tccggcgagg 26160
   agctcaaccc aatccccccg ccgccgcagc cctatcagca gccgcgggcc cttgcttccc 26220
   aggatggcac ccaaaaagaa gctgcagctg ccgccgccgc cacccacgga cgaggaggaa 26280
   tactgggaca gtcaggcaga ggaggttttg gacgaggagg aggagatgat ggaagactgg 26340
   gacagcctag acgaggaagc ttccgaggcc gaagaggtgt cagacgaaac accgtcaccc 26400
   tcggtcgcat tcccctcgcc ggcgccccag aaatcggcaa ccgttcccag cattgctaca 26460
   acctccgctc ctcaggcgcc gccggcactg cccgttcgcc gacccaaccg tagatgggac 26520
   accactggaa ccagggccgg taagtctaag cagccgccgc cgttagccca agagcaacaa 26580
   cagcgccaag gctaccgctc gtggcgcgtg cacaagaacg ccatagttgc ttgcttgcaa 26640
   gactgtgggg gcaacatctc cttcgcccgc cgctttcttc tctaccatca cggcgtggcc 26700
   ttcccccgta acatcctgca ttactaccgt catctctaca gcccctactg caccggcggc 26760
   agcggcagca acagcagcgg ccacgcagaa gcaaaggcga ccggatagca agactctgac 26820
   aaagcccaag aaatccacag cggcggcagc agcaggagga ggagcactgc gtctggcgcc 26880
   caacgaaccc gtatcgaccc gcgagcttag aaacaggatt tttcccactc tgtatgctat 26940
   atttcaacag agcaggggcc aagaacaaga gctgaaaata aaaaacaggt ctctgcgctc 27000
   cctcacccgc agctgcctgt atcacaaaag cgaagatcag cttcggcgca cgctggaaga 27060
   cgcggaggct ctcttcagca aatactgcgc gctgactctt aaggactagt ttcgcgccct 27120
   ttctcaaatt taagcgcgaa aactacgtca tctccagcgg ccacacccgg cgccagcacc 27180
   tgtcgtcagc gccattatga gcaaggaaat tcccacgccc tacatgtgga gttaccagcc 27240
   acaaatggga cttgcggctg gagctgccca agactactca acccgaataa actacatgag 27300
   cgcgggaccc cacatgatat cccgggtcaa cggaatccgc gcccaccgaa accgaattct 27360
   cctcgaacag gcggctatta ccaccacacc tcgtaataac cttaatcccc gtagttggcc 27420
   cgctgccctg gtgtaccagg aaagtcccgc tcccaccact gtggtacttc ccagagacgc 27480
   ccaggccgaa gttcagatga ctaactcagg ggcgcagctt gcgggcggct ttcgtcacag 27540
   ggtgcggtcg cccgggcagg gtataactca cctgaaaatc agagggcgag gtattcagct 27600
   caacgacgag tcggtgagct cctctcttgg tctccgtccg gacgggacat ttcagatcgg 27660
   cggcgctggc cgctcttcat ttacgccccg tcaggcgatc ctaactctgc agacctcgtc 27720
   ctcggagccg cgctccggag gcattggaac tctacaattt attgaggagt tcgtgccttc 27780
   ggtttacttc aacccctttt ctggacctcc cggccactac ccggaccagt ttattcccaa 27840
   ctttgacgcg gtaaaagact cggcggacgg ctacgactga atgaccagtg gagaggcaga 27900
   gcaactgcgc ctgacacacc tcgaccactg ccgccgccac aagtgctttg cccgcggctc 27960
   cggtgagttt tgttactttg aattgcccga agagcatatc gagggcccgg cgcacggcgt 28020
   ccggctcacc acccaggtag agcttacacg tagcctgatt cgggagttta ccaagcgccc 28080
   cctgctagtg gagcgggagc ggggtccctg tgttctgacc gtggtttgca actgtcctaa 28140
   ccctggatta catcaagatc tttgttgtca tctctgtgct gagtataata aatacagaaa 28200
   ttagaatcta ctggggctcc tgtcgccatc ctgtgaacgc caccgttttt acccacccaa 28260
   agcagaccaa agcaaacctc acctccggtt tgcacaagcg ggccaataag taccttacct 28320
   ggtactttaa cggctcttca tttgtaattt acaacagttt ccagcgagac gaagtaagtt 28380
   tgccacacaa ccttctcggc ttcaactaca ccgtcaagaa aaacaccacc accaccctcc 28440
   tcacctgccg ggaacgtacg agtgcgtcac cggttgctgc gcccacacct acagcctgag 28500
   cgtaaccaga cattactccc attttcccaa aacaggaggt gagctcaact cccggaactc 28560
   aggtcaaaaa agcattttgc ggggtgctgg gattttttaa ttaagtatat gagcaattca 28620
   agtaactcta caagcttgtc taatttttct ggaattgggg tcggggttat ccttactctt 28680
   gtaattctgt ttattcttat actagcactt ctgtgcctta gggttgccgc ctgctgcacg 28740
   cacgtttgta cctattgtca gctttttaaa cgctgggggc gacatccaag atgaggtaca 28800
   tgattttagg cttgctcgcc cttgcggcag tctgcagcgc tgccaaaaag gttgagttta 28860
   aggaaccagc ttgcaatgtt acatttaaat cagaagctaa tgaatgcact actcttataa 28920
   aatgcaccac agaacatgaa aagcttatta ttcgccacaa agacaaaatt ggcaagtatg 28980
   ctgtatatgc tatttggcag ccaggtgaca ctaacgacta taatgtcaca gtcttccaag 29040
   gtgaaaatcg taaaactttt atgtataaat ttccatttta tgaaatgtgc gatattacca 29100
   tgtacatgag caaacagtac aagttgtggc ccccacaaaa gtgtttagag aacactggca 29160
   ccttttgttc caccgctctg cttattacag cgcttgcttt ggtatgtacc ttactttatc 29220
   tcaaatacaa aagcagacgc agttttattg atgaaaagaa aatgccttga ttttccgctt 29280
   gcttgtattc ccctggacaa tttactctat gtgggatatg cgccaggcgg gaaagattat 29340
   acccacaacc ttcaaatcaa actttcctgg acgttagcgc ctgacttctg ccagcgcctg 29400
   cactgcaaat ttgatcaaac ccagcttcag cttgcctgct ccagagatga ccggctcaac 29460
   catcgcgccc acaacggact atcgcaacac cactgctacc ggactaaaat ctgccctaaa 29520
   tttaccccaa gttcatgcct ttgtcaatga ctgggcgagc ttgggcatgt ggtggttttc 29580
   catagcgctt atgtttgttt gccttattat tatgtggctt atttgttgcc taaagcgcag 29640
   acgcgccaga ccccccatct ataggcctat cattgtgctc aacccacaca atgaaaaaat 29700
   tcatagattg gacggtctca aaccatgttc tcttctttta cagtatgatt aaatgagaca 29760
   tgattcctcg agtccttata ttattgaccc ttgttgcgct tttctgtgcg tgctctacat 29820
   tggctgcggt cgctcacatc gaagtagatt gcatcccacc tttcacagtt tacctgcttt 29880
   acggatttgt cacccttatc ctcatctgca gcctcgtcac tgtagtcatc gccttcattc 29940
   agttcattga ctggatttgt gtgcgcattg cgtaccttag gcaccatccg caatacagag 30000
   acaggactat agctgatctt ctcagaattc tttaattatg aaacggattg tcacttttgt 30060
   tttgctgatt ttctgcgccc tacctgtgct ttgctcccaa acctcagcgc ctcccaaaag 30120
   acatatttcc tgcagattca ctcaaatatg gaacattccc agctgctaca acaaacagag 30180
   cgatttgtca gaagcctggt tatacgccat catctctgtc atggtttttt gcagtaccat 30240
   ttttgcccta gccatatacc cataccttga cattggttgg aatgccatag atgccatgaa 30300
   ccaccctact ttcccagcgc ccaatgtcat accactgcaa caggttattg ccccaatcaa 30360
   tcagcctcgc cccccttctc ccacccccac tgagattagc tactttaatt tgacaggtgg 30420
   agatgactga atctctagat ctagaattgg atggaattaa caccgaacag cgcctactag 30480
   aaaggcgcaa ggcggcgtcc gagcgagaac gcctaaaaca agaagttgaa gacatggtta 30540
   acctgcacca gtgtaaaaga ggtatctttt gtgtggtcaa gcaggccaaa cttacctacg 30600
   aaaaaaccac taccggcaac cgccttagct acaagctacc cacccagcgc caaaaactgg 30660
   tgcttatggt gggagaaaaa cctatcaccg tcacccagca ctcggcagaa acagaaggct 30720
   gcctgcactt cccctatcag ggtccagagg acctctgcac tcttattaaa accatgtgtg 30780
   gcattagaga tcttattcca ttcaactaac aataaacaca caataaatta cttacttaaa 30840
   atcagtcagc aaatctttgt ccagcttatt cagcatcacc tcctttccct cctcccaact 30900
   ctggtatttc agcagccttt tagctgcgaa ctttctccaa agtctaaatg ggatgtcaaa 30960
   ttcctcatgt tcttgtccct ccgcacccac tatcttcata ttgttgcaga tgaaacgcgc 31020
   cagaccgtct gaagacacct tcaaccctgt gtacccatat gacacggaaa ccggccctcc 31080
   aactgtgcct ttccttaccc ctccctttgt gtcgccaaat gggttccaag aaagtccccc 31140
   cggagtgctt tctttgcgtc tttcagaacc tttggttacc tcacacggca tgcttgcgct 31200
   aaaaatgggc agcggcctgt ccctggatca ggcaggcaac cttacatcaa atacaatcac 31260
   tgtttctcaa ccgctaaaaa aaacaaagtc caatataact ttggaaacat ccgcgcccct 31320
   tacagtcagc tcaggcgccc taaccatggc cacaacttcg cctttggtgg tctctgacaa 31380
   cactcttacc atgcaatcac aagcaccgct aaccgtgcaa gactcaaaac ttagcattgc 31440
   taccaaagag ccacttacag tgttagatgg aaaactggcc ctgcagacat cagcccccct 31500
   ctctgccact gataacaacg ccctcactat cactgcctca cctcctctta ctactgcaaa 31560
   tggtagtctg gctgttacca tggaaaaccc actttacaac aacaatggaa aacttgggct 31620
   caaaattggc ggtcctttgc aagtggccac cgactcacat gcactaacac taggtactgg 31680
   tcagggggtt gcagttcata acaatttgct acatacaaaa gttacaggcg caatagggtt 31740
   tgatacatct ggcaacatgg aacttaaaac tggagatggc ctctatgtgg atagcgccgg 31800
   tcctaaccaa aaactacata ttaatctaaa taccacaaaa ggccttgctt ttgacaacac 31860
   cgcaataaca attaacgctg gaaaagggtt ggaatttgaa acagactcct caaacggaaa 31920
   tcccataaaa acaaaaattg gatcaggcat acaatataat accaatggag ctatggttgc 31980
   aaaacttgga acaggcctca gttttgacag ctccggagcc ataacaatgg gcagcataaa 32040
   caatgacaga cttactcttt ggacaacacc agacccatcc ccaaattgca gaattgcttc 32100
   agataaagac tgcaagctaa ctctggcgct aacaaaatgt ggcagtcaaa ttttgggcac 32160
   tgtttcagct ttggcagtat caggtaatat ggcctccatc aatggaactc taagcagtgt 32220
   aaacttggtt cttagatttg atgacaacgg agtgcttatg tcaaattcat cactggacaa 32280
   acagtattgg aactttagaa acggggactc cactaacggt caaccataca cttatgctgt 32340
   tgggtttatg ccaaacctaa aagcttaccc aaaaactcaa agtaaaactg caaaaagtaa 32400
   tattgttagc caggtgtatc ttaatggtga caagtctaaa ccattgcatt ttactattac 32460
   gctaaatgga acagatgaaa ccaaccaagt aagcaaatac tcaatatcat tcagttggtc 32520
   ctggaacagt ggacaataca ctaatgacaa atttgccacc aattcctata ccttctccta 32580
   cattgcccag gaataaagaa tcgtgaacct gttgcatgtt atgtttcaac gtgtttattt 32640
   ttcaattgca gaaaatttca agtcattttt cattcagtag tatagcccca ccaccacata 32700
   gcttatacta atcaccgtac cttaatcaaa ctcacagaac cctagtattc aacctgccac 32760
   ctccctccca acacacagag tacacagtcc tttctccccg gctggcctta aacagcatca 32820
   tatcatgggt aacagacata ttcttaggtg ttatattcca cacggtctcc tgtcgagcca 32880
   aacgctcatc agtgatgtta ataaactccc cgggcagctc gcttaagttc atgtcgctgt 32940
   ccagctgctg agccacaggc tgctgtccaa cttgcggttg ctcaacgggc ggcgaaggag 33000
   aagtccacgc ctacatgggg gtagagtcat aatcgtgcat caggataggg cggtggtgct 33060
   gcagcagcgc gcgaataaac tgctgccgcc gccgctccgt cctgcaggaa tacaacatgg 33120
   cagtggtctc ctcagcgatg attcgcaccg cccgcagcat aaggcgcctt gtcctccggg 33180
   cacagcagcg caccctgatc tcacttaagt cagcacagta actgcagcac agtaccacaa 33240
   tattgtttaa aatcccacag tgcaaggcgc tgtatccaaa gctcatggcg gggaccacag 33300
   aacccacgtg gccatcatac cacaagcgca ggtagattaa gtggcgaccc ctcataaaca 33360
   cgctggacat aaacattacc tcttttggca tgttgtaatt caccacctcc cggtaccata 33420
   taaacctctg attaaacatg gcgccatcca ccaccatcct aaaccagctg gccaaaacct 33480
   gcccgccggc tatgcactgc agggaaccgg gactggaaca atgacagtgg agagcccagg 33540
   actcgtaacc atggatcatc atgctcgtca tgatatcaat gttggcacaa cacaggcaca 33600
   cgtgcataca cttcctcagg attacaagct cctcccgcgt cagaaccata tcccagggaa 33660
   caacccattc ctgaatcagc gtaaatccca cactgcaggg aagacctcgc acgtaactca 33720
   cgttgtgcat tgtcaaagtg ttacattcgg gcagcagcgg atgatcctcc agtatggtag 33780
   cgcgtgtctc tgtctcaaaa ggaggtaggc gatccctact gtacggagtg cgccgagaca 33840
   accgagatcg tgttggtcgt agtgtcatgc caaatggaac gccggacgta gtcatatttc 33900
   ctgaagcaaa accaggtgcg ggcgtgacaa acagatctgc gtctccggtc tcgtcgctta 33960
   gctcgctctg tgtagtagtt gtagtatatc cactctctca aagcatccag gcgccccctg 34020
   gcttcgggtt ctatgtaaac tccttcatgc gccgctgccc tgataacatc caccaccgca 34080
   gaataagcca cacccagcca acctacacat tcgttctgcg agtcacacac gggaggagcg 34140
   ggaagagctg gaagaaccat gttttttttt tttattccaa aagattatcc aaaacctcaa 34200
   aatgaagatc tattaagtga acgcgctccc ctccggtggc gtggtcaaac tctacagcca 34260
   aagaacagat aatggcattt gtaagatgtt gcacaatggc ttccaaaagg caaactgccc 34320
   tcacgtccaa gtggacgtaa aggctaaacc cttcagggtg aatctcctct ataaacattc 34380
   cagcaccttc aaccatgccc aaataatttt catctcgcca ccttatcaat atgtctctaa 34440
   gcaaatcccg aatattaagt ccggccattg taaaaatctg ctccagagcg ccctccacct 34500
   tcagcctcaa gcagcgaatc atgattgcaa aaattcaggt tcctcacaga cctgtataag 34560
   attcaaaagc ggaacattaa caaaaatacc gcgatcccgt aggtcccttc gcagggccag 34620
   ctgaacataa tcgtgcaggt ctgcacggac cagcgcggcc acttccccgc caggaaccat 34680
   gacaaaagaa cccacactga ttatgacacg catactcgga gctatgctaa ccagcgtagc 34740
   cccgatgtaa gcttgttgca tgggcggcga tataaaatgc aaggtactgc tcaaaaaatc 34800
   aggcaaagcc tcgcgcaaaa aagcaagcac atcgtagtca tgctcatgca gataaaggca 34860
   ggtaagttcc ggaaccacca cagaaaaaga caccattttt ctctcaaaca tgtctgcggg 34920
   ttcctgcata aacacaaaat aaaataacaa aaaaaaaaaa acatttaaac attagaagcc 34980
   tgtnttacaa caggaaaaac aacccttata agcataagac ggactacggc catgccggcg 35040
   tgaccgtaaa aaaactggtc accgtgatta aaaagcacca ccgacagttc ctcggtcatg 35100
   tccggagtca taatgtaaga ctcggtaaac acatcaggtt ggttaacatc ggtcagtgct 35160
   aaaaagcgac cgaaatagcc cgggggaata catacccgca ggcgtagaga caacattaca 35220
   gcccccatag gaggtataac aaaattaata ggagagaaaa acacataaac acctgaaaaa 35280
   ccctcctgcc taggcaaaat agcaccctcc cgctccagaa caacatacag cgcttccaca 35340
   gcggcagcca taacagtcag ccttaccagt aaaaaaacct attaaaaaac accactcgac 35400
   acggcaccag ctcaatcagt cacagtgtaa aaagggccaa gtacagagcg agtatatata 35460
   ggactaaaaa atgacgtaac ggttaaagtc cacaaaaacc acccagaaaa ccgcacgcga 35520
   acctacgccc agaaacgaaa gccaaaaaac ccacaacttc ctcaaatctt cacttccgtt 35580
   ttcccacgat acgtcacttc ccattttaaa aaaaaactac aattcccaat acatgcaagt 35640
   tactccgccc taaaacctac gtcacccgcc ccgttcccac gccccgcgcc acgtcacaaa 35700
   ctccaccccc tcattatcat attggcttca atccaaaata aggtatatta ttgatgatg 35759

<210> 9
<211> 35935
<212> DNA
<213> Adenovirus serotype 5

<400> 9
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa tatttgtcta gggccgcggg 360
   gactttgacc gtttacgtgg agactcgccc aggtgttttt ctcaggtgtt ttccgcgttc 420
   cgggtcaaag ttggcgtttt attattatag tcagctgacg tgtagtgtat ttatacccgg 480
   tgagttcctc aagaggccac tcttgagtgc cagcgagtag agttttctcc tccgagccgc 540
   tccgacaccg ggactgaaaa tgagacatat tatctgccac ggaggtgtta ttaccgaaga 600
   aatggccgcc agtcttttgg accagctgat cgaagaggta ctggctgata atcttccacc 660
   tcctagccat tttgaaccac ctacccttca cgaactgtat gatttagacg tgacggcccc 720
   cgaagatccc aacgaggagg cggtttcgca gatttttccc gactctgtaa tgttggcggt 780
   gcaggaaggg attgacttac tcacttttcc gccggcgccc ggttctccgg agccgcctca 840
   cctttcccgg cagcccgagc agccggagca gagagccttg ggtccggttt ctatgccaaa 900
   ccttgtaccg gaggtgatcg atcttacctg ccacgaggct ggctttccac ccagtgacga 960
   cgaggatgaa gagggtgagg agtttgtgtt agattatgtg gagcaccccg ggcacggttg 1020
   caggtcttgt cattatcacc ggaggaatac gggggaccca gatattatgt gttcgctttg 1080
   ctatatgagg acctgtggca tgtttgtcta cagtaagtga aaattatggg cagtgggtga 1140
   tagagtggtg ggtttggtgt ggtaattttt tttttaattt ttacagtttt gtggtttaaa 1200
   gaattttgta ttgtgatttt tttaaaaggt cctgtgtctg aacctgagcc tgagcccgag 1260
   ccagaaccgg agcctgcaag acctacccgc cgtcctaaaa tggcgcctgc tatcctgaga 1320
   cgcccgacat cacctgtgtc tagagaatgc aatagtagta cggatagctg tgactccggt 1380
   ccttctaaca cacctcctga gatacacccg gtggtcccgc tgtgccccat taaaccagtt 1440
   gccgtgagag ttggtgggcg tcgccaggct gtggaatgta tcgaggactt gcttaacgag 1500
   cctgggcaac ctttggactt gagctgtaaa cgccccaggc cataaggtgt aaacctgtga 1560
   ttgcgtgtgt ggttaacgcc tttgtttgct gaatgagttg atgtaagttt aataaagggt 1620
   gagataatgt ttaacttgca tggcgtgtta aatggggcgg ggcttaaagg gtatataatg 1680
   cgccgtgggc taatcttggt tacatctgac ctcatggagg cttgggagtg tttggaagat 1740
   ttttctgctg tgcgtaactt gctggaacag agctctaaca gtacctcttg gttttggagg 1800
   tttctgtggg gctcatccca ggcaaagtta gtctgcagaa ttaaggagga ttacaagtgg 1860
   gaatttgaag agcttttgaa atcctgtggt gagctgtttg attctttgaa tctgggtcac 1920
   caggcgcttt tccaagagaa ggtcatcaag actttggatt tttccacacc ggggcgcgct 1980
   gcggctgctg ttgctttttt gagttttata aaggataaat ggagcgaaga aacccatctg 2040
   agcggggggt acctgctgga ttttctggcc atgcatctgt ggagagcggt tgtgagacac 2100
   aagaatcgcc tgctactgtt gtcttccgtc cgcccggcga taataccgac ggaggagcag 2160
   cagcagcagc aggaggaagc caggcggcgg cggcaggagc agagcccatg gaacccgaga 2220
   gccggcctgg accctcggga atgaatgttg tacaggtggc tgaactgtat ccagaactga 2280
   gacgcatttt gacaattaca gaggatgggc aggggctaaa gggggtaaag agggagcggg 2340
   gggcttgtga ggctacagag gaggctagga atctagcttt tagcttaatg accagacacc 2400
   gtcctgagtg tattactttt caacagatca aggataattg cgctaatgag cttgatctgc 2460
   tggcgcagaa gtattccata gagcagctga ccacttactg gctgcagcca ggggatgatt 2520
   ttgaggaggc tattagggta tatgcaaagg tggcacttag gccagattgc aagtacaaga 2580
   tcagcaaact tgtaaatatc aggaattgtt gctacatttc tgggaacggg gccgaggtgg 2640
   agatagatac ggaggatagg gtggccttta gatgtagcat gataaatatg tggccggggg 2700
   tgcttggcat ggacggggtg gttattatga atgtaaggtt tactggcccc aattttagcg 2760
   gtacggtttt cctggccaat accaacctta tcctacacgg tgtaagcttc tatgggttta 2820
   acaatacctg tgtggaagcc tggaccgatg taagggttcg gggctgtgcc ttttactgct 2880
   gctggaaggg ggtggtgtgt cgccccaaaa gcagggcttc aattaagaaa tgcctctttg 2940
   aaaggtgtac cttgggtatc ctgtctgagg gtaactccag ggtgcgccac aatgtggcct 3000
   ccgactgtgg ttgcttcatg ctagtgaaaa gcgtggctgt gattaagcat aacatggtat 3060
   gtggcaactg cgaggacagg gcctctcaga tgctgacctg ctcggacggc aactgtcacc 3120
   tgctgaagac cattcacgta gccagccact ctcgcaaggc ctggccagtg tttgagcata 3180
   acatactgac ccgctgttcc ttgcatttgg gtaacaggag gggggtgttc ctaccttacc 3240
   aatgcaattt gagtcacact aagatattgc ttgagcccga gagcatgtcc aaggtgaacc 3300
   tgaacggggt gtttgacatg accatgaaga tctggaaggt gctgaggtac gatgagaccc 3360
   gcaccaggtg cagaccctgc gagtgtggcg gtaaacatat taggaaccag cctgtgatgc 3420
   tggatgtgac cgaggagctg aggcccgatc acttggtgct ggcctgcacc cgcgctgagt 3480
   ttggctctag cgatgaagat acagattgag gtactgaaat gtgtgggcgt ggcttaaggg 3540
   tgggaaagaa tatataaggt gggggtctta tgtagttttg tatctgtttt gcagcagccg 3600
   ccgccgccat gagcaccaac tcgtttgatg gaagcattgt gagctcatat ttgacaacgc 3660
   gcatgccccc atgggccggg gtgcgtcaga atgtgatggg ctccagcatt gatggtcgcc 3720
   ccgtcctgcc cgcaaactct actaccttga cctacgagac cgtgtctgga acgccgttgg 3780
   agactgcagc ctccgccgcc gcttcagccg ctgcagccac cgcccgcggg attgtgactg 3840
   actttgcttt cctgagcccg cttgcaagca gtgcagcttc ccgttcatcc gcccgcgatg 3900
   acaagttgac ggctcttttg gcacaattgg attctttgac ccgggaactt aatgtcgttt 3960
   ctcagcagct gttggatctg cgccagcagg tttctgccct gaaggcttcc tcccctccca 4020
   atgcggttta aaacataaat aaaaaaccag actctgtttg gatttggatc aagcaagtgt 4080
   cttgctgtct ttatttaggg gttttgcgcg cgcggtaggc ccgggaccag cggtctcggt 4140
   cgttgagggt cctgtgtatt ttttccagga cgtggtaaag gtgactctgg atgttcagat 4200
   acatgggcat aagcccgtct ctggggtgga ggtagcacca ctgcagagct tcatgctgcg 4260
   gggtggtgtt gtagatgatc cagtcgtagc aggagcgctg ggcgtggtgc ctaaaaatgt 4320
   ctttcagtag caagctgatt gccaggggca ggcccttggt gtaagtgttt acaaagcggt 4380
   taagctggga tgggtgcata cgtggggata tgagatgcat cttggactgt atttttaggt 4440
   tggctatgtt cccagccata tccctccggg gattcatgtt gtgcagaacc accagcacag 4500
   tgtatccggt gcacttggga aatttgtcat gtagcttaga aggaaatgcg tggaagaact 4560
   tggagacgcc cttgtgacct ccaagatttt ccatgcattc gtccataatg atggcaatgg 4620
   gcccacgggc ggcggcctgg gcgaagatat ttctgggatc actaacgtca tagttgtgtt 4680
   ccaggatgag atcgtcatag gccattttta caaagcgcgg gcggagggtg ccagactgcg 4740
   gtataatggt tccatccggc ccaggggcgt agttaccctc acagatttgc atttcccacg 4800
   ctttgagttc agatgggggg atcatgtcta cctgcggggc gatgaagaaa acggtttccg 4860
   ggtaggggga gatcagctgg gaagaaagca ggttcctgag cagctgcgac ttaccgcagc 4920
   cggtgggccc gtaaatcaca cctattaccg ggtgcaactg gtagttaaga gagctgcagc 4980
   tgccgtcatc cctgagcagg ggggccactt cgttaagcat gtccctgact cgcatgtttt 5040
   ccctgaccaa atccgccaga aggcgctcgc cgcccagcga tagcagttct tgcaaggaag 5100
   caaagttttt caacggtttg agaccgtccg ccgtaggcat gcttttgagc gtttgaccaa 5160
   gcagttccag gcggtcccac agctcggtca cctgctctac ggcatctcga tccagcatat 5220
   ctcctcgttt cgcgggttgg ggcggctttc gctgtacggc agtagtcggt gctcgtccag 5280
   acgggccagg gtcatgtctt tccacgggcg cagggtcctc gtcagcgtag tctgggtcac 5340
   ggtgaagggg tgcgctccgg gctgcgcgct ggccagggtg cgcttgaggc tggtcctgct 5400
   ggtgctgaag cgctgccggt cttcgccctg cgcgtcggcc aggtagcatt tgaccatggt 5460
   gtcatagtcc agcccctccg cggcgtggcc cttggcgcgc agcttgccct tggaggaggc 5520
   gccgcacgag gggcagtgca gacttttgag ggcgtagagc ttgggcgcga gaaataccga 5580
   ttccggggag taggcatccg cgccgcaggc cccgcagacg gtctcgcatt ccacgagcca 5640
   ggtgagctct ggccgttcgg ggtcaaaaac caggtttccc ccatgctttt tgatgcgttt 5700
   cttacctctg gtttccatga gccggtgtcc acgctcggtg acgaaaaggc tgtccgtgtc 5760
   cccgtataca gacttgagag gcctgtcctc gagcggtgtt ccgcggtcct cctcgtatag 5820
   aaactcggac cactctgaga caaaggctcg cgtccaggcc agcacgaagg aggctaagtg 5880
   ggaggggtag cggtcgttgt ccactagggg gtccactcgc tccagggtgt gaagacacat 5940
   gtcgccctct tcggcatcaa ggaaggtgat tggtttgtag gtgtaggcca cgtgaccggg 6000
   tgttcctgaa ggggggctat aaaagggggt gggggcgcgt tcgtcctcac tctcttccgc 6060
   atcgctgtct gcgagggcca gctgttgggg tgagtactcc ctctgaaaag cgggcatgac 6120
   ttctgcgcta agattgtcag tttccaaaaa cgaggaggat ttgatattca cctggcccgc 6180
   ggtgatgcct ttgagggtgg ccgcatccat ctggtcagaa aagacaatct ttttgttgtc 6240
   aagcttggtg gcaaacgacc cgtagagggc gttggacagc aacttggcga tggagcgcag 6300
   ggtttggttt ttgtcgcgat cggcgcgctc cttggccgcg atgtttagct gcacgtattc 6360
   gcgcgcaacg caccgccatt cgggaaagac ggtggtgcgc tcgtcgggca ccaggtgcac 6420
   gcgccaaccg cggttgtgca gggtgacaag gtcaacgctg gtggctacct ctccgcgtag 6480
   gcgctcgttg gtccagcaga ggcggccgcc cttgcgcgag cagaatggcg gtagggggtc 6540
   tagctgcgtc tcgtccgggg ggtctgcgtc cacggtaaag accccgggca gcaggcgcgc 6600
   gtcgaagtag tctatcttgc atccttgcaa gtctagcgcc tgctgccatg cgcgggcggc 6660
   aagcgcgcgc tcgtatgggt tgagtggggg accccatggc atggggtggg tgagcgcgga 6720
   ggcgtacatg ccgcaaatgt cgtaaacgta gaggggctct ctgagtattc caagatatgt 6780
   agggtagcat cttccaccgc ggatgctggc gcgcacgtaa tcgtatagtt cgtgcgaggg 6840
   agcgaggagg tcgggaccga ggttgctacg ggcgggctgc tctgctcgga agactatctg 6900
   cctgaagatg gcatgtgagt tggatgatat ggttggacgc tggaagacgt tgaagctggc 6960
   gtctgtgaga cctaccgcgt cacgcacgaa ggaggcgtag gagtcgcgca gcttgttgac 7020
   cagctcggcg gtgacctgca cgtctagggc gcagtagtcc agggtttcct tgatgatgtc 7080
   atacttatcc tgtccctttt ttttccacag ctcgcggttg aggacaaact cttcgcggtc 7140
   tttccagtac tcttggatcg gaaacccgtc ggcctccgaa cggtaagagc ctagcatgta 7200
   gaactggttg acggcctggt aggcgcagca tcccttttct acgggtagcg cgtatgcctg 7260
   cgcggccttc cggagcgagg tgtgggtgag cgcaaaggtg tccctgacca tgactttgag 7320
   gtactggtat ttgaagtcag tgtcgtcgca tccgccctgc tcccagagca aaaagtccgt 7380
   gcgctttttg gaacgcggat ttggcagggc gaaggtgaca tcgttgaaga gtatctttcc 7440
   cgcgcgaggc ataaagttgc gtgtgatgcg gaagggtccc ggcacctcgg aacggttgtt 7500
   aattacctgg gcggcgagca cgatctcgtc aaagccgttg atgttgtggc ccacaatgta 7560
   aagttccaag aagcgcggga tgcccttgat ggaaggcaat tttttaagtt cctcgtaggt 7620
   gagctcttca ggggagctga gcccgtgctc tgaaagggcc cagtctgcaa gatgagggtt 7680
   ggaagcgacg aatgagctcc acaggtcacg ggccattagc atttgcaggt ggtcgcgaaa 7740
   ggtcctaaac tggcgaccta tggccatttt ttctggggtg atgcagtaga aggtaagcgg 7800
   gtcttgttcc cagcggtccc atccaaggtt cgcggctagg tctcgcgcgg cagtcactag 7860
   aggctcatct ccgccgaact tcatgaccag catgaagggc acgagctgct tcccaaaggc 7920
   ccccatccaa gtataggtct ctacatcgta ggtgacaaag agacgctcgg tgcgaggatg 7980
   cgagccgatc gggaagaact ggatctcccg ccaccaattg gaggagtggc tattgatgtg 8040
   gtgaaagtag aagtccctgc gacgggccga acactcgtgc tggcttttgt aaaaacgtgc 8100
   gcagtactgg cagcggtgca cgggctgtac atcctgcacg aggttgacct gacgaccgcg 8160
   cacaaggaag cagagtggga atttgagccc ctcgcctggc gggtttggct ggtggtcttc 8220
   tacttcggct gcttgtcctt gaccgtctgg ctgctcgagg ggagttacgg tggatcggac 8280
   caccacgccg cgcgagccca aagtccagat gtccgcgcgc ggcggtcgga gcttgatgac 8340
   aacatcgcgc agatgggagc tgtccatggt ctggagctcc cgcggcgtca ggtcaggcgg 8400
   gagctcctgc aggtttacct cgcatagacg ggtcagggcg cgggctagat ccaggtgata 8460
   cctaatttcc aggggctggt tggtggcggc gtcgatggct tgcaagaggc cgcatccccg 8520
   cggcgcgact acggtaccgc gcggcgggcg gtgggccgcg ggggtgtcct tggatgatgc 8580
   atctaaaagc ggtgacgcgg gcgagccccc ggaggtaggg ggggctccgg acccgccggg 8640
   agagggggca ggggcacgtc ggcgccgcgc gcgggcagga gctggtgctg cgcgcgtagg 8700
   ttgctggcga acgcgacgac gcggcggttg atctcctgaa tctggcgcct ctgcgtgaag 8760
   acgacgggcc cggtgagctt gagcctgaaa gagagttcga cagaatcaat ttcggtgtcg 8820
   ttgacggcgg cctggcgcaa aatctcctgc acgtctcctg agttgtcttg ataggcgatc 8880
   tcggccatga actgctcgat ctcttcctcc tggagatctc cgcgtccggc tcgctccacg 8940
   gtggcggcga ggtcgttgga aatgcgggcc atgagctgcg agaaggcgtt gaggcctccc 9000
   tcgttccaga cgcggctgta gaccacgccc ccttcggcat cgcgggcgcg catgaccacc 9060
   tgcgcgagat tgagctccac gtgccgggcg aagacggcgt agtttcgcag gcgctgaaag 9120
   aggtagttga gggtggtggc ggtgtgttct gccacgaaga agtacataac ccagcgtcgc 9180
   aacgtggatt cgttgatatc ccccaaggcc tcaaggcgct ccatggcctc gtagaagtcc 9240
   acggcgaagt tgaaaaactg ggagttgcgc gccgacacgg ttaactcctc ctccagaaga 9300
   cggatgagct cggcgacagt gtcgcgcacc tcgcgctcaa aggctacagg ggcctcttct 9360
   tcttcttcaa tctcctcttc cataagggcc tccccttctt cttcttctgg cggcggtggg 9420
   ggagggggga cacggcggcg acgacggcgc accgggaggc ggtcgacaaa gcgctcgatc 9480
   atctccccgc ggcgacggcg catggtctcg gtgacggcgc ggccgttctc gcgggggcgc 9540
   agttggaaga cgccgcccgt catgtcccgg ttatgggttg gcggggggct gccatgcggc 9600
   agggatacgg cgctaacgat gcatctcaac aattgttgtg taggtactcc gccgccgagg 9660
   gacctgagcg agtccgcatc gaccggatcg gaaaacctct cgagaaaggc gtctaaccag 9720
   tcacagtcgc aaggtaggct gagcaccgtg gcgggcggca gcgggcggcg gtcggggttg 9780
   tttctggcgg aggtgctgct gatgatgtaa ttaaagtagg cggtcttgag acggcggatg 9840
   gtcgacagaa gcaccatgtc cttgggtccg gcctgctgaa tgcgcaggcg gtcggccatg 9900
   ccccaggctt cgttttgaca tcggcgcagg tctttgtagt agtcttgcat gagcctttct 9960
   accggcactt cttcttctcc ttcctcttgt cctgcatctc ttgcatctat cgctgcggcg 10020
   gcggcggagt ttggccgtag gtggcgccct cttcctccca tgcgtgtgac cccgaagccc 10080
   ctcatcggct gaagcagggc taggtcggcg acaacgcgct cggctaatat ggcctgctgc 10140
   acctgcgtga gggtagactg gaagtcatcc atgtccacaa agcggtggta tgcgcccgtg 10200
   ttgatggtgt aagtgcagtt ggccataacg gaccagttaa cggtctggtg acccggctgc 10260
   gagagctcgg tgtacctgag acgcgagtaa gccctcgagt caaatacgta gtcgttgcaa 10320
   gtccgcacca ggtactggta tcccaccaaa aagtgcggcg gcggctggcg gtagaggggc 10380
   cagcgtaggg tggccggggc tccgggggcg agatcttcca acataaggcg atgatatccg 10440
   tagatgtacc tggacatcca ggtgatgccg gcggcggtgg tggaggcgcg cggaaagtcg 10500
   cggacgcggt tccagatgtt gcgcagcggc aaaaagtgct ccatggtcgg gacgctctgg 10560
   ccggtcaggc gcgcgcaatc gttgacgctc tagaccgtgc aaaaggagag cctgtaagcg 10620
   ggcactcttc cgtggtctgg tggataaatt cgcaagggta tcatggcgga cgaccggggt 10680
   tcgagccccg tatccggccg tccgccgtga tccatgcggt taccgcccgc gtgtcgaacc 10740
   caggtgtgcg acgtcagaca acgggggagt gctccttttg gcttccttcc aggcgcggcg 10800
   gctgctgcgc tagctttttt ggccactggc cgcgcgcagc gtaagcggtt aggctggaaa 10860
   gcgaaagcat taagtggctc gctccctgta gccggagggt tattttccaa gggttgagtc 10920
   gcgggacccc cggttcgagt ctcggaccgg ccggactgcg gcgaacgggg gtttgcctcc 10980
   ccgtcatgca agaccccgct tgcaaattcc tccggaaaca gggacgagcc ccttttttgc 11040
   ttttcccaga tgcatccggt gctgcggcag atgcgccccc ctcctcagca gcggcaagag 11100
   caagagcagc ggcagacatg cagggcaccc tcccctcctc ctaccgcgtc aggaggggcg 11160
   acatccgcgg ttgacgcggc agcagatggt gattacgaac ccccgcggcg ccgggcccgg 11220
   cactacctgg acttggagga gggcgagggc ctggcgcggc taggagcgcc ctctcctgag 11280
   cggtacccaa gggtgcagct gaagcgtgat acgcgtgagg cgtacgtgcc gcggcagaac 11340
   ctgtttcgcg accgcgaggg agaggagccc gaggagatgc gggatcgaaa gttccacgca 11400
   gggcgcgagc tgcggcatgg cctgaatcgc gagcggttgc tgcgcgagga ggactttgag 11460
   cccgacgcgc gaaccgggat tagtcccgcg cgcgcacacg tggcggccgc cgacctggta 11520
   accgcatacg agcagacggt gaaccaggag attaactttc aaaaaagctt taacaaccac 11580
   gtgcgtacgc ttgtggcgcg cgaggaggtg gctataggac tgatgcatct gtgggacttt 11640
   gtaagcgcgc tggagcaaaa cccaaatagc aagccgctca tggcgcagct gttccttata 11700
   gtgcagcaca gcagggacaa cgaggcattc agggatgcgc tgctaaacat agtagagccc 11760
   gagggccgct ggctgctcga tttgataaac atcctgcaga gcatagtggt gcaggagcgc 11820
   agcttgagcc tggctgacaa ggtggccgcc atcaactatt ccatgcttag cctgggcaag 11880
   ttttacgccc gcaagatata ccatacccct tacgttccca tagacaagga ggtaaagatc 11940
   gaggggttct acatgcgcat ggcgctgaag gtgcttacct tgagcgacga cctgggcgtt 12000
   tatcgcaacg agcgcatcca caaggccgtg agcgtgagcc ggcggcgcga gctcagcgac 12060
   cgcgagctga tgcacagcct gcaaagggcc ctggctggca cgggcagcgg cgatagagag 12120
   gccgagtcct actttgacgc gggcgctgac ctgcgctggg ccccaagccg acgcgccctg 12180
   gaggcagctg gggccggacc tgggctggcg gtggcacccg cgcgcgctgg caacgtcggc 12240
   ggcgtggagg aatatgacga ggacgatgag tacgagccag aggacggcga gtactaagcg 12300
   gtgatgtttc tgatcagatg atgcaagacg caacggaccc ggcggtgcgg gcggcgctgc 12360
   agagccagcc gtccggcctt aactccacgg acgactggcg ccaggtcatg gaccgcatca 12420
   tgtcgctgac tgcgcgcaat cctgacgcgt tccggcagca gccgcaggcc aaccggctct 12480
   ccgcaattct ggaagcggtg gtcccggcgc gcgcaaaccc cacgcacgag aaggtgctgg 12540
   cgatcgtaaa cgcgctggcc gaaaacaggg ccatccggcc cgacgaggcc ggcctggtct 12600
   acgacgcgct gcttcagcgc gtggctcgtt acaacagcgg caacgtgcag accaacctgg 12660
   accggctggt gggggatgtg cgcgaggccg tggcgcagcg tgagcgcgcg cagcagcagg 12720
   gcaacctggg ctccatggtt gcactaaacg ccttcctgag tacacagccc gccaacgtgc 12780
   cgcggggaca ggaggactac accaactttg tgagcgcact gcggctaatg gtgactgaga 12840
   caccgcaaag tgaggtgtac cagtctgggc cagactattt tttccagacc agtagacaag 12900
   gcctgcagac cgtaaacctg agccaggctt tcaaaaactt gcaggggctg tggggggtgc 12960
   gggctcccac aggcgaccgc gcgaccgtgt ctagcttgct gacgcccaac tcgcgcctgt 13020
   tgctgctgct aatagcgccc ttcacggaca gtggcagcgt gtcccgggac acatacctag 13080
   gtcacttgct gacactgtac cgcgaggcca taggtcaggc gcatgtggac gagcatactt 13140
   tccaggagat tacaagtgtc agccgcgcgc tggggcagga ggacacgggc agcctggagg 13200
   caaccctaaa ctacctgctg accaaccggc ggcagaagat cccctcgttg cacagtttaa 13260
   acagcgagga ggagcgcatt ttgcgctacg tgcagcagag cgtgagcctt aacctgatgc 13320
   gcgacggggt aacgcccagc gtggcgctgg acatgaccgc gcgcaacatg gaaccgggca 13380
   tgtatgcctc aaaccggccg tttatcaacc gcctaatgga ctacttgcat cgcgcggccg 13440
   ccgtgaaccc cgagtatttc accaatgcca tcttgaaccc gcactggcta ccgccccctg 13500
   gtttctacac cgggggattc gaggtgcccg agggtaacga tggattcctc tgggacgaca 13560
   tagacgacag cgtgttttcc ccgcaaccgc agaccctgct agagttgcaa cagcgcgagc 13620
   aggcagaggc ggcgctgcga aaggaaagct tccgcaggcc aagcagcttg tccgatctag 13680
   gcgctgcggc cccgcggtca gatgctagta gcccatttcc aagcttgata gggtctctta 13740
   ccagcactcg caccacccgc ccgcgcctgc tgggcgagga ggagtaccta aacaactcgc 13800
   tgctgcagcc gcagcgcgaa aaaaacctgc ctccggcatt tcccaacaac gggatagaga 13860
   gcctagtgga caagatgagt agatggaaga cgtacgcgca ggagcacagg gacgtgccag 13920
   gcccgcgccc gcccacccgt cgtcaaaggc acgaccgtca gcggggtctg gtgtgggagg 13980
   acgatgactc ggcagacgac agcagcgtcc tggatttggg agggagtggc aacccgtttg 14040
   cgcaccttcg ccccaggctg gggagaatgt tttaaaaaaa aaaaagcatg atgcaaaata 14100
   aaaaactcac caaggccatg gcaccgagcg ttggttttct tgtattcccc ttagtatgcg 14160
   gcgcgcggcg atgtatgagg aaggtcctcc tccctcctac gagagtgtgg tgagcgcggc 14220
   gccagtggcg gcggcgctgg gttctccctt cgatgctccc ctggacccgc cgtttgtgcc 14280
   tccgcggtac ctgcggccta ccggggggag aaacagcatc cgttactctg agttggcacc 14340
   cctattcgac accacccgtg tgtacctggt ggacaacaag tcaacggatg tggcatccct 14400
   gaactaccag aacgaccaca gcaactttct gaccacggtc attcaaaaca atgactacag 14460
   cccgggggag gcaagcacac agaccatcaa tcttgacgac cggtcgcact ggggcggcga 14520
   cctgaaaacc atcctgcata ccaacatgcc aaatgtgaac gagttcatgt ttaccaataa 14580
   gtttaaggcg cgggtgatgg tgtcgcgctt gcctactaag gacaatcagg tggagctgaa 14640
   atacgagtgg gtggagttca cgctgcccga gggcaactac tccgagacca tgaccataga 14700
   ccttatgaac aacgcgatcg tggagcacta cttgaaagtg ggcagacaga acggggttct 14760
   ggaaagcgac atcggggtaa agtttgacac ccgcaacttc agactggggt ttgaccccgt 14820
   cactggtctt gtcatgcctg gggtatatac aaacgaagcc ttccatccag acatcatttt 14880
   gctgccagga tgcggggtgg acttcaccca cagccgcctg agcaacttgt tgggcatccg 14940
   caagcggcaa cccttccagg agggctttag gatcacctac gatgatctgg agggtggtaa 15000
   cattcccgca ctgttggatg tggacgccta ccaggcgagc ttgaaagatg acaccgaaca 15060
   gggcgggggt ggcgcaggcg gcagcaacag cagtggcagc ggcgcggaag agaactccaa 15120
   cgcggcagcc gcggcaatgc agccggtgga ggacatgaac gatcatgcca ttcgcggcga 15180
   cacctttgcc acacgggctg aggagaagcg cgctgaggcc gaagcagcgg ccgaagctgc 15240
   cgcccccgct gcgcaacccg aggtcgagaa gcctcagaag aaaccggtga tcaaacccct 15300
   gacagaggac agcaagaaac gcagttacaa cctaataagc aatgacagca ccttcaccca 15360
   gtaccgcagc tggtaccttg catacaacta cggcgaccct cagaccggaa tccgctcatg 15420
   gaccctgctt tgcactcctg acgtaacctg cggctcggag caggtctact ggtcgttgcc 15480
   agacatgatg caagaccccg tgaccttccg ctccacgcgc cagatcagca actttccggt 15540
   ggtgggcgcc gagctgttgc ccgtgcactc caagagcttc tacaacgacc aggccgtcta 15600
   ctcccaactc atccgccagt ttacctctct gacccacgtg ttcaatcgct ttcccgagaa 15660
   ccagattttg gcgcgcccgc cagcccccac catcaccacc gtcagtgaaa acgttcctgc 15720
   tctcacagat cacgggacgc taccgctgcg caacagcatc ggaggagtcc agcgagtgac 15780
   cattactgac gccagacgcc gcacctgccc ctacgtttac aaggccctgg gcatagtctc 15840
   gccgcgcgtc ctatcgagcc gcactttttg agcaagcatg tccatcctta tatcgcccag 15900
   caataacaca ggctggggcc tgcgcttccc aagcaagatg tttggcgggg ccaagaagcg 15960
   ctccgaccaa cacccagtgc gcgtgcgcgg gcactaccgc gcgccctggg gcgcgcacaa 16020
   acgcggccgc actgggcgca ccaccgtcga tgacgccatc gacgcggtgg tggaggaggc 16080
   gcgcaactac acgcccacgc cgccaccagt gtccacagtg gacgcggcca ttcagaccgt 16140
   ggtgcgcgga gcccggcgct atgctaaaat gaagagacgg cggaggcgcg tagcacgtcg 16200
   ccaccgccgc cgacccggca ctgccgccca acgcgcggcg gcggccctgc ttaaccgcgc 16260
   acgtcgcacc ggccgacggg cggccatgcg ggccgctcga aggctggccg cgggtattgt 16320
   cactgtgccc cccaggtcca ggcgacgagc ggccgccgca gcagccgcgg ccattagtgc 16380
   tatgactcag ggtcgcaggg gcaacgtgta ttgggtgcgc gactcggtta gcggcctgcg 16440
   cgtgcccgtg cgcacccgcc ccccgcgcaa ctagattgca agaaaaaact acttagactc 16500
   gtactgttgt atgtatccag cggcggcggc gcgcaacgaa gctatgtcca agcgcaaaat 16560
   caaagaagag atgctccagg tcatcgcgcc ggagatctat ggccccccga agaaggaaga 16620
   gcaggattac aagccccgaa agctaaagcg ggtcaaaaag aaaaagaaag atgatgatga 16680
   tgaacttgac gacgaggtgg aactgctgca cgctaccgcg cccaggcgac gggtacagtg 16740
   gaaaggtcga cgcgtaaaac gtgttttgcg acccggcacc accgtagtct ttacgcccgg 16800
   tgagcgctcc acccgcacct acaagcgcgt gtatgatgag gtgtacggcg acgaggacct 16860
   gcttgagcag gccaacgagc gcctcgggga gtttgcctac ggaaagcggc ataaggacat 16920
   gctggcgttg ccgctggacg agggcaaccc aacacctagc ctaaagcccg taacactgca 16980
   gcaggtgctg cccgcgcttg caccgtccga agaaaagcgc ggcctaaagc gcgagtctgg 17040
   tgacttggca cccaccgtgc agctgatggt acccaagcgc cagcgactgg aagatgtctt 17100
   ggaaaaaatg accgtggaac ctgggctgga gcccgaggtc cgcgtgcggc caatcaagca 17160
   ggtggcgccg ggactgggcg tgcagaccgt ggacgttcag atacccacta ccagtagcac 17220
   cagtattgcc accgccacag agggcatgga gacacaaacg tccccggttg cctcagcggt 17280
   ggcggatgcc gcggtgcagg cggtcgctgc ggccgcgtcc aagacctcta cggaggtgca 17340
   aacggacccg tggatgtttc gcgtttcagc cccccggcgc ccgcgcggtt cgaggaagta 17400
   cggcgccgcc agcgcgctac tgcccgaata tgccctacat ccttccattg cgcctacccc 17460
   cggctatcgt ggctacacct accgccccag aagacgagca actacccgac gccgaaccac 17520
   cactggaacc cgccgccgcc gtcgccgtcg ccagcccgtg ctggccccga tttccgtgcg 17580
   cagggtggct cgcgaaggag gcaggaccct ggtgctgcca acagcgcgct accaccccag 17640
   catcgtttaa aagccggtct ttgtggttct tgcagatatg gccctcacct gccgcctccg 17700
   tttcccggtg ccgggattcc gaggaagaat gcaccgtagg aggggcatgg ccggccacgg 17760
   cctgacgggc ggcatgcgtc gtgcgcacca ccggcggcgg cgcgcgtcgc accgtcgcat 17820
   gcgcggcggt atcctgcccc tccttattcc actgatcgcc gcggcgattg gcgccgtgcc 17880
   cggaattgca tccgtggcct tgcaggcgca gagacactga ttaaaaacaa gttgcatgtg 17940
   gaaaaatcaa aataaaaagt ctggactctc acgctcgctt ggtcctgtaa ctattttgta 18000
   gaatggaaga catcaacttt gcgtctctgg ccccgcgaca cggctcgcgc ccgttcatgg 18060
   gaaactggca agatatcggc accagcaata tgagcggtgg cgccttcagc tggggctcgc 18120
   tgtggagcgg cattaaaaat ttcggttcca ccgttaagaa ctatggcagc aaggcctgga 18180
   acagcagcac aggccagatg ctgagggata agttgaaaga gcaaaatttc caacaaaagg 18240
   tggtagatgg cctggcctct ggcattagcg gggtggtgga cctggccaac caggcagtgc 18300
   aaaataagat taacagtaag cttgatcccc gccctcccgt agaggagcct ccaccggccg 18360
   tggagacagt gtctccagag gggcgtggcg aaaagcgtcc gcgccccgac agggaagaaa 18420
   ctctggtgac gcaaatagac gagcctccct cgtacgagga ggcactaaag caaggcctgc 18480
   ccaccacccg tcccatcgcg cccatggcta ccggagtgct gggccagcac acacccgtaa 18540
   cgctggacct gcctcccccc gccgacaccc agcagaaacc tgtgctgcca ggcccgaccg 18600
   ccgttgttgt aacccgtcct agccgcgcgt ccctgcgccg cgccgccagc ggtccgcgat 18660
   cgttgcggcc cgtagccagt ggcaactggc aaagcacact gaacagcatc gtgggtctgg 18720
   gggtgcaatc cctgaagcgc cgacgatgct tctgaatagc taacgtgtcg tatgtgtgtc 18780
   atgtatgcgt ccatgtcgcc gccagaggag ctgctgagcc gccgcgcgcc cgctttccaa 18840
   gatggctacc ccttcgatga tgccgcagtg gtcttacatg cacatctcgg gccaggacgc 18900
   ctcggagtac ctgagccccg ggctggtgca gtttgcccgc gccaccgaga cgtacttcag 18960
   cctgaataac aagtttagaa accccacggt ggcgcctacg cacgacgtga ccacagaccg 19020
   gtcccagcgt ttgacgctgc ggttcatccc tgtggaccgt gaggatactg cgtactcgta 19080
   caaggcgcgg ttcaccctag ctgtgggtga taaccgtgtg ctggacatgg cttccacgta 19140
   ctttgacatc cgcggcgtgc tggacagggg ccctactttt aagccctact ctggcactgc 19200
   ctacaacgcc ctggctccca agggtgcccc aaatccttgc gaatgggatg aagctgctac 19260
   tgctcttgaa ataaacctag aagaagagga cgatgacaac gaagacgaag tagacgagca 19320
   agctgagcag caaaaaactc acgtatttgg gcaggcgcct tattctggta taaatattac 19380
   aaaggagggt attcaaatag gtgtcgaagg tcaaacacct aaatatgccg ataaaacatt 19440
   tcaacctgaa cctcaaatag gagaatctca gtggtacgaa actgaaatta atcatgcagc 19500
   tgggagagtc cttaaaaaga ctaccccaat gaaaccatgt tacggttcat atgcaaaacc 19560
   cacaaatgaa aatggagggc aaggcattct tgtaaagcaa caaaatggaa agctagaaag 19620
   tcaagtggaa atgcaatttt tctcaactac tgaggcgacc gcaggcaatg gtgataactt 19680
   gactcctaaa gtggtattgt acagtgaaga tgtagatata gaaaccccag acactcatat 19740
   ttcttacatg cccactatta aggaaggtaa ctcacgagaa ctaatgggcc aacaatctat 19800
   gcccaacagg cctaattaca ttgcttttag ggacaatttt attggtctaa tgtattacaa 19860
   cagcacgggt aatatgggtg ttctggcggg ccaagcatcg cagttgaatg ctgttgtaga 19920
   tttgcaagac agaaacacag agctttcata ccagcttttg cttgattcca ttggtgatag 19980
   aaccaggtac ttttctatgt ggaatcaggc tgttgacagc tatgatccag atgttagaat 20040
   tattgaaaat catggaactg aagatgaact tccaaattac tgctttccac tgggaggtgt 20100
   gattaataca gagactctta ccaaggtaaa acctaaaaca ggtcaggaaa atggatggga 20160
   aaaagatgct acagaatttt cagataaaaa tgaaataaga gttggaaata attttgccat 20220
   ggaaatcaat ctaaatgcca acctgtggag aaatttcctg tactccaaca tagcgctgta 20280
   tttgcccgac aagctaaagt acagtccttc caacgtaaaa atttctgata acccaaacac 20340
   ctacgactac atgaacaagc gagtggtggc tcccgggtta gtggactgct acattaacct 20400
   tggagcacgc tggtcccttg actatatgga caacgtcaac ccatttaacc accaccgcaa 20460
   tgctggcctg cgctaccgct caatgttgct gggcaatggt cgctatgtgc ccttccacat 20520
   ccaggtgcct cagaagttct ttgccattaa aaacctcctt ctcctgccgg gctcatacac 20580
   ctacgagtgg aacttcagga aggatgttaa catggttctg cagagctccc taggaaatga 20640
   cctaagggtt gacggagcca gcattaagtt tgatagcatt tgcctttacg ccaccttctt 20700
   ccccatggcc cacaacaccg cctccacgct tgaggccatg cttagaaacg acaccaacga 20760
   ccagtccttt aacgactatc tctccgccgc caacatgctc taccctatac ccgccaacgc 20820
   taccaacgtg cccatatcca tcccctcccg caactgggcg gctttccgcg gctgggcctt 20880
   cacgcgcctt aagactaagg aaaccccatc actgggctcg ggctacgacc cttattacac 20940
   ctactctggc tctataccct acctagatgg aaccttttac ctcaaccaca cctttaagaa 21000
   ggtggccatt acctttgact cttctgtcag ctggcctggc aatgaccgcc tgcttacccc 21060
   caacgagttt gaaattaagc gctcagttga cggggagggt tacaacgttg cccagtgtaa 21120
   catgaccaaa gactggttcc tggtacaaat gctagctaac tacaacattg gctaccaggg 21180
   cttctatatc ccagagagct acaaggaccg catgtactcc ttctttagaa acttccagcc 21240
   catgagccgt caggtggtgg atgatactaa atacaaggac taccaacagg tgggcatcct 21300
   acaccaacac aacaactctg gatttgttgg ctaccttgcc cccaccatgc gcgaaggaca 21360
   ggcctaccct gctaacttcc cctatccgct tataggcaag accgcagttg acagcattac 21420
   ccagaaaaag tttctttgcg atcgcaccct ttggcgcatc ccattctcca gtaactttat 21480
   gtccatgggc gcactcacag acctgggcca aaaccttctc tacgccaact ccgcccacgc 21540
   gctagacatg acttttgagg tggatcccat ggacgagccc acccttcttt atgttttgtt 21600
   tgaagtcttt gacgtggtcc gtgtgcaccg gccgcaccgc ggcgtcatcg aaaccgtgta 21660
   cctgcgcacg cccttctcgg ccggcaacgc cacaacataa agaagcaagc aacatcaaca 21720
   acagctgccg ccatgggctc cagtgagcag gaactgaaag ccattgtcaa agatcttggt 21780
   tgtgggccat atttttgggc cacctatgac aagcgctttc caggctttgt ttctccacac 21840
   aagctcgcct gcgccatagt caatacggcc ggtcgcgaga ctgggggcgt acactggatg 21900
   gcctttgcct ggaacccgca ctcaaaaaca tgctacctct ttgagccctt tggcttttct 21960
   gaccagcgac tcaagcaggt ttaccagttt gagtacgagt cactcctgcg ccgtagcgcc 22020
   attgcttctt cccccgaccg ctgtataacg ctggaaaagt ccacccaaag cgtacagggg 22080
   cccaactcgg ccgcctgtgg actattctgc tgcatgtttc tccacgcctt tgccaactgg 22140
   ccccaaactc ccatggatca caaccccacc atgaacctta ttaccggggt acccaactcc 22200
   atgctcaaca gtccccaggt acagcccacc ctgcgtcgca accaggaaca gctctacagc 22260
   ttcctggagc gccactcgcc ctacttccgc agccacagtg cgcagattag gagcgccact 22320
   tctttttgtc acttgaaaaa catgtaaaaa taatgtacta gagacacttt caataaaggc 22380
   aaatgctttt atttgtacac tctcgggtga ttatttaccc ccacccttgc cgtctgcgcc 22440
   gtttaaaaat caaaggggtt ctgccgcgca tcgctatgcg ccactggcag ggacacgttg 22500
   cgatactggt gtttagtgct ccacttaaac tcaggcacaa ccatccgcgg cagctcggtg 22560
   aagttttcac tccacaggct gcgcaccatc accaacgcgt ttagcaggtc gggcgccgat 22620
   atcttgaagt cgcagttggg gcctccgccc tgcgcgcgcg agttgcgata cacagggttg 22680
   cagcactgga acactatcag cgccgggtgg tgcacgctgg ccagcacgct cttgtcggag 22740
   atcagatccg cgtccaggtc ctccgcgttg ctcagggcga acggagtcaa ctttggtagc 22800
   tgccttccca aaaagggcgc gtgcccaggc tttgagttgc actcgcaccg tagtggcatc 22860
   aaaaggtgac cgtgcccggt ctgggcgtta ggatacagcg cctgcataaa agccttgatc 22920
   tgcttaaaag ccacctgagc ctttgcgcct tcagagaaga acatgccgca agacttgccg 22980
   gaaaactgat tggccggaca ggccgcgtcg tgcacgcagc accttgcgtc ggtgttggag 23040
   atctgcacca catttcggcc ccaccggttc ttcacgatct tggccttgct agactgctcc 23100
   ttcagcgcgc gctgcccgtt ttcgctcgtc acatccattt caatcacgtg ctccttattt 23160
   atcataatgc ttccgtgtag acacttaagc tcgccttcga tctcagcgca gcggtgcagc 23220
   cacaacgcgc agcccgtggg ctcgtgatgc ttgtaggtca cctctgcaaa cgactgcagg 23280
   tacgcctgca ggaatcgccc catcatcgtc acaaaggtct tgttgctggt gaaggtcagc 23340
   tgcaacccgc ggtgctcctc gttcagccag gtcttgcata cggccgccag agcttccact 23400
   tggtcaggca gtagtttgaa gttcgccttt agatcgttat ccacgtggta cttgtccatc 23460
   agcgcgcgcg cagcctccat gcccttctcc cacgcagaca cgatcggcac actcagcggg 23520
   ttcatcaccg taatttcact ttccgcttcg ctgggctctt cctcttcctc ttgcgtccgc 23580
   ataccacgcg ccactgggtc gtcttcattc agccgccgca ctgtgcgctt acctcctttg 23640
   ccatgcttga ttagcaccgg tgggttgctg aaacccacca tttgtagcgc cacatcttct 23700
   ctttcttcct cgctgtccac gattacctct ggtgatggcg ggcgctcggg cttgggagaa 23760
   gggcgcttct ttttcttctt gggcgcaatg gccaaatccg ccgccgaggt cgatggccgc 23820
   gggctgggtg tgcgcggcac cagcgcgtct tgtgatgagt cttcctcgtc ctcggactcg 23880
   atacgccgcc tcatccgctt ttttgggggc gcccggggag gcggcggcga cggggacggg 23940
   gacgacacgt cctccatggt tgggggacgt cgcgccgcac cgcgtccgcg ctcgggggtg 24000
   gtttcgcgct gctcctcttc ccgactggcc atttccttct cctataggca gaaaaagatc 24060
   atggagtcag tcgagaagaa ggacagccta accgccccct ctgagttcgc caccaccgcc 24120
   tccaccgatg ccgccaacgc gcctaccacc ttccccgtcg aggcaccccc gcttgaggag 24180
   gaggaagtga ttatcgagca ggacccaggt tttgtaagcg aagacgacga ggaccgctca 24240
   gtaccaacag aggataaaaa gcaagaccag gacaacgcag aggcaaacga ggaacaagtc 24300
   gggcgggggg acgaaaggca tggcgactac ctagatgtgg gagacgacgt gctgttgaag 24360
   catctgcagc gccagtgcgc cattatctgc gacgcgttgc aagagcgcag cgatgtgccc 24420
   ctcgccatag cggatgtcag ccttgcctac gaacgccacc tattctcacc gcgcgtaccc 24480
   cccaaacgcc aagaaaacgg cacatgcgag cccaacccgc gcctcaactt ctaccccgta 24540
   tttgccgtgc cagaggtgct tgccacctat cacatctttt tccaaaactg caagataccc 24600
   ctatcctgcc gtgccaaccg cagccgagcg gacaagcagc tggccttgcg gcagggcgct 24660
   gtcatacctg atatcgcctc gctcaacgaa gtgccaaaaa tctttgaggg tcttggacgc 24720
   gacgagaagc gcgcggcaaa cgctctgcaa caggaaaaca gcgaaaatga aagtcactct 24780
   ggagtgttgg tggaactcga gggtgacaac gcgcgcctag ccgtactaaa acgcagcatc 24840
   gaggtcaccc actttgccta cccggcactt aacctacccc ccaaggtcat gagcacagtc 24900
   atgagtgagc tgatcgtgcg ccgtgcgcag cccctggaga gggatgcaaa tttgcaagaa 24960
   caaacagagg agggcctacc cgcagttggc gacgagcagc tagcgcgctg gcttcaaacg 25020
   cgcgagcctg ccgacttgga ggagcgacgc aaactaatga tggccgcagt gctcgttacc 25080
   gtggagcttg agtgcatgca gcggttcttt gctgacccgg agatgcagcg caagctagag 25140
   gaaacattgc actacacctt tcgacagggc tacgtacgcc aggcctgcaa gatctccaac 25200
   gtggagctct gcaacctggt ctcctacctt ggaattttgc acgaaaaccg ccttgggcaa 25260
   aacgtgcttc attccacgct caagggcgag gcgcgccgcg actacgtccg cgactgcgtt 25320
   tacttatttc tatgctacac ctggcagacg gccatgggcg tttggcagca gtgcttggag 25380
   gagtgcaacc tcaaggagct gcagaaactg ctaaagcaaa acttgaagga cctatggacg 25440
   gccttcaacg agcgctccgt ggccgcgcac ctggcggaca tcatttcccc cgaacgcctg 25500
   cttaaaaccc tgcaacaggg tctgccagac ttcaccagtc aaagcatgtt gcagaacttt 25560
   aggaacttta tcctagagcg ctcaggaatc ttgcccgcca cctgctgtgc acttcctagc 25620
   gactttgtgc ccattaagta ccgcgaatgc cctccgccgc tttggggcca ctgctacctt 25680
   ctgcagctag ccaactacct tgcctaccac tctgacataa tggaagacgt gagcggtgac 25740
   ggtctactgg agtgtcactg tcgctgcaac ctatgcaccc cgcaccgctc cctggtttgc 25800
   aattcgcagc tgcttaacga aagtcaaatt atcggtacct ttgagctgca gggtccctcg 25860
   cctgacgaaa agtccgcggc tccggggttg aaactcactc cggggctgtg gacgtcggct 25920
   taccttcgca aatttgtacc tgaggactac cacgcccacg agattaggtt ctacgaagac 25980
   caatcccgcc cgccaaatgc ggagcttacc gcctgcgtca ttacccaggg ccacattctt 26040
   ggccaattgc aagccatcaa caaagcccgc caagagtttc tgctacgaaa gggacggggg 26100
   gtttacttgg acccccagtc cggcgaggag ctcaacccaa tccccccgcc gccgcagccc 26160
   tatcagcagc agccgcgggc ccttgcttcc caggatggca cccaaaaaga agctgcagct 26220
   gccgccgcca cccacggacg aggaggaata ctgggacagt caggcagagg aggttttgga 26280
   cgaggaggag gaggacatga tggaagactg ggagagccta gacgaggaag cttccgaggt 26340
   cgaagaggtg tcagacgaaa caccgtcacc ctcggtcgca ttcccctcgc cggcgcccca 26400
   gaaatcggca accggttcca gcatggctac aacctccgct cctcaggcgc cgccggcact 26460
   gcccgttcgc cgacccaacc gtagatggga caccactgga accagggccg gtaagtccaa 26520
   gcagccgccg ccgttagccc aagagcaaca acagcgccaa ggctaccgct catggcgcgg 26580
   gcacaagaac gccatagttg cttgcttgca agactgtggg ggcaacatct ccttcgcccg 26640
   ccgctttctt ctctaccatc acggcgtggc cttcccccgt aacatcctgc attactaccg 26700
   tcatctctac agcccatact gcaccggcgg cagcggcagc ggcagcaaca gcagcggcca 26760
   cacagaagca aaggcgaccg gatagcaaga ctctgacaaa gcccaagaaa tccacagcgg 26820
   cggcagcagc aggaggagga gcgctgcgtc tggcgcccaa cgaacccgta tcgacccgcg 26880
   agcttagaaa caggattttt cccactctgt atgctatatt tcaacagagc aggggccaag 26940
   aacaagagct gaaaataaaa aacaggtctc tgcgatccct cacccgcagc tgcctgtatc 27000
   acaaaagcga agatcagctt cggcgcacgc tggaagacgc ggaggctctc ttcagtaaat 27060
   actgcgcgct gactcttaag gactagtttc gcgccctttc tcaaatttaa gcgcgaaaac 27120
   tacgtcatct ccagcggcca cacccggcgc cagcacctgt cgtcagcgcc attatgagca 27180
   aggaaattcc cacgccctac atgtggagtt accagccaca aatgggactt gcggctggag 27240
   ctgcccaaga ctactcaacc cgaataaact acatgagcgc gggaccccac atgatatccc 27300
   gggtcaacgg aatccgcgcc caccgaaacc gaattctctt ggaacaggcg gctattacca 27360
   ccacacctcg taataacctt aatccccgta gttggcccgc tgccctggtg taccaggaaa 27420
   gtcccgctcc caccactgtg gtacttccca gagacgccca ggccgaagtt cagatgacta 27480
   actcaggggc gcagcttgcg ggcggctttc gtcacagggt gcggtcgccc gggcagggta 27540
   taactcacct gacaatcaga gggcgaggta ttcagctcaa cgacgagtcg gtgagctcct 27600
   cgcttggtct ccgtccggac gggacatttc agatcggcgg cgccggccgt ccttcattca 27660
   cgcctcgtca ggcaatccta actctgcaga cctcgtcctc tgagccgcgc tctggaggca 27720
   ttggaactct gcaatttatt gaggagtttg tgccatcggt ctactttaac cccttctcgg 27780
   gacctcccgg ccactatccg gatcaattta ttcctaactt tgacgcggta aaggactcgg 27840
   cggacggcta cgactgaatg ttaagtggag aggcagagca actgcgcctg aaacacctgg 27900
   tccactgtcg ccgccacaag tgctttgccc gcgactccgg tgagttttgc tactttgaat 27960
   tgcccgagga tcatatcgag ggcccggcgc acggcgtccg gcttaccgcc cagggagagc 28020
   ttgcccgtag cctgattcgg gagtttaccc agcgccccct gctagttgag cgggacaggg 28080
   gaccctgtgt tctcactgtg atttgcaact gtcctaacct tggattacat caagatcttt 28140
   gttgccatct ctgtgctgag tataataaat acagaaatta aaatatactg gggctcctat 28200
   cgccatcctg taaacgccac cgtcttcacc cgcccaagca aaccaaggcg aaccttacct 28260
   ggtactttta acatctctcc ctctgtgatt tacaacagtt tcaacccaga cggagtgagt 28320
   ctacgagaga acctctccga gctcagctac tccatcagaa aaaacaccac cctccttacc 28380
   tgccgggaac gtacgagtgc gtcaccggcc gctgcaccac acctaccgcc tgaccgtaaa 28440
   ccagactttt tccggacaga cctcaataac tctgtttacc agaacaggag gtgagcttag 28500
   aaaaccctta gggtattagg ccaaaggcgc agctactgtg gggtttatga acaattcaag 28560
   caactctacg ggctattcta attcaggttt ctctagaatc ggggttgggg ttattctctg 28620
   tcttgtgatt ctctttattc ttatactaac gcttctctgc ctaaggctcg ccgcctgctg 28680
   tgtgcacatt tgcatttatt gtcagctttt taaacgctgg ggtcgccacc caagatgatt 28740
   aggtacataa tcctaggttt actcaccctt gcgtcagccc acggtaccac ccaaaaggtg 28800
   gattttaagg agccagcctg taatgttaca ttcgcagctg aagctaatga gtgcaccact 28860
   cttataaaat gcaccacaga acatgaaaag ctgcttattc gccacaaaaa caaaattggc 28920
   aagtatgctg tttatgctat ttggcagcca ggtgacacta cagagtataa tgttacagtt 28980
   ttccagggta aaagtcataa aacttttatg tatacttttc cattttatga aatgtgcgac 29040
   attaccatgt acatgagcaa acagtataag ttgtggcccc cacaaaattg tgtggaaaac 29100
   actggcactt tctgctgcac tgctatgcta attacagtgc tcgctttggt ctgtacccta 29160
   ctctatatta aatacaaaag cagacgcagc tttattgagg aaaagaaaat gccttaattt 29220
   actaagttac aaagctaatg tcaccactaa ctgctttact cgctgcttgc aaaacaaatt 29280
   caaaaagtta gcattataat tagaatagga tttaaacccc ccggtcattt cctgctcaat 29340
   accattcccc tgaacaattg actctatgtg ggatatgctc cagcgctaca accttgaagt 29400
   caggcttcct ggatgtcagc atctgacttt ggccagcacc tgtcccgcgg atttgttcca 29460
   gtccaactac agcgacccac cctaacagag atgaccaaca caaccaacgc ggccgccgct 29520
   accggactta catctaccac aaatacaccc caagtttctg cctttgtcaa taactgggat 29580
   aacttgggca tgtggtggtt ctccatagcg cttatgtttg tatgccttat tattatgtgg 29640
   ctcatctgct gcctaaagcg caaacgcgcc cgaccaccca tctatagtcc catcattgtg 29700
   ctacacccaa acaatgatgg aatccataga ttggacggac tgaaacacat gttcttttct 29760
   cttacagtat gattaaatga gacatgattc ctcgagtttt tatattactg acccttgttg 29820
   cgcttttttg tgcgtgctcc acattggctg cggtttctca catcgaagta gactgcattc 29880
   cagccttcac agtctatttg ctttacggat ttgtcaccct cacgctcatc tgcagcctca 29940
   tcactgtggt catcgccttt atccagtgca ttgactgggt ctgtgtgcgc tttgcatatc 30000
   tcagacacca tccccagtac agggacagga ctatagctga gcttcttaga attctttaat 30060
   tatgaaattt actgtgactt ttctgctgat tatttgcacc ctatctgcgt tttgttcccc 30120
   gacctccaag cctcaaagac atatatcatg cagattcact cgtatatgga atattccaag 30180
   ttgctacaat gaaaaaagcg atctttccga agcctggtta tatgcaatca tctctgttat 30240
   ggtgttctgc agtaccatct tagccctagc tatatatccc taccttgaca ttggctggaa 30300
   acgaatagat gccatgaacc acccaacttt ccccgcgccc gctatgcttc cactgcaaca 30360
   agttgttgcc ggcggctttg tcccagccaa tcagcctcgc cccacttctc ccacccccac 30420
   tgaaatcagc tactttaatc taacaggagg agatgactga caccctagat ctagaaatgg 30480
   acggaattat tacagagcag cgcctgctag aaagacgcag ggcagcggcc gagcaacagc 30540
   gcatgaatca agagctccaa gacatggtta acttgcacca gtgcaaaagg ggtatctttt 30600
   gtctggtaaa gcaggccaaa gtcacctacg acagtaatac caccggacac cgccttagct 30660
   acaagttgcc aaccaagcgt cagaaattgg tggtcatggt gggagaaaag cccattacca 30720
   taactcagca ctcggtagaa accgaaggct gcattcactc accttgtcaa ggacctgagg 30780
   atctctgcac ccttattaag accctgtgcg gtctcaaaga tcttattccc tttaactaat 30840
   aaaaaaaaat aataaagcat cacttactta aaatcagtta gcaaatttct gtccagttta 30900
   ttcagcagca cctccttgcc ctcctcccag ctctggtatt gcagcttcct cctggctgca 30960
   aactttctcc acaatctaaa tggaatgtca gtttcctcct gttcctgtcc atccgcaccc 31020
   actatcttca tgttgttgca gatgaagcgc gcaagaccgt ctgaagatac cttcaacccc 31080
   gtgtatccat atgacacgga aaccggtcct ccaactgtgc cttttcttac tcctcccttt 31140
   gtatccccca atgggtttca agagagtccc cctggggtac tctctttgcg cctatccgaa 31200
   cctctagtta cctccaatgg catgcttgcg ctcaaaatgg gcaacggcct ctctctggac 31260
   gaggccggca accttacctc ccaaaatgta accactgtga gcccacctct caaaaaaacc 31320
   aagtcaaaca taaacctgga aatatctgca cccctcacag ttacctcaga agccctaact 31380
   gtggctgccg ccgcacctct aatggtcgcg ggcaacacac tcaccatgca atcacaggcc 31440
   ccgctaaccg tgcacgactc caaacttagc attgccaccc aaggacccct cacagtgtca 31500
   gaaggaaagc tagccctgca aacatcaggc cccctcacca ccaccgatag cagtaccctt 31560
   actatcactg cctcaccccc tctaactact gccactggta gcttgggcat tgacttgaaa 31620
   gagcccattt atacacaaaa tggaaaacta ggactaaagt acggggctcc tttgcatgta 31680
   acagacgacc taaacacttt gaccgtagca actggtccag gtgtgactat taataatact 31740
   tccttgcaaa ctaaagttac tggagccttg ggttttgatt cacaaggcaa tatgcaactt 31800
   aatgtagcag gaggactaag gattgattct caaaacagac gccttatact tgatgttagt 31860
   tatccgtttg atgctcaaaa ccaactaaat ctaagactag gacagggccc tctttttata 31920
   aactcagccc acaacttgga tattaactac aacaaaggcc tttacttgtt tacagcttca 31980
   aacaattcca aaaagcttga ggttaaccta agcactgcca aggggttgat gtttgacgct 32040
   acagccatag ccattaatgc aggagatggg cttgaatttg gttcacctaa tgcaccaaac 32100
   acaaatcccc tcaaaacaaa aattggccat ggcctagaat ttgattcaaa caaggctatg 32160
   gttcctaaac taggaactgg ccttagtttt gacagcacag gtgccattac agtaggaaac 32220
   aaaaataatg ataagctaac tttgtggacc acaccagctc catctcctaa ctgtagacta 32280
   aatgcagaga aagatgctaa actcactttg gtcttaacaa aatgtggcag tcaaatactt 32340
   gctacagttt cagttttggc tgttaaaggc agtttggctc caatatctgg aacagttcaa 32400
   agtgctcatc ttattataag atttgacgaa aatggagtgc tactaaacaa ttccttcctg 32460
   gacccagaat attggaactt tagaaatgga gatcttactg aaggcacagc ctatacaaac 32520
   gctgttggat ttatgcctaa cctatcagct tatccaaaat ctcacggtaa aactgccaaa 32580
   agtaacattg tcagtcaagt ttacttaaac ggagacaaaa ctaaacctgt aacactaacc 32640
   attacactaa acggtacaca ggaaacagga gacacaactc caagtgcata ctctatgtca 32700
   ttttcatggg actggtctgg ccacaactac attaatgaaa tatttgccac atcctcttac 32760
   actttttcat acattgccca agaataaaga atcgtttgtg ttatgtttca acgtgtttat 32820
   ttttcaattg cagaaaattt caagtcattt ttcattcagt agtatagccc caccaccaca 32880
   tagcttatac agatcaccgt accttaatca aactcacaga accctagtat tcaacctgcc 32940
   acctccctcc caacacacag agtacacagt cctttctccc cggctggcct taaaaagcat 33000
   catatcatgg gtaacagaca tattcttagg tgttatattc cacacggttt cctgtcgagc 33060
   caaacgctca tcagtgatat taataaactc cccgggcagc tcacttaagt tcatgtcgct 33120
   gtccagctgc tgagccacag gctgctgtcc aacttgcggt tgcttaacgg gcggcgaagg 33180
   agaagtccac gcctacatgg gggtagagtc ataatcgtgc atcaggatag ggcggtggtg 33240
   ctgcagcagc gcgcgaataa actgctgccg ccgccgctcc gtcctgcagg aatacaacat 33300
   ggcagtggtc tcctcagcga tgattcgcac cgcccgcagc ataaggcgcc ttgtcctccg 33360
   ggcacagcag cgcaccctga tctcacttaa atcagcacag taactgcagc acagcaccac 33420
   aatattgttc aaaatcccac agtgcaaggc gctgtatcca aagctcatgg cggggaccac 33480
   agaacccacg tggccatcat accacaagcg caggtagatt aagtggcgac ccctcataaa 33540
   cacgctggac ataaacatta cctcttttgg catgttgtaa ttcaccacct cccggtacca 33600
   tataaacctc tgattaaaca tggcgccatc caccaccatc ctaaaccagc tggccaaaac 33660
   ctgcccgccg gctatacact gcagggaacc gggactggaa caatgacagt ggagagccca 33720
   ggactcgtaa ccatggatca tcatgctcgt catgatatca atgttggcac aacacaggca 33780
   cacgtgcata cacttcctca ggattacaag ctcctcccgc gttagaacca tatcccaggg 33840
   aacaacccat tcctgaatca gcgtaaatcc cacactgcag ggaagacctc gcacgtaact 33900
   cacgttgtgc attgtcaaag tgttacattc gggcagcagc ggatgatcct ccagtatggt 33960
   agcgcgggtt tctgtctcaa aaggaggtag acgatcccta ctgtacggag tgcgccgaga 34020
   caaccgagat cgtgttggtc gtagtgtcat gccaaatgga acgccggacg tagtcatatt 34080
   tcctgaagca aaaccaggtg cgggcgtgac aaacagatct gcgtctccgg tctcgccgct 34140
   tagatcgctc tgtgtagtag ttgtagtata tccactctct caaagcatcc aggcgccccc 34200
   tggcttcggg ttctatgtaa actccttcat gcgccgctgc cctgataaca tccaccaccg 34260
   cagaataagc cacacccagc caacctacac attcgttctg cgagtcacac acgggaggag 34320
   cgggaagagc tggaagaacc atgttttttt ttttattcca aaagattatc caaaacctca 34380
   aaatgaagat ctattaagtg aacgcgctcc cctccggtgg cgtggtcaaa ctctacagcc 34440
   aaagaacaga taatggcatt tgtaagatgt tgcacaatgg cttccaaaag gcaaacggcc 34500
   ctcacgtcca agtggacgta aaggctaaac ccttcagggt gaatctcctc tataaacatt 34560
   ccagcacctt caaccatgcc caaataattc tcatctcgcc accttctcaa tatatctcta 34620
   agcaaatccc gaatattaag tccggccatt gtaaaaatct gctccagagc gccctccacc 34680
   ttcagcctca agcagcgaat catgattgca aaaattcagg ttcctcacag acctgtataa 34740
   gattcaaaag cggaacatta acaaaaatac cgcgatcccg taggtccctt cgcagggcca 34800
   gctgaacata atcgtgcagg tctgcacgga ccagcgcggc cacttccccg ccaggaacct 34860
   tgacaaaaga acccacactg attatgacac gcatactcgg agctatgcta accagcgtag 34920
   ccccgatgta agctttgttg catgggcggc gatataaaat gcaaggtgct gctcaaaaaa 34980
   tcaggcaaag cctcgcgcaa aaaagaaagc acatcgtagt catgctcatg cagataaagg 35040
   caggtaagct ccggaaccac cacagaaaaa gacaccattt ttctctcaaa catgtctgcg 35100
   ggtttctgca taaacacaaa ataaaataac aaaaaaacat ttaaacatta gaagcctgtc 35160
   ttacaacagg aaaaacaacc cttataagca taagacggac tacggccatg ccggcgtgac 35220
   cgtaaaaaaa ctggtcaccg tgattaaaaa gcaccaccga cagctcctcg gtcatgtccg 35280
   gagtcataat gtaagactcg gtaaacacat caggttgatt catcggtcag tgctaaaaag 35340
   cgaccgaaat agcccggggg aatacatacc cgcaggcgta gagacaacat tacagccccc 35400
   ataggaggta taacaaaatt aataggagag aaaaacacat aaacacctga aaaaccctcc 35460
   tgcctaggca aaatagcacc ctcccgctcc agaacaacat acagcgcttc acagcggcag 35520
   cctaacagtc agccttacca gtaaaaaaga aaacctatta aaaaaacacc actcgacacg 35580
   gcaccagctc aatcagtcac agtgtaaaaa agggccaagt gcagagcgag tatatatagg 35640
   actaaaaaat gacgtaacgg ttaaagtcca caaaaaacac ccagaaaacc gcacgcgaac 35700
   ctacgcccag aaacgaaagc caaaaaaccc acaacttcct caaatcgtca cttccgtttt 35760
   cccacgttac gtaacttccc attttaagaa aactacaatt cccaacacat acaagttact 35820
   ccgccctaaa acctacgtca cccgccccgt tcccacgccc cgcgccacgt cacaaactcc 35880
   accccctcat tatcatattg gcttcaatcc aaaataaggt atattattga tgatg 35935

<210> 10
<211> 5965
<212> DNA
<213> Artificial Sequence

<220>
<223> NSsuboptmut

<400> 10
gccaccatgg cccccatcac cgcctacagc cagcagacca ggggcctgct gggctgcatc 60
atcaccagcc tgaccggacg cgacaagaac caggtggagg gagaggtgca ggtggtgagc 120
accgctaccc agagcttcct ggccacctgc gtgaacggcg tgtgctggac cgtgtaccac 180
ggagccggaa gcaagaccct ggccggaccc aagggcccta tcacccagat gtacaccaat 240
gtggatcagg atctggtggg ctggcaggcc cctcccggag ccaggagcct gacaccctgt 300
acctgtggaa gcagcgacct gtacctggtg acacgccacg ccgatgtgat ccccgtgagg 360
cgcaggggcg attctcgcgg aagcctgctg agccctaggc ccgtgagcta cctgaagggc 420
agcagcggag gacccctgct gtgtccttct ggccatgccg tgggcatttt tcgcgctgcc 480
gtgtgtacca ggggcgtggc caaagccgtg gattttgtgc ccgtggaaag catggagacc 540
accatgcgca gccctgtgtt caccgacaac agctctcccc ctgccgtgcc ccaatcattc 600
caggtggctc acctgcacgc ccctaccgga tctggcaaga gcaccaaggt gcccgctgcc 660
tacgccgctc agggctacaa ggtgctggtg ctgaacccca gcgtggccgc taccctgggc 720
ttcggcgctt acatgagcaa ggcccatggc atcgacccca acatccgcac aggcgtgcgc 780
accatcacca ccggagctcc cgtgacctac agcacctacg gcaagttcct ggccgatgga 840
ggctgcagcg gaggagccta cgacatcatc atctgcgacg agtgccacag caccgacagc 900
accaccatcc tgggcattgg caccgtgctg gatcaggccg aaacagctgg agccaggctg 960
gtggtgctgg ccacagctac ccctcctggc agcgtgaccg tgccccatcc caatatcgag 1020
gaggtggccc tgagcaacac aggcgagatc cccttctacg gcaaggccat ccccatcgag 1080
gccatccgcg gaggcaggca cctgatcttc tgccacagca agaagaagtg cgacgagctg 1140
gctgccaagc tgagcggact gggcatcaac gccgtggcct actacagggg cctggacgtg 1200
tcagtgatcc ccaccatcgg cgatgtggtg gtggtggcca ccgacgccct gatgacaggc 1260
tacaccggag acttcgacag cgtgatcgac tgcaacacct gcgtgaccca gaccgtggac 1320
ttcagcctgg accccacctt caccatcgaa accaccaccg tgcctcagga tgctgtgagc 1380
aggagccaga ggcgcggacg caccggaagg ggcaggcgcg gaatttatcg ctttgtgacc 1440
cctggcgaaa ggccctctgg catgttcgac agcagcgtgc tgtgcgagtg ctacgacgct 1500
ggctgcgctt ggtacgagct gacacccgct gaaaccagcg tgcgcctgcg cgcttatctg 1560
aatacccctg gcctgcccgt gtgtcaggac cacctggagt tctgggagag cgtgttcaca 1620
ggactgaccc acatcgacgc ccatttcctg agccagacca agcaggctgg cgacaacttc 1680
ccctatctgg tggcctatca ggccaccgtg tgtgctaggg cccaagctcc acctccttca 1740
tgggaccaga tgtggaagtg cctgatccgc ctgaagccca ccctgcacgg ccctacccct 1800
ctgctgtacc gcctgggagc cgtgcagaac gaggtgaccc tgacccaccc catcaccaag 1860
tacatcatgg cctgcatgag cgctgatctg gaagtggtga ccagcacctg ggtgctggtg 1920
ggaggcgtgc tggccgctct ggctgcctac tgcctgacca ccggaagcgt ggtgatcgtg 1980
ggacgcatca tcctgagcgg aaggcccgct atcgtgcccg atcgcgagtt cctgtaccag 2040

gagttcgacg agatggagga gtgtgccagc cacctgccct acatcgagca gggcatgcag 2100
ctggccgaac agttcaagca gaaggccctg ggcctgctgc agacagccac caaacaggcc 2160
gaagctgccg ctcccgtggt ggaaagcaag tggagggccc tggagacctt ctgggctaag 2220
cacatgtgga acttcatctc tggcatccag tacctggccg gactgagcac cctgcctggc 2280
aaccccgcta tcgccagcct gatggccttc accgctagca tcacctctcc cctgaccacc 2340
cagagcaccc tgctgttcaa cattctgggc ggatgggtgg ccgctcagct ggcccctcct 2400
tcagctgctt ctgcctttgt gggcgctggc attgccggag ccgctgtggg cagcattggc 2460
ctgggcaaag tgctggtgga tattctggct ggctatggcg ctggcgtggc cggagccctg 2520
gtggccttca aggtgatgag cggagagatg cccagcaccg aggacctggt gaacctgctg 2580
cctgccattc tgagccctgg agccctggtg gtgggcgtgg tgtgtgctgc cattctgagg 2640
cgccatgtgg gacccggaga gggcgctgtg cagtggatga accgcctgat cgccttcgcc 2700
tctcgcggaa accacgtgag ccctacccac tacgtgcctg agagcgacgc cgctgccagg 2760
gtgacccaga tcctgagcag cctgaccatc acccagctgc tgaagcgcct gcaccagtgg 2820
atcaacgagg actgcagcac accctgcagc ggaagctggc tgagggacgt gtgggactgg 2880
atctgcaccg tgctgaccga cttcaagacc tggctgcaga gcaagctgct gccccaactg 2940
cctggcgtgc ccttcttctc atgccagcgc ggatacaagg gcgtgtggag gggcgatggc 3000
atcatgcaga ccacctgtcc ctgcggagcc cagatcacag gccacgtgaa gaacggcagc 3060
atgcgcatcg tgggccctaa gacctgcagc aacacctggc acggcacctt ccccatcaac 3120
gcctacacca ccggaccctg cacacccagc cctgctccca actacagcag ggccctgtgg 3180
agggtggctg ccgaggagta cgtggaggtg accagggtgg gagacttcca ctacgtgacc 3240
ggaatgacca ccgacaacgt gaagtgtccc tgtcaggtgc ccgctcccga atttttcacc 3300
gaagtggatg gcgtgcgcct gcatcgctat gcccctgcct gtaggcccct gctgcgcgaa 3360
gaagtgacct tccaggtggg cctgaaccag tacctggtgg gcagccagct gccctgcgag 3420
cctgagcccg atgtggccgt gctgaccagc atgctgaccg accccagcca catcacagcc 3480
gaaaccgcta aaaggcgcct ggccaggggc tctcctccaa gcctggcctc aagcagcgct 3540
agccagctgt ctgctcccag cctgaaggcc acctgcacca cccaccacgt gagccccgac 3600
gccgacctga tcgaggccaa cctgctgtgg cgccaggaga tgggcggcaa catcacccgc 3660
gtggagagcg agaacaaggt ggtggtgctg gacagcttcg accccctgcg cgccgaggag 3720
gacgagcgcg aggtgagcgt gcccgccgag atcctgcgca agagcaagaa gttccccgct 3780
gccatgccca tctgggctag acctgattac aaccctcccc tgctggagag ctggaaggac 3840
cctgattacg tgcctccagt ggtgcatggc tgtcctctgc ctcccattaa agcccctcct 3900
attccacctc ctaggcgcaa aaggaccgtg gtgctgacag aaagcagcgt gagctctgct 3960
ctggccgaac tggccaccaa gacctttggc agcagcgaga gctctgccgt ggacagcgga 4020
acagccaccg ctctgcctga ccaggccagc gacgacggcg ataagggcag cgatgtggag 4080
agctatagca gcatgcctcc cctggaaggc gaacctggcg atcccgatct gagcgatggc 4140
agctggagca ccgtgagcga agaggccagc gaggacgtgg tgtgttgcag catgagctac 4200
acctggacag gcgctctgat cacaccctgc gctgccgagg agagcaagct gcccatcaac 4260
gccctgagca acagcctgct gaggcaccac aacatggtgt acgccaccac cagcaggtct 4320
gccggactga ggcagaagaa ggtgaccttc gaccgcctgc aggtgctgga cgaccactac 4380
cgcgatgtgc tgaaggagat gaaggccaag gccagcaccg tgaaggccaa gctgctgagc 4440
gtggaggagg cctgcaagct gacccccccc cacagcgcca agagcaagtt cggctacggc 4500
gccaaggacg tgcgcaacct gagcagcaag gccgtgaacc acatccacag cgtgtggaag 4560
gacctgctgg aggacaccgt gacccccatc gacaccacca tcatggccaa gaacgaggtg 4620
ttctgcgtgc agcccgagaa gggcggccgc aagcccgctc gcctgatcgt gttccccgat 4680
ctgggcgtgc gcgtgtgcga gaagatggcc ctgtacgacg tggtgagcac cctgcctcag 4740
gtggtgatgg gctcaagcta cggcttccag tacagccctg gccagcgcgt ggagttcctg 4800
gtgaacacct ggaagagcaa gaagaacccc atgggcttca gctacgacac acgctgcttc 4860
gacagcaccg tgaccgagaa cgacatccgc gtggaggaga gcatctacca gtgctgcgac 4920
ctggcccctg aggccaggca ggccatcaag agcctgaccg agcgcctgta catcggaggc 4980
cctctgacca acagcaaggg acagaactgc ggatacaggc gctgtagggc ctctggcgtg 5040
ctgaccacca gctgtggcaa caccctgacc tgctacctga aggccagcgc tgcctgtcgc 5100
gctgccaagc tgcaggactg caccatgctg gtgaacgccg ctggcctggt ggtgatttgt 5160
gaaagcgctg gcacccagga agatgctgcc agcctgcgcg tgttcaccga ggccatgacc 5220
aggtactctg cccctcccgg agacccccct cagcccgaat acgacctgga gctgatcacc 5280
agctgctcaa gcaacgtgag cgtggctcac gacgccagcg gaaagcgcgt gtactacctg 5340
acacgcgatc ccaccacccc tctggctcgc gctgcctggg aaaccgctcg ccatacaccc 5400
gtgaacagct ggctgggcaa catcatcatg tacgccccta ccctgtgggc tcgcatgatc 5460
ctgatgaccc acttcttcag catcctgctg gctcaggagc agctggagaa ggccctggac 5520
tgccagattt acggcgcttg ctacagcatc gagcccctgg acctgcccca aatcatcgag 5580
cgcctgcacg gcctgtctgc cttcagcctg cacagctaca gccctggcga aattaatcgc 5640
gtggccagct gtctgcgcaa actgggcgtg cctcctctgc gcgtgtggag gcatagggct 5700
aggagcgtga gggctaggct gctgagccag ggaggcaggg ccgctacctg tggaaagtac 5760
ctgttcaact gggccgtgaa gaccaagctg aagctgaccc ctatccctgc cgctagccag 5820
ctggacctga gcggatggtt cgtggctggc tacagcggag gcgacatcta ccacagcctg 5880
tctcgcgctc gccctcgctg gttcatgctg tgcctgctgc tgctgagcgt gggcgtgggc 5940
atctacctgc tgcccaaccg ctaaa 5965

<210> 11
<211> 5965
<212> DNA
<213> Artificial Sequence

<220>
<223> Chimeric NSsuboptmut

<400> 11
gccaccatgg cccccatcac cgcctacagc cagcagaccc gcggcctgct gggctgcatc 60
atcaccagcc tgaccggccg cgacaagaac caggtggagg gcgaggtgca ggtggtgagc 120
accgccaccc agagcttcct ggccacctgc gtgaacggcg tgtgctggac cgtgtaccac 180
ggcgccggca gcaagaccct ggccggcccc aagggcccca tcacccagat gtacaccaac 240
gtggaccagg acctggtggg ctggcaggcc ccccccggcg cccgcagcct gaccccctgc 300
acctgcggca gcagcgacct gtacctggtg acccgccacg ccgacgtgat ccccgtgcgc 360
cgccgcggcg acagccgcgg cagcctgctg agcccccgcc ccgtgagcta cctgaagggc 420
agcagcggcg gccccctgct gtgccccagc ggccacgccg tgggcatctt ccgcgccgcc 480
gtgtgcaccc gcggcgtggc caaggccgtg gacttcgtgc ccgtggagag catggagacc 540
accatgcgca gccccgtgtt caccgacaac agcagccccc ccgccgtgcc ccagagcttc 600
caggtggccc acctgcacgc ccccaccggc agcggcaaga gcaccaaggt gcccgccgcc 660
tacgccgccc agggctacaa ggtgctggtg ctgaacccca gcgtggccgc caccctgggc 720
ttcggcgcct acatgagcaa ggcccacggc atcgacccca acatccgcac cggcgtgcgc 780
accatcacca ccggcgcccc cgtgacctac agcacctacg gcaagttcct ggccgacggc 840
ggctgcagcg gcggcgccta cgacatcatc atctgcgacg agtgccacag caccgacagc 900
accaccatcc tgggcatcgg caccgtgctg gaccaggccg agaccgccgg cgcccgcctg 960
gtggtgctgg ccaccgccac cccccccggc agcgtgaccg tgccccaccc caacatcgag 1020
gaggtggccc tgagcaacac cggcgagatc cccttctacg gcaaggccat ccccatcgag 1080
gccatccgcg gcggccgcca cctgatcttc tgccacagca agaagaagtg cgacgagctg 1140
gccgccaagc tgagcggcct gggcatcaac gccgtggcct actaccgcgg cctggacgtg 1200
agcgtgatcc ccaccatcgg cgacgtggtg gtggtggcca ccgacgccct gatgaccggc 1260
tacaccggcg acttcgacag cgtgatcgac tgcaacacct gcgtgaccca gaccgtggac 1320
ttcagcctgg accccacctt caccatcgag accaccaccg tgccccagga cgccgtgagc 1380
cgcagccagc gccgcggccg caccggccgc ggccgccgcg gcatctaccg cttcgtgacc 1440
cccggcgagc gccccagcgg catgttcgac agcagcgtgc tgtgcgagtg ctacgacgcc 1500
ggctgcgcct ggtacgagct gacccccgcc gagaccagcg tgcgcctgcg cgcctacctg 1560
aacacccccg gcctgcccgt gtgccaggac cacctggagt tctgggagag cgtgttcacc 1620
ggcctgaccc acatcgacgc ccacttcctg agccagacca agcaggccgg cgacaacttc 1680
ccctacctgg tggcctacca ggccaccgtg tgcgcccgcg cccaggcccc cccccccagc 1740
tgggaccaga tgtggaagtg cctgatccgc ctgaagccca ccctgcacgg ccccaccccc 1800
ctgctgtacc gcctgggcgc cgtgcagaac gaggtgaccc tgacccaccc catcaccaag 1860
tacatcatgg cctgcatgag cgccgacctg gaggtggtga ccagcacctg ggtgctggtg 1920
ggcggcgtgc tggccgccct ggccgcctac tgcctgacca ccggcagcgt ggtgatcgtg 1980
ggccgcatca tcctgagcgg ccgccccgcc atcgtgcccg accgcgagtt cctgtaccag 2040
gagttcgacg agatggagga gtgcgccagc cacctgccct acatcgagca gggcatgcag 2100
ctggccgagc agttcaagca gaaggccctg ggcctgctgc agaccgccac caagcaggcc 2160
gaggccgccg cccccgtggt ggagagcaag tggcgcgccc tggagacctt ctgggccaag 2220
cacatgtgga acttcatcag cggcatccag tacctggccg gcctgagcac cctgcccggc 2280
aaccccgcca tcgccagcct gatggccttc accgccagca tcaccagccc cctgaccacc 2340
cagagcaccc tgctgttcaa catcctgggc ggctgggtgg ccgcccagct ggcccccccc 2400
agcgccgcca gcgccttcgt gggcgccggc atcgccggcg ccgccgtggg cagcatcggc 2460
ctgggcaagg tgctggtgga catcctggcc ggctacggcg ccggcgtggc cggcgccctg 2520
gtggccttca aggtgatgag cggcgagatg cccagcaccg aggacctggt gaacctgctg 2580
cccgccatcc tgagccccgg cgccctggtg gtgggcgtgg tgtgcgccgc catcctgcgc 2640
cgccacgtgg gccccggcga gggcgccgtg cagtggatga accgcctgat cgccttcgcc 2700
agccgcggca accacgtgag ccccacccac tacgtgcccg agagcgacgc cgccgcccgc 2760
gtgacccaga tcctgagcag cctgaccatc acccagctgc tgaagcgcct gcaccagtgg 2820
atcaacgagg actgcagcac cccctgcagc ggcagctggc tgcgcgacgt gtgggactgg 2880
atctgcaccg tgctgaccga cttcaagacc tggctgcaga gcaagctgct gccccagctg 2940
cccggcgtgc ccttcttcag ctgccagcgc ggctacaagg gcgtgtggcg cggcgacggc 3000
atcatgcaga ccacctgccc ctgcggcgcc cagatcaccg gccacgtgaa gaacggcagc 3060
atgcgcatcg tgggccccaa gacctgcagc aacacctggc acggcacctt ccccatcaac 3120
gcctacacca ccggcccctg cacccccagc cccgccccca actacagccg cgccctgtgg 3180
cgcgtggccg ccgaggagta cgtggaggtg acccgcgtgg gcgacttcca ctacgtgacc 3240
ggcatgacca ccgacaacgt gaagtgcccc tgccaggtgc ccgcccccga gttcttcacc 3300
gaggtggacg gcgtgcgcct gcaccgctac gcccccgcct gccgccccct gctgcgcgag 3360
gaggtgacct tccaggtggg cctgaaccag tacctggtgg gcagccagct gccctgcgag 3420
cccgagcccg acgtggccgt gctgaccagc atgctgaccg accccagcca catcaccgcc 3480

gagaccgcca agcgccgcct ggcccgcggc agccccccca gcctggccag cagcagcgcc 3540
agccagctga gcgcccccag cctgaaggcc acctgcacca cccaccacgt gagccccgac 3600
gccgacctga tcgaggccaa cctgctgtgg cgccaggaga tgggcggcaa catcacccgc 3660
gtggagagcg agaacaaggt ggtggtgctg gacagcttcg accccctgcg cgccgaggag 3720
gacgagcgcg aggtgagcgt gcccgccgag atcctgcgca agagcaagaa gttccccgct 3780
gccatgccca tctgggctag acctgattac aaccctcccc tgctggagag ctggaaggac 3840
cctgattacg tgcctccagt ggtgcatggc tgtcctctgc ctcccattaa agcccctcct 3900
attccacctc ctaggcgcaa aaggaccgtg gtgctgacag aaagcagcgt gagctctgct 3960
ctggccgaac tggccaccaa gacctttggc agcagcgaga gctctgccgt ggacagcgga 4020
acagccaccg ctctgcctga ccaggccagc gacgacggcg ataagggcag cgatgtggag 4080
agctatagca gcatgcctcc cctggaaggc gaacctggcg atcccgatct gagcgatggc 4140
agctggagca ccgtgagcga agaggccagc gaggacgtgg tgtgttgcag catgagctac 4200
acctggacag gcgctctgat cacaccctgc gctgccgagg agagcaagct gcccatcaac 4260
gccctgagca acagcctgct gaggcaccac aacatggtgt acgccaccac cagcaggtct 4320
gccggactga ggcagaagaa ggtgaccttc gaccgcctgc aggtgctgga cgaccactac 4380
cgcgatgtgc tgaaggagat gaaggccaag gccagcaccg tgaaggccaa gctgctgagc 4440
gtggaggagg cctgcaagct gacccccccc cacagcgcca agagcaagtt cggctacggc 4500
gccaaggacg tgcgcaacct gagcagcaag gccgtgaacc acatccacag cgtgtggaag 4560
gacctgctgg aggacaccgt gacccccatc gacaccacca tcatggccaa gaacgaggtg 4620
ttctgcgtgc agcccgagaa gggcggccgc aagcccgccc gcctgatcgt gttccccgac 4680
ctgggcgtgc gcgtgtgcga gaagatggcc ctgtacgacg tggtgagcac cctgccccag 4740
gtggtgatgg gcagcagcta cggcttccag tacagccccg gccagcgcgt ggagttcctg 4800
gtgaacacct ggaagagcaa gaagaacccc atgggcttca gctacgacac ccgctgcttc 4860
gacagcaccg tgaccgagaa cgacatccgc gtggaggaga gcatctacca gtgctgcgac 4920
ctggcccccg aggcccgcca ggccatcaag agcctgaccg agcgcctgta catcggcggc 4980
cccctgacca acagcaaggg ccagaactgc ggctaccgcc gctgccgcgc cagcggcgtg 5040
ctgaccacca gctgcggcaa caccctgacc tgctacctga aggccagcgc cgcctgccgc 5100
gccgccaagc tgcaggactg caccatgctg gtgaacgccg ccggcctggt ggtgatctgc 5160
gagagcgccg gcacccagga ggacgccgcc agcctgcgcg tgttcaccga ggccatgacc 5220
cgctacagcg ccccccccgg cgaccccccc cagcccgagt acgacctgga gctgatcacc 5280
agctgcagca gcaacgtgag cgtggcccac gacgccagcg gcaagcgcgt gtactacctg 5340
acccgcgacc ccaccacccc cctggcccgc gccgcctggg agaccgcccg ccacaccccc 5400
gtgaacagct ggctgggcaa catcatcatg tacgccccca ccctgtgggc ccgcatgatc 5460
ctgatgaccc acttcttcag catcctgctg gcccaggagc agctggagaa ggccctggac 5520
tgccagatct acggcgcctg ctacagcatc gagcccctgg acctgcccca gatcatcgag 5580
cgcctgcacg gcctgagcgc cttcagcctg cacagctaca gccccggcga gatcaaccgc 5640
gtggccagct gcctgcgcaa gctgggcgtg ccccccctgc gcgtgtggcg ccaccgcgcc 5700
cgcagcgtgc gcgcccgcct gctgagccag ggcggccgcg ccgccacctg cggcaagtac 5760
ctgttcaact gggccgtgaa gaccaagctg aagctgaccc ccatccccgc cgccagccag 5820
ctggacctga gcggctggtt cgtggccggc tacagcggcg gcgacatcta ccacagcctg 5880
agccgcgccc gcccccgctg gttcatgctg tgcctgctgc tgctgagcgt gggcgtgggc 5940
atctacctgc tgcccaaccg ctaaa 5965

<210> 12
<211> 10
<212> RNA
<213> Artificial Sequence

<220>
<223> Ribosome binding site

<400> 12
gccaccaugg 10

<210> 13
<211> 49
<212> RNA
<213> Artificial Sequence

<220>
<223> Synthetic polyadenylation signal

<400> 13
aauaaaagau cuuuauuuuc auuagaucug uguguugguu uuuugugug 49

<210> 14

<211> 28
<212> DNA
<213> Artificial Sequence

<220>
<223> Additional nucleotides present in pVIJns-NS

<400> 14
tctagagcgt ttaaaccctt aattaagg                                          28

<210> 15
<211> 15
<212> DNA
<213> Artificial Sequence

<220>
<223> Additional nucleotides present in pV1Jns-NSOPTmut

<400> 15
tttaaatgtt taaac                                                        15

<210> 16
<211> 24
<212> DNA
<213> Artificial Sequence

<220>
<223> Oligonucleotide primer

<400> 16
tcgaatcgat acgcgaacct acgc                                              24

<210> 17
<211> 37
<212> DNA
<213> Artificial Sequence

<220>
<223> Oligonucleotide primer

<400> 17
tcgacgtgtc gacttcgaag cgcacaccaa aaacgtc                                37